# EUROPEAN PATENT APPLICATION

(11) **EP 4 810 500 A1**
(43) Date of publication of application: **23.09.2026**
(21) Application number: 24890797.4
(22) Date of filing: 15.11.2024
(51) Int. Cl.: C07D 519/00, A61K 31/437, A61P 3/10

(54) **IMIDAZOL-2-ONE DERIVATIVE AND USE THEREOF IN MEDICINE**

(30) Priority: 16.11.2023 CN 202311530047; 10.01.2024 CN 202410036624; 06.02.2024 CN 202410168226; 15.04.2024 CN 202410449296; 28.05.2024 CN 202410670189; 19.06.2024 CN 202410794160; 29.07.2024 CN 202411022986; 30.08.2024 CN 202411210027; 10.10.2024 CN 202411409623
(71) Applicant: Haisco Pharmaceutical Group Co., Ltd., Shannan, Xizang 856099 (CN)
(72) Inventor: ZHANG, Chen, Chengdu, Sichuan 611130 (CN); LEI, Ming, Chengdu, Sichuan 611130 (CN); LIAO, Yuting, Chengdu, Sichuan 611130 (CN); MOU, Tao, Chengdu, Sichuan 611130 (CN); WANG, Jianmin, Chengdu, Sichuan 611130 (CN); WENG, Guanglin, Chengdu, Sichuan 611130 (CN); MENG, Yifei, Chengdu, Sichuan 611130 (CN); LIN, Renwei, Chengdu, Sichuan 611130 (CN); YUAN, Hongyuan, Chengdu, Sichuan 611130 (CN); HUANG, Anbang, Chengdu, Sichuan 611130 (CN); WANG, Ju, Chengdu, Sichuan 611130 (CN); LI, Yao, Chengdu, Sichuan 611130 (CN); YAN, Pangke, Chengdu, Sichuan 611130 (CN)
(74) Representative: AWA Denmark A/S
(86) International application number: PCT/CN2024/132223
(87) International publication number: WO 2025/103443

(57) **Abstract**

Provided in the present invention are a tetrahydropyridopyrazole derivative and use thereof in medicine. Specifically provided is a compound as shown in general formula (I) or a racemate, stereoisomer, tautomer, deuterated substance, solvate, prodrug, metabolite, pharmaceutically acceptable salt or eutectic crystal thereof, an intermediate thereof, a preparation method therefor, and use thereof in the preparation of a related drug for treating diabetes or obesity.

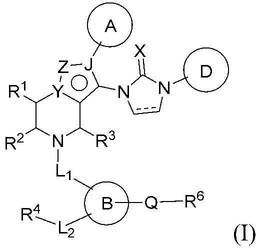

## Description

### Technical Field

The present invention belongs to the field of medicine, and specifically relates to a compound as shown in general formula (I) or a racemate, stereoisomer, tautomer, deuterated substance, solvate, prodrug, metabolite, pharmaceutically acceptable salt or eutectic crystal thereof, an intermediate thereof, a preparation method therefor, and the use thereof in the preparation of a related drug for treating diabetes.

### Background Art

Diabetes is a group of metabolic diseases characterised by hyperglycaemia. Hyperglycaemia is caused by defective insulin secretion or impaired insulin biological action, or both. Long-term hyperglycaemia in diabetes leads to chronic damage and dysfunction of various tissues, especially eyes, kidneys, heart, blood vessels, and nerves. Diabetes is mainly divided into two types: type 1 diabetes which results from destruction of islet B cells, leading to absolute insulin deficiency, and type 2 diabetes which is characterised by insulin resistance as the primary disorder accompanied by relative insulin deficiency, or impaired insulin secretion as the primary disorder accompanied by insulin resistance.

Drugs for type 2 diabetes can be divided into six major classes (insulin, insulin secretagogues, biguanides, glucosidase inhibitors, thiazolidinediones, and SGLT2 inhibitors), each of which works through a different primary mechanism.

GLP-1 is a 30-amino-acid incretin hormone secreted by L cells in the intestine. GLP-1 stimulates insulin secretion, reduces glucagon secretion, inhibits gastric emptying, reduces appetite, and stimulates β-cell proliferation in a physiological and glucose-dependent manner. In nonclinical experiments, GLP-1 promotes the sustainability of β cells by stimulating the transcription of genes important for glucose-dependent insulin secretion and by promoting β cell regeneration. In healthy individuals, GLP-1 plays an important role in the regulation of postprandial blood glucose, leading to increased peripheral glucose uptake by stimulating glucose-dependent insulin secretion from the pancreas. GLP-1 also inhibits glucagon secretion, resulting in decreased hepatic glucose output. In addition, GLP-1 delays gastric emptying, slows small bowel motility, and delays food absorption.

Beyond its potential application in diabetes, research has also revealed that GLP-1 receptor agonists exert antagonistic effects against neurodegenerative pathology and the progression of AD. In AD transgenic mice, systemic administration of liraglutide for 8 weeks can prevent memory impairment, neuronal loss, and deterioration of hippocampal synaptic plasticity. Furthermore, based on the number of activated microglia, liraglutide can significantly reduce amyloid plaque deposition and inflammation. Similarly, in rats receiving intrahippocampal injection of a monoclonal antibody (mAb), liraglutide pretreatment can significantly protect against mAb-induced deficits in spatial memory and long-term potentiation. Another GLP-1 analogue, exenatide, has also shown promising results in preclinical studies against neurodegenerative diseases.

### Summary of the Invention

The object of the present invention is to provide a compound that can agonise GLP-1 receptors or a racemate, stereoisomer, tautomer, deuterated substance, solvate, prodrug, metabolite, pharmaceutically acceptable salt or eutectic crystal thereof, an intermediate thereof, a preparation method therefor, and the use thereof in the preparation of a related drug for treating diabetes or obesity.

The compound of the present invention has good pharmacokinetic performance and bioavailability, oral performance and good safety.

The present invention provides a compound or a racemate, stereoisomer, tautomer, deuterated substance, solvate, prodrug, metabolite, pharmaceutically acceptable salt or eutectic crystal thereof, wherein the compound is a compound as shown in general formula (I), 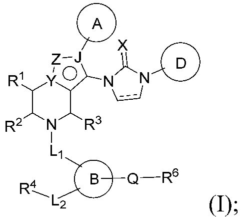
in some embodiments, the compound as shown in general formula (I) is a compound as shown in general formula (Ia), (Ib), (Ic), or (Id), 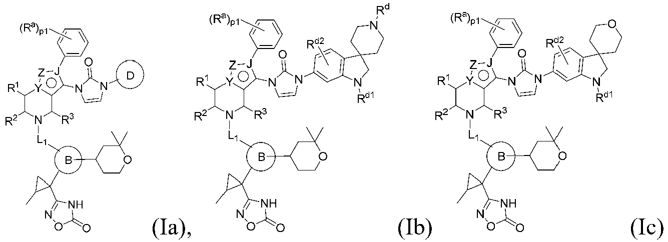 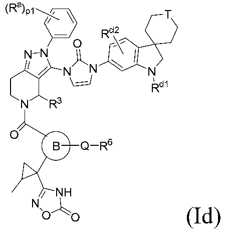
in some embodiments, the compound as shown in general formula (Id) is selected from the compounds as shown in general formula (Id-1), 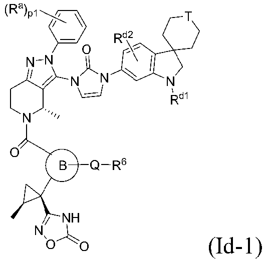
in some embodiments, T is selected from a bond, O, S, CH₂, NH, and N(R^{d3}),
in some embodiments, T is selected from a bond, O, S, CH₂, and NH;
in some embodiments, p1 is selected from 0, 1, 2, 3 or 4;
in some embodiments, 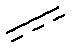 represents a single bond or a double bond;
in some embodiments, Y is selected from C and N, Z is selected from CH or N, J is selected from N or C, and at least one of Y, Z and J is N;
in some embodiments, ring A is selected from C₆₋₁₀ aryl, 5- to 10-membered heteroaryl, C₃₋₁₀ carbocyclyl, 4- to 10-membered heterocyclyl, C₁₁₋₁₅ aryl, 11- to 15-membered heterocyclyl, and 11- to 15-membered heteroaryl, the ring A is optionally substituted with 1 to 4 R^{a}, and the nitrogen atom in the heteroaryl or heterocyclyl is optionally oxidised to form an N-oxide;
in some embodiments, ring A is selected from phenyl, 5- to 6-membered heteroaryl, C₃₋₆ monocyclic carbocyclyl, 4- to 8-membered monocyclic heterocyclyl, and benzo C₇₋₈ carbocyclyl, the ring A is optionally substituted with 1 to 4 R^{a}, and the nitrogen atom in the heteroaryl or heterocyclyl is optionally oxidised to form an N-oxide;
in some embodiments, ring A is selected from one of the following groups optionally substituted with 1 to 4 R^{a}: phenyl, cyclopropyl, cyclobutyl, cyclopentyl, cyclohexyl, azetidinyl, pyrrolidyl, piperidyl, oxetanyl, oxolanyl, oxanyl, 1,3-dioxolanyl, 1,4-dioxanyl, piperazinyl, morpholinyl, pyrazolyl, imidazolyl, oxazolyl, thiazolyl, pyrrolyl, thienyl, triazolyl, pyridyl, pyrimidyl, pyridazinyl, pyrazinyl, triazinyl, 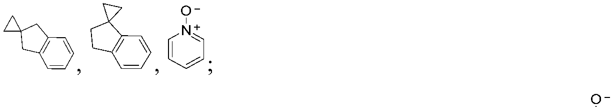
in some embodiments, ring A is selected from phenyl, pyridyl, 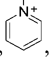 and the ring A is optionally substituted with 1 to 4 R^{a};
in some embodiments, ring A is selected from 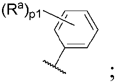 in some embodiments, ring A is selected from 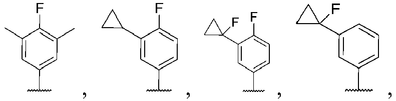 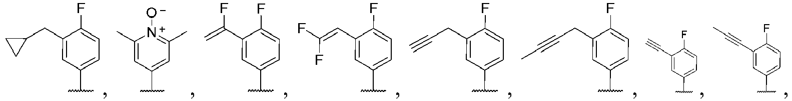 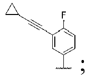 in some embodiments, 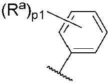 is selected from 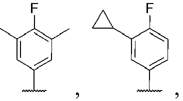 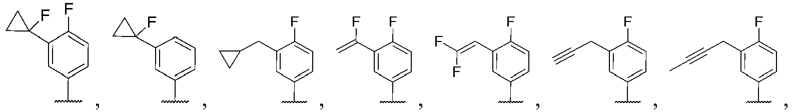 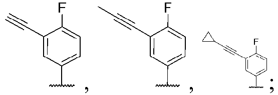
in some embodiments, ring B is selected from C₆₋₁₀ aryl, 5- to 6-membered heteroaryl, 5-membered ring-fused-5-membered heteroaryl, 5-membered ring-fused-6-membered heteroaryl, 6-membered ring-fused-6-membered heteroaryl, C₃₋₁₀ carbocyclyl, and 4- to 10-membered heterocyclyl, and the ring B is optionally substituted with 1 to 4 R^{b};
in some embodiments, ring B is selected from 5-membered ring-fused-5-membered heteroaryl or 5-membered ring-fused-6-membered heteroaryl, and the ring B is optionally substituted with 1 to 4 R^{b};
in some embodiments, ring B is selected from pyrrolothienyl, pyrrolopyrazolyl, pyrrolopyrrolyl, pyrroloimidazolyl, pyrazolothienyl, imidazothienyl, imidazoimidazolyl, pyrazolopyrazolyl, pyrrolothiazolyl, pyrrolofuryl, indolyl, pyrrolopyridyl, pyrrolopyrimidyl, pyrrolopyridazinyl, pyrrolopyrazinyl, pyrrolotriazinyl, pyrazolophenyl, pyrazolopyridyl, pyrazolopyrimidyl, imidazophenyl, imidazopyridyl, imidazopyrimidyl, thienopyridyl, thienophenyl, furopyridyl, and furophenyl, and the ring B is optionally substituted with 1 to 4 R^{b};
in some embodiments, ring B is selected from one of the following groups optionally substituted with 1 to 4 R^{b}: 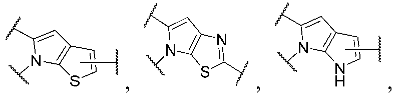 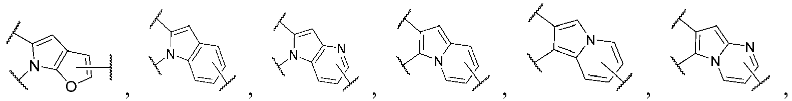 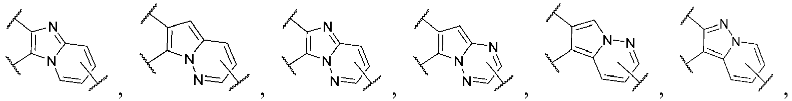 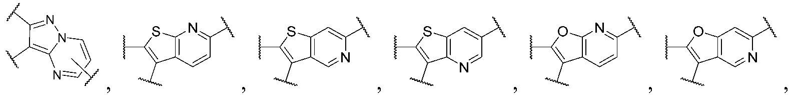 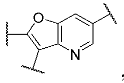 the right side of which is linked to Q;
in some embodiments, ring B is selected from 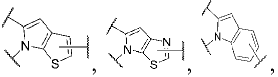 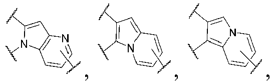 and the ring B is optionally substituted with 1 to 4 R^{b}, the right side of which is linked to Q;
in some embodiments, ring B is selected from 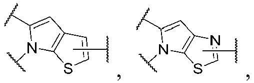 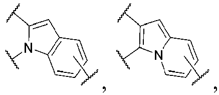 and the ring B is optionally substituted with 1 to 4 R^{b}, the right side of which is linked to Q;
in some embodiments, ring B is selected from 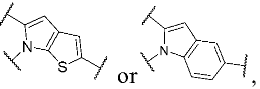 and the ring B is optionally substituted with 1 to 4 R^{b}, the right side of which is linked to Q;
in some embodiments, ring D is selected from 13- to 16-membered tricyclic or tetracyclic heterocyclyl, and 17- to 30-membered tricyclic or tetracyclic heterocyclyl, and the ring D is optionally substituted with 1 to 4 R^{d};
in some embodiments, ring D is selected from partially saturated rings as follows: 13- to 15-membered tricyclic fused heterocyclyl, 10-membered fused carbocycle spiro-linked to C₄₋₆ carbocycle, 9-membered fused carbocycle spiro-linked to C₄₋₆ carbocycle, 11-membered fused carbocycle spiro-linked to C₃₋₅ carbocycle, 10-membered fused heterocycle spiro-linked to C₄₋₆ carbocycle, 11-membered fused heterocycle spiro-linked to C₃₋₅ carbocycle, 10-membered fused carbocycle spiro-linked to 4- to 6-membered heterocycle, 11-membered fused carbocycle spiro-linked to 4- to 5-membered heterocycle, 9-membered fused heterocycle spiro-linked to 4- to 6-membered heterocycle, 9-membered fused heterocycle spiro-linked to 8- to 10-membered heterocycle, 9-membered fused heterocycle spiro-linked to C₃₋₆ carbocycle, 10-membered fused heterocycle spiro-linked to 4- to 6-membered heterocycle, 11-membered fused heterocycle spiro-linked to 4- to 5-membered heterocycle, and 11-membered tricyclic heterocycle spiro-linked to C₃₋₄ carbocycle, and the ring D is optionally substituted with 1 to 4 R^{d};
in some embodiments, ring D is selected from 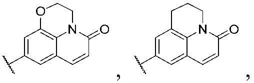 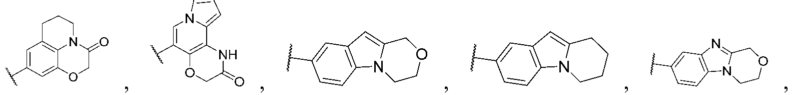 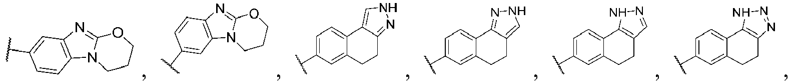 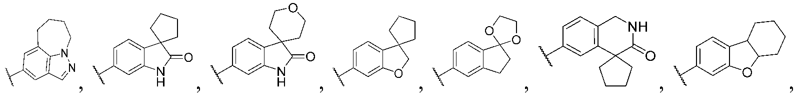 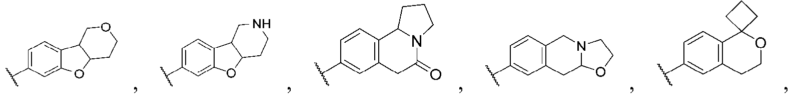 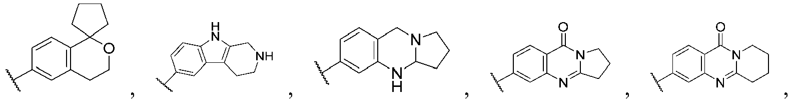 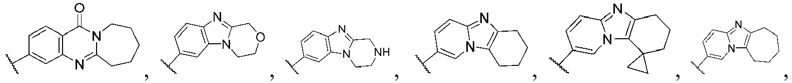 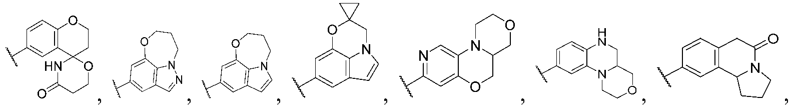 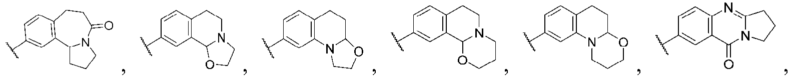 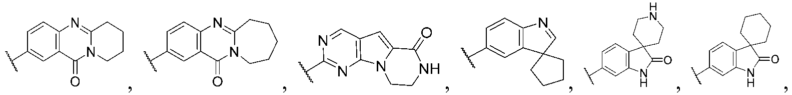 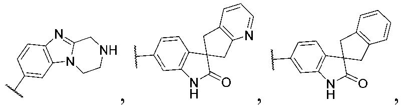 and the ring D is optionally substituted with 1 to 4 R^{d};
in some embodiments, ring D is selected from 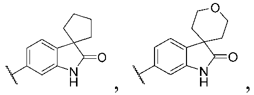 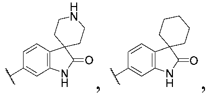 and the ring D is optionally substituted with 1 to 4 R^{d};
in some embodiments, ring D is 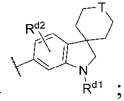 in some embodiments, ring D is selected from 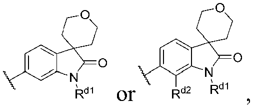 R^{d1} is selected from H or R^{d}, and R^{d2} is selected from H or R^{d}; in some embodiments, ring D is selected from 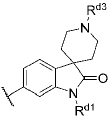 or 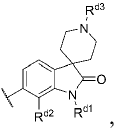 R^{d1} is selected from H or R^{d}, R^{d2} is selected from H or R^{d}, and R^{d3} is selected from H or R^{d};
in some embodiments, Q is selected from a bond, O, S, NH, C₁₋₄ alkylene, 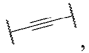 wherein the alkylene is optionally substituted with 1 to 4 R^{k};
in some embodiments, Q is selected from a bond, O, S, NH, CH₂, CH(CH₃), 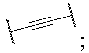
in some embodiments, R⁶ is selected from C₁₋₆ alkyl, -C₁₋₂ alkylene-O-C₁₋₄ alkyl, C₃₋₁₀ carbocyclyl, and 4- to 10-membered heterocyclyl, and the R⁶ is optionally substituted with 1 to 4 R^{6a};
in some embodiments, R⁶ is selected from C₁₋₄ alkyl, -C₁₋₂ alkylene-O-C₁₋₄ alkyl, C₃₋₆monocyclic carbocyclyl, C₆₋₁₀ fused carbocyclyl, C₆₋₁₀ spirocarbocyclyl, C₅₋₁₀ bridged carbocyclyl, 4- to 8-membered monocyclic heterocyclyl, 7- to 10-membered fused heterocyclyl, 7- to 10-membered spiro heterocyclyl, and 6- to 10-membered bridged heterocyclyl, and the R⁶ is optionally substituted with 1 to 4 R^{6a};
in some embodiments, R⁶ is selected from methyl, ethyl, propyl, isopropyl, - CH₂O-CH₃, -CH₂O-CH₂CH₃, -CH₂O-CH(CH₃)₂, -CH₂O-C(CH₃)₃, -CH₂CH₂O-CH₃, -CH₂CH₂O-CH₂CH₃, -CH₂CH₂O-CH(CH₃)₂, -CH₂CH₂O-C(CH₃)₃, cyclopropyl, cyclobutyl, cyclopentyl, cyclohexyl, bicyclo[1.1.1]cyclopentyl, oxetanyl, oxolanyl, oxanyl, azetidinyl, pyrrolidyl, piperidyl, piperazinyl, morpholinyl, phenyl, pyrazolyl, thiazolyl, imidazolyl, oxazolyl, pyrrolyl, thienyl, furyl, pyridyl, pyrimidyl, pyrazinyl, pyridazinyl or 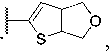 and the R⁶ is optionally substituted with 1 to 4 R^{6a};
in some embodiments, -Q-R⁶ is selected from one of the following optionally substituted groups: ethyl, propyl, isopropyl, 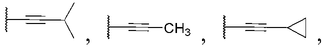 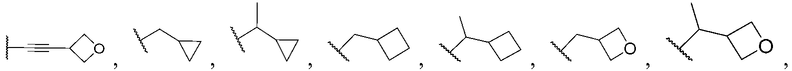 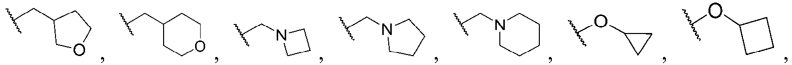 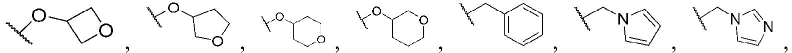 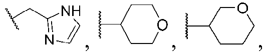 -CH₂CH₂CH₂O-CH₂CH₃, -CH₂CH₂CH₂O-CH(CH₃)₂, - CH₂CH₂CH₂O-C(CH₃)₃, 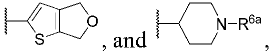 which, when substituted, are substituted with 1 to 4 substituents selected from deuterium, F, Cl, Br, I, =O, CN, OH, CF₃, CHF₂, CH₂F, CD₃, CHD₂, CH₂D, OCD₃, OCF₃, methyl, ethyl, propyl, isopropyl, methoxy or ethoxy;
in some embodiments, R^{6a} is selected from deuterium, F, Cl, Br, I, =O, CN, OH, CF₃, CHF₂, CH₂F, CD₃, CHD₂, CH₂D, OCD₃, OCF₃, methyl, ethyl, propyl, isopropyl, methoxy, ethoxy, cyclopropyl, cyclobutyl, 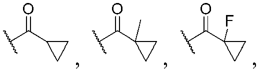 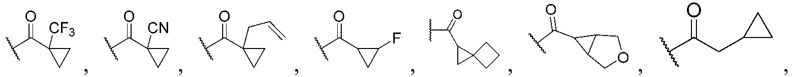 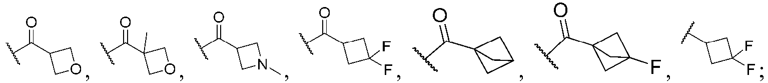
in some embodiments, R^{6a} is selected from methyl, ethyl, propyl, isopropyl, cyclopropyl, cyclobutyl, 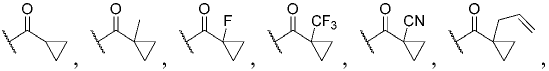 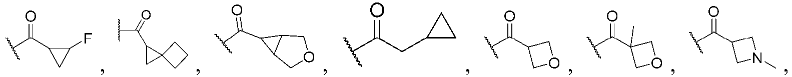 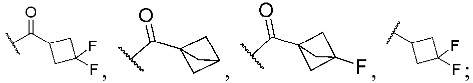
in some embodiments, -Q-R⁶ is selected from one of the following optionally substituted groups: 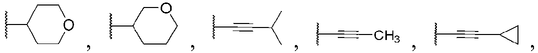 and 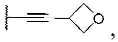 which, when substituted, are substituted with 1 to 4 substituents selected from deuterium, F, Cl, Br, I, =O, CN, OH, CF₃, CHF₂, CH₂F, CD₃, CHD₂, CH₂D, OCD₃, OCF₃, methyl, ethyl, propyl, isopropyl, methoxy or ethoxy;
in some embodiments, -Q-R⁶ is selected from one of the following optionally substituted groups: 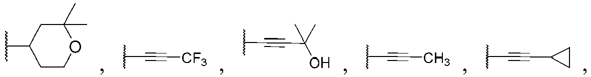 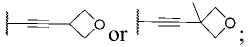
in some embodiments, -Q-R⁶ is optionally substituted 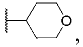 which, when substituted, is substituted with 1 to 4 substituents selected from deuterium, F, Cl, Br, OH, CF₃, CHF₂, CH₂F, CD₃, CHD₂, CH₂D, OCD₃, OCF₃, methyl, ethyl, propyl, isopropyl, methoxy or ethoxy;
in some embodiments, L₁ is selected from -S(=O)₂-, -C(=O)-, and -C(=S)-; in some embodiments, L₁ is -C(=O)-;
in some embodiments, L₂ is -(CR^{L1}R^{L2})ₘ-; in some embodiments, L₂ is selected from -(CR^{L1}R^{L2})-, and -(CR^{L1}R^{L2})₂₋;
in some embodiments, L₂ is selected from CR^{L1}R^{L2}-, or one of the following groups optionally substituted with 1 to 3 R^{k}: 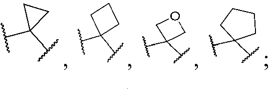 in some embodiments, L₂ is selected from 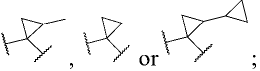
in some embodiments, X is selected from S or O, preferably O;
in some embodiments, m is selected from 1, 2, 3 or 4;
in some embodiments, R⁴ is selected from -C(=O)R^{4a}, -C(=O)OR^{4a}, - C(=O)NR^{4a}R^{4b}, 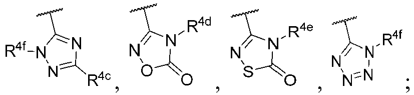 in some embodiments, R⁴ is selected from -C(=O)OH, -C(=O)OCH₃, -C(=O)N(CH₃)₂, 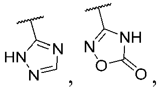 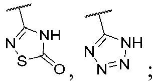 in some embodiments, R⁴ is 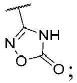
in some embodiments, R^{4a}, R^{4b}, R^{4c}, R^{4d}, R^{4e}, and R^{4f} are each independently selected from H, deuterium, and C₁₋₆ alkyl, wherein the alkyl is optionally substituted with 1 to 4 R^{k};
in some embodiments, R^{4a}, R^{4b}, R^{4c}, R^{4d}, R^{4e}, and R^{4f} are each independently selected from H, deuterium, and C₁₋₄ alkyl, wherein the alkyl is optionally substituted with 1 to 4 R^{k}; in some embodiments, R^{4a}, R^{4b}, R^{4c}, R^{4d}, R^{4e}, and R^{4f} are each independently selected from H, deuterium, methyl, ethyl, propyl, and isopropyl;
in some embodiments, R^{a}, R^{b}, R^{d}, and R^{6a} are each independently selected from H, deuterium, halogen, =O, CN, OH, NO₂, COOH, CONH₂, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₄ alkylene-C₃₋₆ carbocyclyl, - C₁₋₄ alkylene-3- to 7-membered heterocyclyl, -P(=O)R^{5a}R^{5b}, -S(=O)₁₋₂-R^{5c}, - C(=O)R^{5c}, -C(=O)NR^{5d}R^{5e}, -NR^{5d}C(=O)R^{5e}, -C(=O)-M-C(=O)-R^{5c}, -CH₂-M-C(=O)-R^{5c}, -C(=O)-M-CH₂-R^{5c}, -C(=O)-M-R^{5c}, -C(=O)-C₁₋₄ alkylene-OC₀₋₄ alkylene-R^{5c}, C₃₋₁₂ carbocyclyl, 3- to 12-membered heterocyclyl, 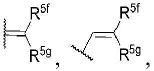 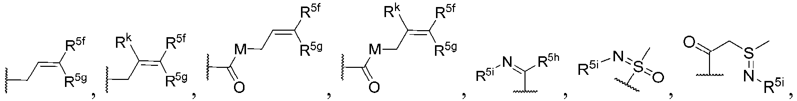 wherein the alkyl, alkylene, alkenyl, alkynyl, carbocyclyl or heterocyclyl is optionally substituted with 1 to 4 R^{k};
in some embodiments, R^{a}, R^{b}, R^{d}, and R^{6a} are each independently selected from H, deuterium, halogen, =O, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₂ alkylene-C₃₋₆ carbocyclyl, -C₁₋₂ alkylene-3- to 7-membered heterocyclyl, -P(=O)R^{5a}R^{5b}, -S(=O)₁₋₂-R^{5c}, -C(=O)R^{5c}, -C(=O)NR^{5d}R^{5e}, - NR^{5d}C(=O)R^{5e}, -C(=O)-M-C(=O)-R^{5c}, -C(=O)-M-R^{5c}, -C(=O)-M-R^{5c}, -C(=O)-C₁₋₂ alkylene-OC₀₋₂ alkylene-R^{5c}, -CH₂-M-C(=O)-R^{5c}, C₃₋₁₁ carbocyclyl, 3- to 11-membered heterocyclyl, 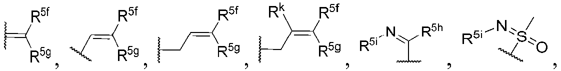 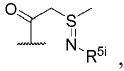 wherein the alkyl, alkenyl, alkynyl, alkylene, carbocyclyl or heterocyclyl is optionally substituted with 1 to 4 R^{k};
in some embodiments, R^{a}, R^{b}, R^{d}, and R^{6a} are each independently selected from H, deuterium, F, Cl, Br, I, =O, CN, OH, NO₂, NH₂, NH(CH₃), N(CH₃)₂, - P(=O)R^{5a}R^{5b}, 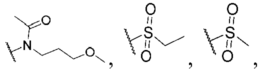 -C(=O)CH₃, -C(=O)CH₂CH₃, or one of the following groups optionally substituted with 1 to 3 R^{k}: methyl, ethyl, propyl, isopropyl, butyl, ethenyl, ethynyl, propynyl, methoxy, ethoxy, isopropoxy, methylthio, cyclopropyl, cyclobutyl, cyclopentyl, cyclohexyl, cyclobutyl-spiro-cyclobutyl, cyclopentyl-spiro-cyclobutyl, cyclopentyl-spiro-cyclopentyl, cyclohexyl-spiro-cyclobutyl, cyclohexyl-spiro-cyclopentyl, cyclohexyl-spiro-cyclohexyl, azetidinyl, oxetanyl, pyrrolidyl, piperidyl, piperazinyl, morpholinyl, morpholinyl-spiro-cyclohexyl, -C(=O)NHCH₃, -C(=O)NH-cyclopropyl, 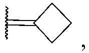 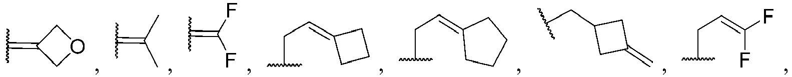 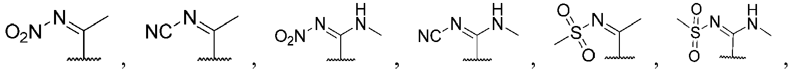 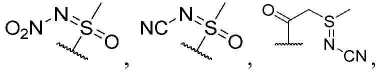 -CH₂-cyclopropyl, -CH₂-cyclobutyl, -CH₂-cyclopentyl, -CH₂-cyclohexyl, -CH₂-azetidinyl, -CH₂-azacyclopentyl, -CH₂-piperidyl, -CH₂-piperazinyl, -C(=O)R^{5c}, -C(=O)-M-C(=O)-R^{5c}, -CH₂-M-C(=O)-R^{5c}, -C(=O)-M-CH₂-R^{5c}, -C(=O)-M-R^{5c}, -C(=O)-CH₂-O-R^{5c}, -C(=O)-CH₂-OCH₂-R^{5c}, - C(=O)-M-R^{5c}, 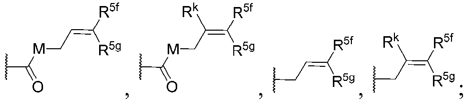
in some embodiments, R^{d} is optionally substituted with 1 to 3 R^{k}, and R^{k} is R^{k1};
in some embodiments, M is selected from C₃₋₆ carbocyclyl or 4- to 7-membered heterocyclyl, and the M is optionally substituted with 1 to 4 R^{k};
in some embodiments, M is selected from C₃₋₆ cycloalkyl, phenyl, 4- to 7-membered heterocycloalkyl or 5- to 6-membered heteroaryl, and the M is optionally substituted with 1 to 4 R^{k};
in some embodiments, M is selected from cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, 3-oxabicyclo[3.1.0]hexyl, and the M is optionally substituted with 1 to 4 R^{k};
in some embodiments, M is selected from cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, and 3-oxabicyclo[3.1.0]hexyl, and the M is optionally substituted with 1 to 4 substituents selected from deuterium, F, Cl, Br, CHF₂, CD₃, methyl, cyclopropyl, oxetanyl, - CH₂-cyclopropyl, -CH₂-oxetanyl;
in some embodiments, each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, propynyl, methoxy, ethoxy, isopropoxy, cyclopropyl, - CH₂OH, -CH₂CH₂OH, -CH₂CN, -CH₂N(CH₃)₂, -CH₂-cyclopropyl, 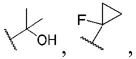 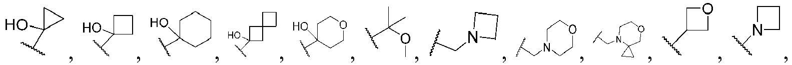 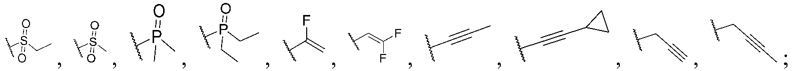 preferably, each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, methoxy, cyclopropyl 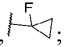
in some embodiments, each R^{b} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, methoxy, ethoxy, isopropoxy, and cyclopropyl;
in some embodiments, each R^{d} is independently selected from deuterium, F, Cl, Br, I, CN, OH, NH₂, NH(CH₃), N(CH₃)₂, CD₃, -CH₂CH₂OCH₃, -CF₃, -CH₂F, - CHF₂, -CH₂CH₂F, -CH₂CH₂CH₂F, -OCH₂CH₂OH, 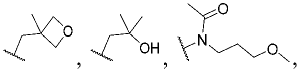 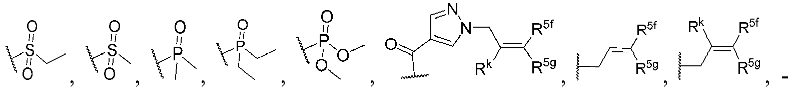 C(=O)NHCH₃, -C(=O)NH-cyclopropyl, 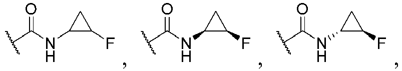 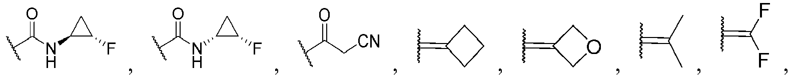 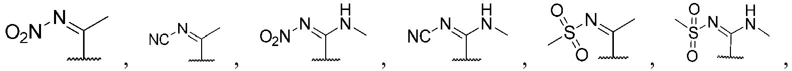 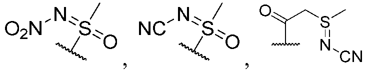 or one of the following groups optionally substituted with 1 to 4 R^{k1}: methyl, ethyl, methoxy, ethoxy, cyclopropyl, oxetanyl, -CH₂-cyclopropyl, -CH₂-cyclobutyl, -CH₂-cyclopentyl, -CH₂-oxetanyl, -CH₂-azetidinyl, -S(=O)₂CH₃, -S(=O)₂ cyclopropyl, -C(=O)NH-cyclopropyl, -C(=O)CH₃, -C(=O)-ethenyl, -C(=O)-cyclopropyl, -C(=O)-cyclobutyl, -C(=O)-cyclopentyl, - C(=O)-bicyclo[2.2.1]heptyl, -C(=O)-bicyclo[1.1.1]pentyl, -C(=O)-cyclobutyl-spiro-cyclobutyl, -C(=O)-oxetanyl, -C(=O)-tetrahydrofuryl, -C(=O)-oxanyl, - C(=O)-azetidinyl, -C(=O)-pyrrolidyl, -C(=O)-piperidyl, -C(=O)-piperazinyl, - C(=O)-pyrazolyl, -C(=O)-phenyl, -C(=O)-azetidinyl-CH₂-cyclopropyl, -C(=O)-pyrazolyl-CH₂-cyclopropyl, -C(=O)-pyrazolyl-CH₂-cyclobutyl, -C(=O)-pyrazolyl-CH₂-cyclopentyl, -C(=O)-pyrrolidyl-CH₂-cyclopropyl, -C(=O)-piperidyl-CH₂-cyclopropyl, -C(=O)-piperazinyl-CH₂-cyclopropyl, -C(=O)-azetidinyl -C(=O)-cyclopropyl, -C(=O)-pyrrolidyl -C(=O)-cyclopropyl, -C(=O)-piperidyl -C(=O)-cyclopropyl, -C(=O)-piperazinyl -C(=O)-cyclopropyl, -CH₂-azetidinyl -C(=O)-cyclopropyl, -CH₂-pyrrolidyl -C(=O)-cyclopropyl, -CH₂-azetidinyl-CH₂-cyclopropyl, -CH₂-pyrrolidyl-CH₂-cyclopropyl, -CH₂-piperidyl-CH₂-cyclopropyl, - CH₂-piperazinyl-CH₂-cyclopropyl, 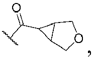 -C(=O)-cyclopropyl-phenyl, -C(=O)-cyclopropyl-pyridyl, -C(=O)-cyclopropyl-thienyl, -C(=O)-cyclopropyl-furyl, - C(=O)-CH₂-O-cyclopropyl, or -C(=O)-CH₂-OCH₂-cyclopropyl;
R^{k1} is selected from deuterium, F, Cl, Br, OH, CN, methyl, ethyl, propyl, isopropyl, ethenyl, propenyl, allyl, -CH₂-cyclopropyl, -CH₂-cyclopropyl, -CH₂-cyclobutyl, cyclopropyl, cyclobutyl, cyclopentyl, cyclohexyl or 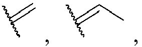 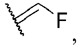 wherein the methyl, ethyl, propyl, isopropyl, ethenyl, propenyl, allyl, - CH₂-cyclopropyl, -CH₂-cyclopropyl, -CH₂-cyclobutyl, cyclopropyl, cyclobutyl, cyclopentyl, or cyclohexyl is optionally substituted with 1 to 4 substituents selected from deuterium, F, Cl, Br, OH, CN, CH₂CN, methyl, ethyl, methoxy or ethoxy;
in some embodiments, each R^{d} is independently selected from deuterium, F, Cl, Br, I, CN, OH, NH₂, NH(CH₃), N(CH₃)₂, CHF₂, CH₂F, CF₃, CD₃, methyl, ethyl, methoxy, ethoxy, cyclopropyl, oxetanyl, -CH₂-cyclopropyl, -CH₂-oxetanyl, - C(=O)CH₃, -C(=O)NHCH₃, -C(=O)NH-cyclopropyl, 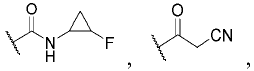 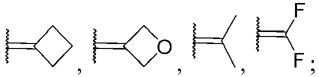
in some embodiments, R^{d1} is selected from H, CHF₂, CD₃, methyl, cyclopropyl, oxetanyl, -CH₂-cyclopropyl, and -CH₂-oxetanyl;
in some embodiments, R^{d2} is selected from H, deuterium, F, Cl, Br, CHF₂, CD₃, methyl, cyclopropyl, oxetanyl, -CH₂-cyclopropyl, and -CH₂-oxetanyl;
in some embodiments, R^{d3} is selected from H or methyl, ethyl, isopropyl, cyclopropyl, cyclobutyl, cyclopentyl, cyclohexyl, -CH₂-cyclopropyl, -CH₂-cyclobutyl, -CH₂-oxetanyl, -CH₂-tetrahydrofuryl, -CH₂-azetidinyl, -CH₂-pyrrolidyl, -CH2-piperidyl, -CH₂-piperazinyl, -S(=O)₁₋₂-R^{5c}, -C(=O)R^{5c}, -C(=O)NR^{5d}R^{5e}, - NR^{5d}C(=O)R^{5e}, -C(=O)-M-C(=O)-R^{5c}, -CH₂-M-C(=O)-R^{5c}, -C(=O)-M-CH₂-R^{5c}, - C(=O)-M-R^{5c}, -C(=O)-M-CH₂-O-R^{5c}, 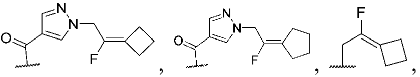 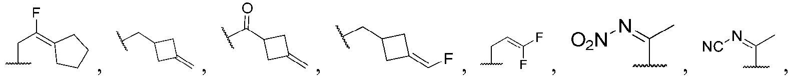 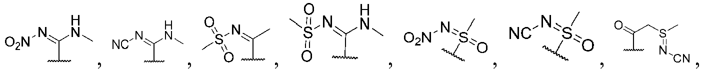 wherein the methyl, ethyl, isopropyl, cyclopropyl, cyclobutyl, cyclopentyl, cyclohexyl, - CH₂-cyclopropyl, -CH₂-cyclobutyl, -CH₂-oxetanyl, -CH₂-tetrahydrofuryl, -CH₂-azetidinyl, -CH₂-pyrrolidyl, -CH₂-piperidyl, or -CH₂-piperazinyl is optionally substituted with 1 to 4 R^{k} (each R^{k} is preferably independently selected from deuterium, F, Cl, Br, CN, OH, CHF₂, CD₃, methyl, ethyl, propyl, isopropyl, cyclopropyl, oxetanyl, -CH₂-cyclopropyl, -CH₂-oxetanyl, methoxy, methoxymethyl, methoxyethyl, CH₂CN, ethenyl, 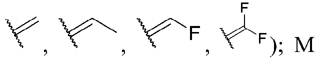 is selected from cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, and 3-oxabicyclo[3.1.0]hexyl, and the M is optionally substituted with 1 to 4 R^{k} (each R^{k} is preferably independently selected from deuterium, F, Cl, Br, CHF₂, CD₃, methyl, cyclopropyl, oxetanyl, -CH₂-cyclopropyl, -CH₂-oxetanyl); R^{5c} is selected from methyl, ethyl, propyl, isopropyl, methoxy, ethoxy, ethenyl, propenyl, allyl, cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, bicyclo[2.2.1]heptyl, cyclopropyl-spiro-cyclobutyl, cyclobutyl-spiro-cyclobutyl, cyclobutyl-spiro-cyclopentyl, cyclobutyl-spiro-cyclohexyl, cyclopentyl-fused-cyclopentyl, 3-oxabicyclo[3.1.0]hexyl, thienyl, furyl, thiazolyl, oxazolyl, methoxymethyl, and ethoxymethyl, and the R^{5c}is optionally substituted with 1 to 4 R^{k} (each R^{k} is preferably independently selected from deuterium, F, Cl, Br, CN, OH, CHF₂, CD₃, methyl, ethyl, propyl, isopropyl, cyclopropyl, oxetanyl, -CH₂-cyclopropyl, -CH₂-oxetanyl, methoxy, methoxymethyl, methoxyethyl, CH₂CN, ethenyl, propenyl, 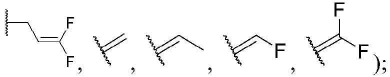
in some embodiments, R^{5a}, R^{5b}, and R^{5c} are each independently selected from C₁₋₆ alkyl, -C₁₋₄ alkyl-OC₁₋₄ alkyl, C₁₋₆ alkoxy, C₂₋₆ alkenyl, C₃₋₁₂ carbocyclyl, and 4- to 12-membered heterocyclyl, wherein the alkyl, alkoxy, alkenyl, carbocyclyl or heterocyclyl is optionally substituted with 1 to 4 R^{k};
in some embodiments, R^{5a}, R^{5b}, and R^{5c} are each independently selected from C₁₋₄ alkyl, -C₁₋₄ alkyl-OC₁₋₄ alkyl, C₁₋₄ alkoxy, C₂₋₄ alkenyl, C₃₋₆ cycloalkyl, C₅₋₈ bridged cycloalkyl, C₅₋₁₁ spirocycloalkyl, C₅₋₁₁ fused cycloalkyl, 4- to 7-membered monocyclic heterocycloalkyl, 6- to 10-membered bridged heterocycloalkyl, 6- to 11-membered spiroheterocycloalkyl, 6- to 11-membered fused heterocycloalkyl, phenyl or 5- to 6-membered heteroaryl, wherein the alkyl, alkoxy, alkenyl, cycloalkyl, heterocycloalkyl, phenyl or heteroaryl is optionally substituted with 1 to 4 R^{k};
in some embodiments, R^{5c} is selected from one of the following groups optionally substituted with 1 to 4 R^{k}: methyl, ethyl, propyl, isopropyl, methoxy, ethoxy, ethenyl, propenyl, allyl, cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, bicyclo[2.2.1]heptyl, cyclopropyl-spiro-cyclobutyl, cyclobutyl-spiro-cyclobutyl, cyclobutyl-spiro-cyclopentyl, cyclobutyl-spiro-cyclohexyl, cyclopentyl-fused-cyclopentyl, 3-oxabicyclo[3.1.0]hexyl, thienyl, furyl, thiazolyl, oxazolyl, methoxymethyl, and ethoxymethyl;
in some embodiments, R^{5c} is selected from methyl, ethyl, propyl, isopropyl, methoxy, ethoxy, ethenyl, propenyl, allyl, cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, bicyclo[2.2.1]heptyl, cyclopropyl-spiro-cyclobutyl, cyclobutyl-spiro-cyclobutyl, cyclobutyl-spiro-cyclopentyl, cyclobutyl-spiro-cyclohexyl, cyclopentyl-fused-cyclopentyl, 3-oxabicyclo[3.1.0]hexyl, thienyl, furyl, thiazolyl, oxazolyl, methoxymethyl, and ethoxymethyl, and the R^{5c}is optionally substituted with 1 to 4 substituents selected from deuterium, F, Cl, Br, CN, OH, CHF₂, CD₃, methyl, ethyl, propyl, isopropyl, cyclopropyl, oxetanyl, -CH₂-cyclopropyl, -CH₂-oxetanyl, methoxy, methoxymethyl, methoxyethyl, CH₂CN, ethenyl, propenyl, 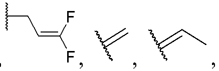 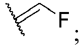
in some embodiments, R^{5d} and R^{5e} are each independently selected from H, C₁₋₆ alkyl, C₃₋₁₀ carbocyclyl, and 4- to 10-membered heterocyclyl, wherein the alkyl, carbocyclyl or heterocyclyl is optionally substituted with 1 to 4 R^{k};
in some embodiments, R^{5d} and R^{5e} are each independently selected from H, C₁₋₄ alkyl, C₃₋₆ cycloalkyl, 4- to 7-membered heterocycloalkyl, phenyl or 5- to 6-membered heteroaryl, wherein the alkyl, cycloalkyl, heterocycloalkyl, phenyl or heteroaryl is optionally substituted with 1 to 4 R^{k};
in some embodiments, R^{5d} and R^{5e} are each independently selected from H, methyl, ethyl, propyl, isopropyl, cyclopropyl, cyclobutyl, cyclopentyl or cyclohexyl, wherein the methyl, ethyl, propyl, isopropyl, cyclopropyl, cyclobutyl, cyclopentyl or cyclohexyl is optionally substituted with 1 to 4 substituents selected from deuterium, F, Cl, Br, CN, OH, CHF₂, CD₃, methyl, and ethyl;
in some embodiments, R^{5d} and R^{5b} are each independently selected from methyl, ethyl, propyl, isopropyl, methoxy, ethoxy, cyclopropyl, cyclobutyl, cyclopentyl or cyclohexyl, wherein the methyl, ethyl, propyl, isopropyl, methoxy, ethoxy, cyclopropyl, cyclobutyl, cyclopentyl or cyclohexyl is optionally substituted with 1 to 3 R^{k};
in some embodiments, R^{5f} and R^{5g} are each independently selected from H, halogen, C₁₋₆ alkyl, and C₃₋₁₀ carbocyclyl, wherein the alkyl or carbocyclyl is optionally substituted with 1 to 4 R^{k}; or R^{5f} and R^{5g} are directly connected to form C₃₋₆ carbocyclyl or 4- to 7-membered heterocyclyl, wherein the carbocyclyl or heterocyclyl is optionally substituted with 1 to 4 R^{k};
in some embodiments, R^{5f} and R^{5g} are each independently selected from H, halogen, C₁₋₄ alkyl, and C₃₋₆ cycloalkyl, wherein the alkyl or cycloalkyl is optionally substituted with 1 to 4 R^{k}; or R^{5f} and R^{5g} are directly connected to form C₃₋₆ carbocyclyl or 4- to 7-membered heterocyclyl, wherein the carbocyclyl or heterocyclyl is optionally substituted with 1 to 4 R^{k};
in some embodiments, R^{5f} and R^{5g} are each independently selected from H, F, Cl, Br, methyl, and ethyl; or R^{5f} and R^{5g} are directly connected to form cyclobutyl, cyclopentyl or cyclohexyl, wherein the cyclobutyl, cyclopentyl or cyclohexyl is optionally substituted with 1 to 4 substituents selected from deuterium, F, Cl, Br, I, CN, OH, SH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, cyclopropyl, and cyclobutyl;
in some embodiments, R^{5f} and R^{5g} are each independently selected from H, F, Cl, Br, methyl, and ethyl; or R^{5f} and R^{5g} are directly connected to form cyclobutyl, cyclopentyl or cyclohexyl;
in some embodiments, R^{5h} is selected from NH₂, NHC₁₋₆ alkyl, N(C₁₋₆ alkyl)₂, C₁₋₆ alkyl, C₃₋₆ carbocyclyl or 4- to 7-membered heterocyclyl, wherein the alkyl, carbocyclyl or heterocyclyl is optionally substituted with 1 to 4 R^{k};
in some embodiments, R⁵ⁱ is selected from CN, NO₂, -S(=O)₁₋₂-C₁₋₆ alkyl, and -S(=O)₁₋₂-C₃₋₆ carbocyclyl, wherein the alkyl or carbocyclyl is optionally substituted with 1 to 4 R^{k};
in some embodiments, R^{5h} is selected from NH₂, NHC₁₋₄ alkyl, N(C₁₋₄ alkyl)₂, C₁₋₆ alkyl, C₃₋₆ cycloalkyl or 4- to 7-membered heterocycloalkyl, wherein the alkyl, cycloalkyl or heterocycloalkyl is optionally substituted with 1 to 4 R^{k}; in some embodiments, R^{5h} is selected from NH₂, NHCH₃, methyl, ethyl, and cyclopropyl;
in some embodiments, R⁵ⁱ is selected from CN, NO₂, -S(=O)₁₋₂-C₁₋₄ alkyl, and -S(=O)₁₋₂-C₃₋₆ cycloalkyl, wherein the alkyl or cycloalkyl is optionally substituted with 1 to 4 R^{k}; in some embodiments, R⁵ⁱ is selected from CN, NO₂, -S(=O)₂₋methyl, and -S(=O)₂-cyclopropyl; in some embodiments, R¹, R², R³, R^{L1}, and R^{L2} are each independently selected from H, deuterium, halogen, CN, OH, NO₂, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, and -SC₁₋₆ alkyl, wherein the alkyl, alkenyl, or alkynyl is optionally substituted with 1 to 4 R^{k};
in some embodiments, R¹, R², R³, R^{L1}, and R^{L2} are each independently selected from H, deuterium, halogen, CN, OH, NO₂, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, and -SC₁₋₄ alkyl, wherein the alkyl, alkenyl, or alkynyl is optionally substituted with 1 to 4 R^{k};
in some embodiments, R¹, R², R³, R^{L1}, and R^{L2} are each independently selected from H, deuterium, F, Cl, Br, I, CN, OH, NO₂, NH₂, NH(CH₃), N(CH₃)₂, or one of the following groups optionally substituted with 1 to 3 R^{k}: methyl, ethyl, ethenyl, ethynyl, methoxy, ethoxy, isopropoxy, and methylthio;
in some embodiments, R¹, R², and R³ are each independently selected from H, deuterium, F, Cl, Br, I, CN, OH, NO₂, NH₂, NH(CH₃), N(CH₃)₂, CD₃, OCD₃, CF₃, CH₂F, CHF₂, CH₂OH, OCF₃, OCHF₂, OCH₂F, methyl, ethyl, ethenyl, ethynyl, methoxy, ethoxy, isopropoxy, and methylthio; preferably, R¹ is H; preferably, R² is selected from H, methyl or CD₃; preferably, R³ is selected from H, methyl or CD₃;
in some embodiments, R^{L1} and R^{L2} are each independently selected from H, deuterium, F, Cl, Br, I, CN, OH, NO₂, NH₂, NH(CH₃), N(CH₃)₂, methyl, ethyl, methoxy, ethoxy, isopropoxy, and methylthio;
in some embodiments, R^{L1} and R^{L2} together with the atom to which they are attached form C₃₋₈ carbocycle or 4- to 8-membered heterocycle, wherein the carbocycle or heterocycle is optionally substituted with 1 to 4 R^{k}; in some embodiments, R^{L1} and R^{L2} together with the carbon atom to which they are attached form C₃₋₆ carbocycle or 4- to 6-membered heterocycle, wherein the carbocycle or heterocycle is optionally substituted with 1 to 4 R^{k}; in some embodiments, R^{L1} and R^{L2} together with the carbon atom to which they are attached form the following groups optionally substituted with 1 to 3 R^{k}: cyclopropyl, cyclobutyl, cyclopentyl, cyclohexyl, azetidinyl, pyrrolidyl, piperidyl, oxetanyl, and tetrahydrofuryl;
in some embodiments, R^{5a} and R^{5b} together with the phosphorus atom to which they are attached form 5- to 8-membered heterocyclyl, wherein the heterocyclyl is optionally substituted with 1 to 4 R^{k}; in some embodiments, R^{5a} and R^{5b} together with the phosphorus atom to which they are attached form 5- to 8-membered monocyclic heterocycle, wherein the heterocycle is optionally substituted with 1 to 4 R^{k}; in some embodiments, R^{5d} and R^{5b} together with the phosphorus atom to which they are attached form the following groups optionally substituted with 1 to 3 R^{k}: 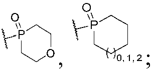
in some embodiments, each R^{k} is independently selected from deuterium, halogen, =O, CN, OH, SH, NO₂, COOH, CONH₂, NH₂, SF₅, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₄ alkylene-C₃₋₆ carbocyclyl, -C₁₋₄ alkylene-3- to 7-membered heterocyclyl, C₃₋₆ carbocyclyl, 3- to 7-membered heterocyclyl, 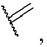 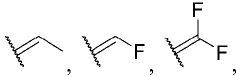 wherein the alkyl, alkylene, alkenyl, alkynyl, carbocyclyl or heterocyclyl is optionally substituted with 1 to 4 substituents selected from deuterium, halogen, CN, OH, NH₂, CH₂CN, C₁₋₆ alkyl, and C₁₋₆ alkoxy;
in some embodiments, each R^{k} is independently selected from deuterium, halogen, =O, CN, OH, SH, NO₂, COOH, CONH₂, NH₂, SF₅, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, -O-C₃₋₆ carbocyclyl, -O-3- to 6-membered heterocyclyl, -NH-C₃₋₆ carbocyclyl, -NH-3- to 6-membered heterocyclyl, -C₁₋₂ alkylene-C₃₋₆ carbocyclyl, -C₁₋₂ alkylene-3- to 6-membered heterocyclyl, C₃₋₆ carbocyclyl, 3- to 6-membered heterocyclyl, 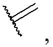 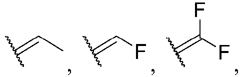 wherein the alkyl, alkylene, alkenyl, alkynyl, carbocyclyl or heterocyclyl is optionally substituted with 1 to 4 substituents selected from deuterium, halogen, CN, OH, NH₂, CH₂CN, C₁₋₄ alkyl, C₁₋₄ alkoxy;
in some embodiments, each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, morpholinyl, 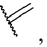 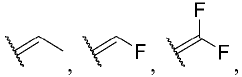 wherein the methyl, ethyl, propyl, isopropyl, methoxy, ethoxy, methylthio, ethenyl, ethynyl, propynyl, propargyl, cyclopropyl, cyclobutyl, azetidinyl, oxetanyl, pyrrolidyl, piperidyl, pyrazolyl, pyrrolyl, or morpholinyl is optionally substituted with 1 to 4 substituents selected from deuterium, F, Cl, Br, I, CN, OH, NH₂, CH₂CN, C₁₋₄ alkyl, and C₁₋₄ alkoxy;
in some embodiments, each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, morpholinyl, 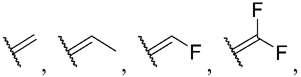 wherein the methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, or morpholinyl is optionally substituted with 1 to 4 substituents selected from deuterium, F, Cl, Br, I, CN, OH, NH₂, CH₂CN, methyl, ethyl, methoxy, and ethoxy;
in some embodiments, R^{k} is R^{k1};
in some embodiments, R^{k1} is selected from deuterium, F, Cl, Br, OH, CN, methyl, ethyl, propyl, isopropyl, ethenyl, propenyl, allyl, -CH₂-cyclopropyl, -CH₂-cyclopropyl, -CH₂-cyclobutyl, cyclopropyl, cyclobutyl, cyclopentyl, cyclohexyl, 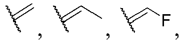 wherein the methyl, ethyl, propyl, isopropyl, ethenyl, propenyl, allyl, -CH₂-cyclopropyl, -CH₂-cyclopropyl, -CH₂-cyclobutyl, cyclopropyl, cyclobutyl, cyclopentyl, or cyclohexyl is optionally substituted with 1 to 4 substituents selected from deuterium, F, Cl, Br, OH, CN, CH₂CN, methyl, ethyl, methoxy or ethoxy;
in some embodiments, ring A is phenyl optionally substituted with 1 to 4 R^{a}; ring B is selected from 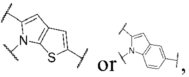 and the ring B is optionally substituted with 1 to 4 R^{b}, the right side of which is linked to Q; -Q-R⁶ is optionally substituted 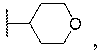 which, when substituted, is substituted with 1 to 4 substituents selected from deuterium, F, Cl, Br, OH, CF₃, CHF₂, CH₂F, CD₃, CHD₂, CH₂D, OCD₃, OCF₃, methyl, ethyl, propyl, isopropyl, methoxy or ethoxy; ring D is selected from 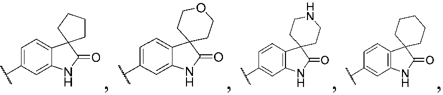 and the ring D is optionally substituted with 1 to 4 R^{d}; X is selected from O or S;
L₁ is -C(=O)-;
L₂ is selected from 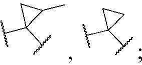 R⁴ is 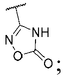
each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, propynyl, methoxy, ethoxy, isopropoxy, cyclopropyl, -CH₂OH, -CH₂CH₂OH, - CH₂CN, -CH₂N(CH₃)₂, -CH₂-cyclopropyl, 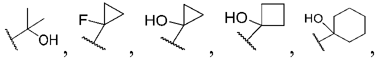 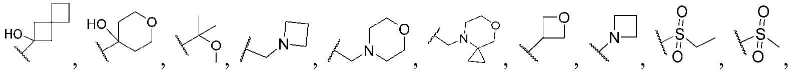 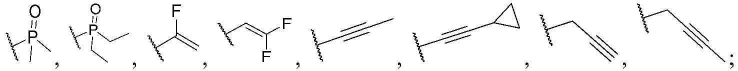
each R^{b} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, methoxy, ethoxy, isopropoxy, and cyclopropyl;
each R^{d} is independently selected from deuterium, F, Cl, Br, I, CN, OH, NH₂, NH(CH₃), N(CH₃)₂, CHF₂, CH₂F, CF₃, CD₃, methyl, ethyl, methoxy, ethoxy, cyclopropyl, oxetanyl, -CH₂-cyclopropyl, -CH₂-oxetanyl, -C(=O)CH₃, and -C(=O)-cyclopropyl.

A first embodiment of the present invention provides the above-described compound as shown in general formula (I) or the racemate, stereoisomer, tautomer, deuterated substance, solvate, prodrug, metabolite, pharmaceutically acceptable salt or eutectic crystal thereof, wherein 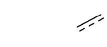 represents a single bond or a double bond;
Y is selected from C and N, Z is selected from CH or N, J is selected from N or C, and at least one of Y, Z and J is N;
ring A is selected from C₆₋₁₀ aryl, 5- to 10-membered heteroaryl, C₃₋₁₀ carbocyclyl, 4- to 10-membered heterocyclyl, C₁₁₋₁₅ aryl, 11- to 15-membered heterocyclyl, and 11- to 15-membered heteroaryl, the ring A is optionally substituted with 1 to 4 R^{a}, and the nitrogen atom in the heteroaryl or heterocyclyl is optionally oxidised to form an N-oxide;
ring B is selected from C₆₋₁₀ aryl, 5- to 6-membered heteroaryl, 5-membered ring-fused-5-membered heteroaryl, 5-membered ring-fused-6-membered heteroaryl, 6-membered ring-fused-6-membered heteroaryl, C₃₋₁₀ carbocyclyl, and 4- to 10-membered heterocyclyl, and the ring B is optionally substituted with 1 to 4 R^{b};
ring D is selected from 13- to 16-membered tricyclic or tetracyclic heterocyclyl, and 17- to 30-membered tricyclic or tetracyclic heterocyclyl, and the ring D is optionally substituted with 1 to 4 R^{d};
L₁ is selected from -S(=O)₂-, -C(=O)-, and -C(=S)-;
L₂ is -(CR^{L1}R^{L2})ₘ-;
X is selected from S or O;
Q is selected from a bond, O, S, NH, C₁₋₄ alkylene, 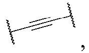 wherein the alkylene is optionally substituted with 1 to 4 R^{k};
m is selected from 1, 2, 3 or 4;
R⁴ is selected from -C(=O)R^{4a}, -C(=O)OR^{4a}, -C(=O)NR^{4a}R^{4b}, 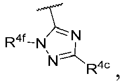 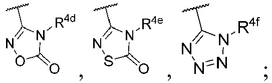
R^{4a}, R^{4b}, R^{4c}, R^{4d}, R^{4e}, and R^{4f} are each independently selected from H, deuterium, and C₁₋₆ alkyl, wherein the alkyl is optionally substituted with 1 to 4 R^{k};
R⁶ is selected from C₁₋₆ alkyl, -C₁₋₂ alkylene-O-C₁₋₄ alkyl, C₃₋₁₀ carbocyclyl, and 4- to 10-membered heterocyclyl, and the R⁶ is optionally substituted with 1 to 4 R^{6a};
R^{a}, R^{b}, R^{d}, and R^{6a} are each independently selected from H, deuterium, halogen, =O, CN, OH, NO₂, COOH, CONH₂, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₄ alkylene-C₃₋₆ carbocyclyl, -C₁₋₄ alkylene-3- to 7-membered heterocyclyl, -P(=O)R^{5a}R^{5b}, -S(=O)₁₋₂-R^{5c}, -C(=O)R^{5c}, -C(=O)NR^{5d}R^{5e}, - NR^{5d}C(=O)R^{5e}, -C(=O)-M-C(=O)-R^{5c} -CH₂-M-C(=O)-R^{5c}, -C(=O)-M-CH₂-R^{5c} - C(=O)-M-R^{5c}, -C(=O)-C₁₋₄ alkylene-OC₀₋₄ alkylene-R^{5c}, -C(=O)-M-C₁₋₄ alkylene-O-R^{5c}, C₃₋₁₂ carbocyclyl, 3- to 12-membered heterocyclyl, 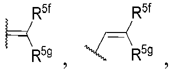 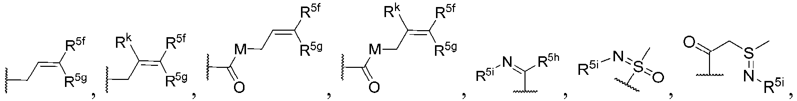 wherein the alkyl, alkylene, alkenyl, alkynyl, carbocyclyl or heterocyclyl is optionally substituted with 1 to 4 R^{k};
M is selected from C₃₋₆ carbocyclyl or 4- to 7-membered heterocyclyl, and the M is optionally substituted with 1 to 4 R^{k};
R^{5a}, R^{5b}, and R^{5c} are each independently selected from C₁₋₆ alkyl, -C₁₋₄ alkyl-OC₁₋₄ alkyl, C₁₋₆ alkoxy, C₂₋₆ alkenyl, C₃₋₁₂ carbocyclyl, and 4- to 12-membered heterocyclyl, wherein the alkyl, alkenyl, alkoxy, carbocyclyl or heterocyclyl is optionally substituted with 1 to 4 R^{k};
R^{5d} and R^{5e} are each independently selected from H, C₁₋₆ alkyl, C₃₋₁₀ carbocyclyl, and 4- to 10-membered heterocyclyl, wherein the alkyl, carbocyclyl or heterocyclyl is optionally substituted with 1 to 4 R^{k};
R^{5f} and R^{5g} are each independently selected from H, halogen, C₁₋₆ alkyl, and C₃₋₁₀ carbocyclyl, wherein the alkyl or carbocyclyl is optionally substituted with 1 to 4 R^{k};
or R^{5f} and R^{5g} are directly connected to form C₃₋₆ carbocyclyl or 4- to 7-membered heterocyclyl, wherein the carbocyclyl or heterocyclyl is optionally substituted with 1 to 4 R^{k};
R^{5h} is selected from NH₂, NHC₁₋₆ alkyl, N(C₁₋₆ alkyl)₂, C₁₋₆ alkyl, C₃₋₆ carbocyclyl or 4- to 7-membered heterocyclyl, wherein the alkyl, carbocyclyl or heterocyclyl is optionally substituted with 1 to 4 R^{k};
R⁵ⁱ is selected from CN, NO₂, -S(=O)₁₋₂-C₁₋₆ alkyl, and -S(=O)₁₋₂-C₃₋₆ carbocyclyl, wherein the alkyl or carbocyclyl is optionally substituted with 1 to 4 R^{k};
R¹, R², R³, R^{L1}, and R^{L2} are each independently selected from H, deuterium, halogen, CN, OH, NO₂, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, and -SC₁₋₆ alkyl, wherein the alkyl, alkenyl, or alkynyl is optionally substituted with 1 to 4 R^{k};
alternatively, R^{L1} and R^{L2} together with the atom to which they are attached form C₃₋₈ carbocyclyl or 4- to 8-membered heterocyclyl, wherein the carbocyclyl or heterocyclyl is optionally substituted with 1 to 4 R^{k};
alternatively, R^{5a} and R^{5b} together with the phosphorus atom to which they are attached form 5- to 8-membered heterocyclyl, wherein the heterocyclyl is optionally substituted with 1 to 4 R^{k};
each R^{k} is independently selected from deuterium, halogen, =O, CN, OH, SH, NO₂, COOH, CONH₂, NH₂, SF₅, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₄ alkylene-C₃₋₆ carbocyclyl, -C₁₋₄ alkylene-3- to 7-membered heterocyclyl, C₃₋₆ carbocyclyl, 3- to 7-membered heterocyclyl, 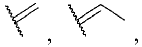 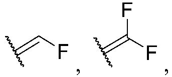 wherein the alkyl, alkylene, alkenyl, alkynyl, carbocyclyl or heterocyclyl is optionally substituted with 1 to 4 substituents selected from deuterium, halogen, CN, OH, NH₂, CH₂CN, C₁₋₆ alkyl, and C₁₋₆ alkoxy.

A second embodiment of the present invention provides the above-described compound as shown in general formula (I) or the racemate, stereoisomer, tautomer, deuterated substance, solvate, prodrug, metabolite, pharmaceutically acceptable salt or eutectic crystal thereof, wherein
ring B is selected from 5-membered ring-fused-5-membered heteroaryl or 5-membered ring-fused-6-membered heteroaryl, and the ring B is optionally substituted with 1 to 4 R^{b};
ring D is selected from partially saturated rings as follows: 13- to 15-membered tricyclic fused heterocyclyl, 10-membered fused carbocycle spiro-linked to C₄₋₆ carbocycle, 9-membered fused carbocycle spiro-linked to C₄₋₆ carbocycle, 11-membered fused carbocycle spiro-linked to C₃₋₅ carbocycle, 10-membered fused heterocycle spiro-linked to C₄₋₆ carbocycle, 11-membered fused heterocycle spiro-linked to C₃₋₅ carbocycle, 10-membered fused carbocycle spiro-linked to 4- to 6-membered heterocycle, 11-membered fused carbocycle spiro-linked to 4- to 5-membered heterocycle, 9-membered fused heterocycle spiro-linked to 4- to 6-membered heterocycle, 9-membered fused heterocycle spiro-linked to C₃₋₆ carbocycle, 9-membered fused heterocycle spiro-linked to 8- to 10-membered heterocycle, 10-membered fused heterocycle spiro-linked to 4- to 6-membered heterocycle, 11-membered fused heterocycle spiro-linked to 4- to 5-membered heterocycle, 11-membered tricyclic heterocycle spiro-linked to C₃₋₄ carbocycle, and the ring D is optionally substituted with 1 to 4 R^{d};
ring A is selected from phenyl, 5- to 6-membered heteroaryl, C₃₋₆ monocyclic carbocyclyl, 4- to 8-membered monocyclic heterocyclyl, and benzo C₇₋₈ carbocyclyl, the ring A is optionally substituted with 1 to 4 R^{a}, and the nitrogen atom in the heteroaryl or heterocyclyl is optionally oxidised to form an N-oxide;
R⁶ is selected from C₁₋₄ alkyl, -C₁₋₂ alkylene-O-C₁₋₄ alkyl, C₃₋₆monocyclic carbocyclyl, C₆₋₁₀ fused carbocyclyl, C₆₋₁₀ spirocarbocyclyl, C₅₋₁₀ bridged carbocyclyl, 4- to 8-membered monocyclic heterocyclyl, 7- to 10-membered fused heterocyclyl, 7- to 10-membered spiro heterocyclyl, and 6- to 10-membered bridged heterocyclyl, and the R⁶ is optionally substituted with 1 to 4 R^{6a};
R^{4a}, R^{4b}, R^{4c}, R^{4d}, R^{4e}, and R^{4f} are each independently selected from H, deuterium, and C₁₋₄ alkyl, wherein the alkyl is optionally substituted with 1 to 4 R^{k};
R^{a}, R^{b}, R^{d}, and R^{6a} are each independently selected from H, deuterium, halogen, =O, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₂ alkylene-C₃₋₆ carbocyclyl, -C₁₋₂ alkylene-3- to 7-membered heterocyclyl, - P(=O)R^{5a}R^{5b}, -S(=O)₁₋₂-R^{5c}, -C(=O)R^{5c}, -C(=O)NR^{5d}R^{5e}, -NR^{5d}C(=O)R^{5e}, -C(=O)-M-C(=O)-R^{5c}, -CH₂-M-C(=O)-R^{5c}, -C(=O)-M-R^{5c}, -C(=O)-M-R^{5c}, -C(=O)-C₁₋₂ alkylene-OC₀₋₂ alkylene-R^{5c}, -C(=O)-M-C₁₋₂ alkylene-O-R^{5c}, C₃₋₁₁ carbocyclyl, 3-to 11-membered heterocyclyl, 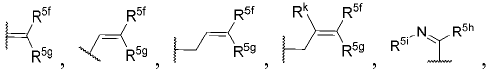 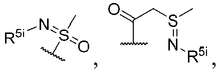 wherein the alkyl, alkenyl, alkynyl, alkylene, carbocyclyl or heterocyclyl is optionally substituted with 1 to 4 R^{k};
M is selected from C₃₋₆ cycloalkyl, phenyl, 4- to 7-membered heterocycloalkyl or 5- to 6-membered heteroaryl, and the M is optionally substituted with 1 to 4 R^{k};
R^{5a}, R^{5b}, and R^{5c} are each independently selected from C₁₋₄ alkyl, -C₁₋₄ alkyl-OC₁₋₄ alkyl, C₁₋₄ alkoxy, C₂₋₄ alkenyl, C₃₋₆ cycloalkyl, C₅₋₈ bridged cycloalkyl, C₅₋₁₁ spirocycloalkyl, C₅₋₁₁ fused cycloalkyl, 4- to 7-membered monocyclic heterocycloalkyl, 6- to 10-membered bridged heterocycloalkyl, 6- to 11-membered spiroheterocycloalkyl, 6- to 11-membered fused heterocycloalkyl, phenyl or 5- to 6-membered heteroaryl, wherein the alkyl, alkoxy, alkenyl, cycloalkyl, heterocycloalkyl, phenyl or heteroaryl is optionally substituted with 1 to 4 R^{k};
R^{5d} and R^{5e} are each independently selected from H, C₁₋₄ alkyl, C₃₋₆ cycloalkyl, 4- to 7-membered heterocycloalkyl, phenyl or 5- to 6-membered heteroaryl, wherein the alkyl, cycloalkyl, heterocycloalkyl, phenyl or heteroaryl is optionally substituted with 1 to 4 R^{k};
R^{5f} and R^{5g} are each independently selected from H, halogen, C₁₋₄ alkyl, and C₃₋₆ cycloalkyl, wherein the alkyl or cycloalkyl is optionally substituted with 1 to 4 R^{k};
or R^{5f} and R^{5g} are directly connected to form C₃₋₆ carbocyclyl or 4- to 7-membered heterocyclyl, wherein the carbocyclyl or heterocyclyl is optionally substituted with 1 to 4 R^{k};
R^{5h} is selected from NH₂, NHC₁₋₄ alkyl, N(C₁₋₄ alkyl)₂, C₁₋₆ alkyl, C₃₋₆ cycloalkyl or 4- to 7-membered heterocycloalkyl, wherein the alkyl, cycloalkyl or heterocycloalkyl is optionally substituted with 1 to 4 R^{k};
R⁵ⁱ is selected from CN, NO₂, -S(=O)₁₋₂-C₁₋₄ alkyl, or -S(=O)₁₋₂-C₃₋₆ cycloalkyl, wherein the alkyl or cycloalkyl is optionally substituted with 1 to 4 R^{k};
R¹, R², R³, R^{L1} and R^{L2} are each independently selected from H, deuterium, halogen, CN, OH, NO₂, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, or -SC₁₋₄ alkyl, wherein the alkyl, alkenyl, or alkynyl is optionally substituted with 1 to 4 R^{k};
alternatively, R^{L1} and R^{L2} together with the carbon atom to which they are attached form C₃₋₆ carbocyclyl or 4- to 6-membered heterocyclyl, wherein the carbocyclyl or heterocyclyl is optionally substituted with 1 to 4 R^{k};
alternatively, R^{5a} and R^{5b} together with the phosphorus atom to which they are attached form 5- to 8-membered monocyclic heterocyclyl, wherein the heterocyclyl is optionally substituted with 1 to 4 R^{k}; the remaining definitions are the same as those in the first embodiment of the present invention.

A third embodiment of the present invention provides the above-described compound as shown in general formula (I) or the racemate, stereoisomer, tautomer, deuterated substance, solvate, prodrug, metabolite, pharmaceutically acceptable salt or eutectic crystal thereof, wherein
ring B is selected from pyrrolothienyl, pyrrolopyrazolyl, pyrrolopyrrolyl, pyrroloimidazolyl, pyrazolothienyl, imidazothienyl, imidazoimidazolyl, pyrazolopyrazolyl, pyrrolothiazolyl, pyrrolofuryl, indolyl, pyrrolopyridyl, pyrrolopyrimidyl, pyrrolopyridazinyl, pyrrolopyrazinyl, pyrrolotriazinyl, pyrazolophenyl, pyrazolopyridyl, pyrazolopyrimidyl, imidazophenyl, imidazopyridyl, imidazopyrimidyl, thienopyridyl, thienophenyl, furopyridyl, and furophenyl, and the ring B is optionally substituted with 1 to 4 R^{b};
L₂ is selected from -(CR^{L1}R^{L2})- and -(CR^{L1}R^{L2})₂-;
each R^{k} is independently selected from deuterium, halogen, =O, CN, OH, SH, NO₂, COOH, CONH₂, NH₂, SF₅, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, -O-C₃₋₆ carbocyclyl, -O-3- to 6-membered heterocyclyl, -NH-C₃₋₆ carbocyclyl, -NH-3- to 6-membered heterocyclyl, -C₁₋₂ alkylene-C₃₋₆ carbocyclyl, -C₁₋₂ alkylene-3- to 6-membered heterocyclyl, C₃₋₆ carbocyclyl, 3- to 6-membered heterocyclyl, 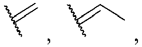 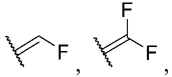 wherein the alkyl, alkylene, alkenyl, alkynyl, carbocyclyl or heterocyclyl is optionally substituted with 1 to 4 substituents selected from deuterium, halogen, CN, OH, NH₂, CH₂CN, C₁₋₄ alkyl, C₁₋₄ alkoxy;
the remaining definitions are the same as those in the first or second embodiment of the present invention.

A fourth embodiment of the present invention provides the above-described compound as shown in general formula (I) or the racemate, stereoisomer, tautomer, deuterated substance, solvate, prodrug, metabolite, pharmaceutically acceptable salt or eutectic crystal thereof, wherein
ring B is selected from one of the following groups optionally substituted with 1 to 4 R^{b}: 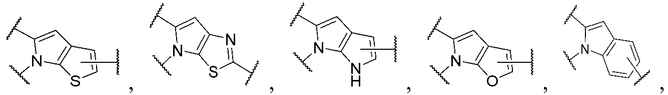 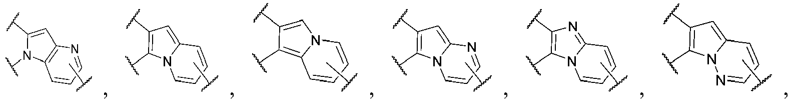 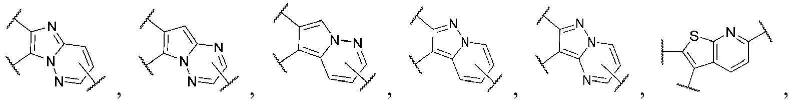 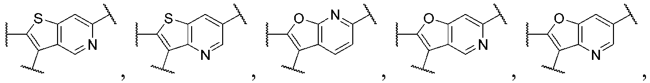 the right side of which is linked to Q;
ring D is selected from 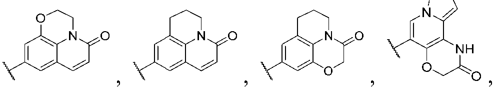 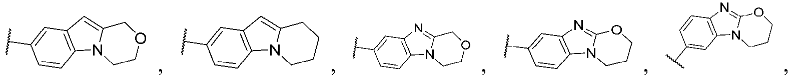 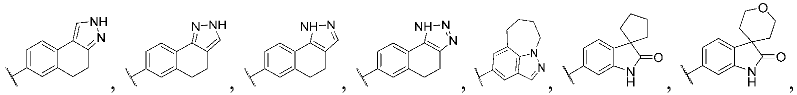 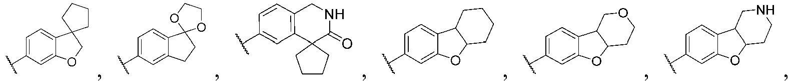 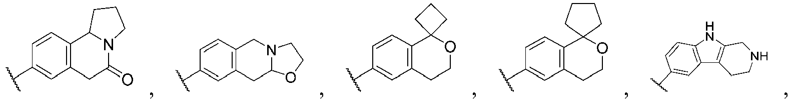 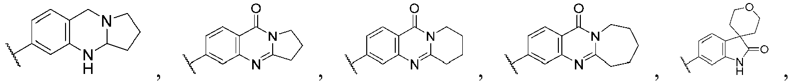 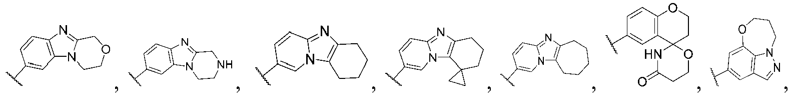 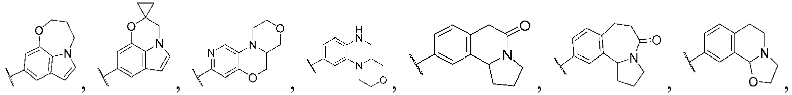 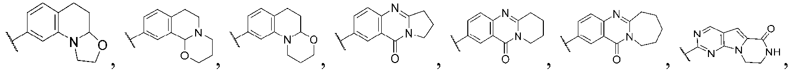 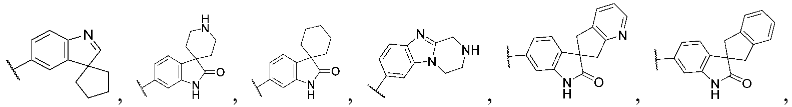 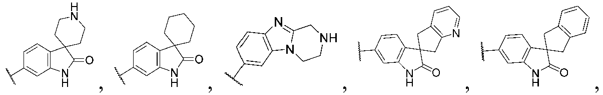 and the ring D is optionally substituted with 1 to 4 R^{d};
Q is selected from a bond, O, S, NH, CH₂, CH(CH₃), 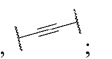
preferably, ring D is selected from 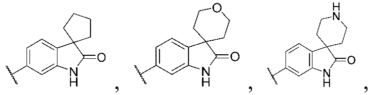 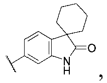 and the ring D is optionally substituted with 1 to 4 R^{d};
R⁶ is selected from methyl, ethyl, isopropyl, propyl, -CH₂O-CH₃, -CH₂O-CH₂CH₃, -CH₂O-CH(CH₃)₂, -CH₂O-C(CH₃)₃, -CH₂CH₂O-CH₃, -CH₂CH₂O-CH₂CH₃, -CH₂CH₂O-CH(CH₃)₂, -CH₂CH₂O-C(CH₃)₃, cyclopropyl, cyclobutyl, cyclopentyl, cyclohexyl, bicyclo[1.1.1]cyclopentyl, oxetanyl, oxolanyl, oxanyl, azetidinyl, pyrrolidyl, piperidyl, piperazinyl, morpholinyl, phenyl, pyrazolyl, thiazolyl, imidazolyl, oxazolyl, pyrrolyl, thienyl, furyl, pyridyl, pyrimidyl, pyrazinyl, pyridazinyl or 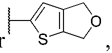 and the R⁶ is optionally substituted with 1 to 4 R^{6a};
R^{4a}, R^{4b}, R^{4c}, R^{4d}, R^{4e}, and R^{4f} are each independently selected from H, deuterium, methyl, ethyl, propyl, and isopropyl;
R^{a}, R^{b}, R^{d}, and R^{6a} are each independently selected from H, deuterium, F, Cl, Br, I, =O, CN, OH, NO₂, NH₂, NH(CH₃), N(CH₃)₂, -P(=O)R^{5a}R^{5b}, 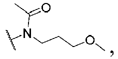 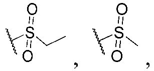 -C(=O)CH₃, -C(=O)CH₂CH₃, or one of the following groups optionally substituted with 1 to 3 R^{k}: methyl, ethyl, propyl, isopropyl, butyl, ethenyl, ethynyl, propynyl, methoxy, ethoxy, isopropoxy, methylthio, cyclopropyl, cyclobutyl, cyclopentyl, cyclohexyl, cyclobutyl-spiro-cyclobutyl, cyclopentyl-spiro-cyclobutyl, cyclopentyl-spiro-cyclopentyl, cyclohexyl-spiro-cyclobutyl, cyclohexyl-spiro-cyclopentyl, cyclohexyl-spiro-cyclohexyl, azetidinyl, oxetanyl, pyrrolidyl, piperidyl, piperazinyl, morpholinyl, morpholinyl-spiro-cyclohexyl, - C(=O)NHCH₃, -C(=O)NH-cyclopropyl, 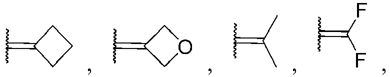 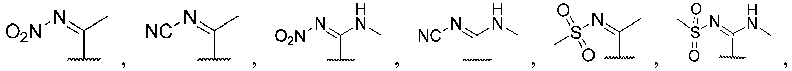 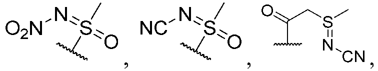 -CH₂-cyclopropyl, -CH₂-cyclobutyl, -CH₂-cyclopentyl, -CH₂-cyclohexyl, -CH₂-azetidinyl, -CH₂-azacyclopentyl, -CH₂-piperidyl, -CH₂-piperazinyl, -CH₂-oxetanyl, -CH₂-tetrahydrofuryl, -CH₂-oxanyl, - C(=O)R^{5c}, -C(=O)-M-C(=O)-R^{5c}, -CH₂-M-C(=O)-R^{5c}, -C(=O)-M-CH₂-R^{5c}, - C(=O)-M-R^{5c}, -C(=O)-CH₂-O-R^{5c}, -C(=O)-CH₂-OCH₂-R^{5c}, -C(=O)-M-CH₂-O-R^{5c}, 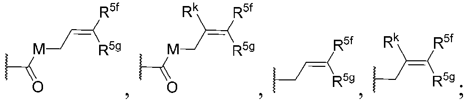
M is selected from cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, and 3-oxabicyclo[3.1.0]hexyl, and the M is optionally substituted with 1 to 4 R^{k};
R^{5c} is selected from one of the following groups optionally substituted with 1 to 4 R^{k}: methyl, ethyl, propyl, isopropyl, methoxy, ethoxy, ethenyl, propenyl, allyl, cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, bicyclo[2.2.1]heptyl, cyclopropyl-spiro-cyclobutyl, cyclobutyl-spiro-cyclobutyl, cyclobutyl-spiro-cyclopentyl, cyclobutyl-spiro-cyclohexyl, cyclopentyl-fused-cyclopentyl, 3-oxabicyclo[3.1.0]hexyl, thienyl, furyl, thiazolyl, oxazolyl, methoxymethyl, and ethoxymethyl;
R^{5f} and R^{5g} are each independently selected from H, F, Cl, Br, methyl, and ethyl;
or R^{5f} and R^{5g} are directly connected to form cyclobutyl, cyclopentyl or cyclohexyl, wherein the cyclobutyl, cyclopentyl or cyclohexyl is optionally substituted with 1 to 4 substituents selected from deuterium, F, Cl, Br, I, CN, OH, SH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, cyclopropyl, and cyclobutyl;
R^{5a} and R^{5b} are each independently selected from methyl, ethyl, propyl, isopropyl, methoxy, ethoxy, cyclopropyl, cyclobutyl, cyclopentyl or cyclohexyl, wherein the methyl, ethyl, propyl, isopropyl, methoxy, ethoxy, cyclopropyl, cyclobutyl, cyclopentyl or cyclohexyl is optionally substituted with 1 to 3 R^{k};
R¹, R², R³, R^{L1}, and R^{L2} are each independently selected from H, deuterium, F, Cl, Br, I, CN, OH, NO₂, NH₂, NH(CH₃), N(CH₃)₂, or one of the following groups optionally substituted with 1 to 3 R^{k}: methyl, ethyl, ethenyl, ethynyl, methoxy, ethoxy, isopropoxy, and methylthio;
alternatively, R^{L1} and R^{L2} together with the carbon atom to which they are attached form the following groups optionally substituted with 1 to 3 R^{k}: cyclopropyl, cyclobutyl, cyclopentyl, cyclohexyl, azetidinyl, pyrrolidyl, piperidyl, oxetanyl, and tetrahydrofuryl;
alternatively, R^{5a} and R^{5b} together with the phosphorus atom to which they are attached form the following groups optionally substituted with 1 to 3 R^{k}: 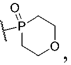 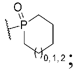
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, morpholinyl, 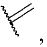 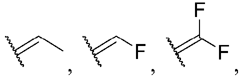 wherein the methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, or morpholinyl is optionally substituted with 1 to 4 substituents selected from deuterium, F, Cl, Br, I, CN, OH, NH₂, C₁₋₄ alkyl, and C₁₋₄ alkoxy;
the remaining definitions are the same as those in the first, second, or third embodiment of the present invention.

A fifth embodiment of the present invention provides the above-described compound as shown in general formula (I) or the racemate, stereoisomer, tautomer, deuterated substance, solvate, prodrug, metabolite, pharmaceutically acceptable salt or eutectic crystal thereof, wherein
R⁴ is selected from -C(=O)OH, -C(=O)OCH₃, -C(=O)N(CH₃)₂, 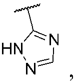 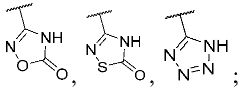
ring A is selected from one of the following groups optionally substituted with 1 to 4 R^{a}: phenyl, cyclopropyl, cyclobutyl, cyclopentyl, cyclohexyl, azetidinyl, pyrrolidyl, piperidyl, oxetanyl, oxolanyl, oxanyl, 1,3-dioxolanyl, 1,4-dioxanyl, piperazinyl, morpholinyl, pyrazolyl, imidazolyl, oxazolyl, thiazolyl, pyrrolyl, thienyl, triazolyl, pyridyl, pyrimidyl, pyridazinyl, pyrazinyl, triazinyl, 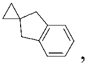 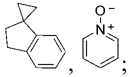
L₂ is selected from -CR^{L1}R^{L2}-, or one of the following groups optionally substituted with 1 to 3 R^{k}: 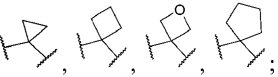
R^{L1} and R^{L2} are each independently selected from H, deuterium, F, Cl, Br, I, CN, OH, NO₂, NH₂, NH(CH₃), N(CH₃)₂, methyl, ethyl, methoxy, ethoxy, isopropoxy, and methylthio;
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, morpholinyl, 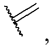 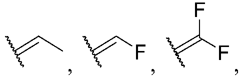 wherein the methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, or morpholinyl is optionally substituted with 1 to 4 substituents selected from deuterium, F, Cl, Br, I, CN, OH, NH₂, CH₂CN, methyl, ethyl, methoxy, and ethoxy;
the remaining definitions are the same as those in the first, second, third, or fourth embodiment of the present invention.

A sixth embodiment of the present invention provides the above-described compound as shown in general formula (I) or the racemate, stereoisomer, tautomer, deuterated substance, solvate, prodrug, metabolite, pharmaceutically acceptable salt or eutectic crystal thereof, wherein
X is selected from O or S;
L₁ is -C(=O)-;
L₂ is selected from 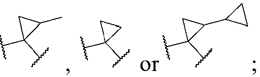
R⁴ is 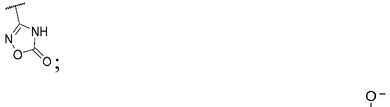
ring A is selected from phenyl, pyridyl, 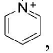 and the ring A is optionally substituted with 1 to 4 R^{a};
ring B is selected from 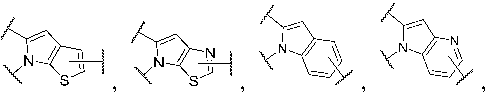 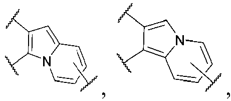 and the ring B is optionally substituted with 1 to 4 R^{b}, the right side of which is linked to Q;
preferably, ring B is selected from 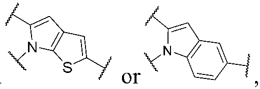 and the ring B is optionally substituted with 1 to 4 R^{b}, the right side of which is linked to Q;
-Q-R⁶ is selected from one of the following optionally substituted groups: ethyl, propyl, isopropyl, 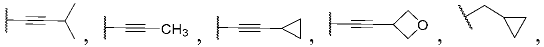 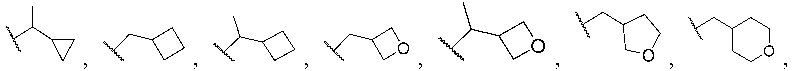 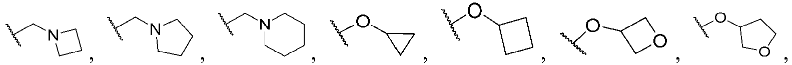 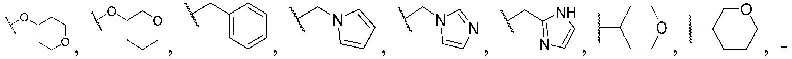 CH₂CH₂CH₂O-CH₂CH₃, -CH₂CH₂CH₂O-CH(CH₃)₂, -CH₂CH₂CH₂O-C(CH₃)₃, 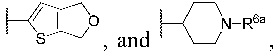 which, when substituted, are substituted with 1 to 4 substituents selected from deuterium, F, Cl, Br, I, =O, CN, OH, CF₃, CHF₂, CH₂F, CD₃, CHD₂, CH₂D, OCD₃, OCF₃, methyl, ethyl, propyl, isopropyl, methoxy or ethoxy;
preferably, -Q-R⁶ is optionally substituted 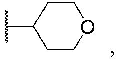 which, when substituted, is substituted with 1 to 4 substituents selected from deuterium, F, Cl, Br, OH, CF₃, CHF₂, CH₂F, CD₃, CHD₂, CH₂D, OCD₃, OCF₃, methyl, ethyl, propyl, isopropyl, methoxy or ethoxy;
R^{6a} is selected from methyl, ethyl, propyl, isopropyl, cyclopropyl, cyclobutyl, 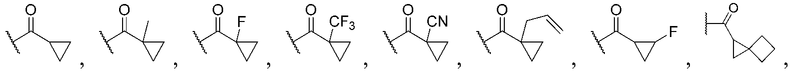 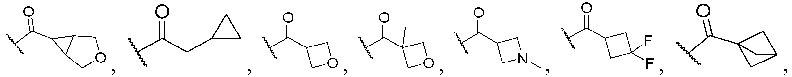 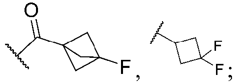
preferably, -Q-R⁶ is selected from one of the following optionally substituted groups: 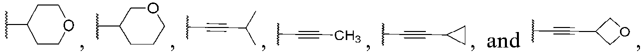 which, when substituted, are substituted with 1 to 4 substituents selected from deuterium, F, Cl, Br, I, =O, CN, OH, CF₃, CHF₂, CH₂F, CD₃, CHD₂, CH₂D, OCD₃, OCF₃, methyl, ethyl, propyl, isopropyl, methoxy or ethoxy;
R¹, R², and R³ are each independently selected from H, deuterium, F, Cl, Br, I, CN, OH, NO₂, NH₂, NH(CH₃), N(CH₃)₂, CD₃, OCD₃, CF₃, CH₂F, CHF₂, CH₂OH, OCF₃, OCHF₂, OCH₂F, methyl, ethyl, ethenyl, ethynyl, methoxy, ethoxy, isopropoxy, and methylthio;
preferably, R¹ is H;
preferably, R² is selected from H, methyl or CD₃;
preferably, R³ is selected from H, methyl or CD₃;
each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, propynyl, methoxy, ethoxy, isopropoxy, cyclopropyl, -CH₂OH, -CH₂CH₂OH, - CH₂CN, -CH₂N(CH₃)₂, -CH₂-cyclopropyl, 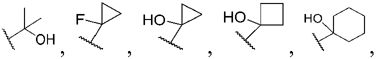 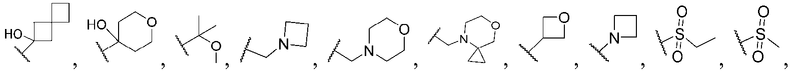 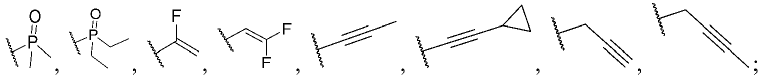
preferably, each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, methoxy, cyclopropyl, 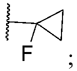
each R^{b} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, methoxy, ethoxy, isopropoxy, and cyclopropyl;
each R^{d} is independently selected from deuterium, F, Cl, Br, I, CN, OH, NH₂, NH(CH₃), N(CH₃)₂, CD₃, -CH₂CH₂OCH₃, -CF₃, -CH₂F, -CHF₂, -CH₂CH₂F, - CH₂CH₂CH₂F, -OCH₂CH₂OH, 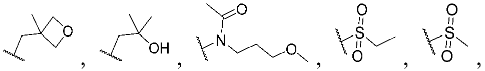 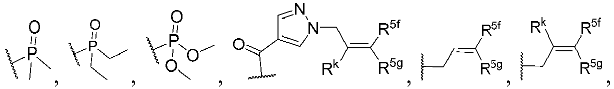 -C(=O)NHCH₃, - C(=O)NH-cyclopropyl, 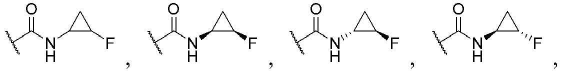 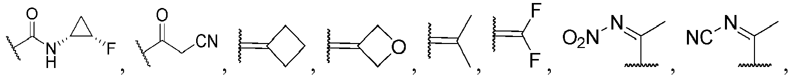 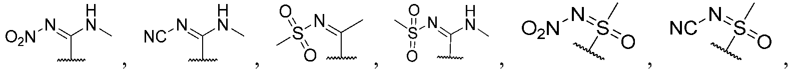 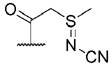 or one of the following groups optionally substituted with 1 to 4 R^{k1}: methyl, ethyl, methoxy, ethoxy, cyclopropyl, oxetanyl, -CH₂-cyclopropyl, -CH₂-cyclobutyl, -CH₂-cyclopentyl, -CH₂-oxetanyl, -CH₂-azetidinyl, -S(=O)₂CH₃, - S(=O)₂ cyclopropyl, -C(=O)NH-cyclopropyl, -C(=O)CH₃, -C(=O)-ethenyl, - C(=O)-cyclopropyl, -C(=O)-cyclobutyl, -C(=O)-cyclopentyl, -C(=O)-bicyclo[2.2.1]heptyl, -C(=O)-bicyclo[1.1.1]pentyl, -C(=O)-cyclobutyl-spiro-cyclobutyl, -C(=O)-oxetanyl, -C(=O)-tetrahydrofuryl, -C(=O)-oxanyl, -C(=O)-azetidinyl, -C(=O)-pyrrolidyl, -C(=O)-piperidyl, -C(=O)-piperazinyl, -C(=O)-pyrazolyl, -C(=O)-phenyl, -C(=O)-azetidinyl-CH₂-cyclopropyl, -C(=O)-pyrazolyl-CH₂-cyclopropyl, -C(=O)-pyrazolyl-CH₂-cyclobutyl, -C(=O)-pyrazolyl-CH₂-cyclopentyl, -C(=O)-pyrrolidyl-CH₂-cyclopropyl, -C(=O)-piperidyl-CH₂-cyclopropyl, -C(=O)-piperazinyl-CH₂-cyclopropyl, -C(=O)-azetidinyl -C(=O)-cyclopropyl, -C(=O)-pyrrolidyl -C(=O)-cyclopropyl, -C(=O)-piperidyl -C(=O)-cyclopropyl, -C(=O)-piperazinyl -C(=O)-cyclopropyl, -CH₂-azetidinyl -C(=O)-cyclopropyl, -CH₂-pyrrolidyl -C(=O)-cyclopropyl, -CH₂-azetidinyl-CH₂-cyclopropyl, -CH₂-pyrrolidyl-CH₂-cyclopropyl, -CH₂-piperidyl-CH₂-cyclopropyl, - CH₂-piperazinyl-CH₂-cyclopropyl, 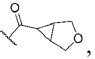 -C(=O)-cyclopropyl-phenyl, -C(=O)-cyclopropyl-pyridyl, -C(=O)-cyclopropyl-thienyl, -C(=O)-cyclopropyl-furyl, - C(=O)-CH₂-O-cyclopropyl, or -C(=O)-CH₂-OCH₂-cyclopropyl;
R^{k1} is selected from methyl, ethyl, propyl, isopropyl, ethenyl, propenyl, allyl, -CH₂-cyclopropyl, -CH₂-cyclopropyl, -CH₂-cyclobutyl, cyclopropyl, cyclobutyl, cyclopentyl, cyclohexyl, 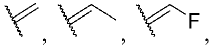 wherein the methyl, ethyl, propyl, isopropyl, ethenyl, propenyl, allyl, -CH₂-cyclopropyl, -CH₂-cyclopropyl, -CH₂-cyclobutyl, cyclopropyl, cyclobutyl, cyclopentyl, or cyclohexyl is optionally substituted with 1 to 4 substituents selected from deuterium, F, Cl, Br, OH, CN, CH₂CN, methyl, ethyl, methoxy or ethoxy;
R^{5f} and R^{5g} are each independently selected from H, F, Cl, Br, methyl, and ethyl; or R^{5f} and R^{5g} are directly connected to form cyclobutyl, cyclopentyl or cyclohexyl;
preferably, each R^{d} is independently selected from deuterium, F, Cl, Br, I, CN, OH, NH₂, NH(CH₃), N(CH₃)₂, CHF₂, CH₂F, CF₃, CD₃, methyl, ethyl, methoxy, ethoxy, cyclopropyl, oxetanyl, -CH₂-cyclopropyl, -CH₂-oxetanyl, -C(=O)CH₃, - C(=O)-cyclopropyl, -C(=O)NHCH₃, -C(=O)NH-cyclopropyl, 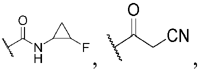 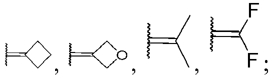
the remaining definitions are the same as those in the first, second, third, fourth, or fifth embodiment of the present invention.

A sixth embodiment of the present invention provides the above-described compound as shown in general formula (I) or the racemate, stereoisomer, tautomer, deuterated substance, solvate, prodrug, metabolite, pharmaceutically acceptable salt or eutectic crystal thereof, wherein
the compound as shown in general formula (I) is a compound as shown in (Id), 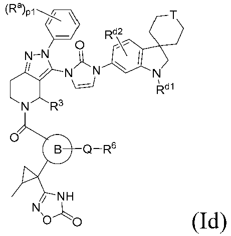
T is selected from a bond, O, S, CH₂, NH, and N (R^{d3}),
p1 is selected from 0, 1, 2, 3 or 4;
R^{d1} is selected from H, CHF₂, CD₃, methyl, cyclopropyl, oxetanyl, -CH₂-cyclopropyl, and -CH₂-oxetanyl;
R^{d2} is selected from H, deuterium, F, Cl, Br, CHF₂, CD₃, methyl, cyclopropyl, oxetanyl, -CH₂-cyclopropyl, and -CH₂-oxetanyl;
R^{d3} is selected from methyl, ethyl, isopropyl, cyclopropyl, cyclobutyl, cyclopentyl, cyclohexyl, -CH₂-cyclopropyl, -CH₂-cyclobutyl, -CH₂-oxetanyl, - CH₂-tetrahydrofuryl, -CH₂-azetidinyl, -CH₂-pyrrolidyl, -CH₂-piperidyl, -CH₂-piperazinyl, -S(=O)₁₋₂-R^{5c}, -C(=O)R^{5c}, -C(=O)NR^{5d}R^{5e}, -NR^{5d}C(=O)R^{5e}, -C(=O)-M-C(=O)-R^{5c}, -CH₂-M-C(=O)-R^{5c}, -C(=O)-M-CH₂-R^{5c}, -C(=O)-M-R^{5c}, -C(=O)-M-CH₂-O-R^{5c}, 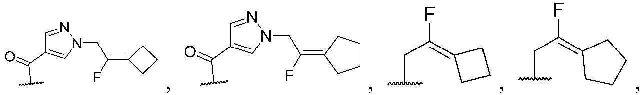 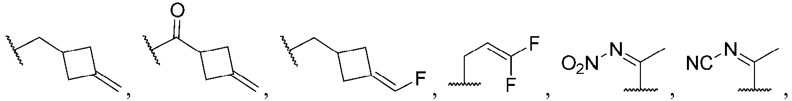 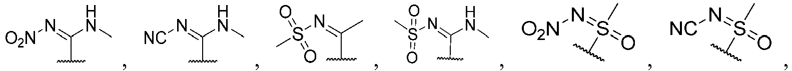 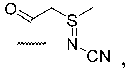 wherein the methyl, ethyl, isopropyl, cyclopropyl, cyclobutyl, cyclopentyl, cyclohexyl, -CH₂-cyclopropyl, -CH₂-cyclobutyl, -CH₂-oxetanyl, - CH₂-tetrahydrofuryl, -CH₂-azetidinyl, -CH₂-pyrrolidyl, -CH₂-piperidyl, or -CH₂-piperazinyl is optionally substituted with 1 to 4 R^{k} (each R^{k} is preferably independently selected from deuterium, F, Cl, Br, CN, OH, CHF₂, CD₃, methyl, ethyl, propyl, isopropyl, cyclopropyl, oxetanyl, -CH₂-cyclopropyl, -CH₂-oxetanyl, methoxy, methoxymethyl, methoxyethyl, CH₂CN, ethenyl, 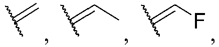 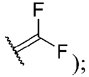
M is selected from cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, 3-oxabicyclo[3.1.0]hexyl, and the M is optionally substituted with 1 to 4 R^{k} (each R^{k} is preferably independently selected from deuterium, F, Cl, Br, CHF₂, CD₃, methyl, cyclopropyl, oxetanyl, - CH₂-cyclopropyl, and -CH₂-oxetanyl);
R^{5c} is selected from methyl, ethyl, propyl, isopropyl, methoxy, ethoxy, ethenyl, propenyl, allyl, cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, bicyclo[2.2.1]heptyl, cyclopropyl-spiro-cyclobutyl, cyclobutyl-spiro-cyclobutyl, cyclobutyl-spiro-cyclopentyl, cyclobutyl-spiro-cyclohexyl, cyclopentyl-fused-cyclopentyl, 3-oxabicyclo[3.1.0]hexyl, thienyl, furyl, thiazolyl, oxazolyl, methoxymethyl, and ethoxymethyl, and the R^{5c} is optionally substituted with 1 to 4 R^{k} (each R^{k} is preferably independently selected from deuterium, F, Cl, Br, CN, OH, CHF₂, CD₃, methyl, ethyl, propyl, isopropyl, cyclopropyl, oxetanyl, -CH₂-cyclopropyl, -CH₂-oxetanyl, methoxy, methoxymethyl, methoxyethyl, CH₂CN, ethenyl, propenyl, 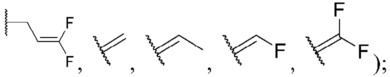
R^{5a} and R^{5e} are each independently selected from H, methyl, ethyl, propyl, isopropyl, cyclopropyl, cyclobutyl, cyclopentyl or cyclohexyl, wherein the methyl, ethyl, propyl, isopropyl, cyclopropyl, cyclobutyl, cyclopentyl or cyclohexyl is optionally substituted with 1 to 4 substituents selected from deuterium, F, Cl, Br, CN, OH, CHF₂, CD₃, methyl, and ethyl;
each R^{b} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, methoxy, ethoxy, isopropoxy, and cyclopropyl;
each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, propynyl, methoxy, ethoxy, isopropoxy, cyclopropyl, -CH₂OH, -CH₂CH₂OH, - CH₂CN, -CH₂N(CH₃)₂, -CH₂-cyclopropyl, 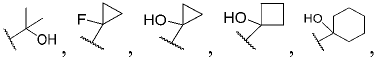 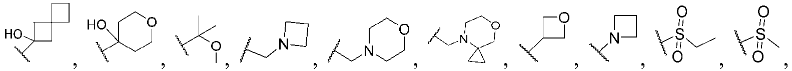 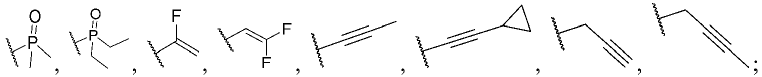
preferably, 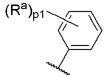 is selected from

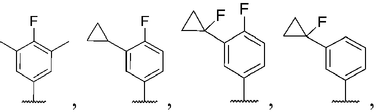

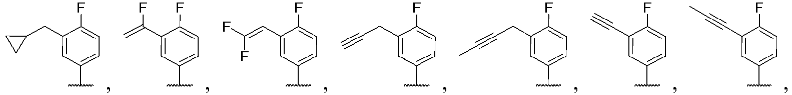

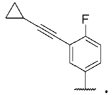

The present invention relates to a compound as described below, or a racemate, stereoisomer, tautomer, deuterated substance, solvate, prodrug, metabolite, pharmaceutically acceptable salt or eutectic crystal thereof, wherein the compound has a structure selected from one of those in Table E-1 below:

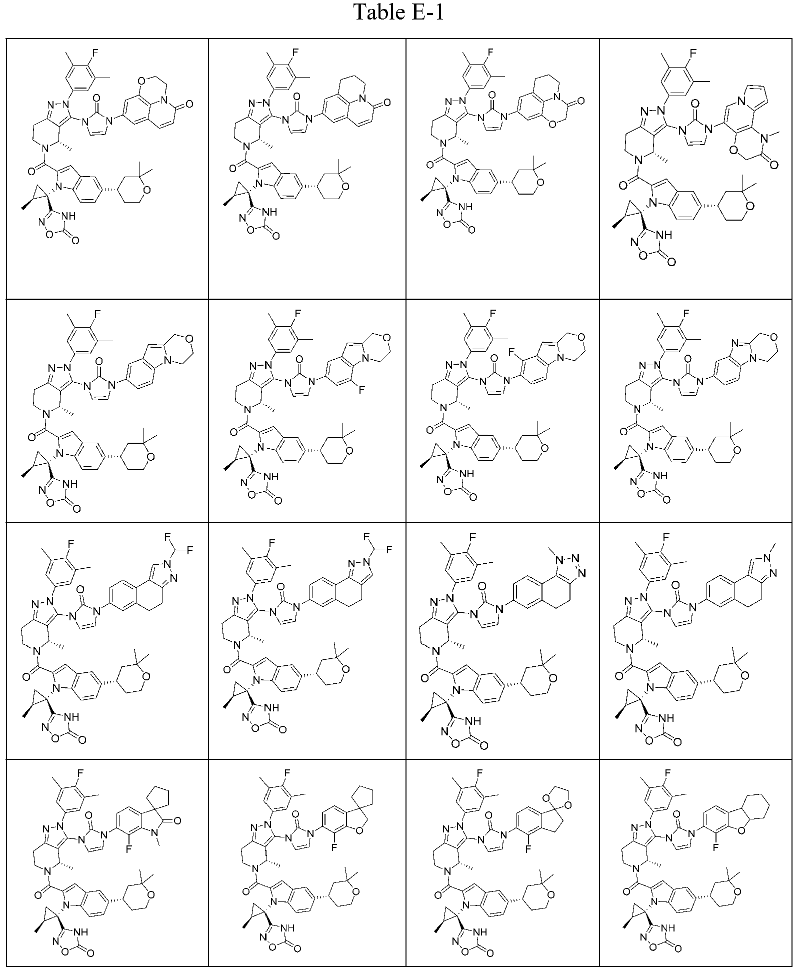

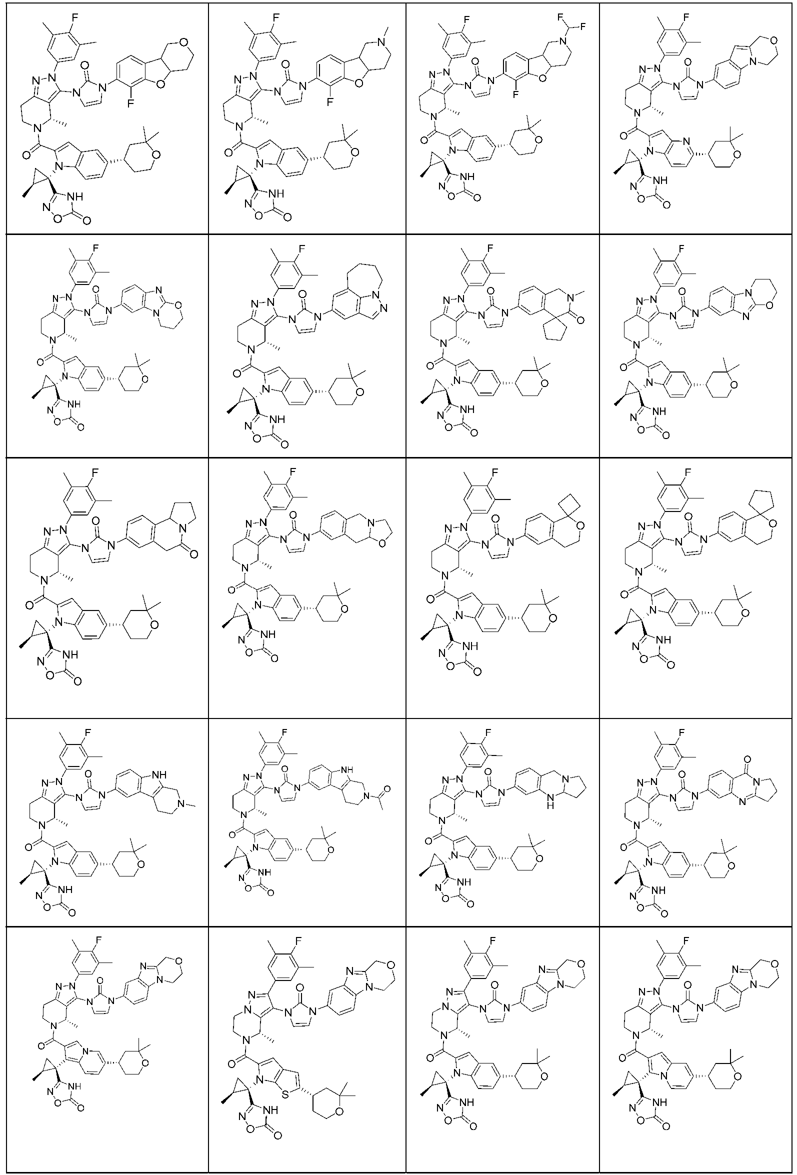

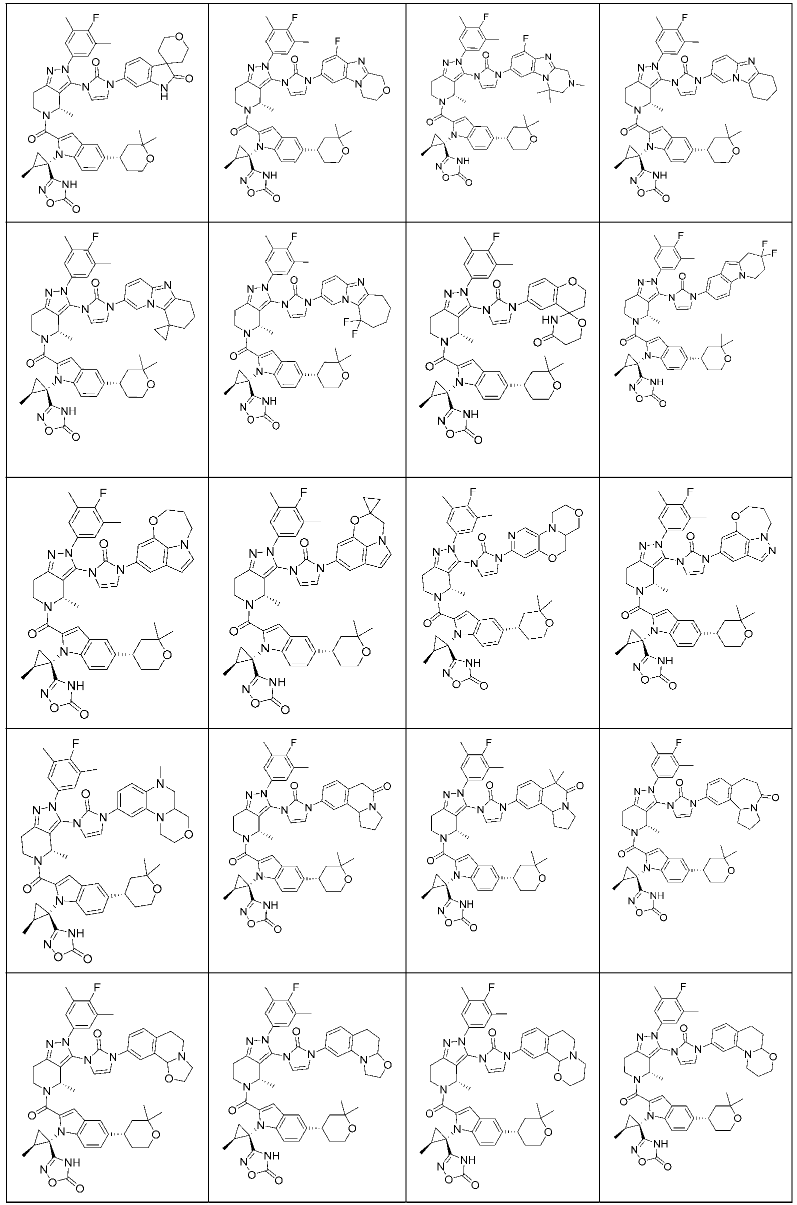

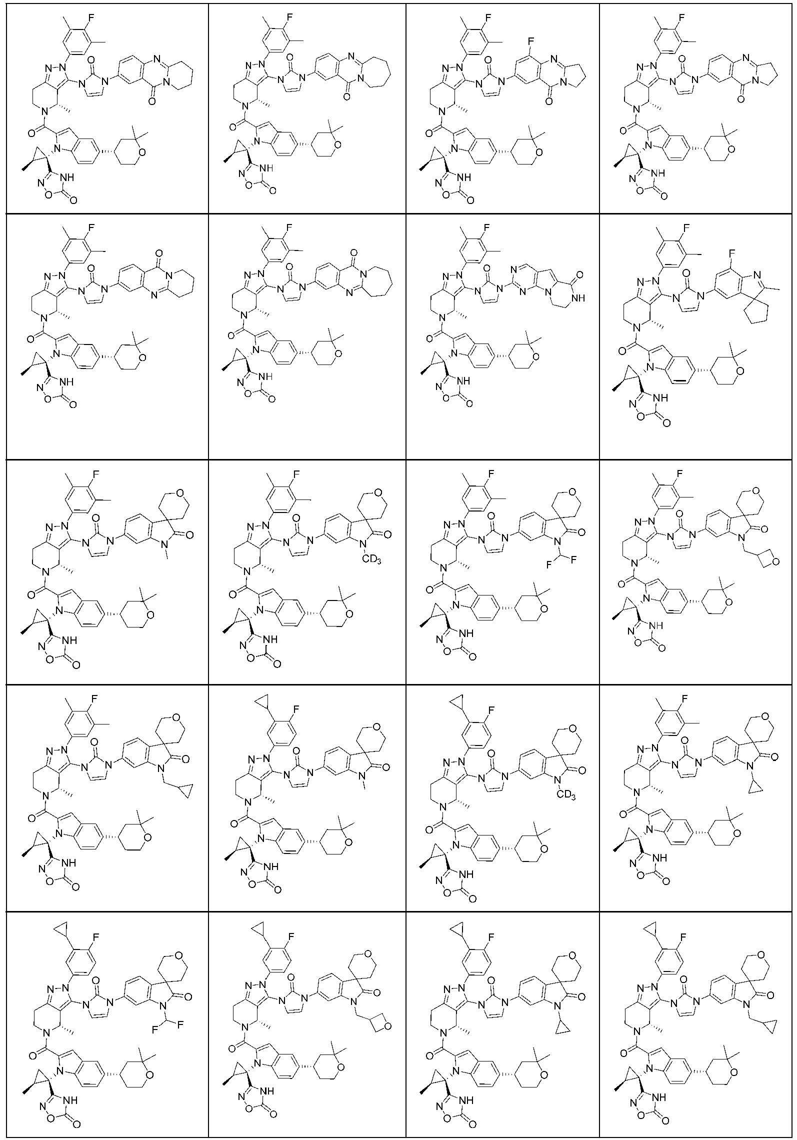

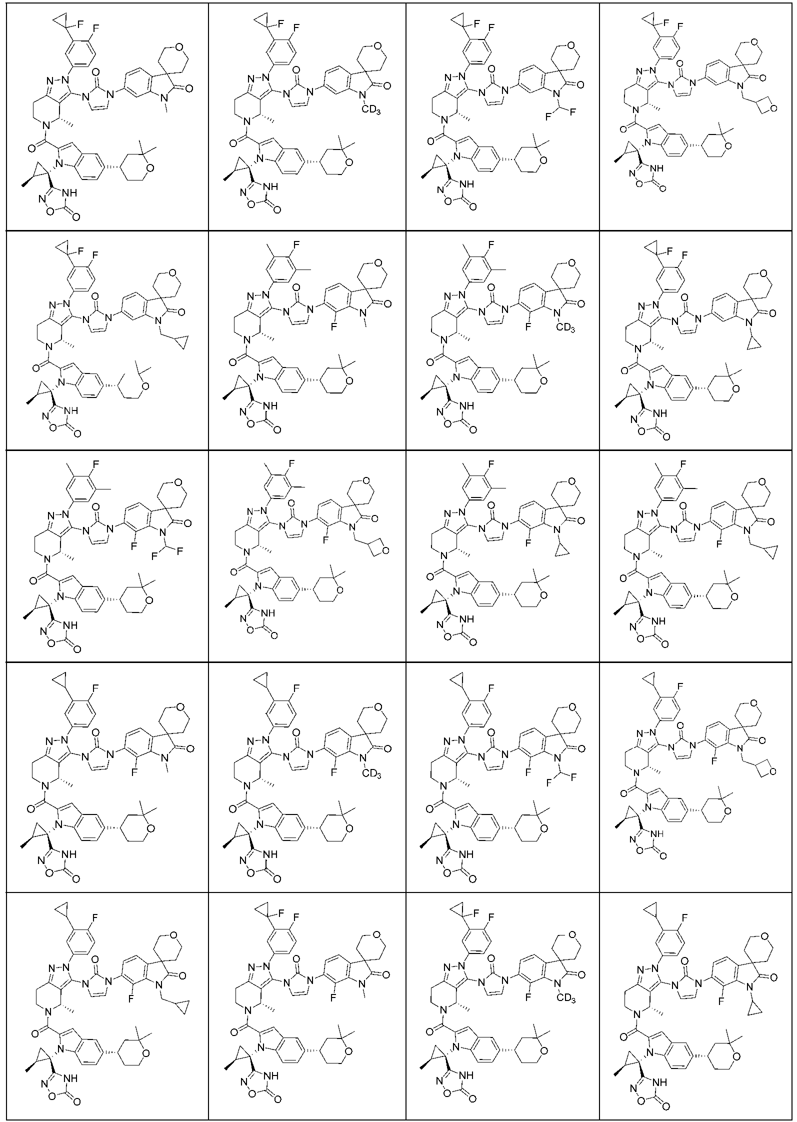

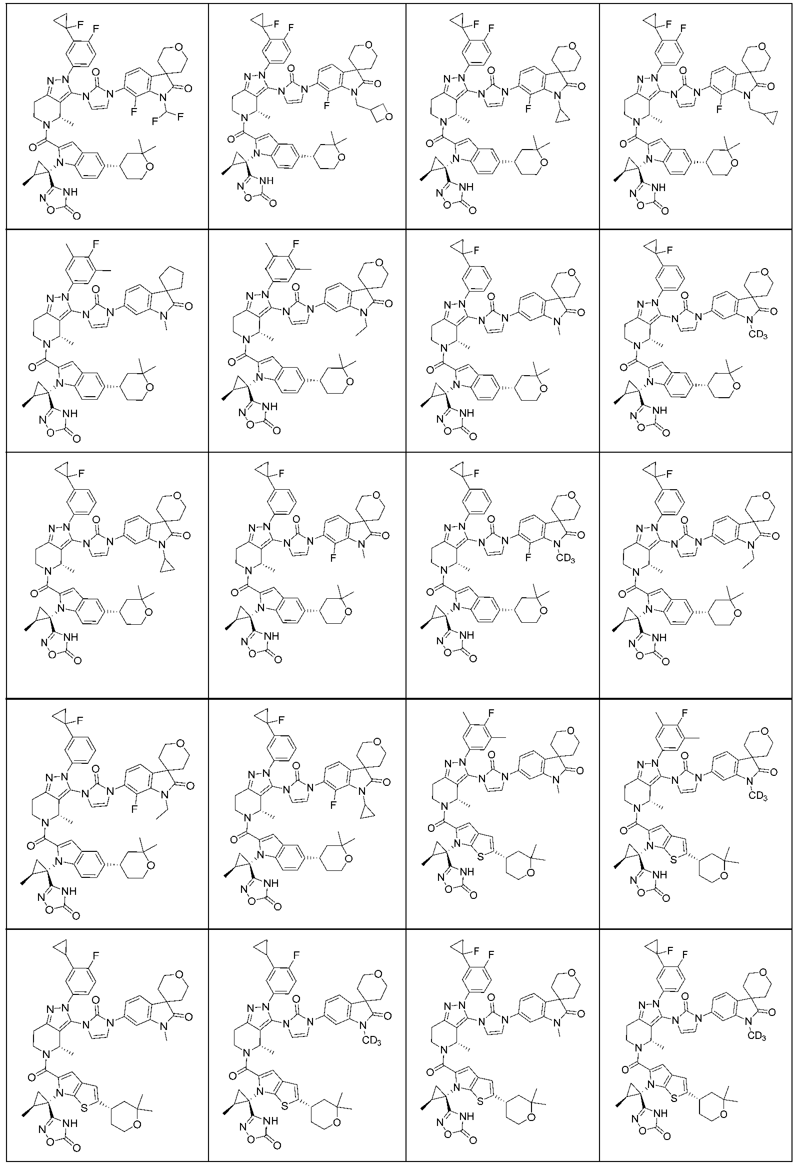

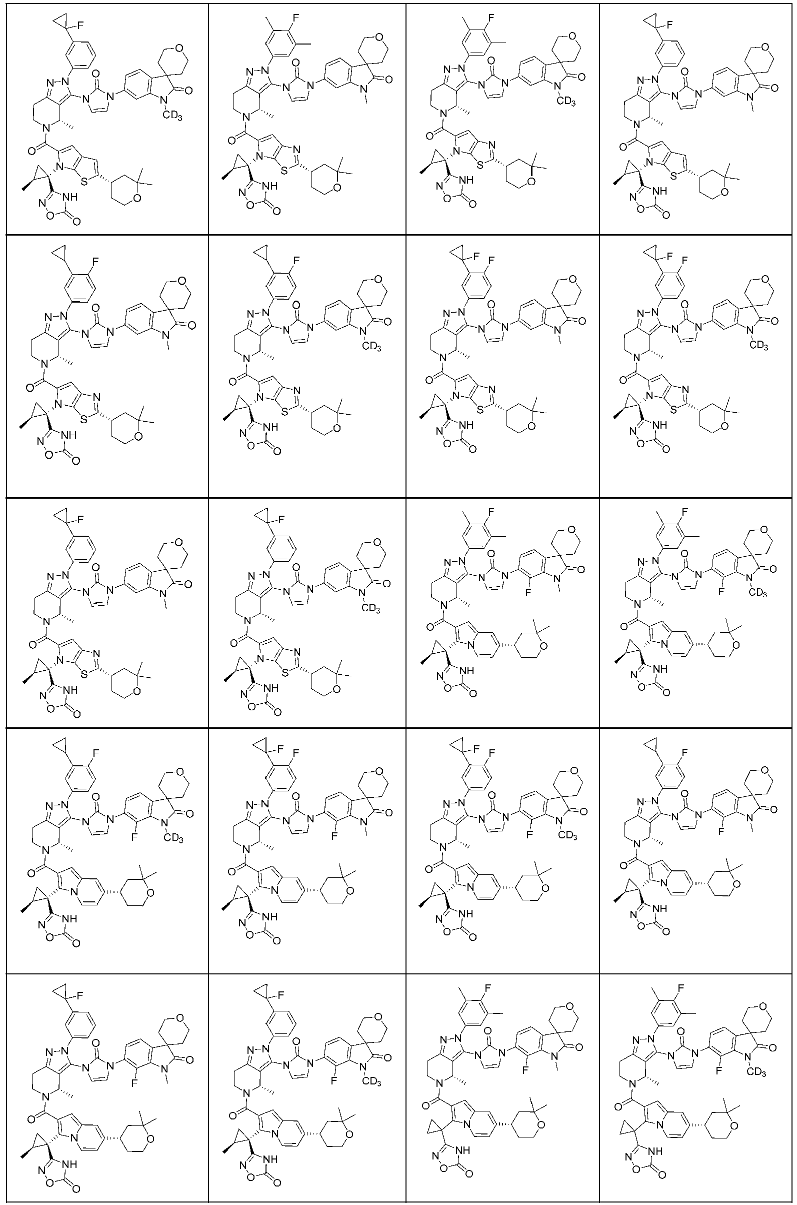

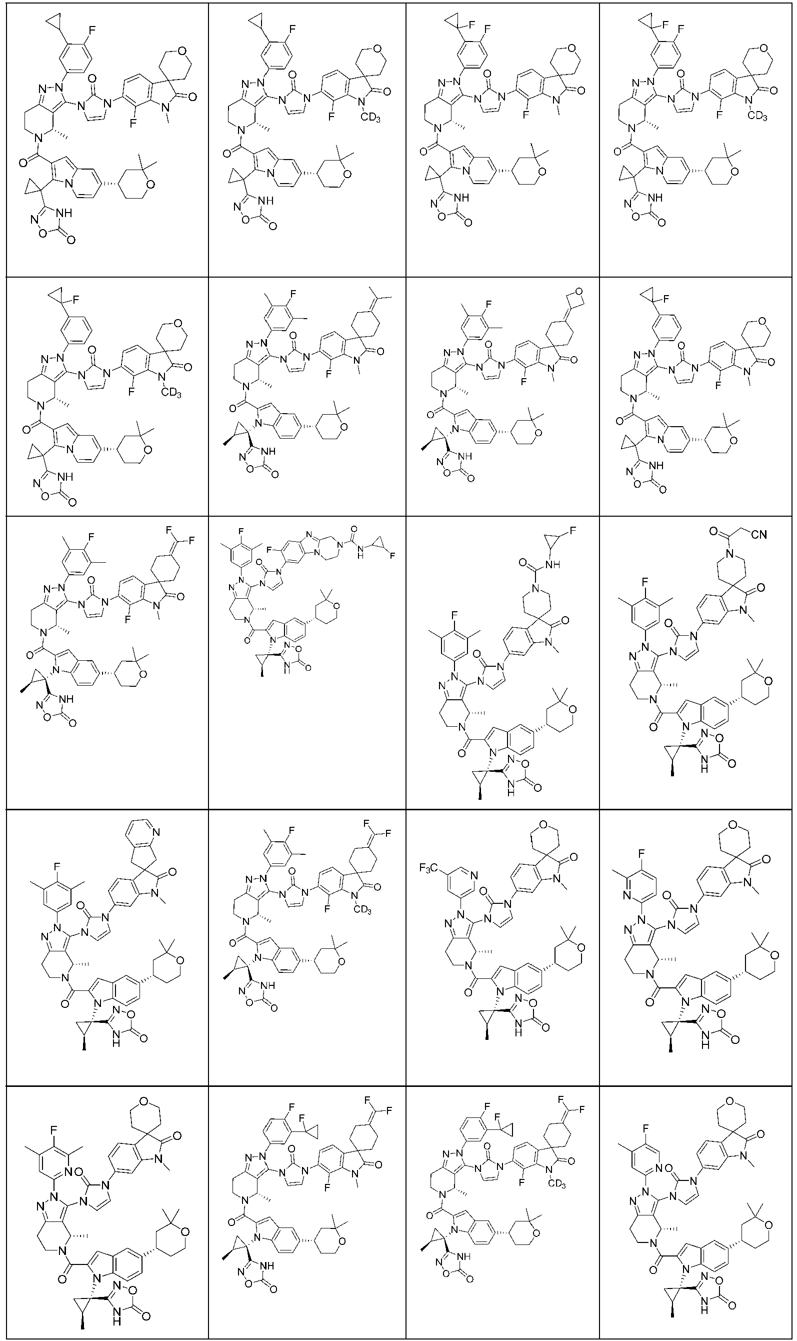

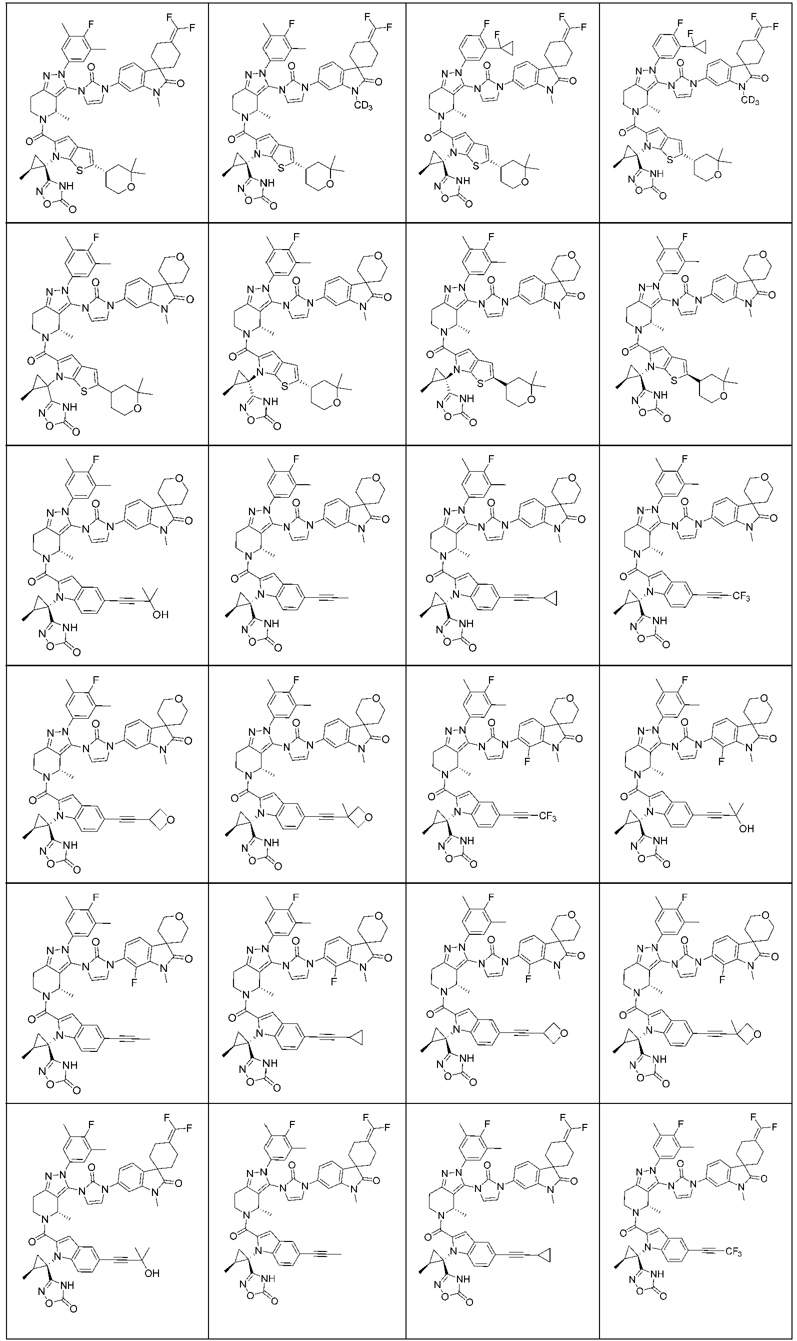

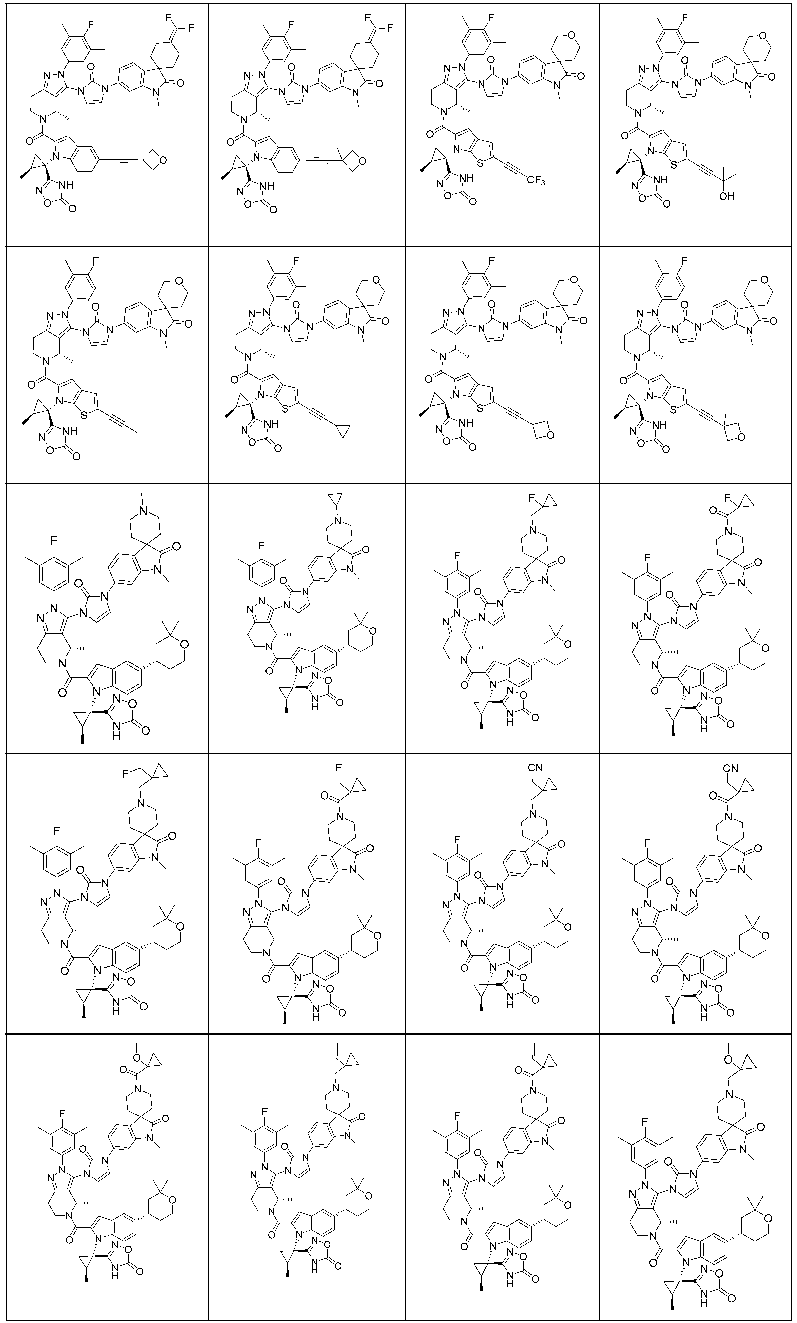

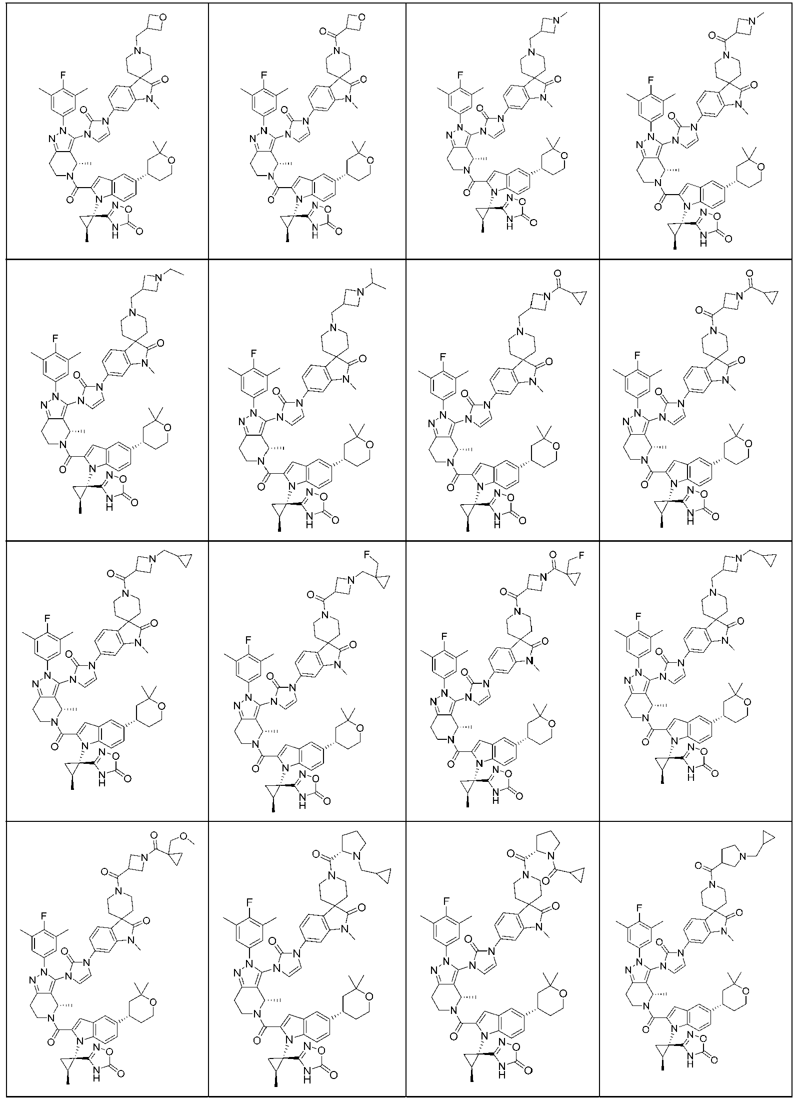

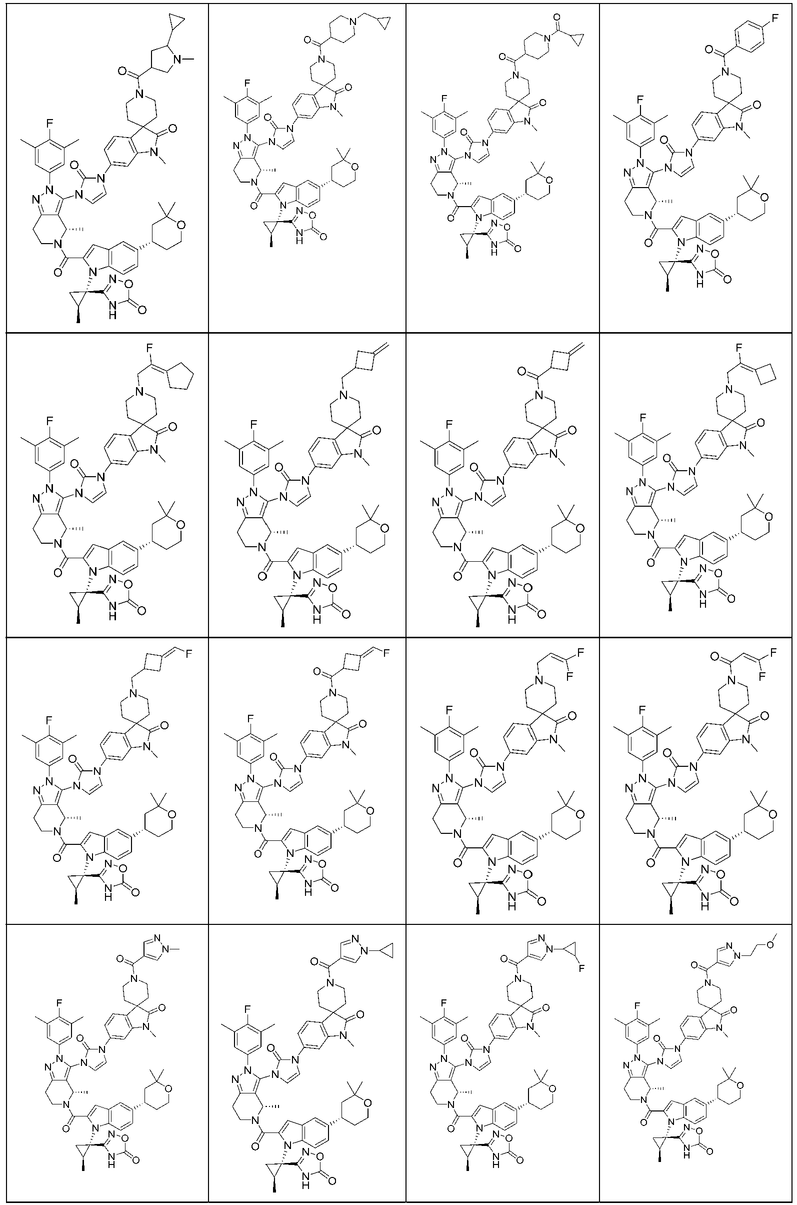

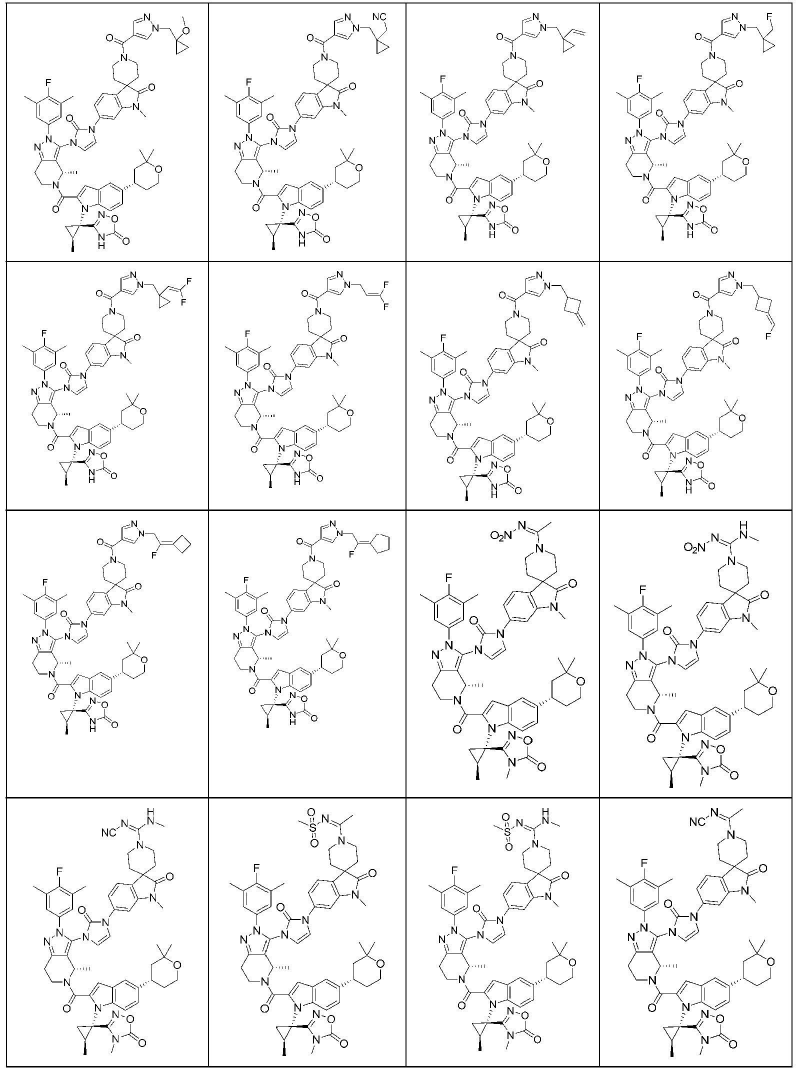

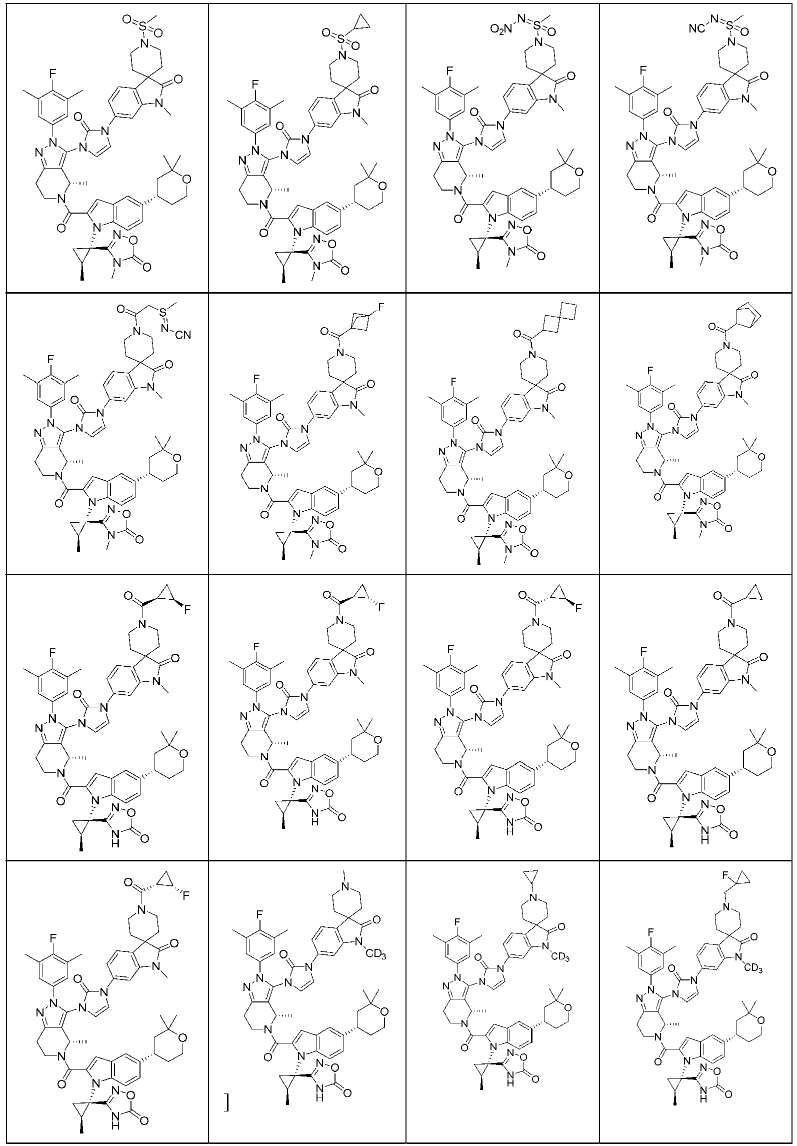

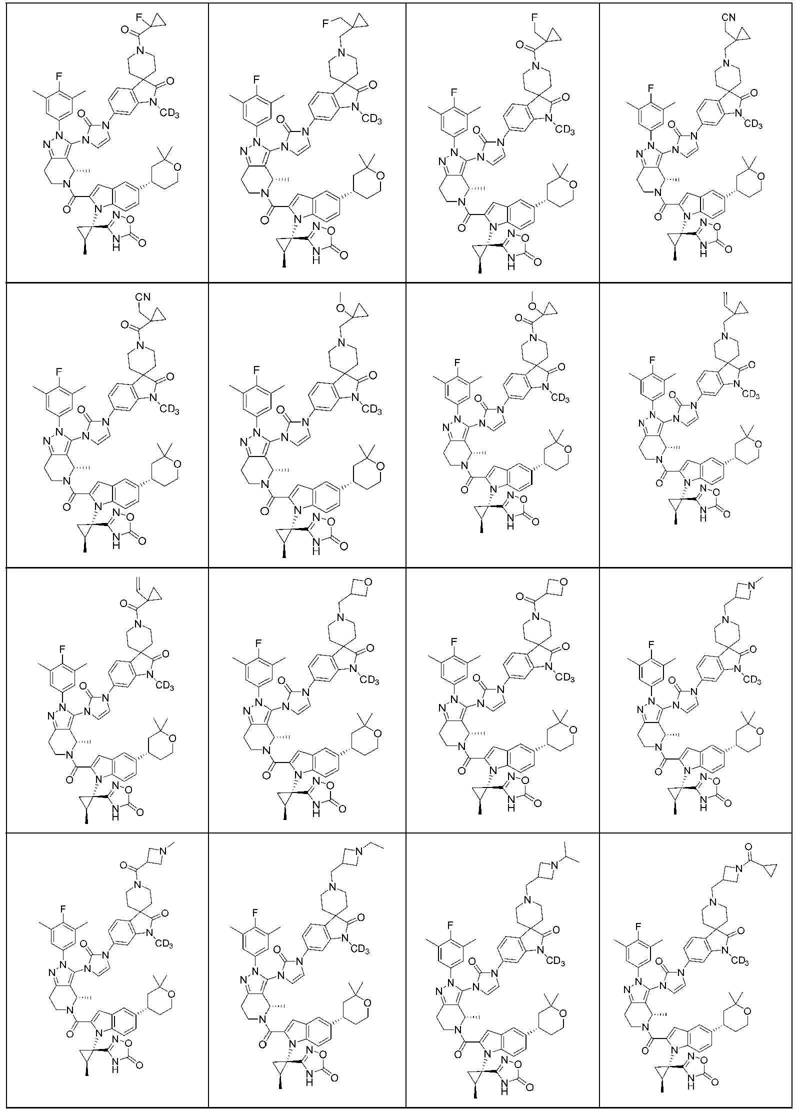

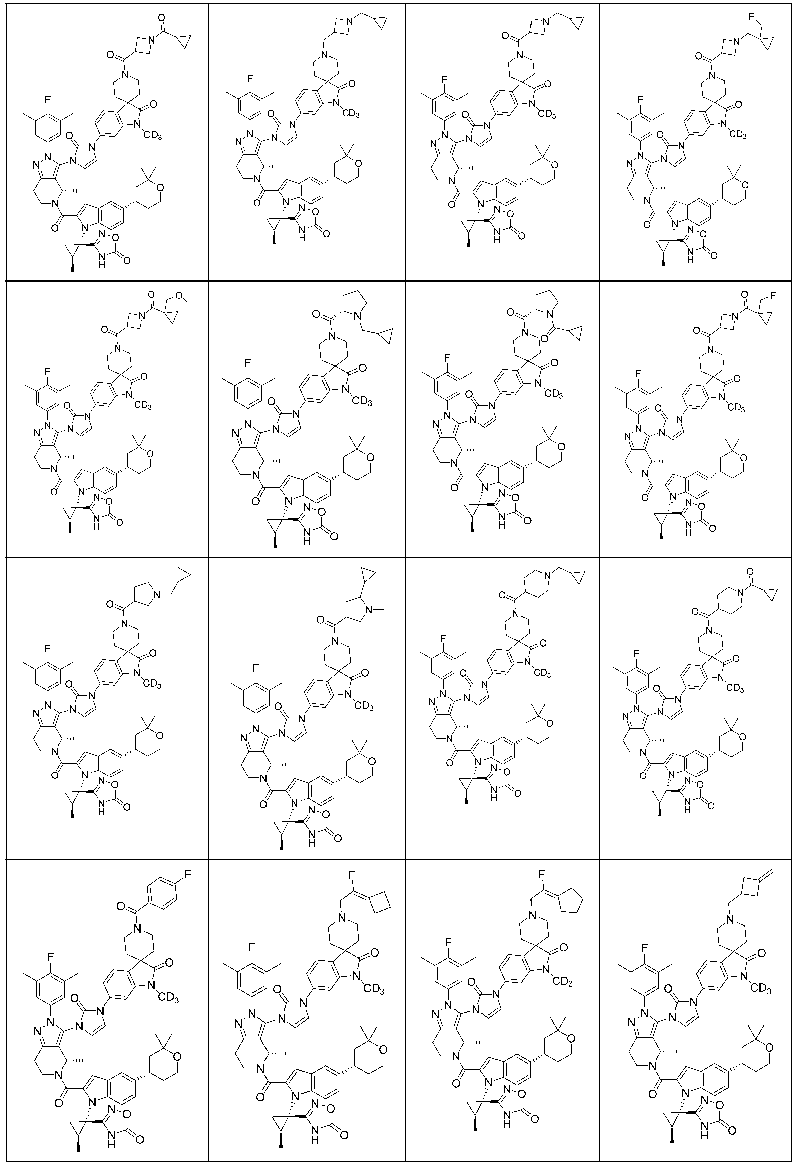

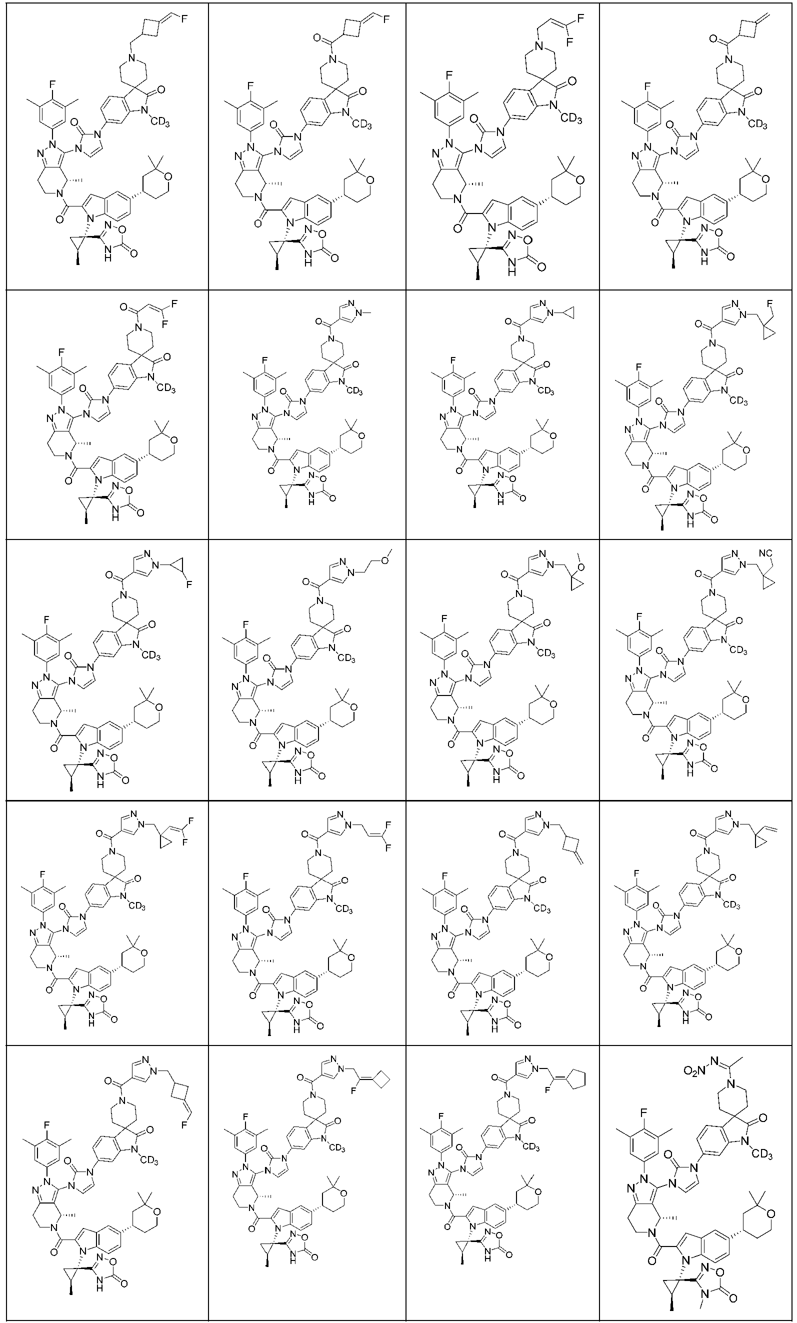

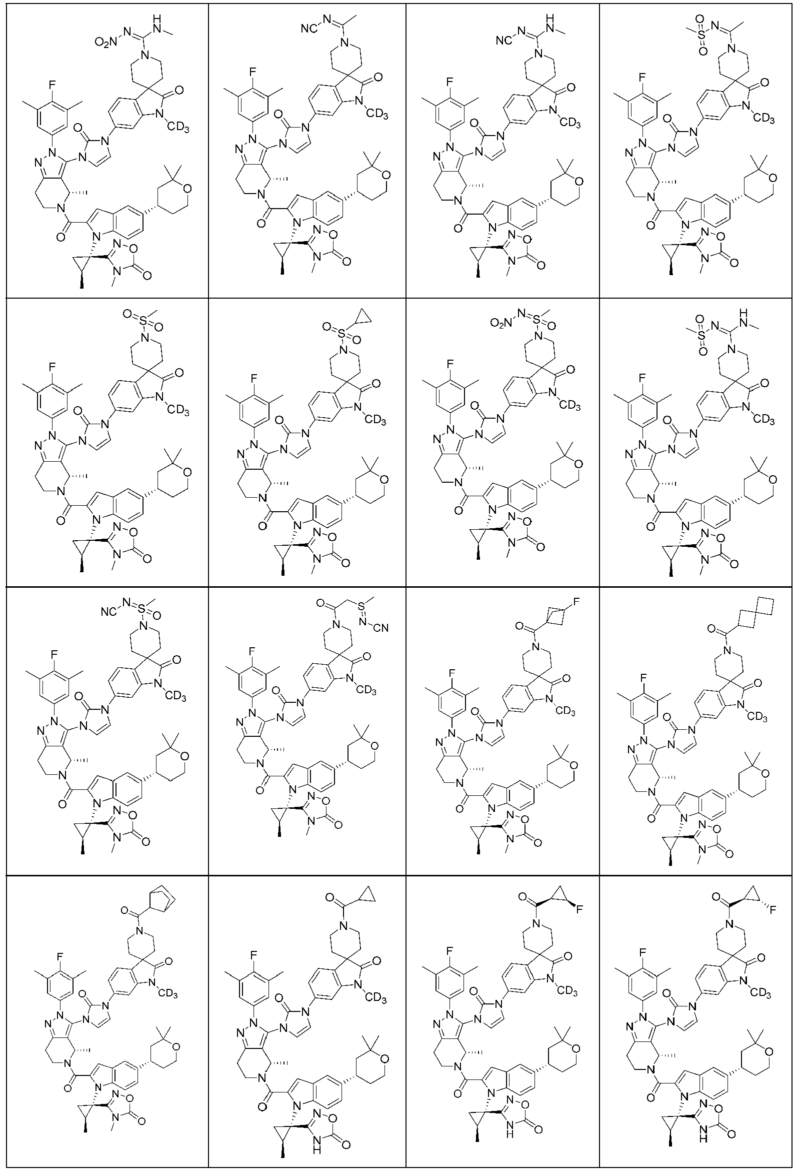

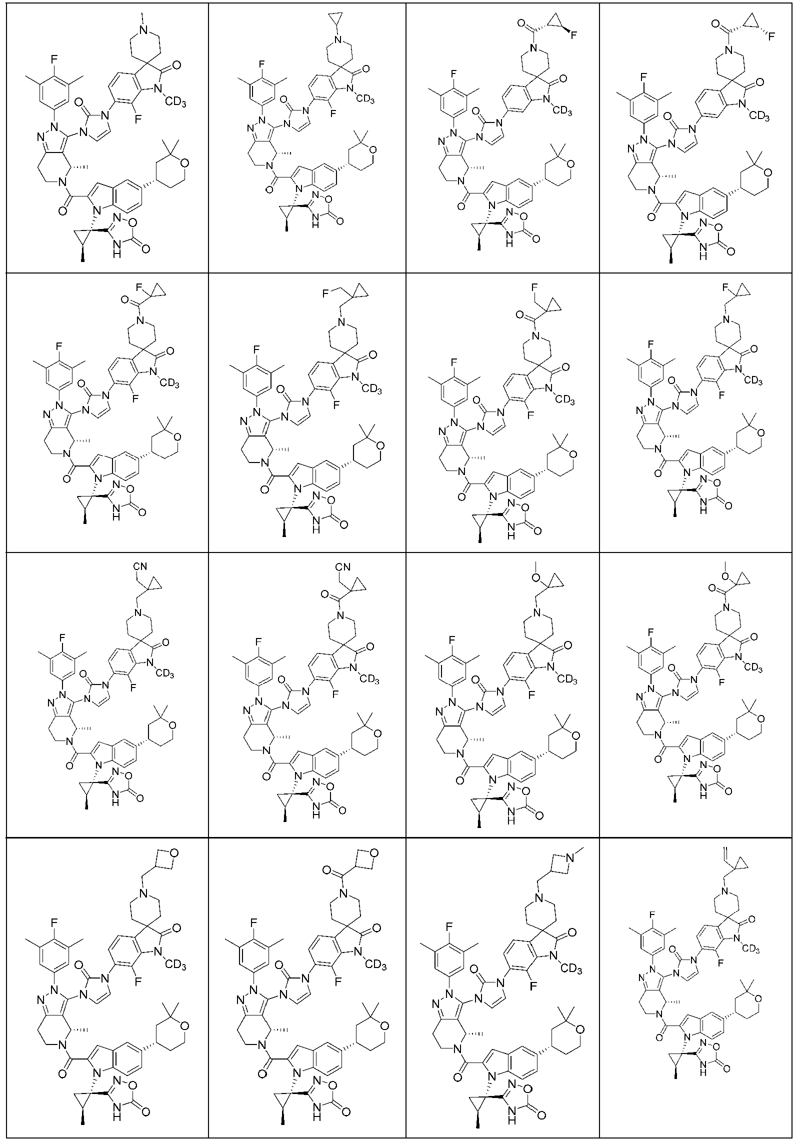

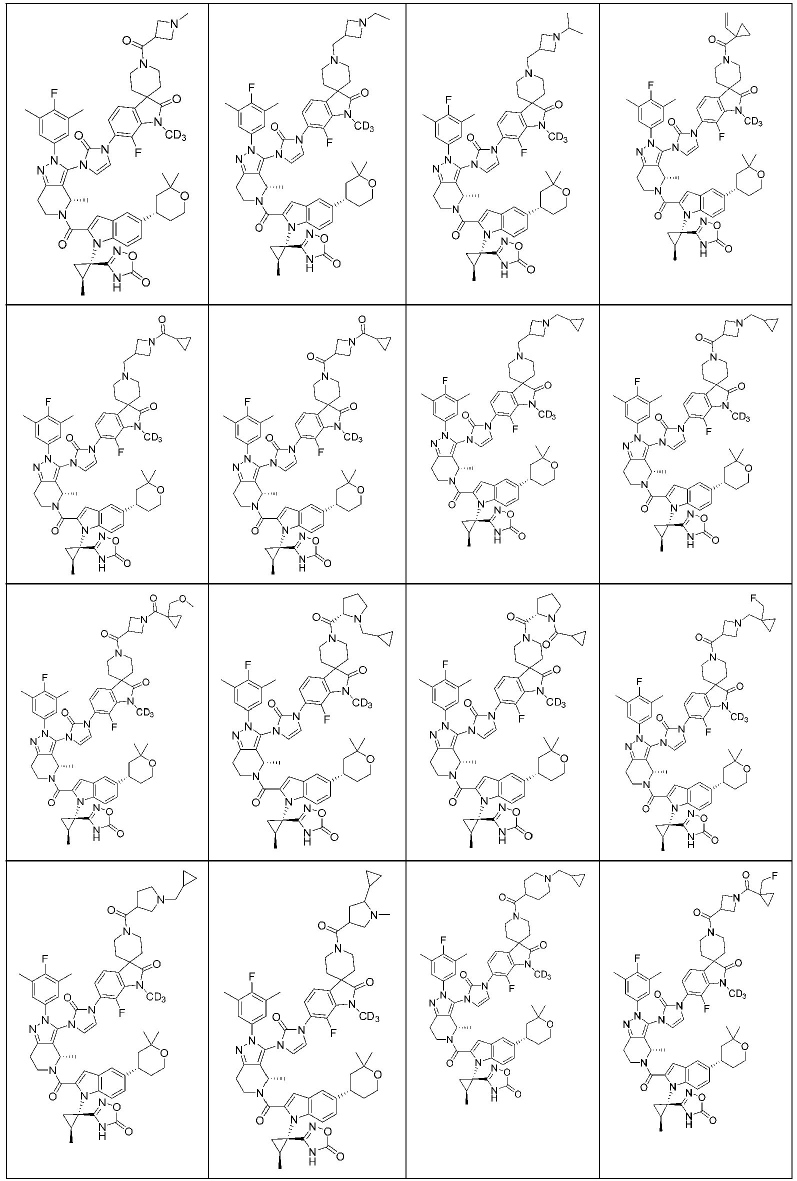

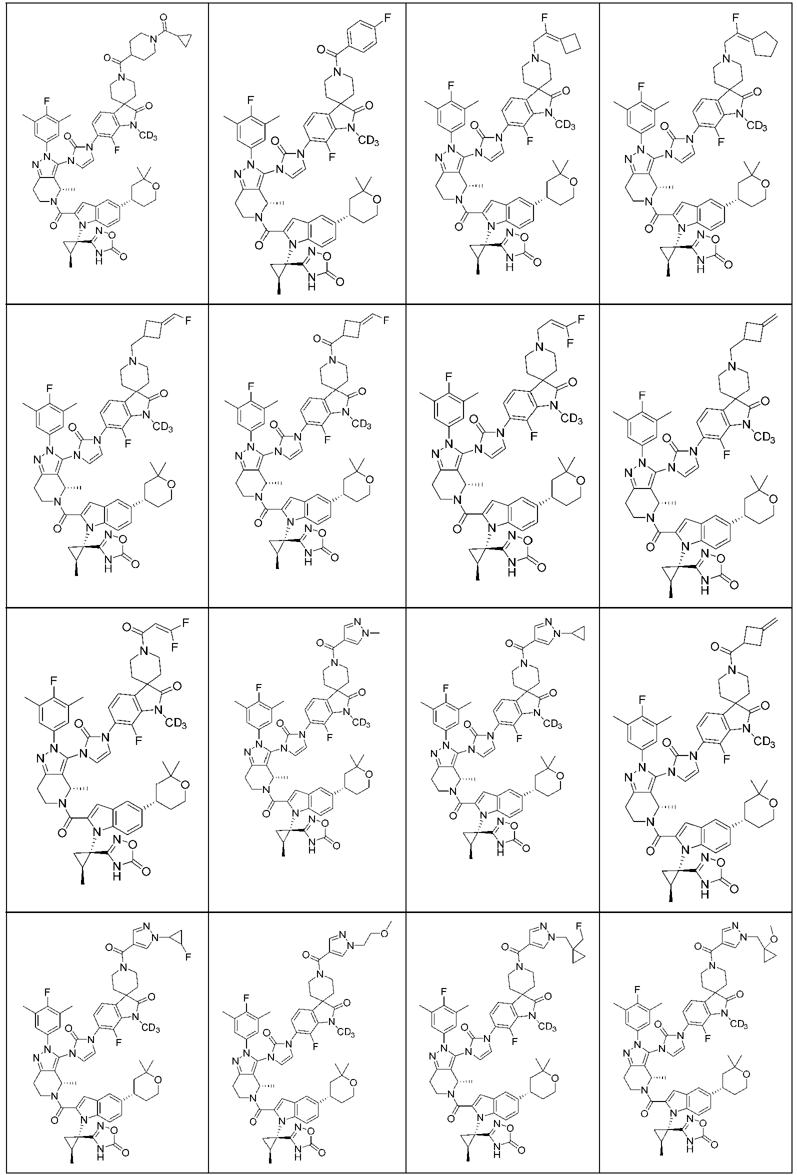

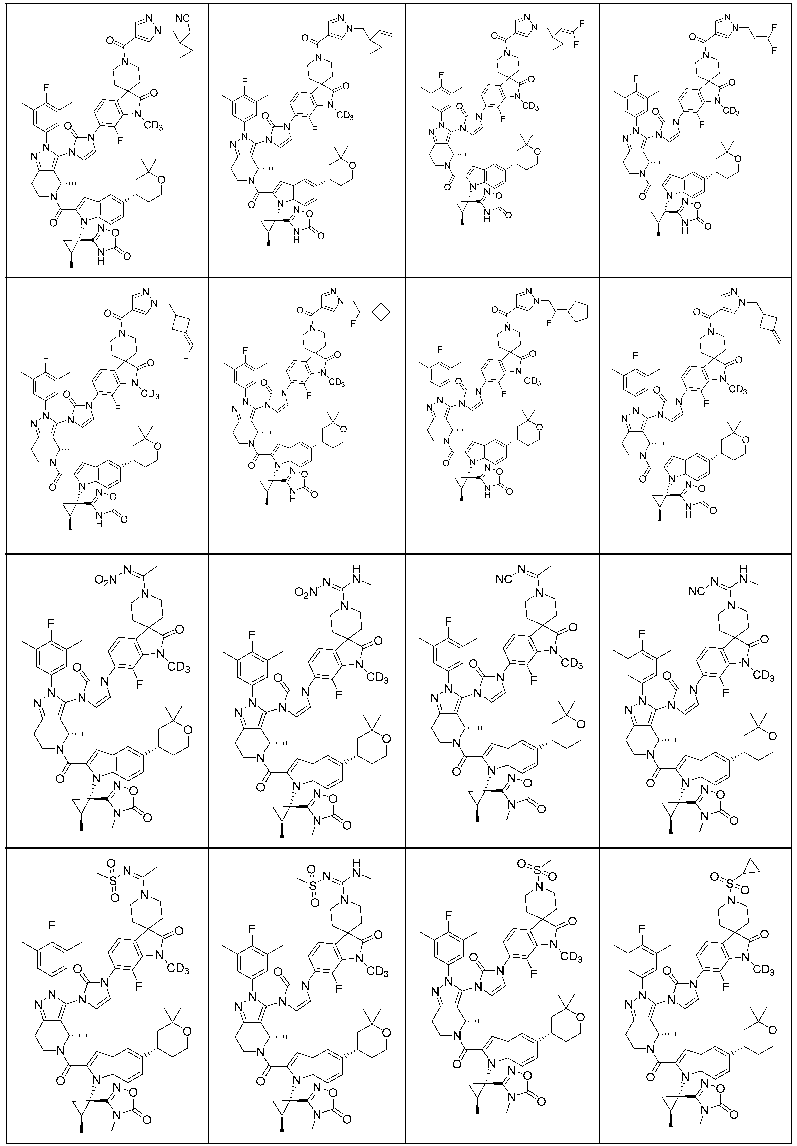

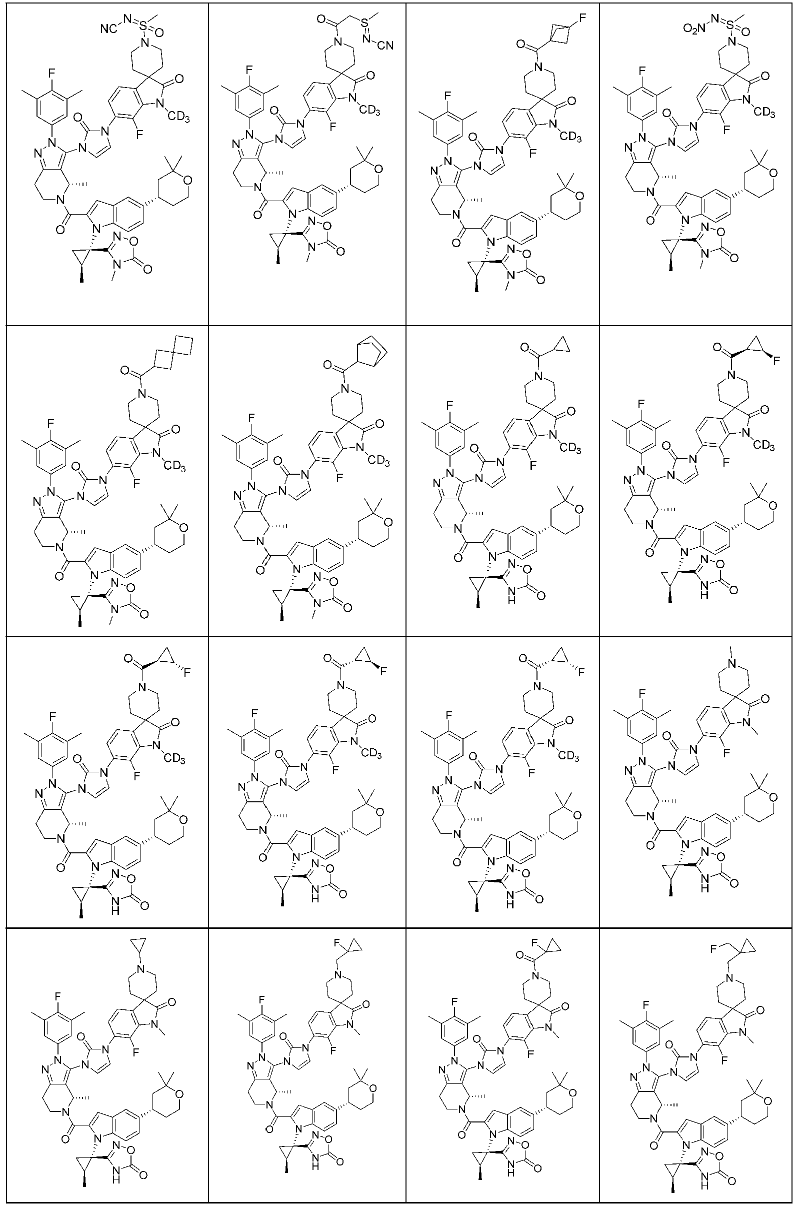

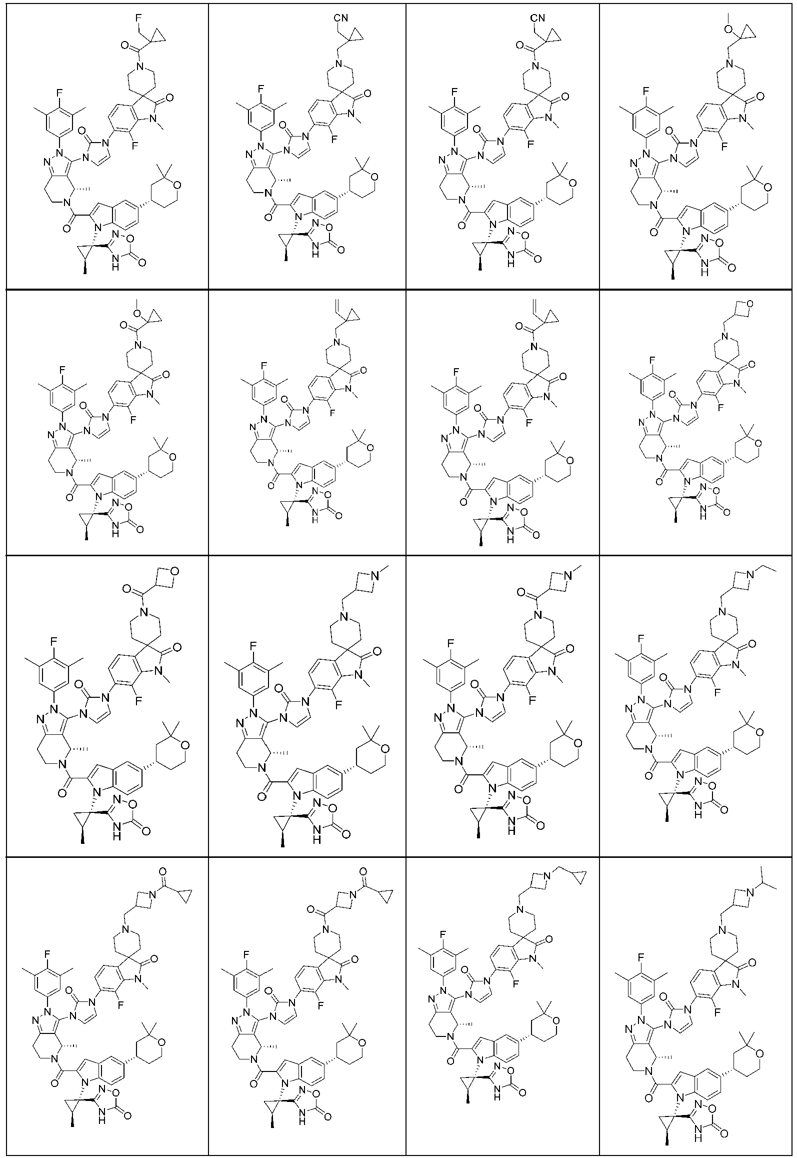

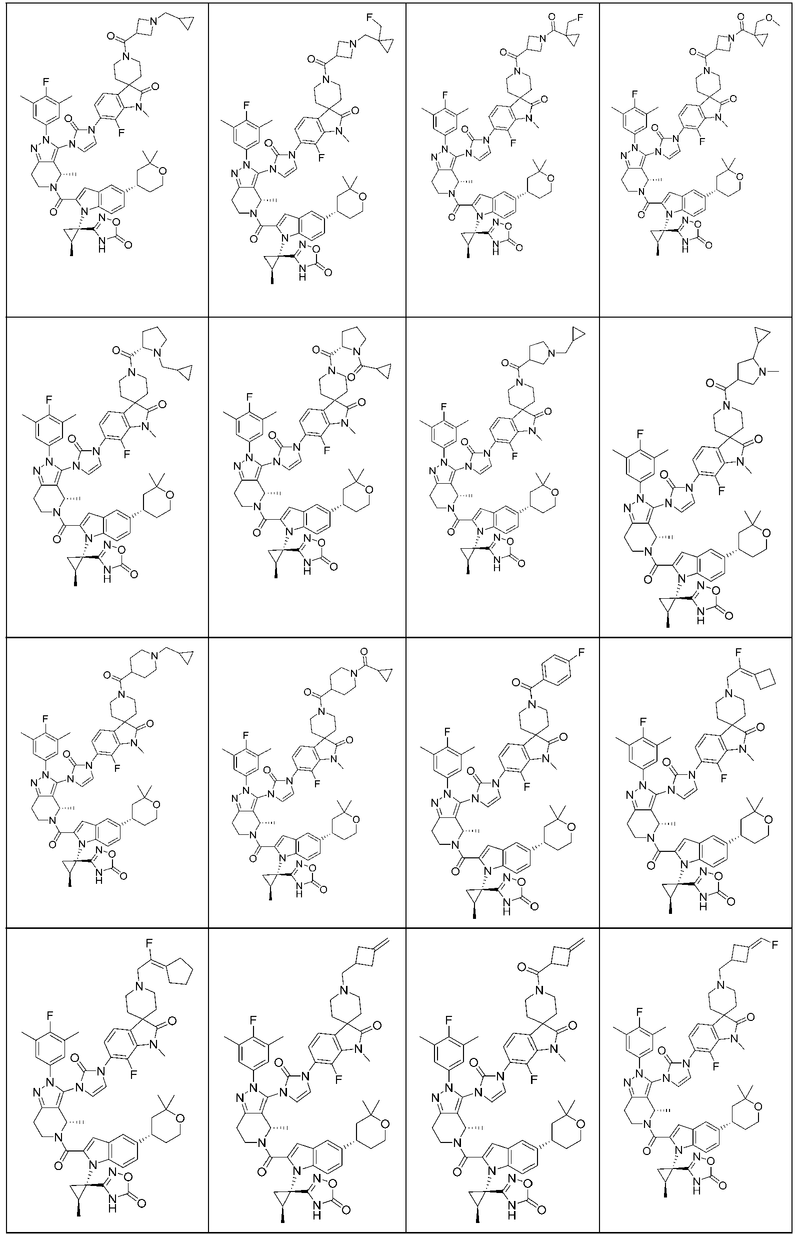

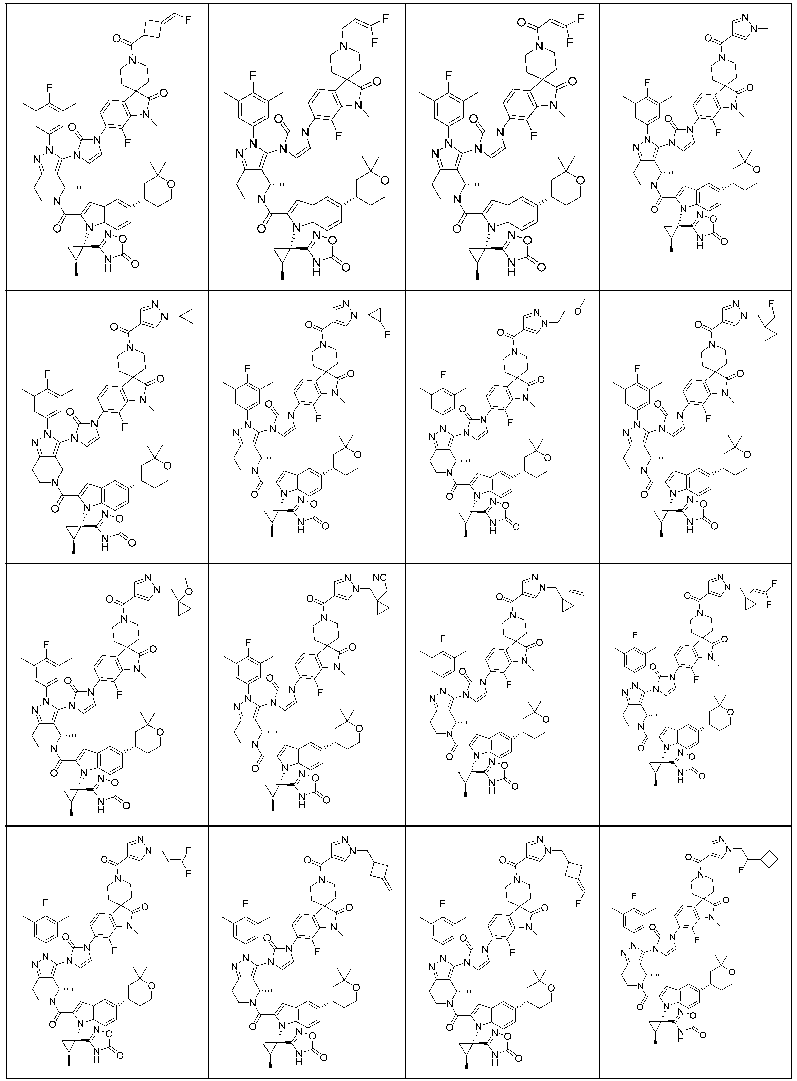

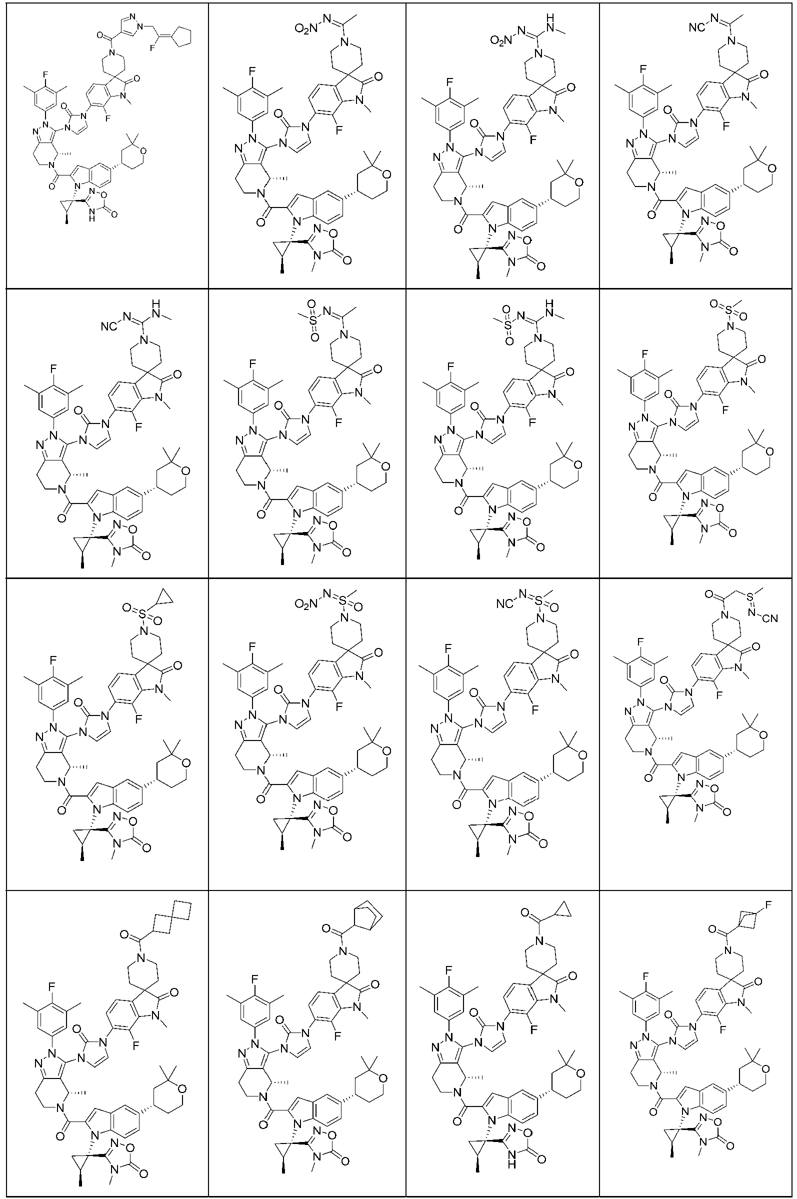

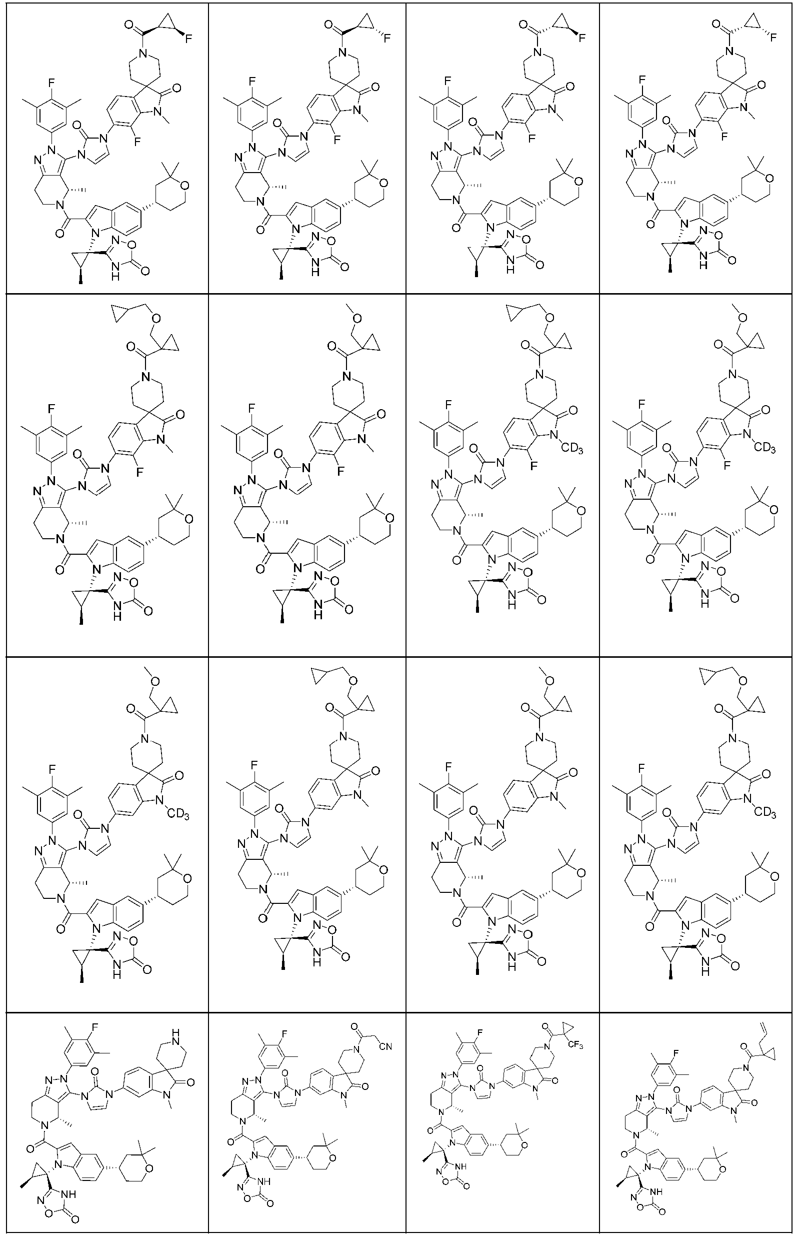

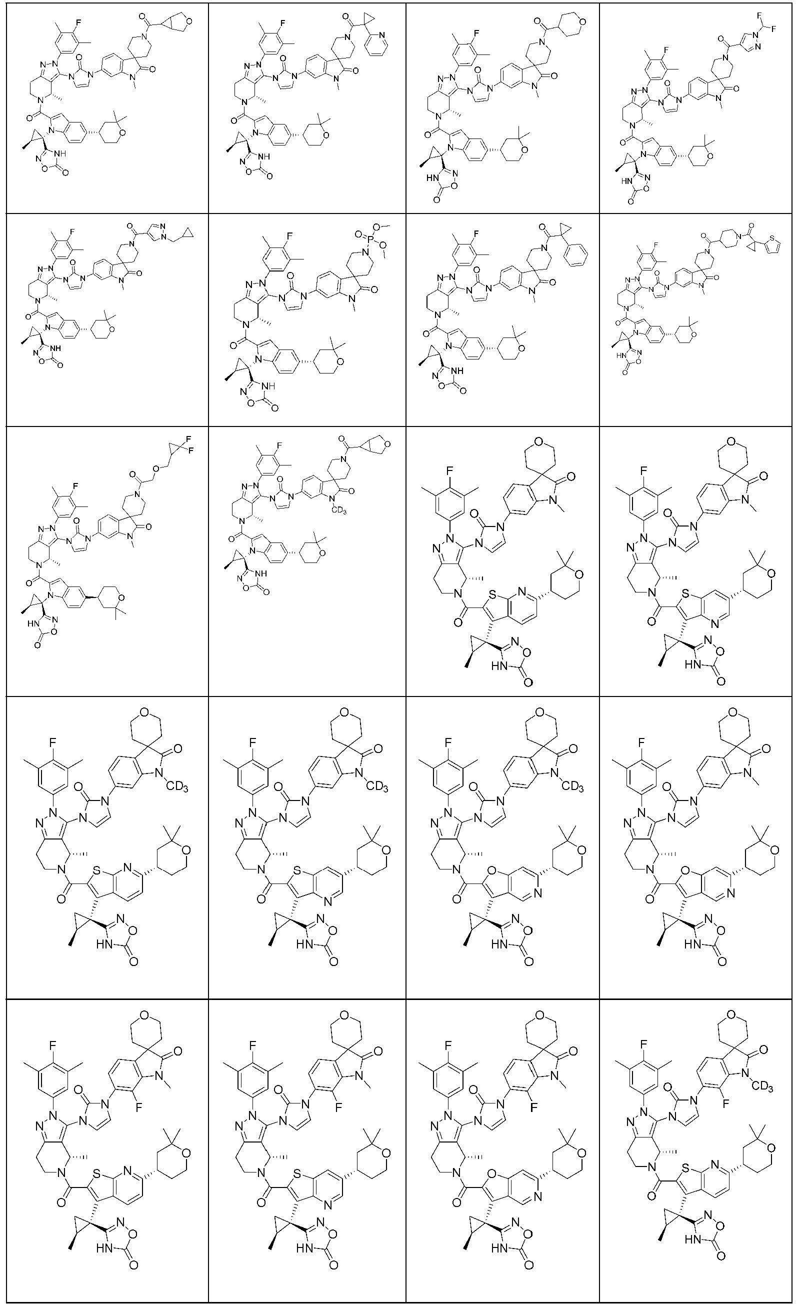

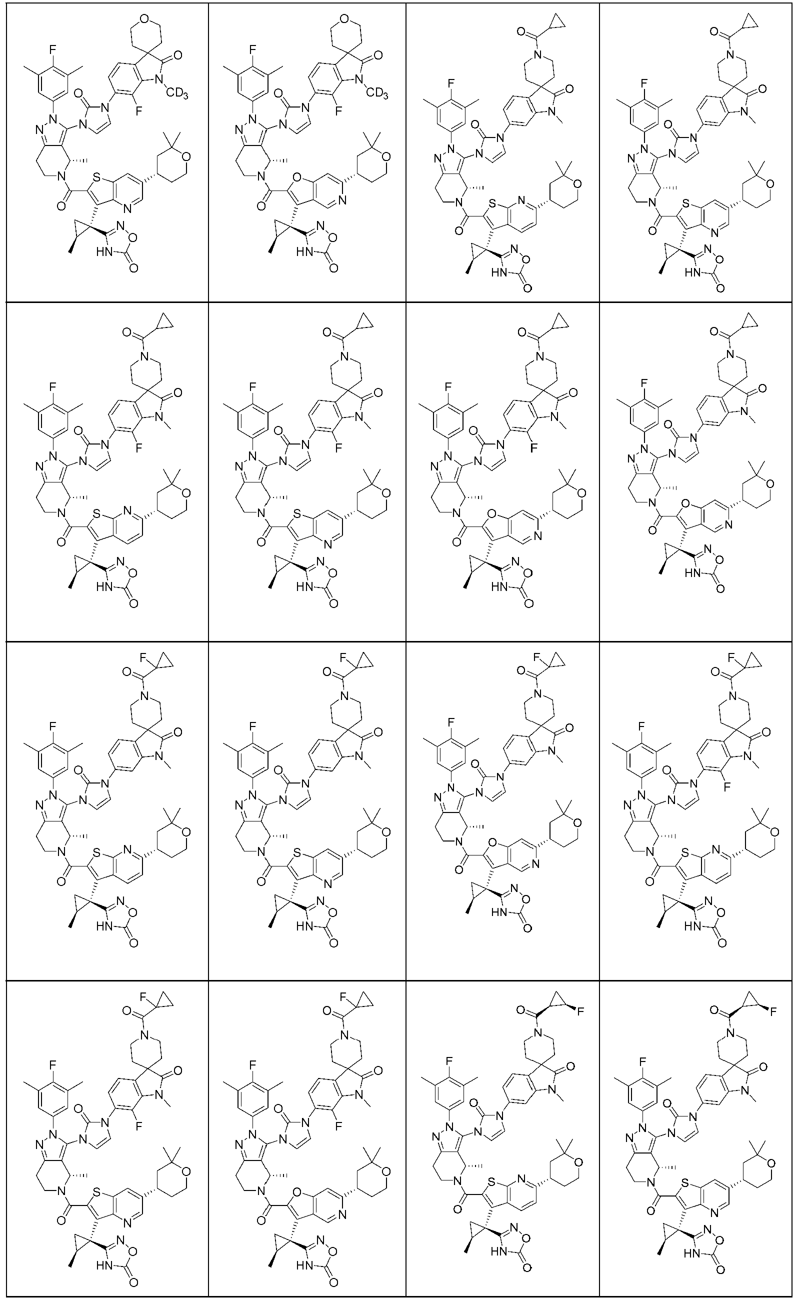

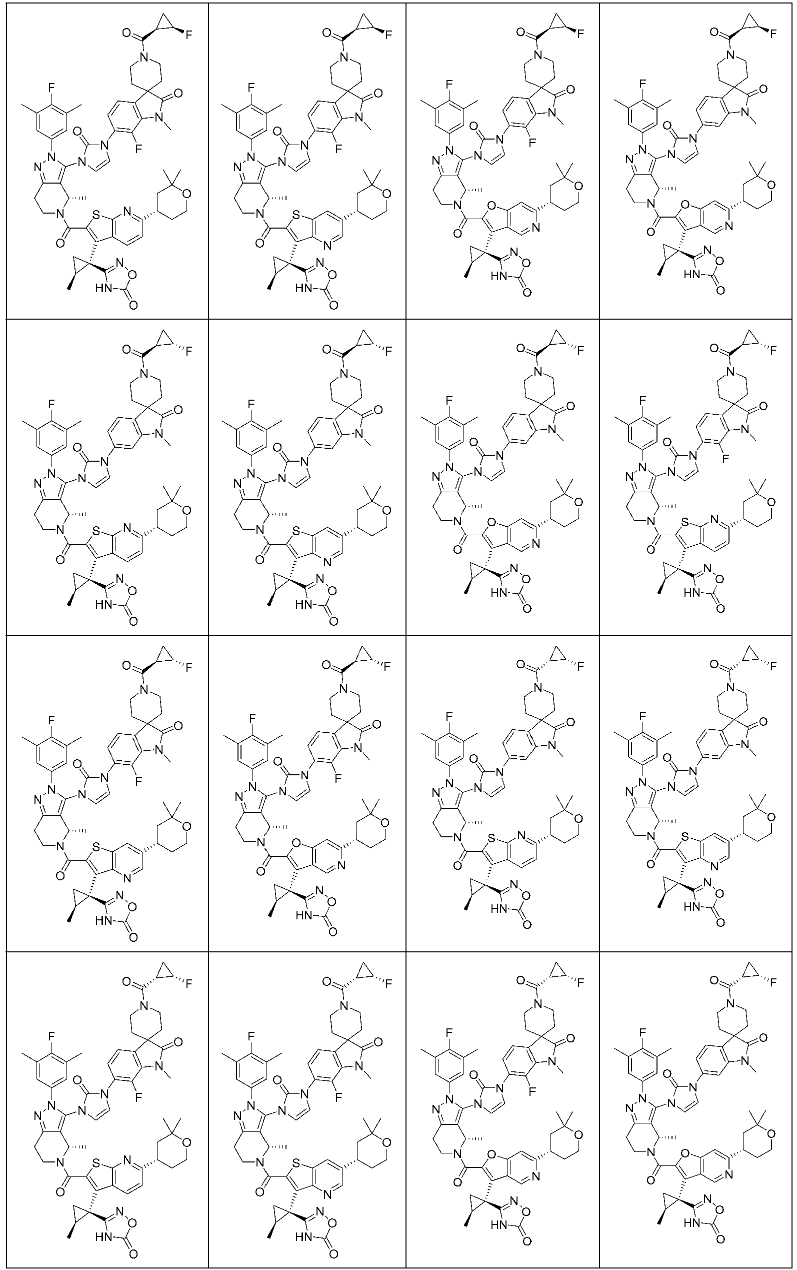

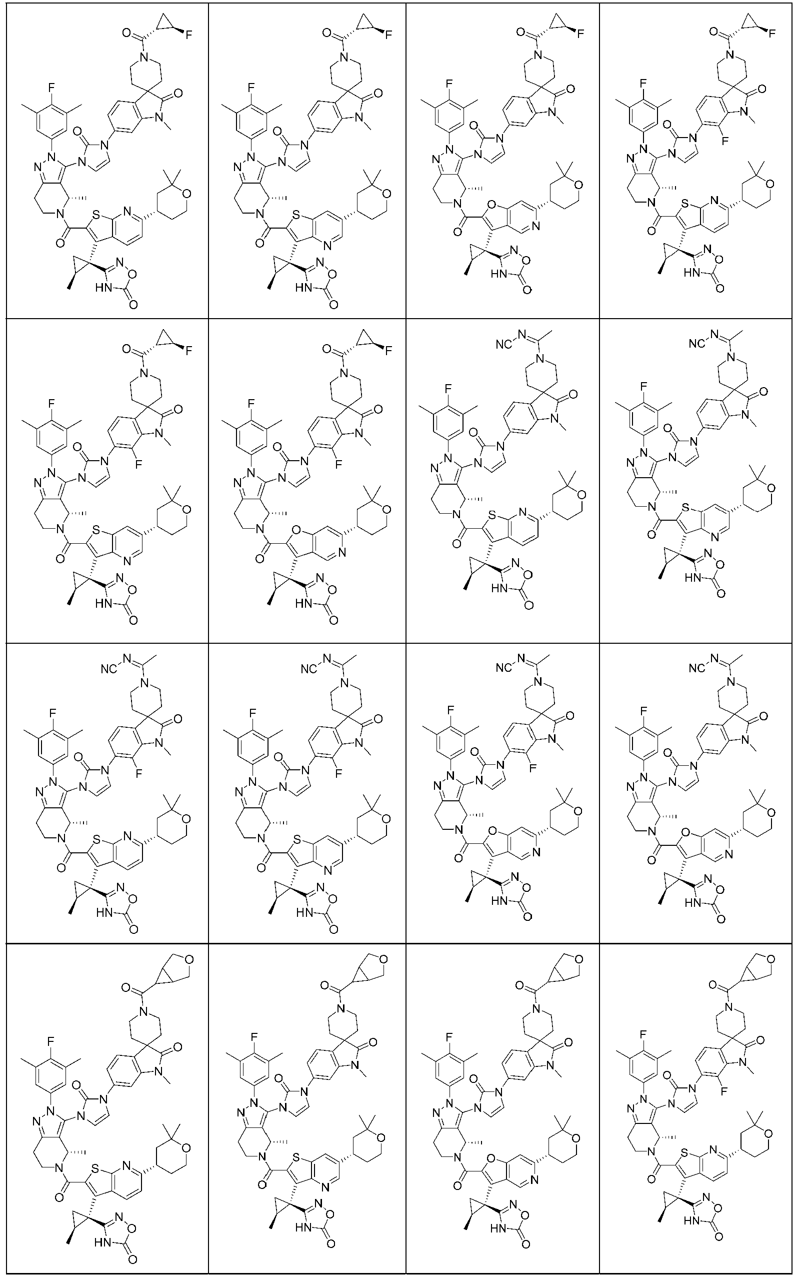

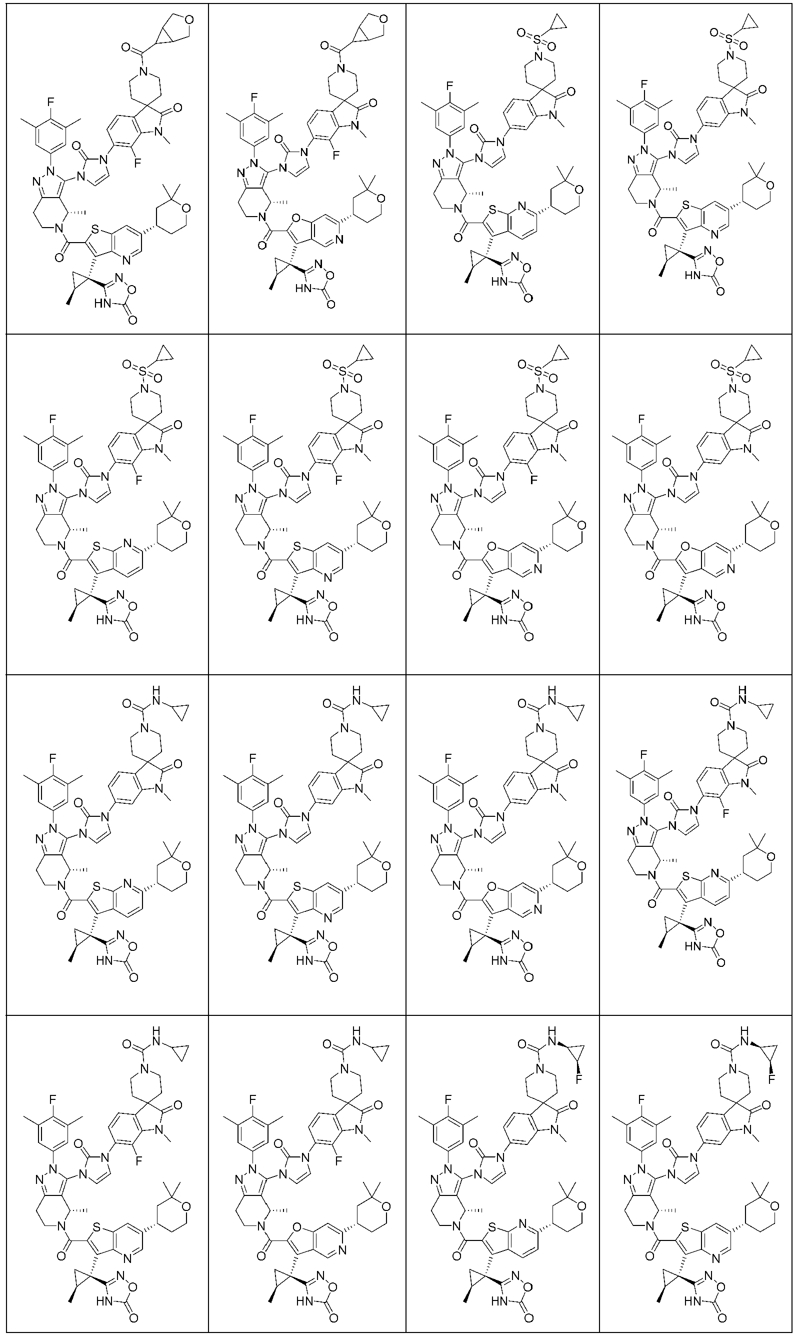

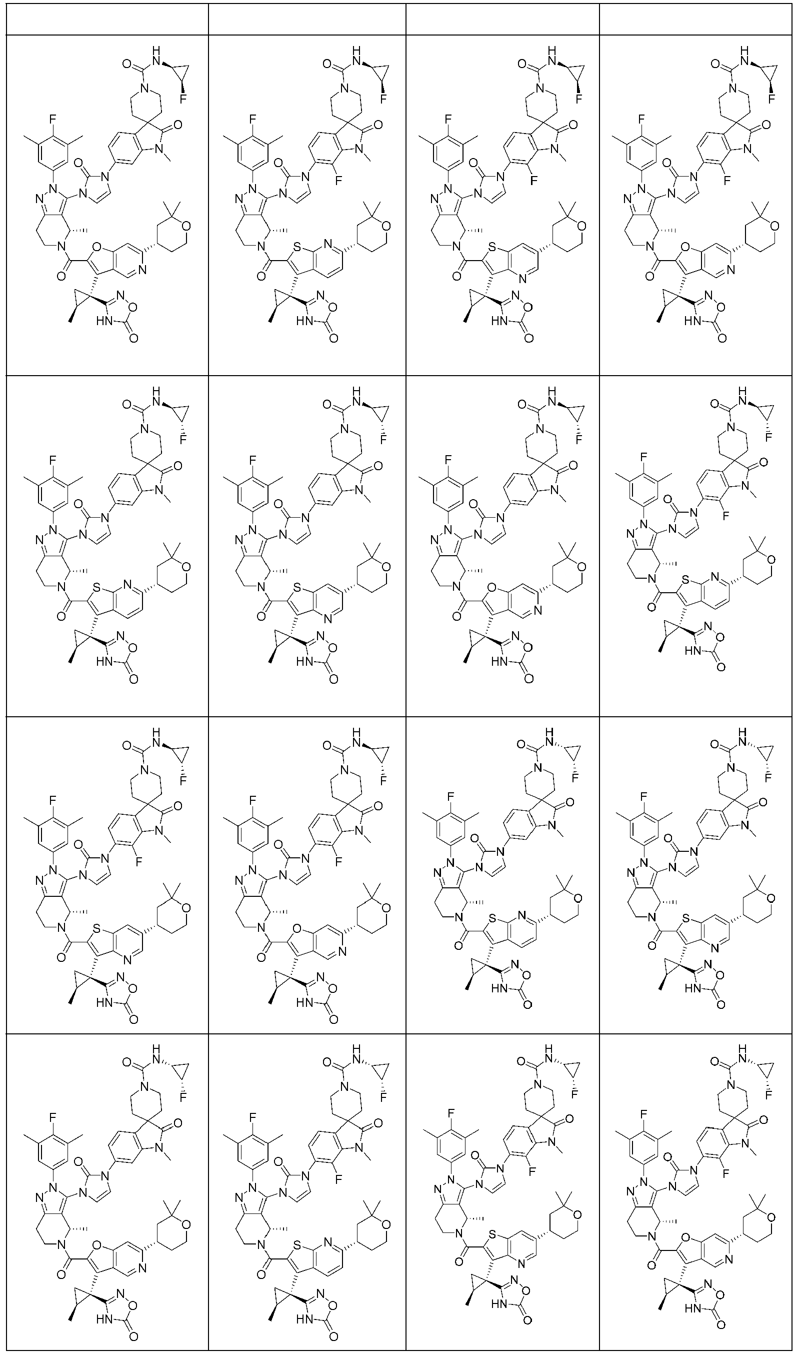

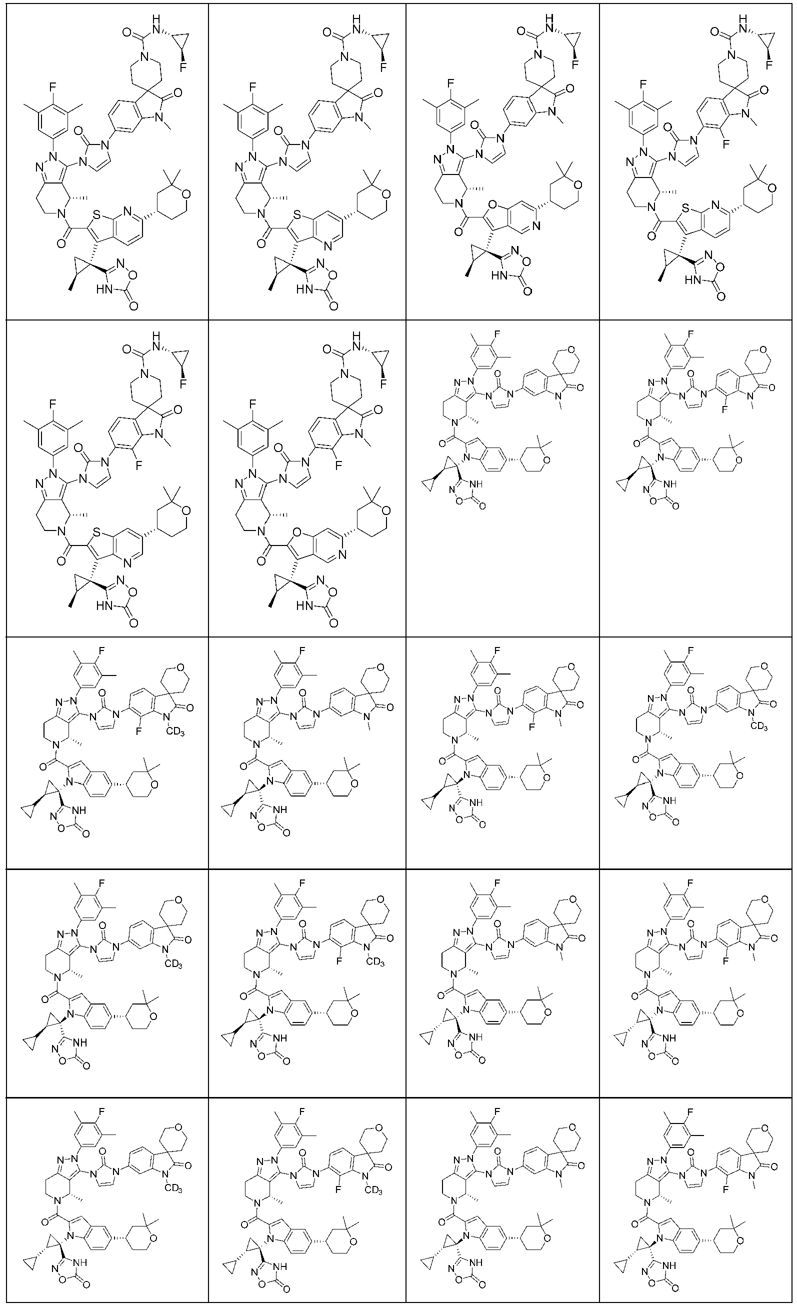

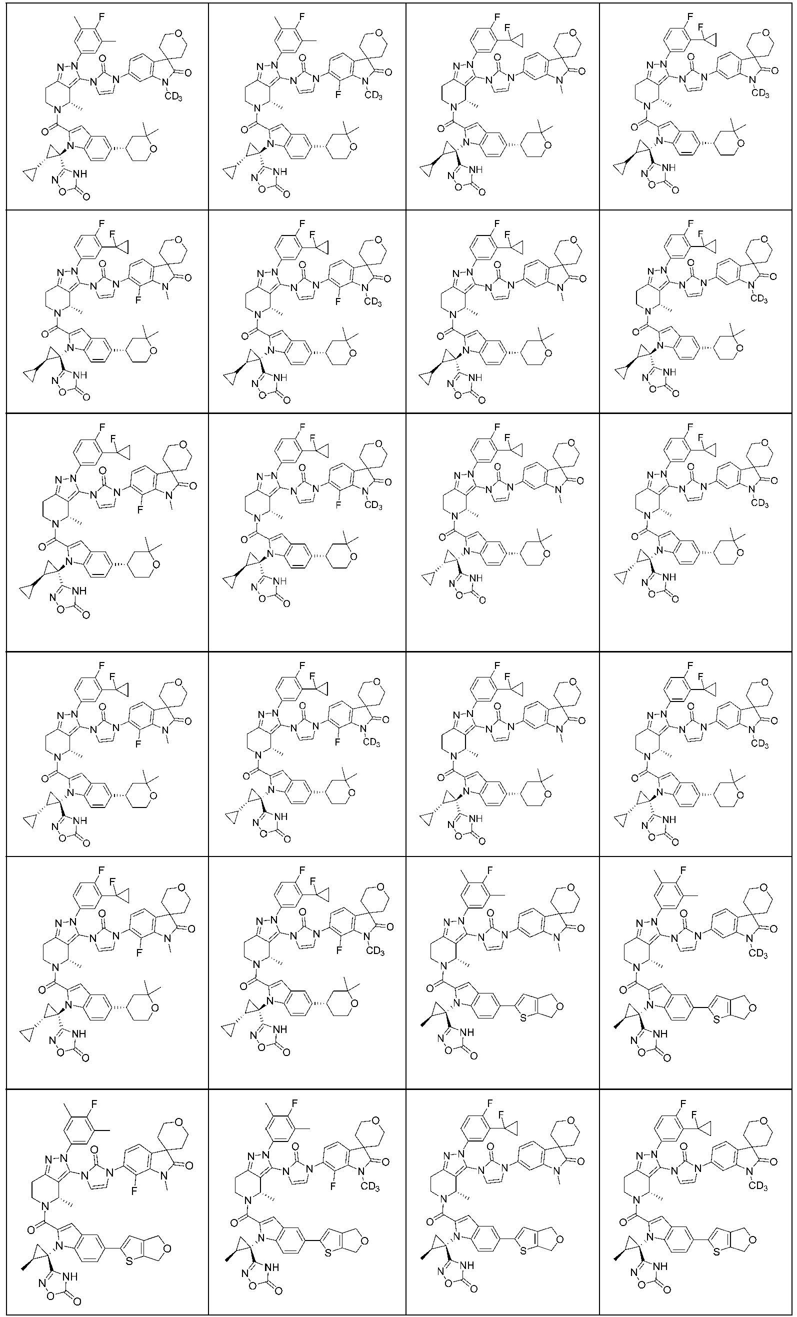

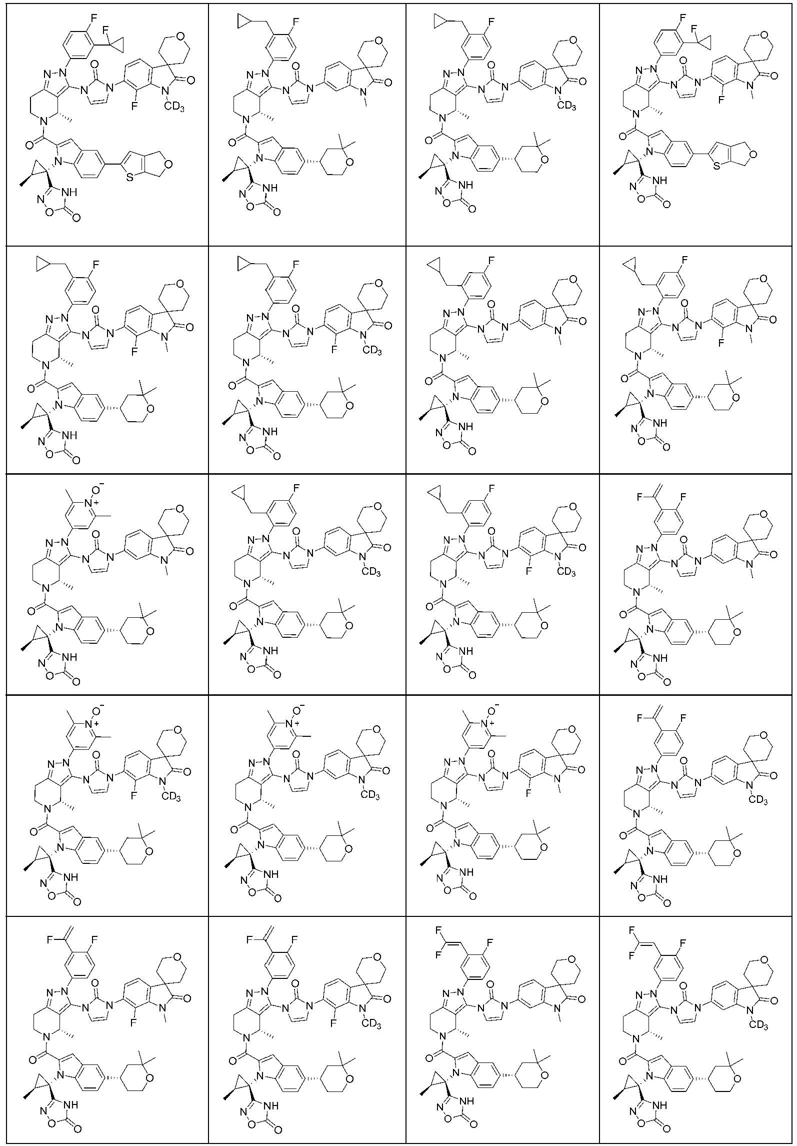

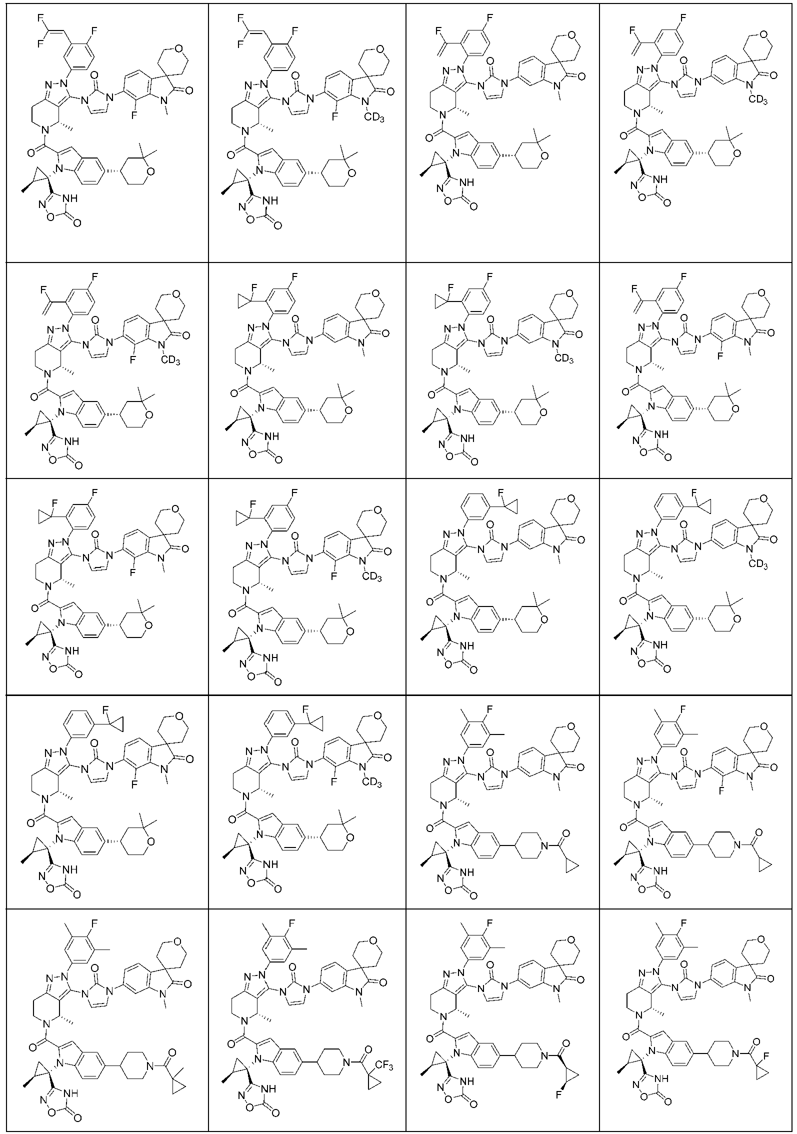

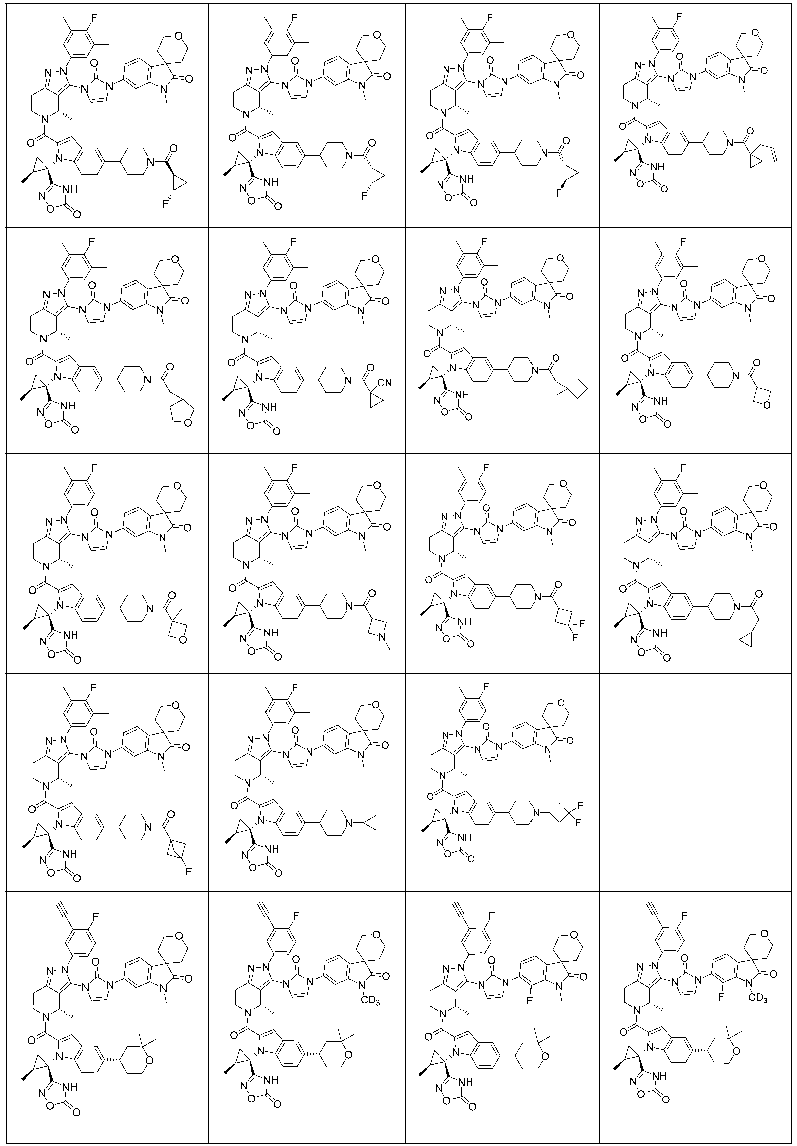

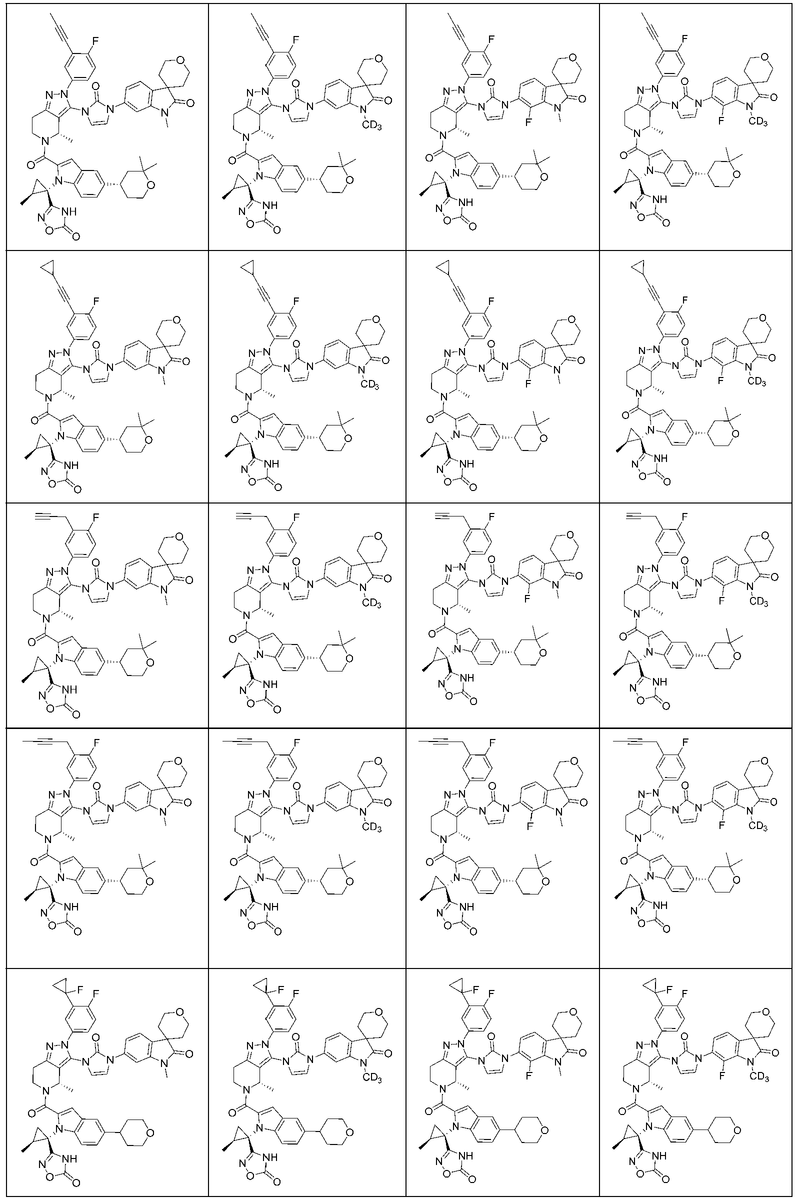

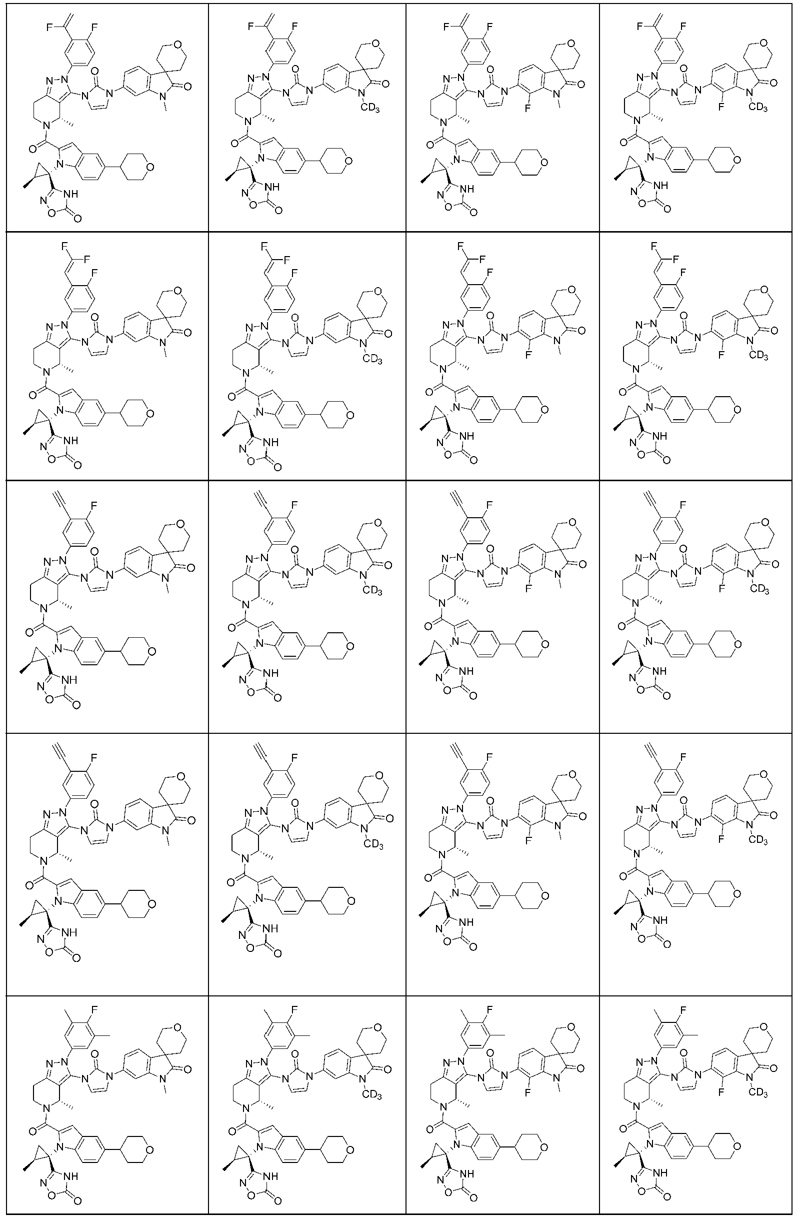

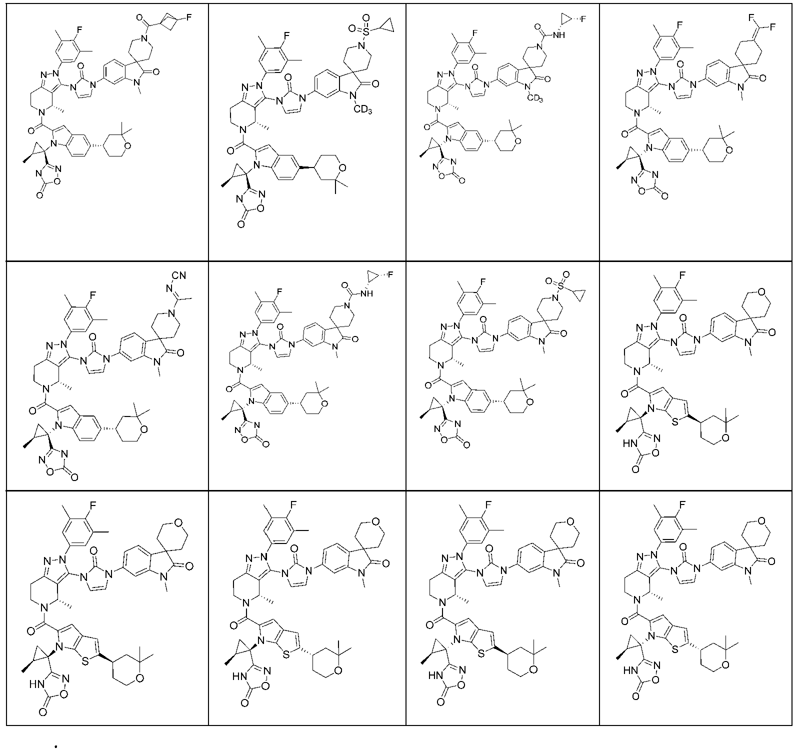

The present invention relates to a pharmaceutical composition comprising the above-described compound or the racemate, stereoisomer, tautomer, deuterated substance, solvate, prodrug, metabolite, pharmaceutically acceptable salt or eutectic crystal thereof, and a pharmaceutically acceptable carrier.

The present invention relates to the use of the above-described compound or the racemate, stereoisomer, tautomer, deuterated substance, solvate, prodrug, metabolite, pharmaceutically acceptable salt or eutectic crystal thereof, or the above-described pharmaceutical composition in the preparation of a drug for treating a disease related to GLP-1R activity or expression level.

The present invention relates to the use of the above-described compound or the racemate, stereoisomer, tautomer, deuterated substance, solvate, prodrug, metabolite, pharmaceutically acceptable salt or eutectic crystal thereof, or the above-described pharmaceutical composition in the preparation of a drug for treating diabetes or obesity.

The present invention relates to a pharmaceutical composition or pharmaceutical preparation, wherein the pharmaceutical composition or pharmaceutical preparation comprises a therapeutically effective amount of the compound or the racemate, stereoisomer, deuterated substance, solvate, prodrug, metabolite, pharmaceutically acceptable salt or eutectic crystal thereof according to the present invention, and a pharmaceutically acceptable excipient. The pharmaceutical composition can be in a unit preparation form (the amount of the active ingredient in the unit preparation is also referred to as the "preparation strength").

The present invention also provides a method for treating a disease in a mammal, wherein the method comprises administering to the mammal a therapeutically effective amount of the compound or the racemate, stereoisomer, deuterated substance, solvate, prodrug, metabolite, pharmaceutically acceptable salt or eutectic crystal thereof, or the pharmaceutical composition according to the present invention. In some embodiments, the mammal described in the present invention comprises a human.

The "effective amount" or "therapeutically effective amount" described in the present application refers to administration of a sufficient amount of the compound disclosed in the present application that will alleviate to some extent one or more symptoms of the diseases or conditions being treated (such as diabetes or obesity). In some embodiments, the outcome is the reduction and/or remission of signs, symptoms, or causes of the disease, or any other desired change in the biological system. For example, an "effective amount" for therapeutic use is an amount of the compound disclosed in the present application that is required to provide a clinically significant reduction of the symptoms of the disease. Examples of the therapeutically effective amount include, but are not limited to 1-1500 mg, 1-1000 mg, 1-800 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, and 150-200 mg;
in some embodiments, the pharmaceutical composition comprises the compound or the racemate, stereoisomer, deuterated substance, solvate, prodrug, metabolite, pharmaceutically acceptable salt or eutectic crystal thereof according to the present invention in an amount including but not limited to 1-1500 mg, 1-1000 mg, 1-800 mg, 1-600 mg, 20-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, and 300 mg.

A method for treating a disease in a mammal, wherein the method comprises administering to a subject a therapeutically effective amount of the compound or the racemate, stereoisomer, deuterated substance, solvate, prodrug, metabolite, pharmaceutically acceptable salt or eutectic crystal thereof according to the present invention, wherein the therapeutically effective amount is preferably 1-1500 mg, and the disease is preferably a disease related to GLP-1R activity or expression level (such as diabetes or obesity).

Provided is a method for treating a disease in a mammal, wherein the method comprises administering to a subject a drug, i.e., the compound or the racemate, stereoisomer, deuterated substance, solvate, prodrug, metabolite, pharmaceutically acceptable salt or eutectic crystal thereof according to the present invention in a daily dose of 1-1500 mg/day, and the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, and 200-400 mg/day; in some embodiments, the daily dose includes, but is not limited to 10 mg/day, 20 mg/day, 25 mg/day, 50 mg/day, 100 mg/day, 125 mg/day, 150 mg/day, 200 mg/day, 400 mg/day, 600 mg/day, and 800 mg/day.

The present invention relates to a kit, wherein the kit may comprise a composition in the form of a single dose or multiple doses and comprises the compound or the racemate, stereoisomer, deuterated substance, solvate, prodrug, metabolite, pharmaceutically acceptable salt or eutectic crystal thereof according to the present invention, and the amount of the compound or the racemate, stereoisomer, deuterated substance, solvate, prodrug, metabolite, pharmaceutically acceptable salt or eutectic crystal thereof according to the present invention is identical to the amount of same in the pharmaceutical composition as described above.

In the present invention, the compound or the racemate, stereoisomer, deuterated substance, solvate, prodrug, metabolite, pharmaceutically acceptable salt or eutectic crystal thereof according to the present invention is calculated in the form of free base in each case.

The term "preparation strength" refers to the weight of the active ingredient contained in each vial, tablet or other unit preparation.

### Synthesis method I:

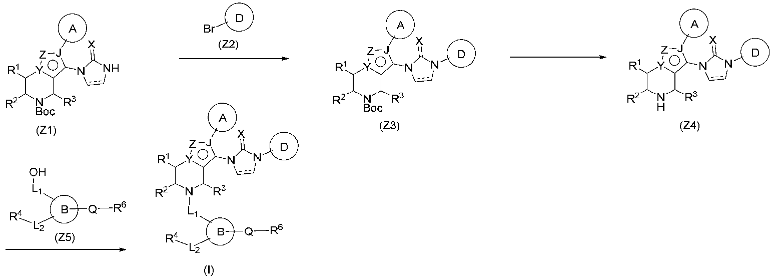

general formula (Z1) and general formula (Z2) undergo a coupling reaction catalysed by a heavy metal catalyst to afford general formula (Z3). The protecting group of general formula (Z3) is removed under acidic conditions to yield general formula (Z4). General formula (Z4) is then reacted with general formula (Z5) in the presence of a condensing agent (e.g., HATU) to afford general formula (I).

Unless otherwise stated, the terms used in the description and claims have the following meanings.

The carbon, hydrogen, oxygen, sulphur, nitrogen, F, Cl, Br and I involved in the groups and compounds of the present invention all comprise their isotopes, and the carbon, hydrogen, oxygen, sulphur or nitrogen involved in the groups and compounds of the present invention is optionally further replaced with one or more of their corresponding isotopes, wherein the isotopes of carbon comprise ¹²C, ¹³C and ¹⁴C, the isotopes of hydrogen comprise protium (H), deuterium (D, also known as heavy hydrogen) and tritium (T, also known as superheavy hydrogen), the isotopes of oxygen comprise ¹⁶O, ¹⁷O and ¹⁸O, the isotopes of sulphur comprise ³²S, ³³S, ³⁴S and ³⁶S, the isotopes of nitrogen comprise ¹⁴N and ¹⁵N, the isotopes of fluorine comprise ¹⁷F and ¹⁹F, the isotopes of chlorine comprise ³⁵Cl and ³⁷Cl, and the isotopes of bromine comprise ⁷⁹Br and ⁸¹Br.

"CN" refers to cyano.

"Halogen" refers to F, Cl, Br or I.

"Halogen-substituted" refers to substitution with F, Cl, Br, or I, including but not limited to substitution with 1 to 10 substituents selected from F, Cl, Br, or I, or substitution with 1 to 6 substituents selected from F, Cl, Br, or I, or substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The alkyl can be monovalent, divalent, trivalent or tetravalent.

"Alkylene" refers to a substituted or unsubstituted linear or branched divalent saturated hydrocarbon group, including -(CH₂)ᵥ- (v is an integer from 1 to 10), and examples of alkylene include, but are not limited to, methylene, ethylene, propylene, butylene, etc.

"Cycloalkyl" refers to a substituted or unsubstituted saturated carbocyclic hydrocarbon group, typically having 3 to 12 carbon atoms, and the cycloalkyl can be monocyclic, fused, bridged or spirocyclic. Non-limiting examples include cyclopropyl, cyclobutyl, cyclopentyl, cyclohexyl, cycloheptyl, cyclobutyl fused cyclobutyl, cyclobutyl spiro cyclobutyl, adamantane, etc. The cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 12 atoms, 3 to 8 atoms, or 1 to 3 heteroatoms selected from N, O, S, P or Se. The C, N, S and P in the ring of the heterocycloalkyl can be oxidised to various oxidation states. The heterocycloalkyl can be monocyclic, fused, bridged or spirocyclic. The heterocycloalkyl can be connected to a heteroatom or a carbon atom, and non-limiting examples include oxiranyl, aziridinyl, oxetanyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl, piperazinyl, 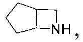 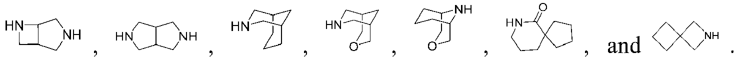 The heterocycloalkyl can be monovalent, divalent, trivalent or tetravalent.

"Alkenyl" refers to a substituted or unsubstituted linear or branched unsaturated hydrocarbon group, having at least 1, typically 1, 2 or 3 carbon-carbon double bonds, with a main chain including but not limited to, 2 to 10, 2 to 6, or 2 to 4 carbon atoms. Examples of the alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, and 2-methyl-3-butenyl. The alkenyl can be monovalent, divalent, trivalent or tetravalent.

"Alkynyl" refers to a substituted or unsubstituted linear or branched unsaturated hydrocarbon group, having at least 1, typically 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of the alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, and 2-methyl-3-butynyl. The alkynyl can be monovalent, divalent, trivalent or tetravalent.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system. The carbocyclyl can be connected to an aromatic ring or non-aromatic ring, and the ring is optionally monocyclic, fused, bridged or spirocyclic. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring, 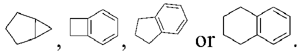 The "carbocyclyl" or "carbocyclic ring" can be monovalent, divalent, trivalent or tetravalent.

"Heterocyclyl" or "heterocyclic ring" refers to a substituted or unsubstituted aromatic ring or non-aromatic ring, wherein the aromatic ring or non-aromatic ring can be a 3- to 8-membered monocyclic ring, 4- to 12-membered bicyclic ring, 10-to 15-membered tricyclic ring, or 12- to 18-membered tetracyclic ring system, and contains 1 or more (including but not limited to 2, 3, 4 or 5) heteroatoms selected from N, O, S, P or Se, and C, N, S, P or Se (which may be selectively substituted) in the ring of the heterocyclyl can be oxidised to various oxidation states. Heterocyclyl can be connected to a heteroatom or a carbon atom; heterocyclyl can be connected to an aromatic ring or a non-aromatic ring; and heterocyclyl is optionally monocyclic, bridged, fused, or spiro. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl, 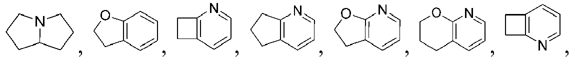 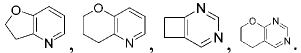 The "heterocyclyl" or "heterocyclic ring" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and may optionally contain 0 to 5 heteroatoms selected from N, O or S(=O)ₙ (n is 0, 1, or 2). Non-limiting examples include: 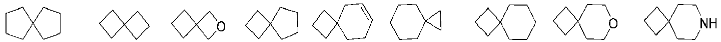 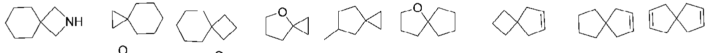 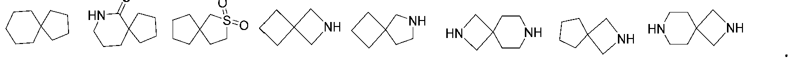 The "spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, S(=O)ₙ or O, wherein n is 0, 1 or 2). The number of ring atoms in the fused ring system includes, but is not limited to 5 to 20, 5 to 14, 5 to 12, or 5 to 10. Non-limiting examples include: 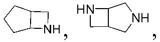 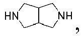 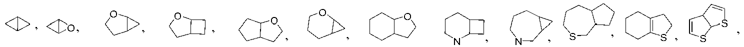 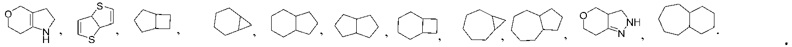 The "fused ring" or "fused ring group" can be monovalent, divalent, trivalent or tetravalent.

"Bridged ring" or "bridged ring group" means a substituted or unsubstituted polycyclic group containing any two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes, but is not limited to 5 to 20, 5 to 14, 5 to 12, or 5 to 10. Non-limiting examples include 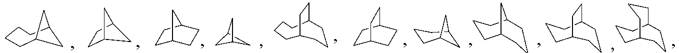 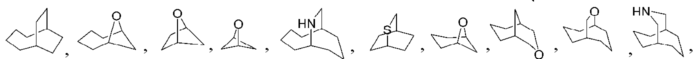 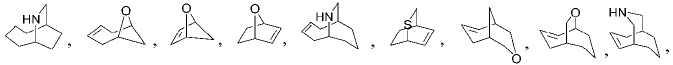 cubane, or adamantane. The "bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused-heterocyclic ring", "fused-heterocyclyl", "fused ring heterocyclyl" or "fused-heterocyclic ring group" refers to a "fused ring" containing a heteroatom.

"Spiro-heterocyclic ring", "spiro-heterocyclyl", "spiro ring heterocyclyl" or "spiro-heterocyclic ring group" refers to a "spiro ring" containing a heteroatom.

"Bridged-heterocyclic ring", "bridged-heterocyclyl", "bridged ring heterocyclyl" or "bridged-heterocyclic ring group" refers to a "bridged ring" containing a heteroatom.

"Aryl" or "aromatic ring" refers to a substituted or unsubstituted aromatic hydrocarbon group with a monocyclic ring or a fused ring, wherein the number of ring atoms in the aromatic ring includes, but is not limited to 6 to 18, 6 to 12, or 6 to 10 carbon atoms. The aryl ring can be fused to a saturated or unsaturated carbocyclic ring, wherein the ring connected to the parent structure is an aryl ring. Non-limiting examples include a benzene ring, a naphthalene ring, and 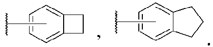 The "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O, S(=O)n, or Se(=O)n, wherein n is 0, 1 or 2), wherein the number of ring atoms in the heteroaromatic ring includes, but is not limited to, 5 to 15, 5 to 10, or 5 to 6. The atoms C, N, and S in the ring are optionally oxidised (i.e., C(=O), NO, S(=O)n, and Se(=O)n, wherein n is 1 or 2). Non-limiting examples of heteroaryl include, but are not limited to pyridyl, furyl, thienyl, selenophenyl, pyridyl, pyranyl, N-alkylpyrrolyl, pyrimidyl, pyrazinyl, pyridazinyl, imidazolyl, benzopyrazolyl, benzimidazolyl, benzopyridyl, pyrrolopyridyl, pyridonyl, etc. The heteroaryl ring can be fused to a saturated or unsaturated carbocyclic ring or heterocyclic ring, wherein the ring connected to the parent structure is an aryl ring. Non-limiting examples include 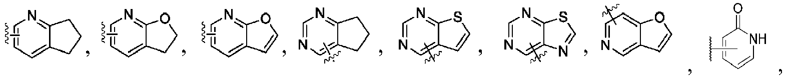 and 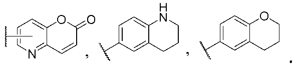 The definition of the heteroaryl herein is consistent with this definition. The heteroaryl can be monovalent, divalent, trivalent or tetravalent. When divalent, trivalent or tetravalent, the points of connection are on the aromatic ring.

"Substitution" or "substituted" refers to substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, a bridged ring group, a spiro ring group, a fused ring group, hydroxyalkyl, =O, carbonyl, aldehyde, carboxylic acid, carboxylate, -(CH2)ₘ-C(=O)-R^{a}, -O-(CH2)ₘ-C(=O)-R^{a}, -(CH2)ₘ-C(=O)-NR^{b}R^{c}, -(CH₂)ₘS(=O)ₙR^{a}, -(CH2)ₘ-alkenyl-R^{a}, OR^{d}, -(CH2)ₘ₋alkynyl-R^{a} (wherein m and n are 0, 1 or 2), arylthio, thiocarbonyl, silyl, -NR^{b}R^{c}, etc., wherein R^{b} and R^{c} are independently selected from H, hydroxyl, amino, carbonyl, alkyl, alkoxy, cycloalkyl, heterocyclyl, aryl, heteroaryl, sulphonyl, or trifluoromethylsulphonyl; alternatively, R^{b} and R^{c} may form five- or six-membered cycloalkyl or heterocyclyl; R^{a} and R^{d} are each independently selected from aryl, heteroaryl, alkyl, alkoxy, cycloalkyl, heterocyclyl, carbonyl, an ester group, a bridged ring group, a spiro ring group or a fused ring group.

"Substituted with 1 to X substituents selected from ..." refers to a substitution with 1, 2, 3 ... X substituents selected from ..., wherein X is selected from any integer between 1 and 10. For example, "substituted with 1 to 4 R^{k}" refers to a substitution with 1, 2, 3 or 4 R^{k}. For example, "substituted with 1 to 5 substituents selected from ..." refers to a substitution with 1, 2, 3, 4 or 5 substituents selected from ... For example, "bridged-heterocyclic ring is optionally substituted with 1 to 4 substituents selected from H or F" means that the bridged-heterocyclic ring is optionally substituted with 1, 2, 3 or 4 substituents selected from H or F.

An X- to Y-membered ring (X and Y are integers, 3 ≤ X < Y, and X < Y ≤ 20 (i.e., any integer from 4 to 20)) includes X-, X+1-, X+2-, X+3-, X+4-,...or Y-membered rings. Rings include heterocyclyl, carbocyclyl, an aromatic ring group, aryl, heteroaryl, cycloalkyl, a mono-heterocyclic ring, a fused-heterocyclic ring, a spiro-heterocyclic ring, or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

A C_{x-y} carbocyclyl (including aryl, cycloalkyl, monocyclic carbocyclyl, spiro carbocyclyl, fused carbocyclyl, or bridged carbocyclyl) includes Cₓ-, Cₓ₊₁-, Cₓ₊₂-, Cₓ₊₃-, Cₓ₊₄-,...or C_{y}-membered rings (x is an integer, 3 ≤ x < y, and y is any integer from 4 to 20). For example, "C₃₋₆ cycloalkyl" refers to C₃, C₄, C₅ or C₆ cycloalkyl.

When a group has one or more connectable sites, any one or more sites of the group can be connected to other groups via chemical bonds. When the connection mode of the chemical bond is indeterminate and there are hydrogen atoms at the connectable sites, the number of H atoms at the site will decrease correspondingly with the number of connected chemical bonds when the chemical bond is connected, forming a group with the corresponding valence. For example, 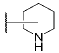 indicates that any connectable site on the piperidyl group can be connected to other groups via one chemical bond, including at least 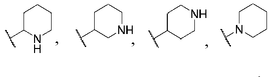 these 4 connection modes. Even if an H atom is shown on the N atom, 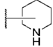 also includes 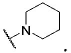 For example, 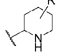 indicates that the R group on the piperidyl group can be located on C or N, including at least

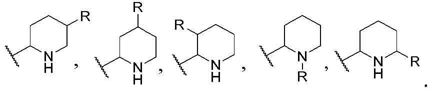

When the connecting groups listed do not specify their connection direction, their connection directions include both the left-to-right and right-to-left reading orders. For example, for A-L-B, wherein L is -M-W-, it includes both A-M-W-B and A-W-M-B.

The term "preparation strength" refers to the weight of the active ingredient contained in each vial, tablet or other unit preparation.

"Carrier" refers to a material that does not cause significant irritation to an organism and does not eliminate the biological activity and characteristics of a compound administered.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerisation and amide-imino alcohol isomerisation.

### Detailed Description of Embodiments

The technical solutions of the present invention will be described in detail by the following examples, but the scope of protection of the present invention includes but is not limited thereto.

The structures of the compounds are determined by nuclear magnetic resonance (NMR) or (and) mass spectrometry (MS). The NMR shift (δ) is given in the unit of 10⁻⁶ (ppm). NMR is measured with NMR instruments (Bruker Avance III 400 and Bruker Avance 300), and the solvent for determination is deuterated dimethyl sulphoxide (DMSO-d₆), deuterated chloroform (CDCl₃), and deuterated methanol (CD₃OD), and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM).

Yantai Huanghai HSGF₂₅₄ or Qingdao GF₂₅₄ silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm-0.5 mm;
and for the column chromatography, Yantai Huanghai silica gel of 200-300 mesh is generally used as a carrier.

The *abs1 or *abs2 in the structural formula represents that the chiral centre is of unspecified but single configuration.

HATU: CAS148893-10-1; LiHMDS: lithium bis(trimethylsilyl)amide NMP: N-methylpyrrolidone; DIPEA: N,N-diisopropylethylamine.

### Example 1: Preparation of compound 1

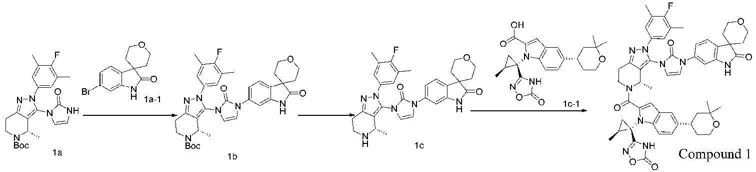

### Step 1: Preparation of 1b

1a (220 mg, 0.50 mmol) and 1a-1 (155 mg, 0.55 mmol) were dissolved in NMP (4 mL). CuI (50 mg, 0.26 mmoL), (1S,2S)-N,N'-dimethyl-1,2-cyclohexanediamine (50 mg, 0.35 mmoL), and anhydrous potassium carbonate (250 mg, 1.81 mmol) were added. Under a nitrogen atmosphere, the mixture was reacted at 130°C for 2 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 3 : 1) to obtain 1b (300 mg, yield: 93.67%). (1a was synthesised with reference to US 2019225604 A1).

LCMS m/z = 643.5 [M+H]⁺

### Step 2: Preparation of 1c

1b (300 mg, 0.47 mmol) was dissolved in dichloromethane (2 mL). 4 M HCl-dioxane solution (2 mL) was added, and the mixture was reacted at room temperature for 16 h. The system was concentrated to dryness. 2 mL of saturated aqueous potassium carbonate solution was added, and the mixture was extracted with ethyl acetate (2 mL×3). The organic layers were combined, dried over anhydrous sodium sulphate and filtered, and the filtrate was concentrated under reduced pressure. The residue was separated and purified by column chromatography (eluent: dichloromethane : methanol (v/v) = 10 : 1) to obtain 1c (170 mg, yield: 67.12%).

LCMS m/z = 543.4 [M+H]⁺

### Step 3: Preparation of compound 1

1c (70 mg, 0.13 mmol) was dissolved in super-dry DMF (2 mL). HATU (55 mg, 0.14 mmol), triethylamine (0.1 mL, 0.54 mmol), and 1c-1 (53 mg, 0.13 mmol) were added. Under a nitrogen atmosphere, the mixture was reacted at room temperature for 2 h. The filtrate was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 1 (60 mg, 49.69%). (1c-1 was synthesised with reference to US 2019225604 A1).

LCMS m/z = 936.5 [M+H]⁺

### Example 2: Preparation of compound 2

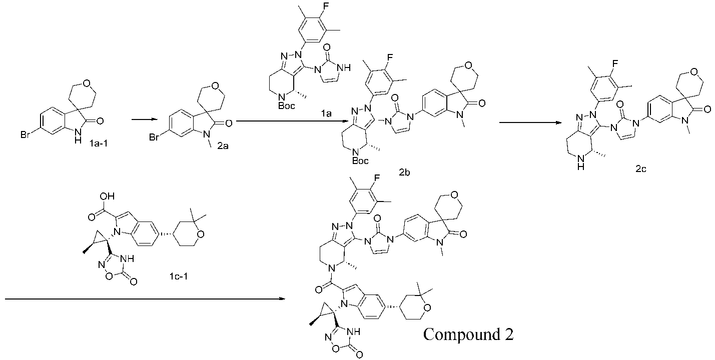

### Step 1: Preparation of 2a

1a-1 (280 mg, 0.99 mmol) was dissolved in super-dry THF (5 mL). Iodomethane (156 mg, 1.10 mmoL) and anhydrous potassium carbonate (200 mg, 1.45 mmoL) were added. Under a nitrogen atmosphere, the mixture was reacted at 45°C for 16 h, and cooled to room temperature. The reaction solution was filtered, and the filtrate was concentrated under reduced pressure. The residue was separated and purified by column chromatography (eluent: petroleum ether : ethyl acetate (v/v) = 5 : 1) to obtain 2a (175 mg, yield: 59.54%).

LCMS m/z = 296.0 [M+H]⁺
¹H NMR (400 MHz, DMSO-*d6*) δ 7.49 (d, 1H), 7.27 (d, 1H), 7.24-7.19 (m, 1H), 4.13-3.95 (m, 2H), 3.88-3.74 (m, 2H), 3.12 (s, 3H) ), 1.80-1.64 (m, 4H).

### Step 2: Preparation of 2b

2a (175 mg, 0.59 mmol) and 1a (218 mg, 0.49 mmol) were dissolved in NMP (5 mL). CuI (142.5 mg, 0.75 mmol), (1S,2S)-N,N'-dimethyl-1,2-cyclohexanediamine (107 mg, 0.75 mmoL), and anhydrous potassium carbonate (138 mg, 1 mmol) were added. Under a nitrogen atmosphere, the mixture was reacted at 130°C for 2 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 1/1) to obtain 2b (288 mg, yield: 88.81%).

LCMS m/z = 657.4 [M+H]⁺

### Step 3: Preparation of 2c

2b (200 mg, 0.3 mmol) was dissolved in dichloromethane (2 mL). 4 M HCl-dioxane solution (2 mL) was added, and the mixture was reacted at room temperature for 16 h. The system was concentrated to dryness. 2 mL of saturated aqueous potassium carbonate solution was added to the reaction solution, and the mixture was extracted with ethyl acetate (2 mL×3). The organic layers were combined, dried over anhydrous sodium sulphate and filtered, and the filtrate was concentrated under reduced pressure. The residue was separated and purified by column chromatography (eluent: dichloromethane : methanol (v/v) = 10/1) to obtain 2c (105 mg, yield: 61.94%).

LCMS m/z = 557.8 [M+H]⁺
¹H NMR (400 MHz, DMSO-*d6*) δ 7.64 (d, 1H), 7.39-7.21 (m, 3H), 7.11 (d, 2H), 6.91 (s, 1H), 4.12-3.97 (m, 2H), 3.92-3.73 (m, 3H), 3.24-3.09 (m, 4H), 2.91-2.76 (m, 1H), 2.70-2.54 (m, 2H), 2.44-2.25 (m, 1H), 2.19 (s, 6H), 1.82-1.65 (m, 4H) ), 1.08 (d, 3H).

### Step 4: Preparation of compound 2

2c (85 mg, 0.15 mmol) was dissolved in super-dry DMF (2 mL). HATU (87 mg, 0.23 mmol), triethylamine (60 mg, 0.46 mmol), and 1c-1 (63 mg, 0.15 mmol) were added. Under a nitrogen atmosphere, the mixture was reacted at room temperature for 2 h. The reaction solution was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 2 (25 mg, 17.19%).

LCMS m/z = 950.4 [M+H]⁺
¹H NMR (400 MHz, CF₃COOD) δ 7.70-7.58 (m, 3H), 7.39 (d, 1H), 7.35-7.25 (m, 3H), 7.23-7.18 (m, 1H), 7.10-6.80 (m, 3H), 6.15-6.05 (m, 1H), 4.95-4.79 (m, 1H), 4.60-4.51 (m, 2H), 4.30-4.07 (m, 4H), 3.97-3.83 (m, 1H), 3.63-3.42 (m, 2H), 3.39 (s, 3H), 3.28 -3.17 (m, 1H), 2.38 (s, 6H), 2.19-2.01 (m, 5H), 2.00-1.70 (m, 9H), 1.55 (s, 3H) ), 1.49 (s, 3H), 1.34 (d, 3H).

### Example 3: Preparation of compound 3

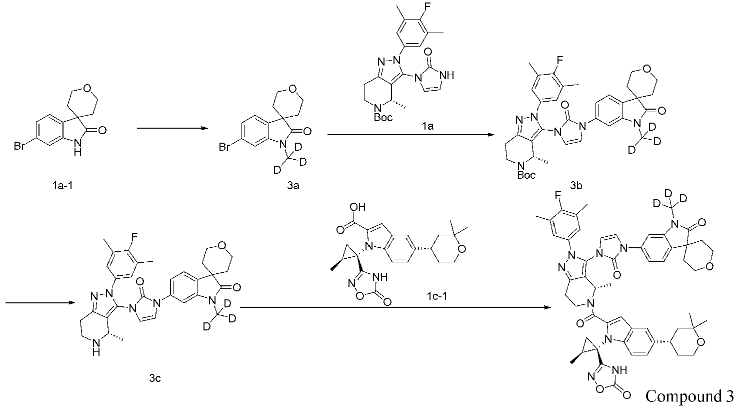

### Step 1: Preparation of 3a

1a-1 (200 mg, 0.71 mmol) was dissolved in super-dry DMF (5 mL). In an ice bath, sodium hydride (57 mg, 1.43 mmol, 60% wt) was added, and the mixture was stirred for 5 min. Then, deuterated iodomethane (114 mg, 0.79 mmol) was added. Under a nitrogen atmosphere, the mixture was reacted at room temperature for 2 h. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 3a (191 mg, yield: 90.06%).

### Step 2: Preparation of 3b

3a (175 mg, 0.58 mmol) and 1a (218 mg, 0.49 mmol) were dissolved in NMP (5 mL). CuI (143 mg, 0.75 mmol), (1S,2S)-N,N'-dimethyl-1,2-cyclohexanediamine (110 mg, 0.77 mmoL), and anhydrous potassium carbonate (140 mg, 1.01 mmol) were added. Under a nitrogen atmosphere, the mixture was reacted at 130°C for 2 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 1 : 1) to obtain 3b (285 mg, yield: 87.48%).

LCMS m/z = 660.5 [M+H]⁺

### Step 3: Preparation of 3c

3b (200 mg, 0.3 mmol) was dissolved in dichloromethane (3 mL). 4 M HCl-dioxane solution (4 mL) was added, and the mixture was reacted at room temperature for 16 h. The system was concentrated to dryness to obtain 3c.

### Step 4: Preparation of compound 3

3c (180 mg, 0.3 mmol) was dissolved in super-dry DMF (5 mL). HATU (171 mg, 0.45 mmol), triethylamine (0.2 mL, 1.21 mmol), and 1c-1 (124 mg, 0.3 mmol) were added. Under a nitrogen atmosphere, the mixture was reacted at room temperature for 2 h. The reaction solution was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 3 (30 mg, 10.42%).

LCMS m/z = 953.4 [M+H]⁺
¹H NMR (400 MHz, CF₃COOD) δ 7.69-7.58 (m, 3H), 7.39 (d, 1H), 7.35-7.25 (m, 3H), 7.23-7.17 (m, 1H), 7.11-6.80 (m, 3H), 6.16-6.04 (m, 1H), 4.93-4.79 (m, 1H), 4.60-4.51 (m, 2H), 4.30-4.07 (m, 4H), 3.98-3.83 (m, 1H), 3.59-3.38 (m, 2H), 3.28 -3.17 (m, 1H), 2.38 (s, 6H), 2.19-1.72 (m, 14H), 1.55 (s, 3H) ), 1.49 (s, 3H), 1.34 (d, 3H).

### Example 4: Preparation of compound 4

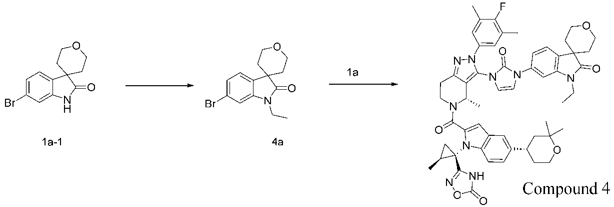

### Step 1: Preparation of 4a

1a-1 (200 mg, 0.71 mmol) was dissolved in super-dry DMF (5 mL). In an ice bath, sodium hydride (58 mg, 1.45 mmol, 60% wt) was added, and the mixture was stirred for 5 min. Then, iodoethane (122 mg, 0.78 mmol) was added. Under a nitrogen atmosphere, the mixture was reacted at room temperature for 2 h. 10 mL of water was added to the reaction solution, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 4a (215 mg, yield: 97.78%).

LCMS m/z =310.1, 312.1 [M+H]⁺
Using 4a as a substrate, and with reference to the synthetic methods from steps 2 to 4 of Example 2, compound 4 (15 mg) was obtained.

LCMS m/z = 964.6 [M+H]⁺
¹H NMR (400 MHz, CF₃COOD) δ 7.70-7.57 (m, 3H), 7.39 (d, 1H), 7.35-7.23 (m, 3H), 7.19 (s, 1H), 7.06-7.00 (m, 1H), 6.95-6.88 (m, 1H), 6.15-6.04 (m, 1H), 4.92-4.80 (m, 1H), 4.59-4.51 (m, 2H), 4.30-4.07 (m, 4H), 4.00-3.84 (m, 3H), 3.59-3.36 (m, 2H), 3.28-3.15 (m, 1H), 2.38 (s, 6H), 2.19-1.70 (m, 14H), 1.55 (s, 3H) ), 1.49 (s, 3H), 1.41-1.27 (m, 6H).

### Example 5: Preparation of compound 5

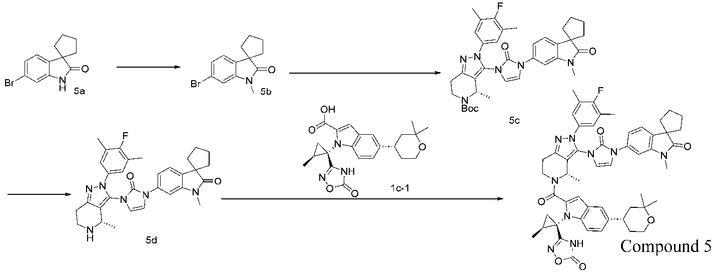

### Step 1: Preparation of 5b

5a (120 mg, 0.45 mmol) was dissolved in super-dry DMF (5 mL). Under a nitrogen atmosphere and at 0°C, sodium hydride (40 mg, 1.0 mmoL, 60% wt) was added, and the mixture was stirred for 1 h. Iodomethane (130 mg, 0.92 mmoL) was added, and the mixture was reacted at room temperature for 2 h. 5 mL of saturated aqueous ammonium chloride solution was added to the reaction solution, and the mixture was extracted with ethyl acetate (5 mL×3) and washed with a saturated aqueous sodium chloride solution (5 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 10 : 1) to obtain 5b (120 mg, yield: 94.99%).

LCMS m/z = 280.0, 282.0 [M+H]⁺

### Step 2: Preparation of 5c

1a (160 mg, 0.36 mmol) and 5b (120 mg, 0.43 mmol) were dissolved in NMP (3 mL). CuI (40 mg, 0.21 mmol), (1S,2S)-N,N'-dimethyl-1,2-cyclohexanediamine (40 mg, 0.28 mmoL), and anhydrous potassium carbonate (200 mg, 1.45 mmol) were added. Under a nitrogen atmosphere, the mixture was reacted at 130°C for 2 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 3 : 1) to obtain 5c (220 mg, yield: 94.74%).

LCMS m/z = 641.3 [M+H]⁺

### Step 3: Preparation of 5d

5c (220 mg, 0.34 mmol) was dissolved in dichloromethane (2 mL). 4 M HCl-dioxane solution (2 mL) was added, and the mixture was reacted at room temperature for 3 h. The system was concentrated to dryness. 5 mL of saturated aqueous potassium carbonate solution was added, and the mixture was extracted with ethyl acetate (5 mL×3). The organic layers were combined, dried over anhydrous sodium sulphate and filtered, and the filtrate was concentrated under reduced pressure. The residue was separated and purified by column chromatography (eluent: dichloromethane : methanol (v/v) = 10 : 1) to obtain 5d (160 mg, yield: 86.20%).

LCMS m/z = 541.4 [M+H]⁺

### Step 4: Preparation of compound 5

5d (160 mg, 0.30 mmol) was dissolved in super-dry DMF (4 mL). HATU (150 mg, 0.39 mmol), triethylamine (0.4 mL, 2.88 mmol), and 1c-1 (160 mg, 0.39 mmol) were added. Under a nitrogen atmosphere, the mixture was reacted at room temperature for 2 h. The reaction solution was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 5 (80 mg, 28.94%).

LCMS m/z = 934.4 [M+H]⁺
¹H NMR (400 MHz, CF₃COOD) δ 7.67-7.56 (m, 2H), 7.51-7.45 (m, 1H), 7.43-7.37 (m, 1H), 7.36-7.28 (m, 2H), 7.27-7.21 (m, 1H), 7.17-7.08 (m, 1H), 7.05-6.97 (m, 1H), 6.93-6.87 (m, 1H), 6.19-6.00 (m, 1H), 4.96-4.78 (m, 1H), 4.26-4.03 (m, 2H), 3.96-3.83 (m, 1H), 3.60-3.14 (m, 6H), 2.38 (s, 6H),2.34-1.68 (m, 18H), 1.55 (s, 3H),1.49 (s, 3H), 1.42-1.26 (m, 3H).

### Example 6: Preparation of compound 6

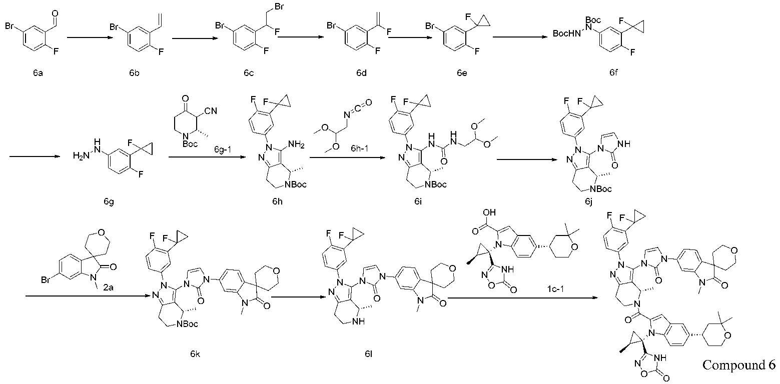

### Step 1: Preparation of 6b

In an ice bath, methyltriphenylphosphonium bromide (20.0 g, 55.99 mmol) was dissolved in super-dry THF (100 mL). 1 M potassium tert-butoxidetetrahydrofuran solution (60.0 mL, 60.0 mmol) was added. Under a nitrogen atmosphere, the mixture was reacted for 0.5 h. 6a (8.0 g, 39.41 mmol) was added, and the mixture was reacted at room temperature for 1 h. The reaction solution was concentrated under reduced pressure. The residue was separated and purified by column chromatography (eluent: petroleum ether) to obtain 6b (3.46 g, yield: 43.67%).

### Step 2: Preparation of 6c

6b (2.0 g, 9.95 mmol) was dissolved in dichloromethane (20 mL). In an ice bath, bromosuccinimide (4.4 g, 24.72 mmol) and triethylamine trihydrofluoride (4.8 g, 29.77 mmol) were added, and the mixture was reacted at room temperature for 16 h. 50 mL of water was added, and the mixture was extracted with dichloromethane (30 mL×3). The organic layers were combined, dried over anhydrous sodium sulphate and filtered, and the filtrate was concentrated under reduced pressure. The residue was separated and purified by column chromatography (eluent: petroleum ether) to obtain 6c (600 mg, yield: 20.11%).

### Step 3: Preparation of 6d

6c (550 mg, 1.83 mmol) was dissolved in petroleum ether (10 mL). Potassium tert-butoxide (1.2 g, 10.69 mmol) was added, and the mixture was reacted at room temperature for 16 h and filtered. The filtrate was concentrated under reduced pressure. The residue was separated and purified by column chromatography (eluent: petroleum ether) to obtain 6d (400 mg, yield: 99.59%).

### Step 4: Preparation of 6e

In an ice bath, 1 M diethylzinc in n-hexane (4 mL, 4 mmol) was dissolved in super-dry dichloromethane (4 mL), and trifluoroacetic acid (0.3 mL, 4.04 mmol) was slowly added dropwise. Under a nitrogen atmosphere, the mixture was reacted for 0.5 h. Diiodomethane (0.35 mL, 4.34 mmol) was added, and the mixture was stirred for 20 minutes. Then, 6d (400 mg, 1.83 mmoL) was added, and the mixture was reacted at room temperature for 16 h. 10 mL of saturated aqueous ammonium chloride solution was added, and the mixture was extracted with dichloromethane (10 mL×3). The reaction solution was concentrated under reduced pressure. The residue was separated and purified by column chromatography (eluent: petroleum ether) to obtain 6e (370 mg, yield: 86.94%).

### Step 5: Preparation of 6f

6e (370 mg, 1.59 mmol) was dissolved in super-dry THF (5 mL). At -78°C, 2.5 M n-butyllithium in tetrahydrofuran (0.7 mL, 1.75 mmol) was added, and the mixture was reacted for 1 h. Di-tert-butyl azodicarboxylate (400 mg, 1.74 mmol) was added, and the mixture was reacted at room temperature for 2 h. 10 mL of water was added, and the mixture was extracted with ethyl acetate (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 10 : 1) to obtain 6f (239 mg, yield: 39.16%).

### Step 6: Preparation of 6g

6f (230 mg, 0.60 mmol) was dissolved in NMP (2 mL). Methanesulphonic acid (0.3 mL, 4.62 mmol) was added, and the mixture was reacted at 80°C for 16 h. 5 mL of water was added, and the mixture was extracted with ethyl acetate (5 mL×3) and washed with a saturated aqueous sodium chloride solution (5 mL×3). The organic layers were combined and dried over anhydrous sodium sulphate, and the filtrate was concentrated under reduced pressure to obtain 6g (100 mg).

### Step 7: Preparation of 6h

6g (100 mg, 0.54 mmol) was dissolved in toluene (2 mL). 6g-1 (145 mg, 0.61 mmol) and pyridine hydrochloride (10 mg, 0.087 mmol) were added, and the mixture was reacted at 90°C for 1 h. The system was concentrated under reduced pressure. The reaction solution was concentrated under reduced pressure. The residue was separated and purified by column chromatography (eluent: petroleum ether : ethyl acetate (v/v) = 5 : 1) to obtain 6h (54 mg, yield: 24.59%).

LCMS m/z = 405.3 [M+H]⁺

### Step 8: Preparation of 6i

6h (50 mg, 0.12 mmol) was dissolved in pyridine (1 mL). 6h-1 (40 mg, 0.31 mmol) and diethylamine (20 mg, 0.27 mmol) were added, and the mixture was reacted at room temperature for 0.5 h. 5 mL of water was added to the system, and the mixture was extracted with ethyl acetate (5 mL×3) and washed with a saturated aqueous sodium chloride solution (5 mL×3). The organic layers were combined and dried over anhydrous sodium sulphate, and the filtrate was concentrated under reduced pressure to obtain 6i (60 mg).

### Step 9: Preparation of 6j

6i (60 mg, 0.11 mmol) was dissolved in super-dry THF (2 mL). Methanesulphonic acid (0.1 mL, 1.54 mmol) was added, and the mixture was reacted at 60°C for 0.5 h. 5 mL of saturated aqueous sodium bicarbonate solution was added, and the mixture was extracted with ethyl acetate (5 mL×3). The organic layers were combined and dried over anhydrous sodium sulphate, and the filtrate was concentrated under reduced pressure to obtain 6j (50 mg, yield: 94.66%)
LCMS m/z = 472.2 [M+H]⁺

### Step 10: Preparation of 6k

6j (80 mg, 0.17 mmol) and 2a (55 mg, 0.19 mmol) were dissolved in NMP (2 mL). CuI (30 mg, 0.16 mmoL), (1S,2S)-N,N'-dimethyl-1,2-cyclohexanediamine (30 mg, 0.21 mmoL), and anhydrous potassium carbonate (120 mg, 0.87 mmol) were added. Under a nitrogen atmosphere, the mixture was reacted at 130°C for 2 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 6k (100 mg, yield: 85.82%).

LCMS m/z = 687.3 [M+H]⁺

### Step 11: Preparation of 6l

6k (100 mg, 0.15 mmol) was dissolved in dichloromethane (1 mL). 4 M HCl-dioxane solution (1 mL) was added, and the mixture was reacted at room temperature for 3 h. The system was concentrated to dryness. 2 mL of saturated aqueous potassium carbonate solution was added, and the mixture was extracted with ethyl acetate (2 mL×3). The organic layers were combined, dried over anhydrous sodium sulphate and filtered, and the filtrate was concentrated under reduced pressure. The residue was separated and purified by column chromatography (eluent: dichloromethane : methanol (v/v) = 10 : 1) to obtain 6l (80 mg, yield: 93.65%).

LCMS m/z = 587.2 [M+H]⁺

### Step 12: Preparation of compound 6

6l (80 mg, 0.14 mmol) was dissolved in super-dry DMF (2 mL). HATU (70 mg, 0.18 mmol), triethylamine (0.2 mL, 1.44 mmol), and 1c-1 (66 mg, 0.16 mmol) were added. Under a nitrogen atmosphere, the mixture was reacted at room temperature for 16 h. The reaction solution was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 6 (50 mg, 37.41%).

LCMS m/z = 980.6 [M+H]⁺
¹H NMR (400 MHz, CF₃COOD) δ 7.90-7.82 (m, 1H), 7.71-7.51 (m, 4H), 7.44-7.26 (m, 3H), 7.24-7.16 (m, 1H), 7.05-6.99 (m, 1H), 6.98-6.91 (m, 1H), 6.15-6.03 (m, 1H), 4.91-4.79 (m, 1H), 4.61-4.46 (m, 2H), 4.31-4.05 (m, 4H), 3.97-3.82 (m, 1H), 3.60-3.12 (m, 6H), 2.26-1.15 (m, 27H).

### Example 7: Preparation of compound 7

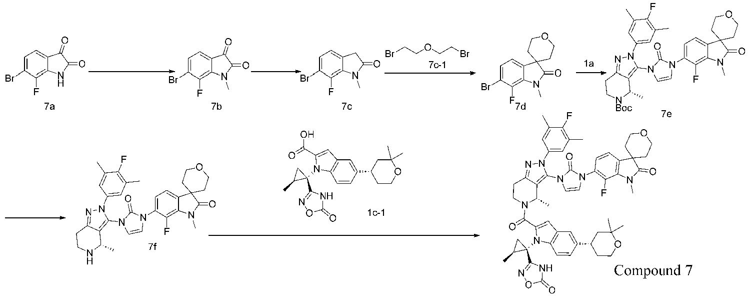

### Step 1: Preparation of 7b

7a (2.44 g, 10.0 mmol) was dissolved in super-dry DMF (10 mL). Iodomethane (2.14 g, 15.0 mmoL) and anhydrous potassium carbonate (2.76 g, 20.0 mmoL) were added. Under a nitrogen atmosphere, the mixture was reacted at 60°C for 2 h, and cooled to room temperature. 20 mL of saturated aqueous ammonium chloride solution was added, and the mixture was extracted with ethyl acetate (20 mL×3) and washed with a saturated aqueous sodium chloride solution (20 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 7b (2.0 g, yield: 77.51%).

LCMS m/z = 258.0, 260.0 [M+H]⁺

### Step 2: Preparation of 7c

7b (200 mg, 0.78 mmol) was dissolved in ethanol (2 mL). Hydrazine hydrate (1 mL, 80% wt) was added, and the mixture was reacted at 130°C for 2 h, and cooled to room temperature. The reaction solution was concentrated under reduced pressure. The residue was separated and purified by column chromatography (eluent: petroleum ether : ethyl acetate (v/v) = 5 : 1) to obtain 7c (100 mg, yield: 52.86%).

### Step 3: Preparation of 7d

7c (100 mg, 0.41 mmol) was dissolved in super-dry DMF (3 mL). 7c-1 (190 mg, 0.82 mmoL), caesium carbonate (270 mg, 0.82 mmoL), and sodium iodide (120 mg, 0.82 mmoL) were added. Under a nitrogen atmosphere, the mixture was reacted at 50°C for 16 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 7d (60 mg, yield: 46.61%).

LCMS m/z = 314.0, 316.0 [M+H]⁺

### Step 4: Preparation of 7e

1a (65 mg, 0.15 mmol) and 7d (50 mg, 0.16 mmol) were dissolved in super-dry NMP (2 mL). CuI (15 mg, 0.079 mmol), (1S,2S)-N,N'-dimethyl-1,2-cyclohexanediamine (15 mg, 0.11 mmoL), and anhydrous potassium carbonate (90 mg, 0.65 mmol) were added. Under a nitrogen atmosphere, the mixture was reacted at 130°C for 5 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: dichloromethane : methanol (v/v) = 10 : 1) to obtain 7e (90 mg, yield: 90.60%).

LCMS m/z = 675.4 [M+H]⁺

### Step 5: Preparation of 7f

7e (90 mg, 0.13 mmol) was dissolved in dichloromethane (2 mL). 4 M HCl-dioxane solution (2 mL) was added, and the mixture was reacted at room temperature for 16 h. The system was concentrated to dryness. 2 mL of saturated aqueous potassium carbonate solution was added, and the mixture was extracted with ethyl acetate (2 mL×3). The organic layers were combined, dried over anhydrous sodium sulphate and filtered, and the filtrate was concentrated under reduced pressure to obtain 7f (70 mg).

LCMS m/z = 574.9 [M+H]⁺

### Step 6: Preparation of compound 7

1c-1 (50 mg, 0.12 mmol) and 7f (70 mg, 0.12 mmol) were dissolved in super-dry DMF (2 mL). HATU (55 mg, 0.14 mmol) and triethylamine (0.1 mL, 0.72 mmol) were added. Under a nitrogen atmosphere, the mixture was reacted at room temperature for 16 h. The reaction solution was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 7 (30 mg, 25.50%).

LCMS m/z = 968.0 [M+H]⁺
¹H NMR (400 MHz, CF₃COOD) δ 7.77-7.29 (m, 7H),7.08-6.94 (m, 2H), 6.25-6.14 (m, 1H), 5.03-4.89 (m, 1H), 4.71-4.57 (m, 2H), 4.40-4.14 (m, 4H), 4.08-3.93 (m, 1H), 3.76-3.44 (m, 5H), 3.38-3.24 (m, 1H), 2.49 (s, 6H),2.39-1.72 (m, 14H), 1.65 (s, 3H), 1.58 (s, 3H), 1.52-1.35 (m, 3H).

### Example 8: Preparation of compound 8

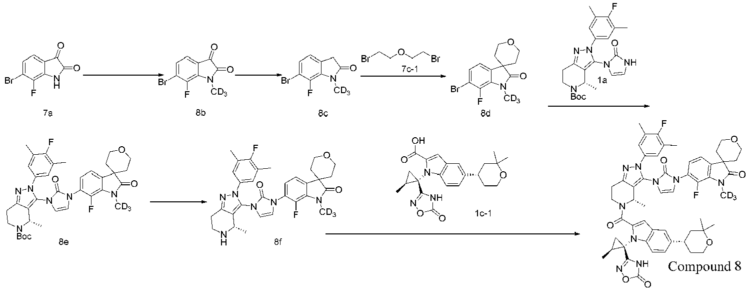

### Step 1: Preparation of 8b

7a (2.44 g, 10.0 mmol) was dissolved in super-dry DMF (10 mL). Deuterated iodomethane (2.17 g, 15.0 mmoL) and anhydrous potassium carbonate (2.76 g, 20.0 mmoL) were added. Under a nitrogen atmosphere, the mixture was reacted at 60°C for 1 h, and cooled to room temperature. 20 mL of saturated aqueous ammonium chloride solution was added, and the mixture was extracted with ethyl acetate (20 mL×3) and washed with a saturated aqueous sodium chloride solution (20 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 8b (2.4 g, yield: 91.94%).

### Step 2: Preparation of 8c

8b (2.4 g, 9.19 mmol) was dissolved in ethanol (15 mL). Hydrazine hydrate (3 mL, 80% wt) was added, and the mixture was then reacted at 130°C for 2 h, and cooled to room temperature. The reaction solution was concentrated under reduced pressure. The residue was separated and purified by column chromatography (eluent: petroleum ether : ethyl acetate (v/v) = 5 : 1) to obtain 8c (1.75 g, yield: 77.04%).

LCMS m/z =247.0, 249.0 [M+H]⁺

### Step 3: Preparation of 8d

8c (1.7 g, 6.88 mmol) was dissolved in super-dry DMF (20 mL). 7c-1 (3.19 g, 13.76 mmoL), caesium carbonate (4.48 g, 13.76 mmoL), and sodium iodide (2.06 g, 13.76 mmoL) were added. Under a nitrogen atmosphere, the mixture was reacted at 60°C for 16 h, and cooled to room temperature. 20 mL of water was added, and the mixture was extracted with ethyl acetate (20 mL×3) and washed with a saturated aqueous sodium chloride solution (20 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 8d (1.5 g, yield: 68.74%).

LCMS m/z = 317.0, 319.0 [M+H]⁺

### Step 4: Preparation of 8e

1a (100 mg, 0.23 mmol) and 8d (80 mg, 0.25 mmol) were dissolved in super-dry NMP (4 mL). CuI (40 mg, 0.21 mmol), (1S,2S)-N,N'-dimethyl-1,2-cyclohexanediamine (40 mg, 0.28 mmoL), and anhydrous potassium carbonate (150 mg, 1.09 mmol) were added. Under a nitrogen atmosphere, the mixture was reacted at 130°C for 5 h, and cooled to room temperature. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: dichloromethane : methanol (v/v) = 10 : 1) to obtain 8e (120 mg, yield: 78.17%).

LCMS m/z = 678.5 [M+H]⁺

### Step 5: Preparation of 8f

8e (120 mg, 0.18 mmol) was dissolved in dichloromethane (2 mL). 4 M HCl-dioxane solution (2 mL) was added, and the mixture was reacted at room temperature for 16 h. The system was concentrated to dryness. 2 mL of saturated aqueous potassium carbonate solution was added, and the mixture was extracted with ethyl acetate (2 mL×3). The organic layers were combined, dried over anhydrous sodium sulphate and filtered, and the filtrate was concentrated under reduced pressure to obtain 8f (90 mg).

LCMS m/z = 578.1 [M+H]⁺

### Step 6: Preparation of compound 8

1c-1 (60 mg, 0.15 mmol) and 8f (90 mg, 0.16 mmol) were dissolved in super-dry DMF (2 mL). HATU (90 mg, 0.24 mmol) and triethylamine (0.1 mL, 0.72 mmol) were added. Under a nitrogen atmosphere, the mixture was reacted at room temperature for 16 h. The reaction solution was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 8 (90 mg, 63.56%).

LCMS m/z = 971.5 [M+H]⁺
¹H NMR (400 MHz, CF₃COOD) δ 7.67-7.57 (m, 2H), 7.48-7.36 (m, 2H), 7.35-7.24 (m, 3H), 6.95-6.84 (m, 2H), 6.15-6.04 (m, 1H), 4.93-4.79 (m, 1H), 4.62-4.47 (m, 2H), 4.32-4.04 (m, 4H), 3.97-3.83 (m, 1H), 3.61-3.32 (m, 2H), 3.29-3.13 (m, 1H), 2.39 (s, 6H),2.26-2.14 (m, 2H), 2.09-1.71 (m, 12H),1.55 (s, 3H), 1.49 (s, 3H), 1.39-1.30 (m, 3H).

### Example 9: Preparation of compound 9

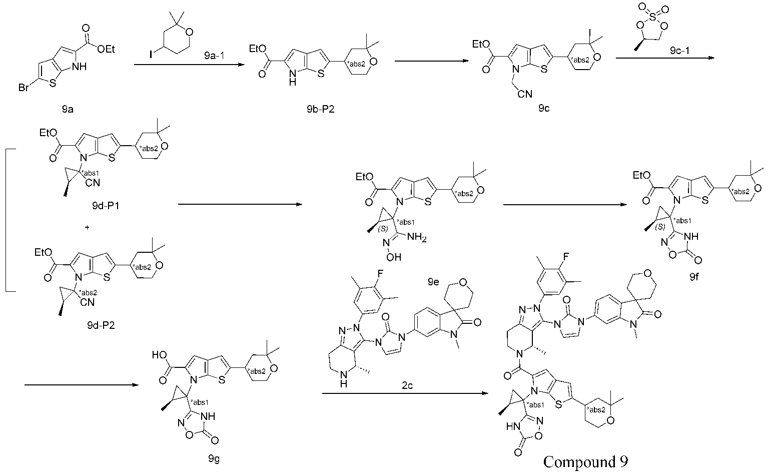

### Step 1: Preparation of 9b-P2

At room temperature, zinc powder (15.21 g, 232.58 mmol, purity: 99.999%) was dissolved in DMA (120 mL). Chlorotrimethylsilane (6.50 g, 59.85 mmol) and 1,2-dibromoethane (5.65 g, 30.08 mmol) were sequentially added dropwise, and a solution of 9a-1 (37.22 g, 155.05 mmol) in DMA (30 mL) was added slowly. Under a nitrogen atmosphere, the mixture was reacted at room temperature for 2 h. 9a (10.00 g, 31 mmol), Pd(dppf)Cl₂.CH₂Cl₂ (3.04 g, 3.72 mmol) and CuI (0.94 g, 4.96 mmol) were sequentially added to the system (under a nitrogen atmosphere). Under a nitrogen atmosphere, the mixture was reacted at 80°C for 2 h. The reaction was cooled to room temperature and quenched with a saturated aqueous ammonium chloride solution (500 mL). The system was filtered. The filter cake was washed with ethyl acetate (10 mL×3), and the filtrate was extracted with ethyl acetate (100 mL×3). The organic phases were combined, backwashed with a saturated aqueous sodium chloride solution (300 mL×2), and collected. The collected organic phase was dried over anhydrous sodium sulphate, filtered and concentrated. The crude was purified by silica gel column chromatography (eluent: petroleum ether : ethyl acetate (v/v) = 5 : 1) to obtain **racemate 9b** (4.1 g, yield: 43.01 %).

LCMS m/z =308.2 [M+H]⁺

SFC preparative conditions:
instrument: SFC Prep 150 AP; preparative column: AD (19 mm×250 mm); preparation method: the sample was dissolved in methanol and filtered through a 0.45 µm filter head to prepare a sample solution; mobile phase system: carbon dioxide/isopropanol, with methanol content of 52%; flow rate: 38 mL/min.

4.1 g of the racemate 9b was subjected to preparative SFC and concentrated to obtain compound 9b-P1 (1.86 g, retention time: 4.67 min) and compound 9b-P2 (2.08 g, retention time: 6.32 min) as white solids.

### Step 2: Preparation of 9c

9b-P2 (2.08 g, 6.77 mmol) was dissolved in DMF (25 mL). Under a nitrogen atmosphere and in an ice bath, sodium hydride (0.24 g, 10.15 mmol, purity 60%) was added in batches to the system. Under a nitrogen atmosphere and in an ice bath, the mixture was reacted for 30 minutes. Chloroacetonitrile (1.02 g, 13.54 mmol) was added dropwise, and then the mixture was slowly warmed to room temperature and reacted for 18 h. In an ice bath, the reaction was quenched with a saturated aqueous ammonium chloride solution (100 mL) and extracted with ethyl acetate (50 mL×3). The organic phases were combined, backwashed with a saturated aqueous sodium chloride solution (150 mL), and collected. The collected organic phase was dried over anhydrous sodium sulphate, filtered and concentrated to obtain a crude product. The crude product was purified by silica gel column chromatography (eluent: petroleum ether : ethyl acetate (v/v) = 5 : 1) to obtain 9c (2.13 g, yield: 90.87%).

LCMS m/z =347.1 [M+H]⁺

### Step 3: Preparation of 9d

9c (2.13 g, 6.15 mmol) and 9c-1 (2.55 g, 18.45 mmol) were dissolved in tetrahydrofuran (25 mL). In an ice bath, lithium bis(trimethylsilyl)amide (4.12 g, 24.6 mmol) was slowly added dropwise, and the mixture was reacted at 0°C for 2 h. In an ice bath, the reaction was quenched with a saturated aqueous ammonium chloride solution (50 mL) and extracted with ethyl acetate (30 mL×3). The organic phases were combined, dried over anhydrous sodium sulphate, filtered and concentrated to obtain a crude. The crude was purified by silica gel column chromatography (eluent: petroleum ether : ethyl acetate (v/v) = 5 : 1) to obtain 9d (1.65 g, yield: 69.43%).

LCMS m/z =387.2 [M+H]⁺

SFC preparative conditions:
instrument: SFC Prep 150 AP; preparative column: IC (19 mm×250 mm); preparation method: the sample was dissolved in methanol and filtered through a 0.45 µm filter head to prepare a sample solution; mobile phase system: carbon dioxide/isopropanol, with methanol content of 33%; flow rate: 40 mL/min.

9d (1.65 g) was subjected to SFC chiral resolution to obtain 9d-P1 (950 mg, retention time: 9.88 min) and 9d-P2 (600 mg, retention time: 11.78 min).

### Step 4: Preparation of 9e

Substrate 9d-P1 (913 mg, 2.36 mmol) and hydroxylamine hydrochloride (1.64 g, 23.60 mmol) were dissolved in dimethyl sulphoxide (10 mL). Sodium carbonate (2.50 g, 23.60 mmol) was added. Under a nitrogen atmosphere, the mixture was reacted at 60°C for 18 h. The reaction was cooled to room temperature, quenched with water (30 mL), and extracted with ethyl acetate (10 mL×3). The organic phases were combined, backwashed with saturated sodium chloride (30 mL), and collected. The collected organic phase was dried over anhydrous sodium sulphate, filtered and concentrated to obtain 9e (990 mg, yield: 99.90%).

LCMS m/z =420.1 [M+H]⁺

### Step 5: Preparation of 9f

9e (990 mg, 2.36 mmol) was dissolved in DMSO (10 mL). N,N'-carbonyldiimidazole (765.35 mg, 4.72 mmol) and DBU (898.22 mg, 5.90 mmol) were sequentially added. Under a nitrogen atmosphere, the mixture was reacted at room temperature for 2 h. The reaction was quenched with water (50 mL), adjusted to pH=6-7 with 1 N hydrochloric acid, and extracted with ethyl acetate (30 mL×3). The organic phases were combined, backwashed with a saturated aqueous sodium chloride solution (100 mL), and collected. The collected organic phase was dried over anhydrous sodium sulphate, filtered and concentrated to obtain a crude. The crude was purified by silica gel column chromatography (eluent: dichloromethane : ethyl acetate (v/v) = 5 : 1) to obtain 9f (624 mg, yield: 59.35%).

LCMS m/z =444.2 [M-H]⁻

### Step 6: Preparation of 9g

Substrate 9f (622 mg, 1.40 mmol) was dissolved in DMSO (7 mL). Sodium hydroxide (168.00 mg, 4.20 mmol) was prepared as a 2 M aqueous solution and added dropwise to the system. After the addition, the mixture was reacted at 30°C for 16 h. The reaction was quenched by adding water (30 mL) to the reaction system, and adjusted to pH=1-2 by adding 1 N hydrochloric acid dropwise. The aqueous phase was extracted with ethyl acetate (30 mL×3). The organic phases were combined, backwashed with saturated sodium chloride (100 mL), and collected. The collected organic phase was dried over anhydrous sodium sulphate, filtered and concentrated to obtain 9g (582 mg, yield: 99.86%).

LCMS m/z =416.2 [M-H]⁻

### Step 7: Preparation of compound 9

9g (41.75 mg, 0.1 mmol) was dissolved in DMF (3 mL). 2c (67 mg, 0.12 mmol), HATU (57 mg, 0.15 mmol) and DIPEA (130 mg, 1 mmol) were sequentially added. Under a nitrogen atmosphere, the mixture was reacted at 60°C for 24 h. In an ice bath, the reaction was quenched with water (30 mL), and extracted with ethyl acetate (10 mL×3). The organic phases were combined, backwashed with a saturated aqueous sodium chloride solution (30 mL), and collected. The collected organic phase was dried over anhydrous sodium sulphate, filtered and concentrated to obtain a crude. The crude was separated and purified by preparative liquid chromatography (instrument: waters 2767 preparative liquid chromatographic instrument; chromatographic column: SunFire@ Prep C18 (19 mm×150 mm); composition of mobile phases: mobile phase A: acetonitrile, and mobile phase B: water (containing 0.1% TFA)) and subjected to lyophilisation to obtain compound 9 (21 mg, yield: 21.96%).

LCMS m/z = 956.3 [M+H]⁺
¹H NMR (400 MHz, CF₃COOD) δ 7.65 (d, 1H), 7.38 - 7.22 (m, 3H), 7.21 - 7.13 (m, 1H), 7.06 - 6.97 (m, 1H), 6.95 - 6.78 (m, 3H), 6.10 - 5.97 (m, 1H), 5.03 - 4.86 (m, 1H), 4.62 - 4.48 (m, 2H), 4.33 - 4.20 (m, 2H), 4.19 - 4.01 (m, 2H), 3.96 - 3.77 (m, 1H), 3.53 - 3.28 (m, 6H), 2.38 (s, 6H), 2.24 - 1.77 (m, 11H), 1.72 (d, 3H), 1.51 (s, 3H), 1.48 (s, 3H), 1.33 (d, 3H).

### Example 10: Preparation of compound 10

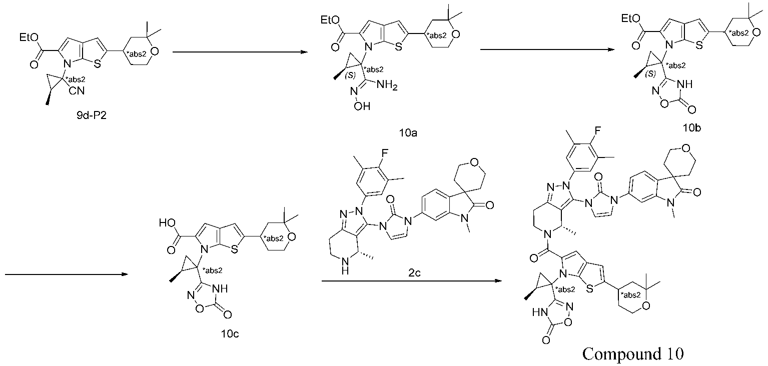

With reference to the synthetic method of compound 9, compound 10 (15 mg, yield: 15.69%) was obtained.

LCMS m/z = 956.3 [M+H]⁺
¹H NMR (400 MHz, CF₃COOD) δ 7.65 (d, 1H), 7.37-7.22 (m, 3H), 7.20-7.12 (m, 1H), 7.05-6.79 (m, 4H), 6.40-5.97 (m, 1H), 5.34-4.93 (m, 1H), 4.59-4.49 (m, 2H), 4.30-4.21 (m, 2H), 4.19-4.02 (m, 2H), 4.01-3.82 (m, 1H),3.53-3.06 (m, 7H), 2.37 (s, 6H), 2.31-1.72 (m, 11H), 1.71-1.59 (m, 3H), 1.51 (s, 3H) ), 1.48 (s, 3H), 1.30-1.14 (m, 3H).

### Example 11: Preparation of compound 11

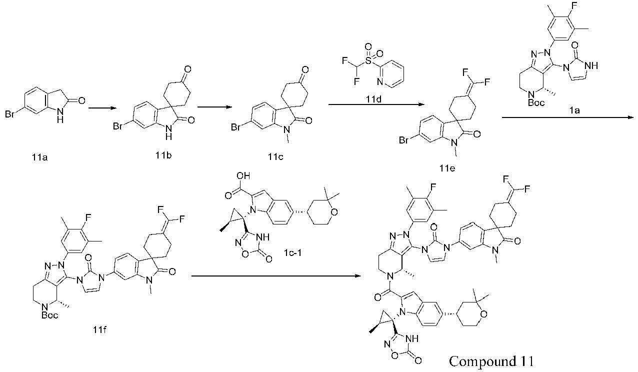

### Step 1: Preparation of 11b

11a (10.6 g, 50 mmol) was dissolved in super-dry DMSO (60 mL). Potassium tert-butoxide (5 mL, 1 M in THF) was added at room temperature, and the mixture was reacted for 30 minutes. A mixture of ethyl acrylate (13.6 mL, 125 mmol) and DMSO (10 mL) was added and reacted at 45°C for 3 h. Potassium tert-butoxide (5 mL, 1 M in THF) was added dropwise, and the mixture was reacted at 60°C for 3 h. Water (200 mL) was added, and the mixture was reacted at 80°C for 18 h. The reaction was cooled to room temperature, and extracted with ethyl acetate (200 mL×3). The organic phases were combined, washed with a saturated aqueous sodium chloride solution, dried over anhydrous sodium sulphate and then filtered. The filtrate was concentrated under reduced pressure. The residue was separated and purified by column chromatography (eluent: petroleum ether : ethyl acetate (v/v) = 5 : 1) to obtain 11b (6.18 g, yield: 42.2%).

LCMS m/z = 294.0, 296.0 [M+H]⁺

### Step 2: Preparation of 11c

11b (6.18 g, 21.12 mmol), iodomethane (6.0 g, 42.24 mmol) and caesium carbonate (13.77 g, 42.24 mmol) were dissolved in acetonitrile (200 mL). Under a nitrogen atmosphere, the mixture was reacted at 70°C for 16 h, cooled to room temperature, and filtered. The filtrate was concentrated under reduced pressure. The residue was separated and purified by column chromatography (eluent: petroleum ether : ethyl acetate (v/v) = 5 : 1) to obtain 11c (5.9 g, yield: 91%).

LCMS m/z = 308.0, 310.0 [M+H]⁺

### Step 3: Preparation of 11e

11c (3.07 g, 10 mmol) and 11d (960 mg, 5.0 mmol) were dissolved in DMF (40 mL). Hexamethylphosphoric triamide (4 mL) was added, and the mixture was purged with nitrogen, and then stirred evenly at -60°C. To the system was slowly added dropwise LiHMDS (10 mL, 1 M in THF), and the mixture was further reacted at this temperature for 1 h. To the system was added 2 M hydrochloric acid (10 mL), and the mixture was stirred at room temperature for 30 minutes. Then, to the system was added 12 M hydrochloric acid (15 mL), and the mixture was reacted at 130°C for 10 h. The reaction was cooled to room temperature, and extracted with ethyl acetate (100 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 5 : 1) to obtain 11e (610 mg, yield: 17.9%).

LCMS m/z =342.0, 344.0 [M+H]⁺

### Step 4: Preparation of 11f

1a (110 mg, 0.25 mmol) and 11e (102 mg, 0.3 mmol) were dissolved in NMP (6 mL). CuI (71.6 mg, 0.374 mmol), (1S,2S)-N,N'-dimethyl-1,2-cyclohexanediamine (53.2 mg, 0.374 mmoL), and anhydrous potassium carbonate (103 mg, 0.74 mmol) were added. Under a nitrogen atmosphere, the mixture was reacted at 130°C for 4 h, and cooled to room temperature. 20 mL of water was added to the reaction solution, and the mixture was extracted with ethyl acetate (30 mL×3), and washed with a saturated aqueous sodium chloride solution (50 mL). The organic layers were combined, dried over anhydrous sodium sulphate and filtered, and the filtrate was concentrated under reduced pressure. The residue was separated and purified by column chromatography (dichloromethane : ethyl acetate (v/v) = 30 : 1) to obtain 11f (153 mg, yield: 87.1%).

LCMS m/z =703.3 [M+H]⁺

### Step 5: Preparation of compound 11

11f (153 mg, 0.218 mmol) was dissolved in dichloromethane (2 mL). 4 M HCl-dioxane solution (4 mL) was added, and the mixture was reacted at room temperature for 3 h. The system was concentrated to dryness. The resulting residue was dissolved in DMF (4 mL). 1c-1 (89 mg, 0.218 mmol), HATU (114 mg, 0.3 mmol) and DIPEA (51.6 mg, 0.4 mmol) were added, and the mixture was reacted at room temperature for 18 h. In an ice bath, the reaction was quenched with water (10 mL), and extracted with ethyl acetate (20 mL×3). The organic phases were combined, washed with a saturated aqueous sodium chloride solution (30 mL), and collected. The collected organic phase was dried over anhydrous sodium sulphate, filtered and concentrated to obtain a crude. The crude was separated and purified by preparative liquid chromatography (instrument: waters 2767 preparative liquid chromatographic instrument; chromatographic column: SunFire@ Prep C18 (19 mm × 150 mm); composition of mobile phases: mobile phase A: acetonitrile, and mobile phase B: water (containing 0.1% TFA)) and subjected to lyophilisation to obtain compound 11 (57 mg, yield: 26.2%).

LCMS m/z = 996.3 [M+H]⁺
¹H NMR (400 MHz, CF₃COOD) δ 7.80-7.60 (m, 3H), 7.55-7.29 (m, 4H), 7.26 (s, 1H), 7.11 (d, 1H), 7.00 (d, 1H), 6.30-6.06 (m, 1H), 5.02-4.84 (m, 1H), 4.33-4.11 (m, 2H), 4.07-3.91 (m, 1H), 3.70-3.55 (m, 1H), 3.54-3.39 (m, 4H), 3.37 - 3.22 (m, 1H), 2.88-2.75 (m, 2H), 2.62-2.50 (m, 2H), 2.46 (s, 6H), 2.21-1.70 (m, 15H), 1.63 (s, 3H) ), 1.57 (s, 3H), 1.42 (d, 3H).

### Example 12: Preparation of compound 12

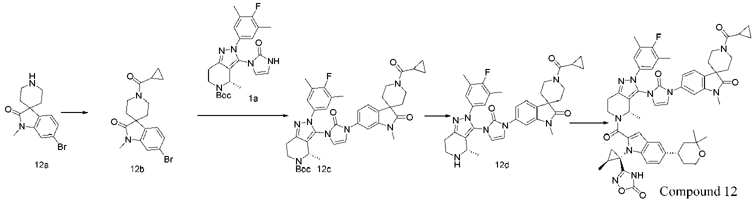

### Step 1: Preparation of 12b

12a (CAS: 1552637-88-3,80 mg, 0.27 mmol) was dissolved in super-dry DMF (2 mL). HATU (154 mg, 0.41 mmol), N,N-diisopropylethylamine (70 mg, 0.54 mmol), and cyclopropanecarboxylic acid (30.22 mg, 0.35 mmol) were added. Under a nitrogen atmosphere, the mixture was reacted at room temperature for 16 h. 10 mL of water was added, and the mixture was extracted with ethyl acetate (10 mL×3) and washed with a saturated aqueous sodium chloride solution (10 mL×3). The organic layers were combined, dried over anhydrous sodium sulphate and filtered, and the filtrate was concentrated under reduced pressure. The residue was separated and purified by column chromatography (eluent: petroleum ether : ethyl acetate (v/v) = 3 : 1) to obtain 12b (98 mg, yield: 99.54%).

With reference to the synthetic method of compound 1, steps 2 to 4 were carried out to obtain compound 12 (10 mg).

LCMS m/z = 509.4 [M+2H]²⁺

### Example 13: Preparation of compound 13

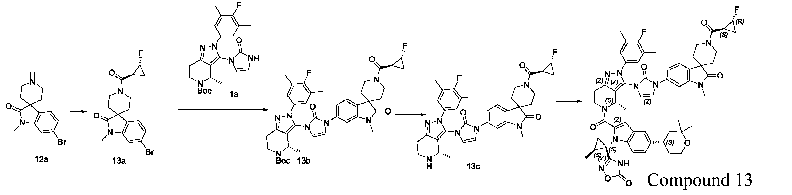

With reference to the synthetic method of compound 12, compound 13 (10 mg) was obtained.

LCMS m/z = 518.3 [M+2H]²⁺

### Example 14: Preparation of compound 14

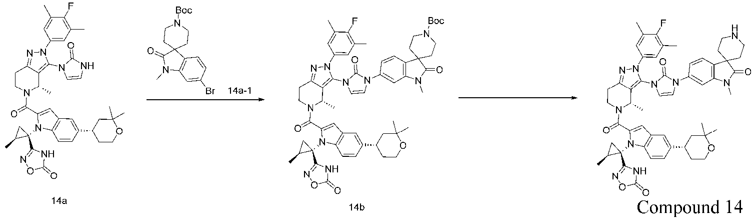

### Step 1: Preparation of 14b

14a (CAS: 2212020-29-4, 1.8 g, 2.45 mmol) was dissolved in NMP (20 mL). 14a-1 (CAS: 2491752-99-7, 1.02 g, 2.57 mmol), CuI (699.90 mg, 3.68 mmol), (1S,2S)-(+)-N,N-dimethylcyclohexanediamine (522.73 mg, 3.68 mmol) and potassium carbonate (1.02 g, 7.35 mmol) were sequentially added. Under a nitrogen atmosphere, the mixture was reacted at 130°C for 3 h, and cooled to room temperature. Water (100 mL) was added, and the mixture was filtered. The filter cake was washed with ethyl acetate (10 mL×2), and the filtrate was collected and extracted with ethyl acetate (40 mL×3). The organic phases were combined, backwashed with a saturated aqueous sodium chloride solution (150 mL), and collected. The collected organic phase was dried over anhydrous sodium sulphate, filtered and concentrated to obtain a crude. The crude was purified by silica gel column chromatography (eluent: dichloromethane : ethyl acetate (v/v) = 30 : 1) to obtain 14b (1.27 g, yield: 49.68%).

### Step 2: Preparation of compound 14

Substrate 14b (1.27 g, 1.21 mmol) was dissolved in dichloromethane (10 mL). 4 M HCl in 1,4-dioxane (10 mL) was added dropwise, and the mixture was reacted at room temperature for 2 h. The reaction solution was concentrated under reduced pressure to obtain a hydrochloride crude. The crude was dissolved in water (20 mL). In an ice bath, the mixture was adjusted to pH=7-8 by slowly adding saturated sodium bicarbonate, and extracted with dichloromethane : methanol (10 : 1, 30 mL×3). The organic phases were combined, dried over anhydrous sodium sulphate, filtered and concentrated to obtain compound 14 (975 mg, yield: 84.87%).

LCMS m/z = 949.5 [M+H]⁺
¹H NMR (400 MHz, CF₃COOD) δ7.73-7.17 (m, 8H), 7.04 (s, 1H), 7.92 (s, 1H), 6.13 (s, 1H), 4.87 (s, 1H), 4.37-4.05 (m, 4H), 4.01-4.85 (m, 1H), 3.74 (d, 2H), 3.67-3.30 (m, 5H), 3.27-3.11 (m, 1H), 2.63-2.48 (m, 2H), 2.39 (s, 6H), 2.19 (d, 2H), 2.11-1.65 (m, 10H), 1.55 (s, 3H), 1.54-1.42 (m, 4H), 1.33 (m, 3H).

### Example 15: Preparation of compound 15

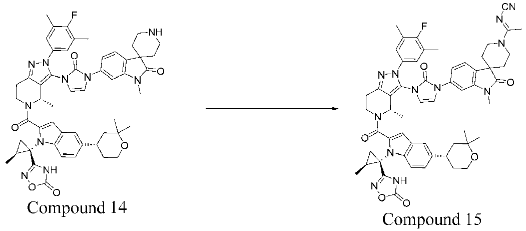

Compound 14 (40.81 mg, 0.043 mmol) was dissolved in methanol (3 mL). Methyl N-cyanoethanimidate (cas: 5652-84-6,6.3 mg, 0.065 mmol) and DBU (7.9 mg, 0.052 mmol) were added. After the addition, the mixture was reacted at 65°C under a nitrogen atmosphere for 2 h. The reaction was concentrated to obtain a crude. The crude was purified by silica gel column chromatography (eluent: dichloromethane : methanol (v/v) = 30 : 1) to obtain compound 15 (14.5 mg, yield: 28.90%).

LCMS m/z = 508.4 [M+2H]²⁺
¹H NMR (400 MHz, CF₃COOD) δ 7.66-7.57 (m, 2H), 7.56-7.48 (m, 1H), 7.43-7.37 (m, 1H), 7.36-7.25 (m, 4H), 7.07-7.00 (m, 1H), 6.92-6.87 (m, 1H), 6.15-6.05 (m, 1H), 4.92-4.80 (m, 1H), 4.79-4.57 (m, 1H), 4.52-4.29 (m, 2H), 4.23-4.05 (m, 2H), 3.97-3.82 (m, 1H), 3.58-3.42 (m, 2H), 3.40 (s, 3H), 3.29-3.17 (m, 1H), 3.06-2.94 (m, 3H), 2.61-2.41 (m, 2H), 2.38 (s, 6H), 2.33-2.16 (m, 2H), 2.09-1.76 (m, 8H), 1.73 (d, 3H), 1.55 (s, 3H), 1.48 (s, 3H), 1.33 (d, 3H).

### Example 16: Preparation of compound 16

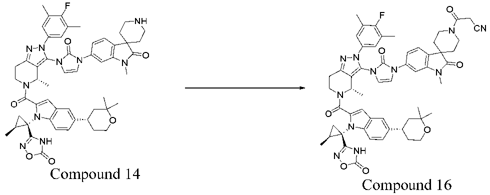

Compound 14 (20 mg, 0.02 mmol) was dissolved in DMF (2 mL). Cyanoacetic acid (cas: 372-09-8, 2.5 mg, 0.03 mmol), DIPEA (4 mg, 0.03 mmol) and HATU (11.4 mg, 0.03 mmol) were added. After the addition, the mixture was reacted at room temperature for 2 h. The reaction was concentrated to obtain a crude. The crude was purified by silica gel column chromatography (eluent: dichloromethane : ethyl acetate (v/v) = 30 : 1) to obtain compound 16 (9 mg, yield: 44.33%).

LCMS m/z = 1016.6 [M+H]⁺
¹H NMR (400 MHz, CF₃COOD) δ7.70-7.50 (m, 3H), 7.48-7.14 (m, 5H), 7.02 (d, 1H), 6.92 (s, 1H), 6.12 (s, 1H), 4.95-4.76 (m, 1H), 4.49-4.24 (m, 2H), 4.23-4.04 (m, 3H), 4.00-3.82 (m, 2H), 3.64-3.33 (m, 5H), 3.28-3.16 (m, 1H), 2.38 (s, 6H), 2.25-1.84 (m, 11H), 1.83-1.66 (m, 4H), 1.55 (s, 3H), 1.54-1.43 (m, 4H), 1.42-1.27 (m, 3H).

### Example 17: Preparation of compound 17

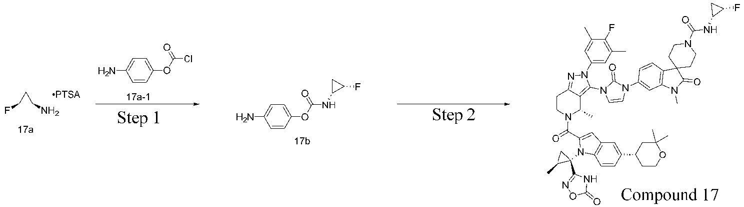

### Step 1: Preparation of compound 17b

Compound 17a (494 mg, 2.0 mmol) was dissolved in dichloromethane (20 mL). Under a nitrogen atmosphere and at -20°C, triethylamine (607 mg, 6.0 mmol) and 17a-1 (483.6 mg, 2.4 mmol) were added. After the addition, the mixture was further reacted at this temperature for 2 h. The reaction was concentrated to obtain a crude. The crude was purified by silica gel column chromatography (eluent: petroleum ether : ethyl acetate (v/v) = 3 : 1) to obtain compound 17b (201 mg, yield: 41.8%).

LCMS m/z =241.1 [M+H]⁺

### Step 2: Preparation of compound 17

Compound 14 (35 mg, 0.037 mmol), 17b (12 mg, 0.05 mmol) and pyridine (3 mL) were added to a sealed tube, and the mixture was reacted at 106°C for 3 h. The reaction solution was cooled to room temperature, and concentrated under reduced pressure to remove the solvent. The residue was separated and purified by silica gel column chromatography (eluent: dichloromethane : ethyl acetate (v/v) = 30 : 1) to obtain compound 17 (19 mg, yield: 48.95%)
LCMS m/z =1050.4[M+H]⁺
¹H NMR (400 MHz, CF₃COOD) δ 7.68-7.49 (m, 3H), 7.19 (d, 1H), 7.35-7.27 (m, 3H), 7.24 (s, 1H), 7.03 (d, 1H), 6.90(d, 1H), 6.19-6.03 (m, 1H), 4.97-4.71 (m, 2H), 4.31-3.98(m, 6H), 3.90 (t, 1H), 3.59-3.47 (m, 1H), 3.46-3.32 (m, 4H), 3.29-3.15 (m, 1H), 2.96-2.86 (m, 1H), 2.38 (s, 6H), 2.24-2.09 (m, 4H), 2.03 (t, 1H), 1.99-1.80 (m, 6H), 1.74 (d, 3H), 1.55 (s, 3H), 1.49 (s, 3H), 1.45-1.36 (m, 1H), 1.33 (d, 3H), 1.32-1.22 (m, 1H).

### Example 20: Preparation of compound 20

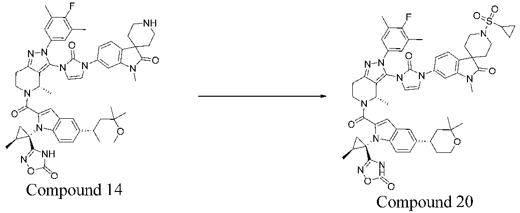

Compound 14 (40 mg, 0.042 mmol) was dissolved in dichloromethane (2 mL). Triethylamine (8.5 mg, 0.084 mmol) was added. In an ice bath, cyclopropanesulphonyl chloride (7 mg, 0.05 mmol) was added. The mixture was reacted in an ice bath for 2 h. The reaction was concentrated to obtain a crude. The crude was purified by silica gel column chromatography (eluent: dichloromethane : ethyl acetate (v/v) = 30 : 1) to obtain compound 20 (25 mg, yield: 56.6%).

LCMS m/z = 527.3 [M+2H]²⁺
¹H NMR (400 MHz, CF₃COOD) δ 7.69-7.50 (m, 3H), 7.40 (d, 1H), 7.36-7.23 (m, 3H), 7.20 (s, 1H), 7.02 (d, 1H), 6.91 (d, 1H), 6.19-6.02 (m, 1H), 4.96-4.76 (m, 1H), 4.23-4.06 (m, 2H), 3.99 (t, 2H), 3.89-3.80 (m, 2H), 3.62-3.40 (m, 2H), 3.38 (s, 3H), 3.28-3.15 (m, 1H), 2.72-2.59 (m, 1H), 2.38 (s, 6H), 2.25-2.15 (m, 2H), 2.12-2.01 (m, 3H), 1.98-1.84 (m, 5H), 1.78-1.70 (m, 3H), 1.55 (s, 3H), 1.48 (s, 3H), 1.39-1.32 (m, 7H), 1.30-1.25 (m, 2H).

Starting from compound 14, the following compounds were synthesised under similar synthetic conditions as described in the above examples.

| Compound | Structure | LCMS m/z | ¹H NMR |
|---|---|---|---|
| Compound 18 | 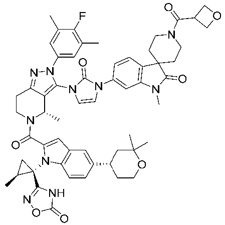 | 1033.4[M+H]⁺ | |
| Compound 19 | 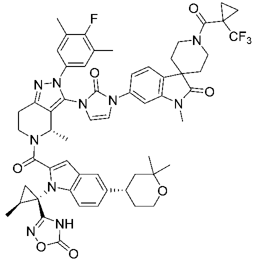 | 1185.6[M+H]⁺ | (400 MHz, CF₃COOD) δ 7.68-7.49 (m, 3H), 7.40 (d, 1H), 7.35-7.25 (m, 3H), 7.23 (s, 1H), 7.03 (d, 1H), 6.91 (d, 1H), 6.18-6.06 (m, 1H), 4.97-4.77 (m, 1H), 4.53-4.33 (m, 3H), 4.27-4.06 (m, 3H), 3.90 (t, 1H), 3.59-3.47 (m, 1H), 3.46-3.33 (m, 4H), 3.28-3.16 (m, 1H), 2.38 (s, 6H), 2.24-2.08 (m, 4H), 2.04 (t, 1H), 2.00-1.78 (m, 6H), 1.74 (d, 3H), 1.69-1.60 (m, 2H), 1.55 (s, 3H), 1.51-1.44 (m, 5H), 1.34 (d, 3H). |
| Compound 21 | 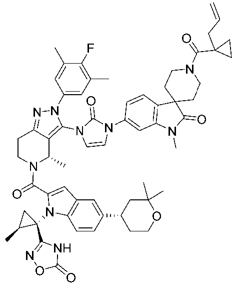 | 1057.6[M+H]⁺ | (400 MHz, CF₃COOD) δ 7.69-7.45 (m, 3H), 7.40 (d, 1H), 7.37-7.28 (m, 3H), 7.27 (s, 1H), 7.04 (d, 1H), 7.90 (d, 1H), 6.17-6.04 (m, 1H), 6.02-1.86 (m, 1H), 5.42-5.30 (m, 2H), 4.93-4.80 (m, 1H), 4.70-4.50 (m, 2H), 4.50 (t, 1H), 4.37-4.23 (m, 2H), 3.90 (t, 1H), 3.59-3.47 (m, 1H), 3.46-3.33 (m, 4H), 3.3.28-3.17 (m, 1H), 2.68-2.48 (m, 2H), 2.38 (s, 6H), 2.34-2.16 (m, 4H), 2.03 (t, 1H), 2.00-1.76 (m, 6H), 1.74 (d, 3H), 1.55 (s, 3H), 1.52-1.45 (m, 4H), 1.44-1.37 (m, 2H), 1.33 (d, 3H), 1.31-1.24 (m, 2H). |
| Compound 22 | 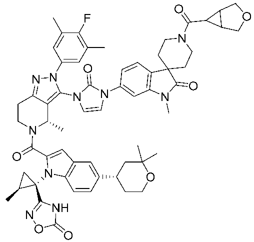 | 1059.3[M+H]⁺ | (400 MHz, CF₃COOD) δ 7.66-7.53 (m, 3H), 7.40 (d, 1H), 7.35-7.26 (m, 3H), 7.23 (s, 1H), 7.03 (d, 1H), 6.91 (d, 1H), 6.17-6.04 (m, 1H), 4.96-4.79 (m, 1H), 4.47-4.27 (m, 6H), 4.22-4.06 (m, 4H), 3.97-3.83 (m, 1H), 3.61-3.48 (m, 1H), 3.45-3.36 (m, 4H), 3.30-3.15 (m, 1H), 2.66 (s, 2H), 2.44-2.29 (m, 7H), 2.22-2.09 (m, 4H), 2.03 (t, 1H), 2.00-1.78 (m, 6H), 1.74 (d, 3H), 1.55 (s, 3H), 1.49 (s, 3H), 1.33 (d, 3H). |
| Compound 23 | 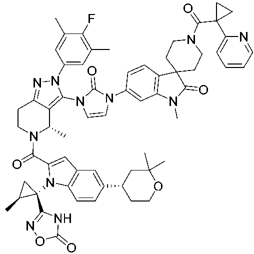 | 1094.6[M+H]⁺ | (400 MHz, CF₃COOD) δ8.82 (d, 1H), 8.70 (t, 1H), 8.17-8.01 (m, 2H), 7.68-7.46 (m, 3H), 7.39 (d, 1H), 7.36-7.25 (m, 3H), 7.23 (s, 1H), 7.03 (d, 1H), 6.89 (d, 1H), 6.17-6.03 (m, 1H), 4.98-4.76 (m, 1H), 4.47-4.04 (m, 6H), 3.90 (t, 1H), 3.60-3.47 (m, 1H), 3.46-3.30 (m, 4H), 3.28-3.15 (m, 1H), 2.38 (s, 6H), 2.19-1.77 (m, 15H), 1.73 (d, 3H), 1.55 (s, 3H), 1.49 (s, 3H) ), 1.33 (d, 3H). |
| Compound 24 | 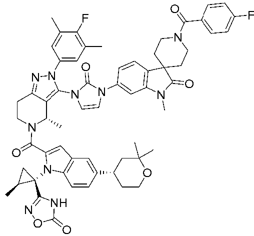 | 1071.6[M+H]⁺ | (400 MHz, CF3COOD) δ 7.79-7.71 (m, 2H), 7.61 (t, 3H), 7.44-7.29 (m, 6H), 7.26 (s, 1H), 7.04 (d, 1H), 6.91 (d, 1H), 6.17-6.04 (d, 1H), 4.96-4.79 (m, 1H), 4.78-4.61 (m, 1H), 4.60-4.42 (m, 2H), 4.23-4.03 (m, 3H), 3.96-3.83 (m, 1H), 3.61-3.48 (m, 1H), 3.46-3.36 (m, 4H), 3.28-3.16 (m, 1H), 2.42-2.32 (m, 6H), 2.30-2.11 (m, 3H), 2.06-2.00 (m, 1H), 1.99-1.77 (m, 7H), 1.74 (d, 3H), 1.55 (s, 3H), 1.49 (s, 3H), 1.34 (d, 3H). |
| Compound 25 | 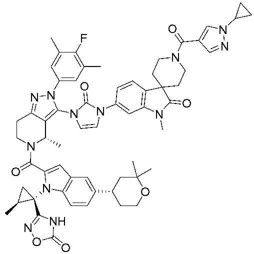 | 1083.6[M+H]⁺ | |
| Compound 26 | 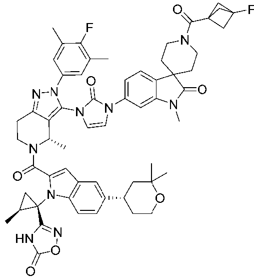 | 531.3[M+2H]^{2 +} | (400 MHz, CF₃COOD) δ 7.67-7.59 (m, 2H), 7.55 (d, 1H), 7.40 (d, 1H), 7.35-7.27 (m, 3H), 7.24 (s, 1H), 7.07-7.00 (m, 1H), 6.93-6.87 (m, 1H), 6.16-6.05 (m, 1H), 4.93-4.79 (m, 1H), 4.63-4.01 (m, 6H), 3.96-3.83 (m, 1H), 3.60-3.41 (m, 2H), 3.39 (s, 3H), 3.29-3.15 (m, 1H), 2.74 (s, 6H), 2.38 (s, 6H), 2.26-2.08 (m, 4H), 2.06-1.71 (m, 10H), 1.55 (s, 3H) ), 1.49 (s, 3H), 1.34 (d, 3H). |
| Compound 27 | 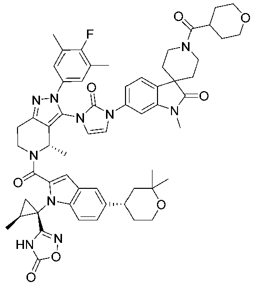 | 531.3[M+2H]^{2 +} | (400 MHz, CF₃COOD) δ 7.65-7.59 (m, 2H), 7.56 (d, 1H), 7.40 (d, 1H), 7.34-7.26 (m, 3H), 7.23 (s, 1H), 7.03 (d, 1H), 6.91 (d, 1H), 6.15-6.05 (m, 1H), 4.93-4.80 (m, 1H), 4.51-4.22 (m, 5H), 4.21-4.08 (m, 2H), 3.95-3.75 (m, 3H), 3.60-3.42 (m, 2H), 3.39 (s, 3H), 3.36-3.15 (m, 2H), 2.38 (s, 6H), 2.30-2.08 (m, 6H), 2.06-1.77 (m, 10H), 1.74 (d, 3H), 1.55 (s, 3H), 1.48 (s, 3H), 1.34 (d, 3H). |
| Compound 28 | 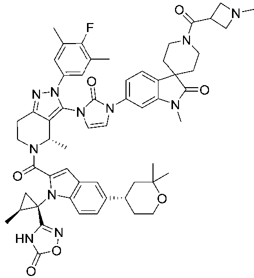 | 963.4[M+H]⁺ | (400 MHz, CF₃COOD) δ 7.66-7.58 (m, 2H), 7.54 (d, 1H), 7.40 (d, 1H), 7.34-7.26 (m, 3H), 7.25-7.20 (m, 1H), 7.06-7.00 (m, 1H), 6.92-6.86 (m, 1H), 6.16-6.05 (m, 1H), 4.96-4.78 (m, 3H), 4.65-4.34 (m, 2H), 4.28-4.04 (m, 5H), 3.98-3.73 (m, 2H), 3.61-3.41 (m, 2H), 3.39 (s, 3H), 3.26-3.15 (m, 4H), 2.38 (s, 6H), 2.24-2.08 (m, 2H), 2.07-1.78 (m, 10H), 1.74 (d, 3H), 1.055 (s, 3H), 1.48 (s, 3H), 1.33(d, 3H). |
| Compound 29 | 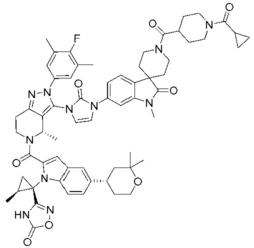 | 564.8[M+2H]^{2 +} | (400 MHz, CF₃COOD) δ 7.66-7.54 (m, 3H), 7.40 (d, 1H), 7.36-7.27 (m, 3H), 7.24 (s, 1H), 7.04 (d, 1H), 6.90 (d, 1H), 6.15-6.05 (m, 1H), 4.91-4.80 (m, 1H), 4.79-4.65 (m, 2H), 4.38-4.25 (m, 2H), 4.21-4.06 (m, 3H), 3.96-3.82 (m, 1H), 3.71-3.42 (m, 5H), 3.40 (s, 3H), 3.28-3.17(m, 1H), 2.38 (s, 6H), 2.31-2.14 (m, 7H), 2.13-2.00 (m, 4H), 1.99-1.77 (m, 6H), 1.74 (d, 3H), 1.55 (s, 3H), 1.49 (s, 3H), 1.46-1.39 (m, 4H), 1.34 (d, 3H). |
| Compound 30 | 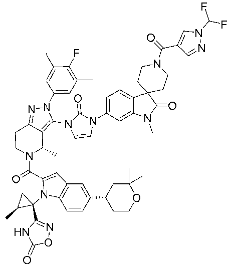 | 547.3[M+2H]^{2 +} | |
| Compound 32 | 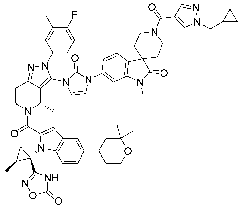 | 1097.6[M+H]⁺ | (400 MHz, CF₃COOD) δ 8.74 (s, 1H), 8.63 (s, 1H), 7.67-7.53 (m, 3H), 7.40 (d, 1H), 7.36-7.28 (m, 3H), 7.25 (s, 1H), 7.03 (d, 1H), 6.90 (d, 1H), 6.16-6.06 (m, 1H), 4.94-4.81 (m, 1H), 4.57-4.30 (m, 4H), 4.23-4.01 (m, 3H), 3.96-3.82 (m, 1H), 3.61-3.47 (m, 1H), 3.45-3.35 (m, 4H), 3.29-3.15 (m, 1H), 2.43-2.32 (m, 7H), 2.24-2.09 (m, 4H), 2.03 (t, 1H), 1.99-1.84 (m, 6H), 1.83-1.77 (m, 1H), 1.74 (d, 3H), 1.55 (s, 3H), 1.49 (s, 3H), 1.33 (d, 3H), 1.03-0.92 (m, 2H), 0.70-0.59 (m, 2H). |
| Compound 33 | 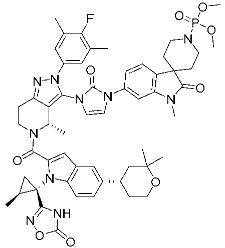 | 529.5[M+2H]^{2 +} | (400 MHz, CF₃COOD) δ 7.66-7.49 (m, 3H), 7.40 (d, 1H), 7.37-7.21 (m, 4H), 7.04 (s, 1H), 7.90 (s, 1H), 6.17-6.04 (m, 1H), 4.97-4.73 (m, 1H), 4.33-4.07 (m, 4H), 3.97 (s, 3H), 3.94 (s, 3H), 3.92-3.82 (m, 1H), 3.78-3.64 (m, 2H), 3.59-3.47 (m, 1H), 3.46-3.32 (m, 4H), 3.31-3.15 (m, 1H), 2.60-2.45 (m, 2H), 2.38 (s, 6H), 2.20 (d, 2H), 2.03 (t, 1H), 2.00-1.76 (m, 7H), 1.74 (d, 3H), 1.54 (s, 3H), 1.48 (s, 3H), 1.35-1.33 (m, 3H). |
| Compound 34 | 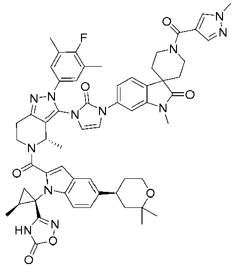 | 523.9[M+2H]^{2 +} | (400 MHz, CF₃COOD) δ 8.58 (d, 2H), 7.66-7.52 (m, 3H), 7.40 (d, 1H), 7.36-7.27 (m, 3H), 7.25 (s, 1H), 7.03 (s, 1H), 6.90 (s, 1H), 6.17-6.05 (m, 1H), 4.95-4.79 (m, 1H), 4.54-4.38 (m, 2H), 4.36 (s, 3H), 4.22-4.04 (m, 3H), 3.96-3.83 (m, 1H), 3.64-3.42 (m, 2H), 3.40 (s, 3H), 3.29-3.16 (m, 1H), 2.38 (s, 6H), 2.24-2.07 (m, 4H), 2.06-2.00 (m, 1H), 1.99-1.77 (m, 7H), 1.76-1.67 (m, 3H), 1.55 (s, 3H), 1.48 (s, 3H), 1.34 (d, 3H). |
| Compound 35 | 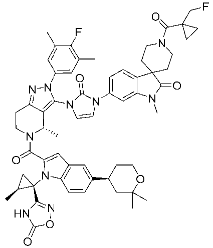 | 525.3[M+2H]^{2 +} | |
| Compound 36 | 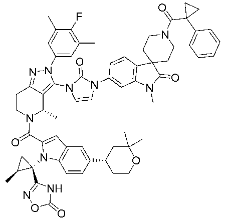 | 547.3[M+2H]^{2 +} | (400 MHz, CF₃COOD) δ 7.68-7.57 (m, 2H), 7.56-7.43 (m, 3H), 7.43-7.35 (m, 3H), 7.32-7.25 (m, 4H), 7.21 (s, 1H), 7.02 (d, 1H), 6.89 (d, 1H), 6.15-6.05 (m, 1H), 4.96-4.78 (m, 1H), 4.68-4.33 (m, 4H), 4.25-4.05 (m, 2H), 3.96-3.81 (m, 1H), 3.59-3.47 (m, 1H), 3.43 (s, 1H), 3.36 (s, 3H), 3.27-3.16 (m, 1H), 2.55-2.09 (m, 9H), 2.07-2.00 (m, 1H), 2.00-1.85 (m, 7H), 1.81-1.67 (m, 6H), 1.65-1.58 (m, 1H), 1.55 (s, 3H), 1.49 (s, 3H), 1.33 (d, 3H). |
| Compound 37 | 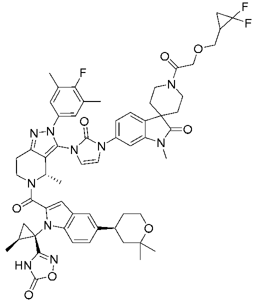 | 549.3[M+2H]^{2 +} | (400 MHz, CF₃COOD) δ 7.67-7.57 (m, 2H), 7.52 (d, 1H), 7.40 (d, 1H), 7.36-7.28 (m, 3H), 7.26 (s, 1H), 7.04 (d, 1H), 6.90 (d, 1H), 6.15-6.06 (m, 1H), 4.94-4.70 (m, 3H), 4.49-4.31 (m, 2H), 4.28-4.06 (m, 3H), 3.99-3.83 (m, 2H), 3.77-3.60 (m, 1H), 3.58-3.42 (m, 2H), 3.40 (s, 3H), 3.29-3.16 (m, 1H), 2.38 (s, 6H), 2.35-2.12 (m, 4H), 2.10-1.76 (m, 9H), 1.74 (d, 3H), 1.70-1.60 (m, 1H), 1.55 (s, 3H), 1.49 (s, 3H), 1.33 (d, 3H), 1.30-1.18 (m, 1H). |
| Compound 38 | 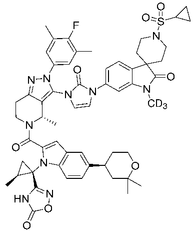 | 529.0[M+2H]^{2 +} | δ 7.67-7.59 (m, 2H), 7.58-7.52 (m, 1H), 7.45-7.23 (m, 4H), 7.22-7.17 (m, 1H), 7.06-7.00 (m, 1H), 7.96-6.86 (m, 1H), 6.18-6.06 (m, 1H), 4.95-4.80 (m, 1H), 4.26-4.06 (m, 2H), 4.05-3.79 (m, 5H), 3.63-3.33 (m, 2H), 3.29-3.19 (m, 1H), 2.69-2.61 (m, 1H), 2.38 (s, 6H), 2.26-.214 (m, 2H), 2.13-2.01 (m, 3H), 1.99-1.84 (m, 5H), 1.81-1.72 (m, 3H), 1.55 (s, 3H), 1.48 (s, 3H), 1.41-.1.26 (m, 8H). |
| Compound 39 | 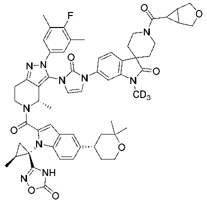 | 1062.6[M+H]⁺ | (400 MHz, CF₃COOD) δ 7.66-7.57 (m, 2H), 7.56-7.45 (m, 1H), 7.44-7.36 (m, 1H), 7.35-7.27 (m, 3H), 7.24 (s, 1H), 7.03 (s, 1H), 6.90 (s, 1H), 6.22-5.96 (m, 1H), 5.26-5.02 (m, 1H), 4.94-4.78 (m,1H), 4.62-4.35 (m, 4H), 4.23-4.03 (m, 2H), 3.96-3.80 (m, 1H), 3.64-3.34 (m, 2H), 3.29-3.13 (m, 1H), 2.44-2.13 (m, 12H), 2.08-1.67 (m, 14H), 1.55 (s, 3H), 1.48 (s, 3H), 1.34 (d, 3H). |
| Compound 40 | 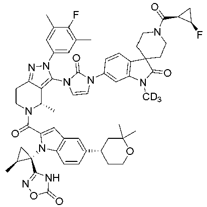 | 1038.6[M+H]⁺ | (400 MHz, CF₃COOD) δ 7.66-7.59 (m, 2H), 7.59-7.52 (m, 1H), 7.43-7.36 (m, 1H), 7.34-7.27 (m, 3H), 7.23 (s, 1H), 7.03 (d, 1H), 6.91 (d, 1H), 6.16-6.04 (m, 1H), 4.97-4.79 (m, 1H), 4.31 (d, 3H), 4.21-4.15 (m, 1H), 4.10 (d, 3H), 3.95-3.83 (m, 1H), 3.60-3.48 (m, 1H), 3.45-3.35 (m, 1H), 3.27-3.17 (m, 1H), 2.66 (s, 2H), 2.40-2.35 (m, 6H), 2.29-2.23 (m, 1H), 2.21-2.11 (m, 4H), 2.07-2.00 (m, 1H), 1.98-1.84 (m, 5H), 1.82-1.78 (m, 1H), 1.74 (d, 3H), 1.55 (s, 3H), 1.48 (s, 3H), 1.34 (d, 3H). |
| Compound 41 | 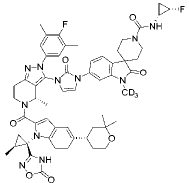 | 1053.6[M+H]⁺ | |

### Example 42: Preparation of compound 42

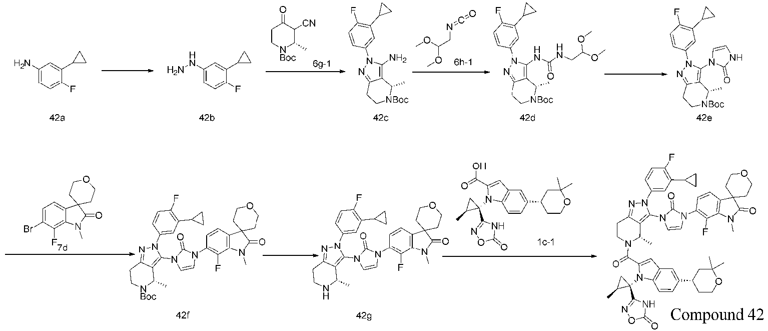

### Step 1: Preparation of 42b

42a (2 g, 13.23 mmol) was dissolved in water (30 mL). Concentrated hydrochloric acid (20 mL) was added, and the mixture was stirred at room temperature for 5 min. Then, sodium nitrite (1.2 g, 17.33 mmol) was added, and the mixture was stirred at 0°C for 30 min. At 0°C, tin chloride (5 g, 26.37 mmol) was slowly added, and the mixture was reacted at room temperature for 2 h. Potassium carbonate solids were added until no more bubbles were generated, and the mixture was extracted with ethyl acetate (30 mL×3), and washed with a saturated aqueous sodium chloride solution (30 mL×3). The organic layers were combined, dried over anhydrous sodium sulphate and filtered, and the filtrate was concentrated to dryness so as to obtain 42b (2 g, yield: 90.91%), which was directly used in the next reaction.

### Step 2: Preparation of compound 42

Using 42b as a substrate, and with reference to the synthetic method of Example 6, the target compound 42 (59 mg, yield: 61.92%) was obtained.

LCMS m/z =490.9 [M+2H]²⁺
¹H NMR (400 MHz, CF₃COOD) δ 7.66-7.57 (m, 2H), 7.46-7.36 (m, 3H), 7.32-7.23 (m, 2H), 7.22-7.16 (m, 1H), 6.95-6.86 (m, 2H), 6.14-6.03 (m, 1H), 4.92-4.78 (m, 1H), 4.63-4.48 (m, 2H), 4.31-4.04 (m, 4H), 3.96-3.82 (m, 1H), 3.66-3.46 (m, 4H), 3.44-3.35 (m, 1H), 3.28-3.15 (m, 1H), 2.28-2.13 (m, 3H), 2.10-1.71 (m, 12H),1.55 (s, 3H), 1.49 (s, 3H), 1.39-1.30 (m, 3H), 1.22-1.11 (m, 2H), 0.85-0.69 (m, 2H).

### Example 43: Preparation of compound 43

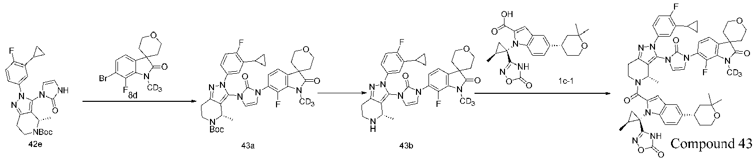

Using 42e as a substrate, and with reference to the synthetic method of Example 6, the target compound 43 (73 mg, 61.10%) was obtained.

LCMS m/z =492.2 [M+2H]²⁺
¹H NMR (400 MHz, CF₃COOD) δ 7.66-7.55 (m, 2H), 7.47-7.36 (m, 3H), 7.32-7.24 (m, 2H), 7.22-7.15 (m, 1H), 6.96-6.85 (m, 2H), 6.15-6.02 (m, 1H), 4.91-4.78 (m, 1H), 4.62-4.48 (m, 2H), 4.30-4.04 (m, 4H), 3.95-3.83 (m, 1H), 3.58-3.34 (m, 2H), 3.28-3.14 (m, 1H), 2.29-1.71 (m, 15H),1.55 (s, 3H), 1.49 (s, 3H), 1.40-1.29 (m, 3H), 1.22-1.12 (m, 2H), 0.85-0.69 (m, 2H).

### Example 44: Preparation of compound 44

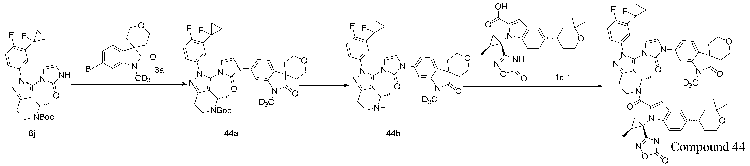

Using 6j as a substrate, and with reference to the synthetic method of Example 6, the target compound 44 (60 mg, 41.85%) was obtained.

LCMS m/z =983.5 [M+H]⁺
¹H NMR (400 MHz, CF₃COOD) δ 7.93 - 7.81 (m, 1H), 7.75 - 7.47 (m, 4H), 7.45 - 7.25 (m, 3H), 7.23 - 7.13 (m, 1H), 7.07 - 6.97 (m, 1H), 6.96 - 6.86 (m, 1H), 6.14 - 6.03 (m, 1H), 4.91 - 4.78 (m, 1H), 4.62 - 4.46 (m, 2H), 4.32 - 4.05 (m, 4H), 3.96 - 3.81 (m, 1H), 3.60 - 3.45 (m, 1H), 3.44 - 3.32 (m, 1H), 3.30 - 3.14 (m, 1H), 2.21 - 1.67 (m, 15H), 1.61 - 1.43 (m, 8H), 1.42 - 1.28 (m, 4H).

### Example 45: Preparation of compound 45

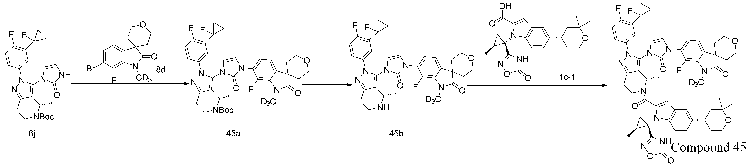

Using 6j as a substrate, and with reference to the synthetic method of Example 6, the target compound 45 (56 mg, 38.36%) was obtained.

LCMS m/z =1001.5 [M+H]⁺
¹H NMR (400 MHz, CF₃COOD) δ 7.90 - 7.80 (m, 1H), 7.73 - 7.19 (m, 7H), 7.01 - 6.84 (m, 2H), 6.16 - 6.00 (m, 1H), 4.92 - 4.76 (m, 1H), 4.63 - 4.47 (m, 2H), 4.30 - 4.05 (m, 4H), 3.96 - 3.81 (m, 1H), 3.58 - 3.30 (m, 2H), 3.29 - 3.13 (m, 1H), 2.26 - 1.67 (m, 15H), 1.61 - 1.44 (m, 8H), 1.42 - 1.29 (m, 4H).

### Example 46: Preparation of compound 46

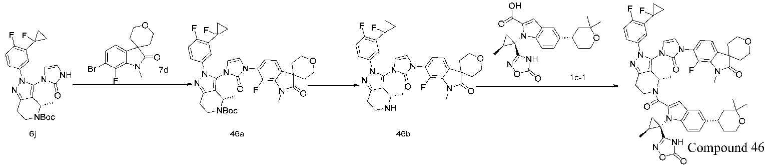

Using 6j as a substrate, and with reference to the synthetic method of Example 6, the target compound 46 (66 mg, 45.35%) was obtained.

LCMS m/z =998.4 [M+H]⁺
¹H NMR (400 MHz, CF₃COOD) δ 7.90 - 7.79 (m, 1H), 7.76 - 7.19 (m, 7H), 7.02 - 6.85 (m, 2H), 6.16 - 6.01 (m, 1H), 4.92 - 4.77 (m, 1H), 4.63 - 4.45 (m, 2H), 4.33 - 4.03 (m, 4H), 3.99 - 3.81 (m, 1H), 3.65 - 3.31 (m, 5H), 3.28 - 3.15 (m, 1H), 2.26 - 1.68 (m, 15H), 1.62 - 1.44 (m, 8H), 1.44 - 1.28 (m, 4H).

### Example 47: Preparation of compound 47

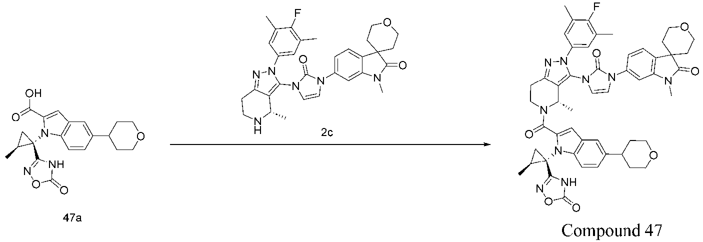

### Step 1: Preparation of compound 47

47a (76.68 mg, 0.2 mmol, with reference to WO 2023016546) was dissolved in DMF (3 mL). 2c (110 mg, 0.2 mmol), HATU (114.07 mg, 0.3 mmol) and DIPEA (258.48 mg, 2.0 mmol) were sequentially added. Under a nitrogen atmosphere, the mixture was reacted at room temperature for 18 h. Water (30 mL) was added, and the mixture was extracted with ethyl acetate (15 mL×3). The organic phases were combined, backwashed with a saturated aqueous sodium chloride solution (20 mL×2), and collected. The collected organic phase was dried over anhydrous sodium sulphate, filtered and concentrated. The residue was purified by silica gel column chromatography (eluent: dichloromethane : methanol (v/v) = 30 : 1) to obtain compound 47 (76 mg, yield: 41.21%).

LCMS m/z = 922.4 [M+H]⁺
¹H NMR (400 MHz, CF₃COOD) δ 7.71-7.56 (m, 3H), 7.40 (d, 1H), 7.36-7.25 (m, 3H), 7.20 (s, 1H), 7.03 (s, 1H), 6.95-6.87 (m, 1H), 6.17-6.06 (m, 1H), 4.92-4.79 (m, 1H), 4.61-4.47 (m, 2H), 4.45-4.33 (m, 2H), 4.31-4.18 (m, 2H), 3.96-3.78 (m, 3H), 3.60-3.41 (m, 2H), 3.39 (s, 3H), 3.08-2.95 (m, 1H), 2.38 (s, 6H), 2.23-1.64 (m, 14H), 1.34 (d, 3H).

### Example 48: Preparation of compound 48

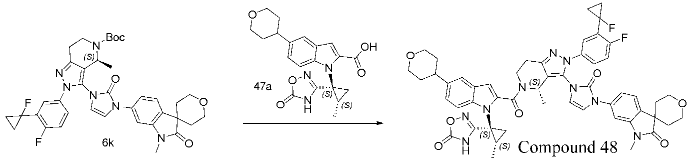

6k (41 mg, 0.06 mmol) and 4 mol/L HCl in 1,4-dioxane (5 mL) were respectively added to the reaction flask, and the mixture was stirred at room temperature for 3 h. The reaction system was concentrated under reduced pressure to obtain a crude (45 mg). The crude (45 mg) was dissolved in 2.5 mL of DMF. 47a (25 mg, 0.065 mmol) (synthesised with reference to WO 2021155841), HATU (33 mg, 0.087 mmol) and DIPEA (38 mg, 0.29 mmol) were respectively added, and the mixture was reacted at room temperature for 16 h. The system was concentrated under reduced pressure, and the resulting crude was subjected to Pre-HPLC (instrument and preparative column: Shimadzu LC-20AP preparative liquid chromatography, C18 preparative column, inner diameter × length = 19 mm × 250 mm). Preparative method: a solution of the crude in DMF was filtered through a 0.45 µm filter membrane to prepare a sample solution; mobile phase system: ammonium bicarbonate in water (10 mmol/L)/acetonitrile; gradient elution method: gradient elution with acetonitrile from 30% to 60% (elution time: 16 min). Lyophilisation was performed to obtain compound 48 (17 mg, yield: 30%).

LCMS m/z = 952.3 [M+1]⁺
¹H NMR (400 MHz, CF₃COOD) δ 7.93 - 7.77 (m, 1H), 7.73 - 7.10 (m, 8H), 7.07 - 6.85 (m, 2H), 6.18 - 5.99 (m, 1H), 4.94 - 4.76 (m, 1H), 4.63 - 4.47 (m, 2H), 4.46 - 4.33 (m, 2H), 4.32 - 4.17 (m, 2H), 4.00 - 3.77 (m, 3H), 3.61 - 3.31 (m, 5H), 3.12 - 2.92 (m, 1H), 2.21 - 1.64 (m, 15H), 1.63 - 1.47 (m, 2H), 1.45 - 1.24 (m, 4H).

### Example 49: Preparation of compound 49

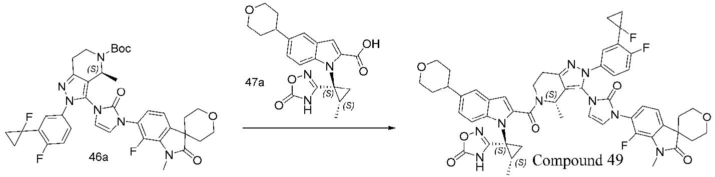

Starting from compounds 46a and 47a, and with reference to the synthetic method of Example 48, compound 49 was obtained.

LCMS m/z = 970.4 [M+1]⁺
¹H NMR (400 MHz, CF₃COOD) δ 7.94 - 7.76 (m, 1H), 7.74 - 7.17 (m, 7H), 7.04 - 6.82 (m, 2H), 6.17 - 6.01 (m, 1H), 4.94 - 4.77 (m, 1H), 4.63 - 4.47 (m, 2H), 4.46 - 4.33 (m, 2H), 4.31 - 4.15 (m, 2H), 3.98 - 3.78 (m, 3H), 3.64 - 3.30 (m, 5H), 3.09 - 2.93 (m, 1H), 2.25 - 1.67 (m, 15H), 1.63 - 1.47 (m, 2H), 1.44 - 1.29 (m, 4H).

### Example 50: Preparation of compound 50

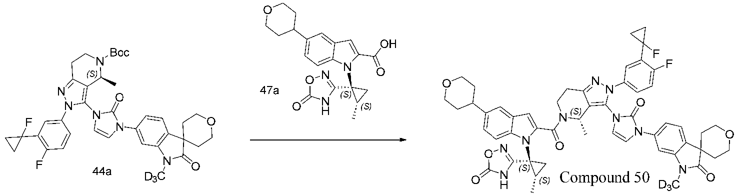

Starting from compounds 44a and 47a, and with reference to the synthetic method of Example 48, compound 50 was obtained.

LCMS m/z = 955.3 [M+1]⁺

### Example 51: Preparation of compound 51

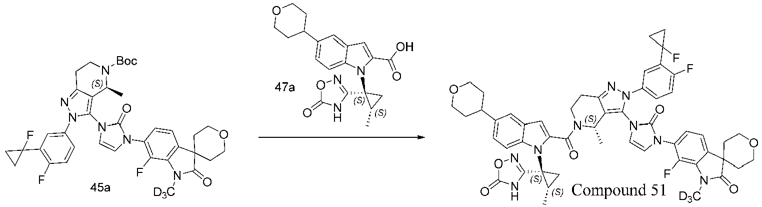

Starting from compounds 45a and 47a, and with reference to the synthetic method of Example 48, compound 51 was obtained.

LCMS m/z = 973.3 [M+1]⁺

### Example 52: Preparation of compound 52

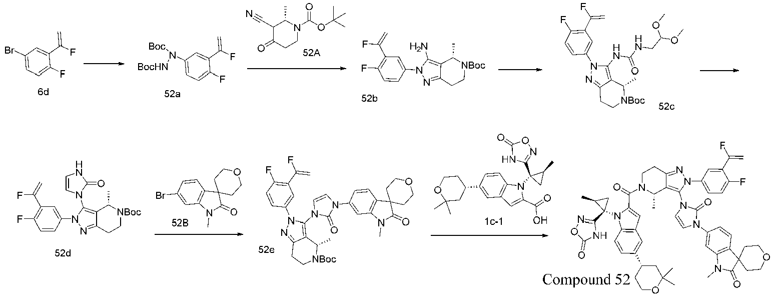

### Step 1: Preparation of 52a

6d (22 g, 0.1 mol) was dissolved in 300 mL of THF, and the temperature was cooled to -78°C. 40 mL of 2.5 mol/L n-butyllithium solution was added, and the mixture was reacted at -78°C for 1 h. Then, di-tert-butyl diazene-1,2-dicarboxylate (34.5 g, 0.15 mol) was slowly added, and the mixture was reacted at room temperature for 2 h. 100 mL of saturated aqueous ammonium chloride solution and 500 mL of ethyl acetate were added. The organic phase was dried over anhydrous sodium sulphate, and concentrated under reduced pressure. The crude was separated and purified by silica gel column chromatography (petroleum ether : ethyl acetate (v/v) = 4 : 1) to obtain 52a (11 g, yield: 30%).

### Step 2: Preparation of 52b

52a (3.7 g, 9.99 mmol) was dissolved in 30 mL of dichloromethane. 4 mol/L HCl in 1,4-dioxane (30 mL) was added, and the mixture was reacted at 25°C for 3 h. The reaction solution was concentrated under reduced pressure, and 52A (2.4 g, 10.07 mmol), pyridine hydrochloride (0.12 g, 1 mmol) and 100 mL of toluene were respectively added, and the mixture was reacted at 90°C for 1 h. The reaction solution was cooled to room temperature, and concentrated under reduced pressure. The crude was separated and purified by silica gel column chromatography (petroleum ether : ethyl acetate (v/v) = 3 : 1) to obtain 52b (1.5 g, yield: 38%).

LCMS m/z = 391.3 [M+1] ⁺

### Step 3: Preparation of 52c

52b (1.5 g, 3.8 mmol) was dissolved in 20 mL of pyridine. 2-Isocyanato-1,1-dimethoxyethane (1.3 g, 9.9 mmol) was added, and the mixture was reacted at room temperature for 8 h. The reaction solution was concentrated under reduced pressure. The crude was separated and purified by silica gel column chromatography (petroleum ether : ethyl acetate (v/v) = 1 : 1) to obtain 52c (1.4 g, yield: 71%).

LCMS m/z = 522.4 [M+1] ⁺

### Step 4: Preparation of 52d

52c (1.4 g, 2.69 mmol) was dissolved in 20 mL of tetrahydrofuran. Methanesulphonic acid (0.58 g, 6.03 mmol) was added, and the mixture was reacted at 60°C for 2 h. The mixture was cooled to room temperature, and adjusted to pH 9.0 with a saturated potassium carbonate solution. Ditert-butyl dicarbonate (0.59 g, 2.7 mmol) was added, and the mixture was reacted at room temperature for 1 h. 30 mL of purified water and 50 mL of ethyl acetate were added. The organic phase was dried over anhydrous sodium sulphate, and concentrated under reduced pressure. The crude was separated and purified by silica gel column chromatography (petroleum ether : ethyl acetate (v/v) = 1 : 1) to obtain 52d (0.6 g, yield: 49%).

LCMS m/z = 458.4 [M+1]⁺

### Step 5: Preparation of 52e

52d (0.14 g, 0.31 mmol) was dissolved in 6 mL of NMP. 52B (0.15 g, 0.51 mmol), CuI (0.086 g, 0.45 mmol) and (1S,2S)-(+)-N,N'-dimethyl-1,2-cyclohexanediamine (0.064 g, 0.45 mmol) were respectively added, and the mixture was reacted at 130°C for 3 h. The reaction solution was cooled to room temperature. 30 mL of purified water and 30 mL of ethyl acetate were added. The organic phase was dried over anhydrous sodium sulphate, and concentrated under reduced pressure. The crude was separated and purified by silica gel column chromatography (petroleum ether : ethyl acetate (v/v) = 1 : 1) to obtain 52e (0.1 g, yield: 48%).

### Step 6: Preparation of compound 52

52e (0.1 g, 0.15 mmol) was dissolved in 3 mL of dichloromethane. 3 mL of 4 mol/L HCl in 1,4-dioxane was added, and the mixture was reacted at 25°C for 3 h. The reaction solution was concentrated under reduced pressure. 1c-1 (0.066 g, 0.16 mmol), HATU (0.068 g, 0.18 mmol) and 5 mL of DMF were respectively added, and the mixture was reacted at room temperature for 8 h. The reaction solution was concentrated under reduced pressure, and the crude was separated and purified by silica gel column chromatography (petroleum ether : ethyl acetate (v/v) = 1 : 2). The resulting crude was subjected to Pre-HPLC (instrument and preparative column: Shimadzu LC-20AP preparative liquid chromatography, C18 preparative column, inner diameter × length = 19 mm × 250 mm). Preparative method: a solution of the crude in acetonitrile was filtered through a 0.45 µm filter membrane to prepare a sample solution; mobile phase system: ammonium bicarbonate in water (10 mmol/L)/acetonitrile; gradient elution method: gradient elution with acetonitrile from 30% to 50% (elution time: 14 min). Lyophilisation was performed to obtain compound 52 (80 mg, yield: 55%).

LCMS m/z = 966.4 [M+1] ⁺
¹H NMR (400 MHz, CF₃COOD)δ 7.91 - 7.80 (m, 1H), 7.78 - 7.49 (m, 4H), 7.48 - 7.35 (m, 2H), 7.34 - 7.25 (m, 1H), 7.24 - 7.17 (m, 1H), 7.05 - 6.98 (m, 1H), 6.97 - 6.87 (m, 1H), 6.14 - 6.01 (m, 1H), 5.64 - 5.43 (m, 1H), 5.42 - 5.30 (m, 1H), 4.90 - 4.76 (m, 1H), 4.62 - 4.44 (m, 2H), 4.33 - 4.05 (m, 4H), 3.97 - 3.81 (m, 1H), 3.59 - 3.44 (m, 1H), 3.44 - 3.32 (m, 4H), 3.27 - 3.15 (m, 1H), 2.19 - 1.66 (m, 14H), 1.52 (d, 6H), 1.34 (d, 3H).

Starting from compound 52d, the following compounds were synthesised under similar synthetic conditions as described in the above examples.

| Compound | Structure | LCMS m/z | ¹H NMR |
|---|---|---|---|
| Compound 53 | 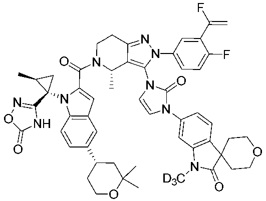 | 967.4 [M-H]⁻ | (400 MHz, CF₃COOD) δ 7.91 - 7.80 (m, 1H), 7.77 - 7.48 (m, 4H), 7.48 - 7.35 (m, 2H), 7.34 - 7.25 (m, 1H), 7.24 - 7.17 (m, 1H), 7.05 - 6.98 (m, 1H), 6.96 - 6.87 (m, 1H), 6.14 - 6.01 (m, 1H), 5.64 - 5.44 (m, 1H), 5.42 - 5.29 (m, 1H), 4.90 - 4.77 (m, 1H), 4.62 - 4.47 (m, 2H), 4.32 - 4.04 (m, 4H), 3.99 - 3.80 (m, 1H), 3.58 - 3.44 (m, 1H), 3.44 - 3.30 (m, 1H), 3.29 - 3.13 (m, 1H), 2.20 - 1.68 (m, 14H), 1.61 - 1.44 (m, 6H), 1.41 - 1.23 (m, 3H). |
| Compound 54 | 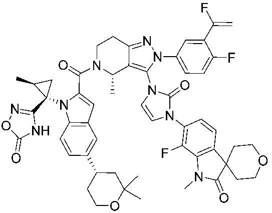 | 984.3 [M+H]⁺ | (400 MHz, CF₃COOD) δ 7.92 - 7.78 (m, 1H), 7.75 - 7.55 (m, 3H), 7.50 - 7.35 (m, 3H), 7.34 - 7.20 (m, 1H), 7.02 - 6.81 (m, 3H), 6.15 - 6.00 (m, 1H), 5.65 - 5.43 (m, 1H), 5.42 - 5.27 (m, 1H), 4.96 - 4.74 (m, 1H), 4.66 - 4.43 (m, 2H), 4.33 - 4.03 (m, 4H), 3.97 - 3.79 (m, 1H), 3.65 - 3.29 (m, 5H), 3.29 - 3.12 (m, 1H), 2.25 - 2.12 (m, 2H), 2.10 - 1.70 (m, 12H), 1.55 (s, 3H), 1.49 (s, 3H), 1.35 (d, 3H). |
| Compound 55 | 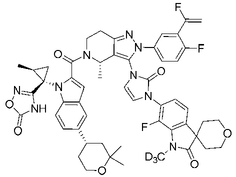 | 987.3 [M+H]⁺ | (400 MHz, CF₃COOD) δ 7.93 - 7.77 (m, 1H), 7.75 - 7.52 (m, 3H), 7.50 - 7.33 (m, 3H), 7.33 - 7.20 (m, 1H), 7.02 - 6.84 (m, 3H), 6.14 - 6.00 (m, 1H), 5.65 - 5.43 (m, 1H), 5.41 - 5.28 (m, 1H), 4.91 - 4.75 (m, 1H), 4.65 - 4.44 (m, 2H), 4.31 - 4.02 (m, 4H), 3.97 - 3.81 (m, 1H), 3.58 - 3.30 (m, 2H), 3.28 - 3.13 (m, 1H), 2.25 - 2.11 (m, 2H), 2.10 - 1.68 (m, 12H), 1.55 (s, 3H), 1.49 (s, 3H), 1.35 (d, 3H). |

Starting from compound 47a, the following compounds were synthesised under similar synthetic conditions as described in the above examples.

| Compound | Structure | LCMS m/z | ¹H NMR |
|---|---|---|---|
| Compound 56 | 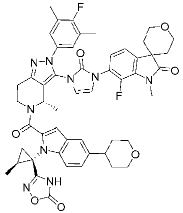 | 940.0 [M+H]⁺ | (400 MHz, CF₃COOD) δ 7.66-7.58 (m, 2H), 7.46-7.37 (m, 2H), 7.35-7.25 (m, 3H), 6.95-6.85 (m, 2H), 6.17- 6.05 (m, 1H), 4.95-4.81 (m, 1H), 4.60-4.50 (m, 2H), 4.44-4.36 (m, 2H), 4.29-4.19 (m, 2H), 3.93-3.80 (m, 3H), 3.59 (s, 3H), 3.54-3.36 (m, 2H), 3.07-2.97 (m, 1H), 2.40 (s, 6H), 2.25-2.14 (m, 2H), 2.13-1.95 (m, 7H), 1.92-1.84 (m, 1H), 1.83-1.71 (m, 4H), 1.35 (d, 3H). |
| Compound 57 | 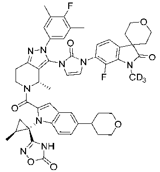 | 943.0 [M+H]⁺ | (400 MHz, CF₃COOD) δ 7.67-7.53 (m, 2H), 7.47-7.37 (m, 2H), 7.36-7.24 (m, 3H),6.97-6.85(m,2H), 6.17-6.01 (m, 1H), 4.95-4.77 (m, 1H), 4.61-4.48 (m, 2H), 4.45-4.34 (m, 2H), 4.31-4.15 (m, 2H), 3.98-3.74 (m, 3H), 3.60-3.47 (m, 1H), 3.46-3.34 (m, 1H), 3.09-2.92 (m, 1H), 2.46-2.32 (m, 6H), 2.26-1.66(m, 14H), 1.35 (d, 3H). |
| Compound 58 | 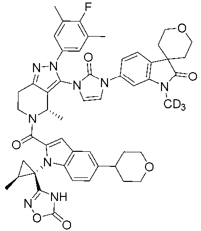 | 925.4 [M+H]⁺ | ¹H NMR (400 MHz, CF₃COOD) δ 7.70-7.58 (m, 3H), 7.40 (d, 1H), 7.35-7.24 (m, 3H), 7.20 (s, 1H), 7.03 (d, 1H), 6.94-6.88 (m, 1H), 6.16-6.06 (m, 1H), 4.94-4.79 (m, 1H), 4.65-4.46 (m, 2H), 4.45-4.35 (m, 2H), 4.31-4.20 (m, 2H), 3.96-3.79 (m, 3H), 3.61-3.47 (m, 1H), 3.46-3.35 (m, 1H), 3.07-2.97 (m, 1H), 2.42-2.35 (m, 6H), 2.20-1.94 (m, 9H), 1.91-1.83 (m, 1H), 1.83-1.78 (m, 1H), 1.74 (d, 3H), 1.34 (d, 3H). |

### Example 59: Preparation of compound 59

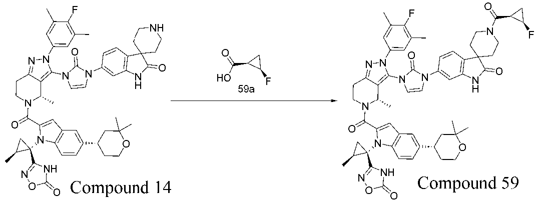

Compound 14 (114 mg, 0.12 mmol), 59a (21 mg, 0.2 mmol), HATU (76 mg, 0.2 mmol) and DIPEA (65 mg, 0.5 mmol) were dissolved in N,N-dimethylformamide (5 mL), and the mixture was reacted overnight at room temperature. 15 mL of water was added, and the mixture was extracted with ethyl acetate (20 mL×3), and washed with a saturated aqueous sodium chloride solution (20 mL). The organic layers were combined, dried over anhydrous sodium sulphate and filtered, and the filtrate was concentrated under reduced pressure. The residue was separated and purified by column chromatography (eluent: dichloromethane : methanol (v/v) = 30 : 1) to obtain compound 59 (60 mg, yield: 48.35%).

LCMS m/z = 518.3 [M+2H]⁺
¹H NMR (400 MHz, CF₃COOD) δ 7.69-7.46 (m, 3H), 7.39 (d, 1H), 7.36-7.27 (m, 3H), 7.25 (s, 1H), 7.03 (d, 1H), 6.91 (d, 1H), 6.21-6.00 (m, 1H), 5.27-5.00 (m, 1H), 4.93-4.79 (m, 1H), 4.75-4.25 (m, 4H), 4.23-4.05 (m, 2H), 3.90 (t, 1H), 3.59-3.47 (m, 1H), 3.46-3.35 (m, 4H), 3.27-3.17 (m, 1H), 2.44-2.13 (m, 11H), 2.08 (t, 1H), 2.00-1.79 (m, 7H), 1.74 (d, 3H), 1.67-1.58 (m, 1H), 1.55 (s, 3H), 1.49 (s, 3H), 1.33 (d, 3H).

### Biological test example 1: In vitro activity assay

HEK293-CRE-luc-GLP-1R cells were seeded into a 384-well cell culture plate (Greiner #781946) at a certain cell density (5 µL/well). 5 µl/well of 2× working solution was added to the 384-well microplate. The microplate was sealed with parafilm and incubated in a 37°C 5% CO2 incubator (Thermo) for 1 h. Subsequently, the detection was performed using the cAMP-GS HIRANGE KIT (PerkinElmer, 62AM6PEB), and detection reagents were prepared according to the kit instruction manual. After the incubation, the detection reagents were added to the 384-well microplate, and the microplate was incubated for 1 h at room temperature in the dark. HTRF signals at 665 nm and 620 nm were then measured using a multimode microplate reader. Data were exported from the microplate reader in Excel format. GraphPad Prism was opened. The logarithm of the sample concentration was calculated. Using the logarithm of concentration as the x-axis and the 665 nm/620 nm ratio as the y-axis, four-parameter logistic fitting was performed to obtain the EC50 value of the curve.

**Table 1: EC₅₀ of compounds against GLP-1**

| Compound | EC₅₀ (nM) | Compound | EC₅₀ (nM) |
|---|---|---|---|
| Compound 2 | A | Compound 35 | A |
| Compound 3 | A | Compound 37 | A |
| Compound 5 | A | Compound 38 | A |
| Compound 6 | A | Compound 39 | A |
| Compound 7 | A | Compound 40 | A |
| Compound 8 | A | Compound 41 | A |
| Compound 9 | A | Compound 42 | A |
| Compound 12 | A | Compound 43 | A |
| Compound 13 | A | Compound 44 | A |
| Compound 15 | A | Compound 45 | A |
| Compound 17 | A | Compound 46 | A |
| Compound 18 | A | Compound 47 | A |
| Compound 20 | A | Compound 49 | A |
| Compound 22 | A | Compound 51 | A |
| Compound 23 | A | Compound 52 | A |
| Compound 25 | A | Compound 53 | A |
| Compound 26 | A | Compound 54 | A |
| Compound 29 | A | Compound 55 | A |
| Compound 30 | A | Compound 56 | A |
| Compound 32 | A | Compound 57 | A |
| Compound 33 | A | Compound 58 | A |
| Compound 34 | A | Compound 59 | A |

$A < 1 nM$

Conclusion: the compounds of the present invention, such as the example compounds, exhibit good agonistic effects on GLP-1 receptors.

### Biological test example 2:

### Oral glucose tolerance test (OGTT)

OGTT experiment was performed in male C57BL/6-G1p1rem2 (hGLP1R) Smoc mice to evaluate the hypoglycaemic effect of GLP-1 receptor agonists. The animals were fasted for more than 16 hours and randomly grouped. Tail tip blood was collected and basal fasting blood glucose (FBG) was measured using a blood glucose meter. Mice with FBG in the range of 3.5-6.5 mmol/L were selected and orally administered, and the control group was administered the vehicle. 15 min after administration, each group of animals was intragastrically administered with 25% glucose solution. Blood glucose levels were measured in tail tip blood collected 15 min, 30 min, 45 min, 1 h, and 2 h after glucose administration. 0-2 h blood glucose AUC_{0-2 h} was calculated using the Graphpad Prism software, and the inhibition rate was further calculated. 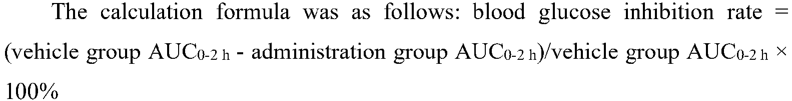 The calculation formula was as follows: blood glucose inhibition rate = (vehicle group AUC0-2 h - administration group AUC0-2 h)/vehicle group AUC0-2 h × 100%

Conclusion: the compounds of the present invention, such as the example compounds, exhibit good oral hypoglycaemic effects via the GLP-1 receptor. For example, compound 2 and compound 3 exhibit excellent hypoglycaemic performance at a dose of 3 mg/kg, wherein compound 2 shows a blood glucose inhibition rate of -25.5% at 3 mg/kg, compound 3 shows a blood glucose inhibition rate of -37.1% at 3 mg/kg, and the control compound Orforglipron shows a blood glucose inhibition rate of -9.8% at 3 mg/kg.

### Biological test example 3: Mouse pharmacokinetic assay

Experimental animals: male C57 mice, 22-25 g, 6 mice/compound.

Experimental design: on the day of the experiment, C57 mice were randomly grouped according to body weight. The animals were fasted with water available for 12 h to 14 h one day before the administration, and were fed 4 h after the administration.

**Table 2. Administration information**

| Administration dosage (mg/kg) | Administration concentration (mg/mL) | Administration volume (mL/kg) | Collected sample | Administration mode |
|---|---|---|---|---|
| 2.5 | 0.5 | 5 | Plasma | Intravenous administration |
| 10 | 1 | 10 | Plasma | Intragastric administration |

| | | | | |
|---|---|---|---|---|
| Notes: vehicle for intravenous administration: 5% DMA+5% Solutol+90% Saline; vehicle for intragastric administration: 5% DMSO + 5% Solutol + 10% PEG400 + 80% (20% SBE-β-CD); (DMA: dimethylacetamide; Solutol: polyethylene glycol-15-hydroxystearate; Saline: physiological saline; DMSO: dimethyl sulphoxide; PEG400: polyethylene glycol 400; SBE-β-CD: sulphobutyl-β-cyclodextrin) | | | | |

Before and after the administration, 0.06 mL of blood was taken from the orbits under isoflurane anaesthesia and placed in an EDTAK2 centrifuge tube. The blood was centrifuged at 5000 rpm at 4°C for 10 min, and the plasma was collected. The blood collection time points for the intravenous administration group and intragastric administration group were 0, 5 min, 15 min, 30 min, 1 h, 2 h, 4 h, 7 h and 24 h. Before analysis and detection, all samples were stored at -80°C, and the samples were quantitatively analysed by LC-MS/MS.

**Table 2-1 Pharmacokinetic results of test compounds in mice**

| Test compound | Administration dosage | AUC₀₋ₜ (hr×ng/mL) | F% |
|---|---|---|---|
| Orforglipron | Intragastric administration 10 mg/kg | 3255 | 7.93 |
| Compound 2 | Intragastric administration 10 mg/kg | 23230 | 70.3 |
| Compound 54 | Intragastric administration 10 mg/kg | 12141 | 33.8 |
| Compound 55 | Intragastric administration 10 mg/kg | 8666 | 25.1 |

Conclusion: the compounds of the present invention, such as the example compounds, exhibit good oral performance in mice.

### Biological test example 4: Monkey pharmacokinetic assay

Experimental animals: male cynomolgus monkeys, 3-5 kg, 3-6 years old, 5 monkeys/compound, purchased from Hainan New Source Biotechnology Co., Ltd.

Experimental method: On the day of the experiment, five monkeys were randomly grouped according to body weight, with two in the intravenous administration group and three in the intragastric administration group. The animals were fasted with water available for 14 h to 18 h one day before the administration, and were fed 4 h after the administration.

**Table 3. Administration information**

| Administration dosage (mg/kg) | Administration concentration (mg/mL) | Administration volume (mL/kg) | Collected sample | Administration mode |
|---|---|---|---|---|
| 1 | 1 | 1 | Plasma | Intravenous administration |
| 5 | 1 | 5 | Plasma | Intragastric administration |

| | | | | |
|---|---|---|---|---|
| Notes: vehicle for intravenous administration: 5% DMA + 5% Solutol + 90% Saline; and vehicle for intragastric administration: 5% DMSO+5% Solutol+ 90% (0.5% MC); before and after the administration, 1.0 mL of blood was taken from the limb veins and placed in an EDTAK2 centrifuge tube. The tube was centrifuged at 5000 rpm at 4°C for 10 min, and the plasma was collected. The blood collection time points for the intravenous administration group and intragastric administration group were 0 min, 5 min, 15 min, 30 min, 1 h, 2 h, 4 h, 6 h, 8 h, 10 h, 12 h, 24 h and 48 h. Before analysis and detection, all samples were stored at -80°C, and the samples were quantitatively analysed by LC-MS/MS. | | | | |

**Table 3-1. Pharmacokinetic parameters of test compound in plasma of monkeys**

| Test compound | Administration dosage | AUC₀₋ₜ (hr*ng/mL) | F (%) |
|---|---|---|---|
| Orforglipron | Intragastric administration 5 mg/kg | 584 | 1.93 |
| Compound 2 | Intragastric administration 5 mg/kg | 11636 | 32.6 |

Conclusion: the compounds of the present invention, such as the example compounds, exhibit good oral performance in monkeys.

### Biological test example 5: CYP450 enzyme activity inhibition assay

The purpose of this study was to evaluate the effects of the test compounds on the activities of five isoenzymes (CYP1A2, CYP2C9, CYP2C19, CYP2D6, and CYP3A4) of human liver microsomal cytochrome P450 (CYP) using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates. Changes in CYP enzyme activity were determined, and IC₅₀ value was calculated to evaluate the inhibitory potential of the test compound on each CYP enzyme subtype.

**Table 4**

| Test compound | IC50 (µM) | | | | |
|---|---|---|---|---|---|
| | CYP1A2 | CYP2C9 | CYP2C19 | CYP2D6 | CYP3A4 |
| Orforglipron | >30 | 0.905 | >30 | >30 | >30 |
| Compound 3 | >30 | >30 | >30 | >30 | >30 |
| Compound 6 | >30 | >30 | >30 | >30 | >30 |
| Compound 9 | >30 | 3.31 | >30 | >30 | >30 |

Conclusion: the compounds of the present invention, such as the example compounds, exhibit lower CYP inhibitory potential than the control compound Orforglipron.

### Biological test example 6: SLC transporter inhibition assay

The objective of this study was to evaluate the inhibitory effects of the test substance on the activities of the transporters OATP1B1 and OATP1B3.

HEK293-OATP1B1 and OATP1B3 cells were incubated with and without the test substance (0-30 µM) for the corresponding time. Samples were then collected, and the substrate content in the samples was detected by liquid chromatography-tandem mass spectrometry (LC-MS/MS). By calculating the transport activity of the transporter with and without the test substance, the percentage of transporter activity of the transporter-expressing cells at different administration concentrations of the test substance (%VC (Vehicle Control, solvent control), the percentage of transport activity of the transporter with and without the test substance) was obtained, and the half-maximal inhibitory concentration (IC₅₀) was calculated from this.

Conclusion: the compounds of the present invention, such as the example compounds, exhibit weaker inhibitory effects on OATP1B1 and OATP1B3. For example, compound 6 exhibits IC₅₀ > 2 µM against OATP1B1 and IC₅₀ > 1 µM against OATP1B3.

## Claims

1. A compound or a racemate, stereoisomer, tautomer, deuterated substance, solvate, prodrug, metabolite, pharmaceutically acceptable salt or eutectic crystal thereof, **characterised in that** the compound is a compound as shown in general formula (I), 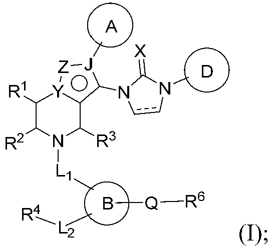 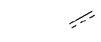 represents a single bond or a double bond;
Y is selected from C and N, Z is selected from CH or N, J is selected from N or C, and at least one of Y, Z and J is N;
ring A is selected from C₆₋₁₀ aryl, 5- to 10-membered heteroaryl, C₃₋₁₀ carbocyclyl, 4- to 10-membered heterocyclyl, C₁₁₋₁₅ aryl, 11- to 15-membered heterocyclyl, and 11- to 15-membered heteroaryl, the ring A is optionally substituted with 1 to 4 R^{a}, and the nitrogen atom in the heteroaryl or heterocyclyl is optionally oxidised to form an N-oxide;
ring B is selected from C₆₋₁₀ aryl, 5- to 6-membered heteroaryl, 5-membered ring-fused-5-membered heteroaryl, 5-membered ring-fused-6-membered heteroaryl, 6-membered ring-fused-6-membered heteroaryl, C₃₋₁₀ carbocyclyl, and 4- to 10-membered heterocyclyl, and the ring B is optionally substituted with 1 to 4 R^{b};
ring D is selected from 13- to 16-membered tricyclic or tetracyclic heterocyclyl, and 17- to 30-membered tricyclic or tetracyclic heterocyclyl, and the ring D is optionally substituted with 1 to 4 R^{d};
L₁ is selected from -S(=O)₂-, -C(=O)-, and -C(=S)-;
L₂ is -(CR^{L1}R^{L2})ₘ-;
X is selected from S or O;
Q is selected from a bond, O, S, NH, C₁₋₄ alkylene, 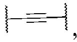 wherein the alkylene is optionally substituted with 1 to 4 R^{k};
m is selected from 1, 2, 3 or 4;
R⁴ is selected from -C(=O)R^{4a}, -C(=O)OR^{4a}, -C(=O)NR^{4a}R^{4b}, 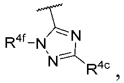 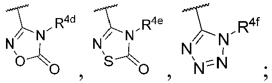
R^{4a}, R^{4b}, R^{4c}, R^{4d}, R^{4e}, and R^{4f} are each independently selected from H, deuterium, and C₁₋₆ alkyl, wherein the alkyl is optionally substituted with 1 to 4 R^{k};
R⁶ is selected from C₁₋₆ alkyl, -C₁₋₂ alkylene-O-C₁₋₄ alkyl, C₃₋₁₀ carbocyclyl, and 4- to 10-membered heterocyclyl, and the R⁶ is optionally substituted with 1 to 4 R^{6a};
R^{a}, R^{b}, R^{d}, and R^{6a} are each independently selected from H, deuterium, halogen, =O, CN, OH, NO₂, COOH, CONH₂, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₄ alkylene-C₃₋₆ carbocyclyl, -C₁₋₄ alkylene-3- to 7-membered heterocyclyl, -P(=O)R^{5a}R^{5b}, -S(=O)₁₋₂-R^{5c}, -C(=O)R^{5c}, -C(=O)NR^{5d}R^{5e}, - NR^{5d}C(=O)R^{5e}, -C(=O)-M-C(=O)-R^{5c}, -CH₂-M-C(=O)-R^{5c}, -C(=O)-M-CH₂-R^{5c}, - C(=O)-M-R^{5c}, -C(=O)-C₁₋₄ alkylene-OC₀₋₄ alkylene-R^{5c}, -C(=O)-M-C₁₋₄ alkylene-O-R^{5c}, C₃₋₁₂ carbocyclyl, 3- to 12-membered heterocyclyl, 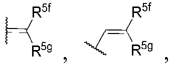 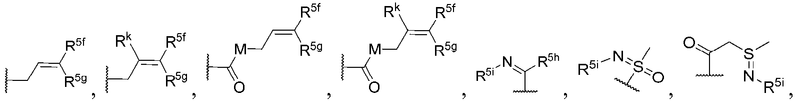 wherein the alkyl, alkylene, alkenyl, alkynyl, carbocyclyl or heterocyclyl is optionally substituted with 1 to 4 R^{k};
M is selected from C₃₋₆ carbocyclyl or 4- to 7-membered heterocyclyl, and the M is optionally substituted with 1 to 4 R^{k};
R^{5a}, R^{5b}, and R^{5c} are each independently selected from C₁₋₆ alkyl, -C₁₋₄ alkyl-OC₁₋₄ alkyl, C₁₋₆ alkoxy, C₂₋₆ alkenyl, C₃₋₁₂ carbocyclyl, and 4- to 12-membered heterocyclyl, wherein the alkyl, alkenyl, alkoxy, carbocyclyl or heterocyclyl is optionally substituted with 1 to 4 R^{k};
R^{5d} and R^{5e} are each independently selected from H, C₁₋₆ alkyl, C₃₋₁₀ carbocyclyl, and 4- to 10-membered heterocyclyl, wherein the alkyl, carbocyclyl or heterocyclyl is optionally substituted with 1 to 4 R^{k};
R^{5f} and R^{5g} are each independently selected from H, halogen, C₁₋₆ alkyl, and C₃₋₁₀ carbocyclyl, wherein the alkyl or carbocyclyl is optionally substituted with 1 to 4 R^{k};
or R^{5f} and R^{5g} are directly connected to form C₃₋₆ carbocyclyl or 4- to 7-membered heterocyclyl, wherein the carbocyclyl or heterocyclyl is optionally substituted with 1 to 4 R^{k};
R^{5h} is selected from NH₂, NHC₁₋₆ alkyl, N(C₁₋₆ alkyl)₂, C₁₋₆ alkyl, C₃₋₆ carbocyclyl or 4- to 7-membered heterocyclyl, wherein the alkyl, carbocyclyl or heterocyclyl is optionally substituted with 1 to 4 R^{k};
R⁵ⁱ is selected from CN, NO₂, -S(=O)₁₋₂-C₁₋₆ alkyl, and -S(=O)₁₋₂-C₃₋₆ carbocyclyl, wherein the alkyl or carbocyclyl is optionally substituted with 1 to 4 R^{k};
R¹, R², R³, R^{L1}, and R^{L2} are each independently selected from H, deuterium, halogen, CN, OH, NO₂, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, and -SC₁₋₆ alkyl, wherein the alkyl, alkenyl, or alkynyl is optionally substituted with 1 to 4 R^{k};
alternatively, R^{L1} and R^{L2} together with the atom to which they are attached form C₃₋₈ carbocyclyl or 4- to 8-membered heterocyclyl, wherein the carbocyclyl or heterocyclyl is optionally substituted with 1 to 4 R^{k};
alternatively, R^{5a} and R^{5b} together with the phosphorus atom to which they are attached form 5- to 8-membered heterocyclyl, wherein the heterocyclyl is optionally substituted with 1 to 4 R^{k};
each R^{k} is independently selected from deuterium, halogen, =O, CN, OH, SH, NO₂, COOH, CONH₂, NH₂, SF₅, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₄ alkylene-C₃₋₆ carbocyclyl, -C₁₋₄ alkylene-3- to 7-membered heterocyclyl, C₃₋₆ carbocyclyl, 3- to 7-membered heterocyclyl, 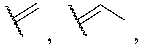 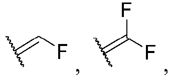 wherein the alkyl, alkylene, alkenyl, alkynyl, carbocyclyl or heterocyclyl is optionally substituted with 1 to 4 substituents selected from deuterium, halogen, CN, OH, NH₂, CH₂CN, C₁₋₆ alkyl, and C₁₋₆ alkoxy.

2. The compound or the racemate, stereoisomer, tautomer, deuterated substance, solvate, prodrug, metabolite, pharmaceutically acceptable salt or eutectic crystal thereof according to claim 1, **characterised in that**
ring B is selected from 5-membered ring-fused-5-membered heteroaryl or 5-membered ring-fused-6-membered heteroaryl, and the ring B is optionally substituted with 1 to 4 R^{b};
ring D is selected from partially saturated rings as follows: 13- to 15-membered tricyclic fused heterocyclyl, 10-membered fused carbocycle spiro-linked to C₄₋₆ carbocycle, 9-membered fused carbocycle spiro-linked to C₄₋₆ carbocycle, 11-membered fused carbocycle spiro-linked to C₃₋₅ carbocycle, 10-membered fused heterocycle spiro-linked to C₄₋₆ carbocycle, 11-membered fused heterocycle spiro-linked to C₃₋₅ carbocycle, 10-membered fused carbocycle spiro-linked to 4- to 6-membered heterocycle, 11-membered fused carbocycle spiro-linked to 4- to 5-membered heterocycle, 9-membered fused heterocycle spiro-linked to 4- to 6-membered heterocycle, 9-membered fused heterocycle spiro-linked to C₃₋₆ carbocycle, 9-membered fused heterocycle spiro-linked to 8- to 10-membered heterocycle, 10-membered fused heterocycle spiro-linked to 4- to 6-membered heterocycle, 11-membered fused heterocycle spiro-linked to 4- to 5-membered heterocycle, 11-membered tricyclic heterocycle spiro-linked to C₃₋₄ carbocycle, and the ring D is optionally substituted with 1 to 4 R^{d};
ring A is selected from phenyl, 5- to 6-membered heteroaryl, C₃₋₆ monocyclic carbocyclyl, 4- to 8-membered monocyclic heterocyclyl, and benzo C₇₋₈ carbocyclyl, the ring A is optionally substituted with 1 to 4 R^{a}, and the nitrogen atom in the heteroaryl or heterocyclyl is optionally oxidised to form an N-oxide;
R⁶ is selected from C₁₋₄ alkyl, -C₁₋₂ alkylene-O-C₁₋₄ alkyl, C₃₋₆monocyclic carbocyclyl, C₆₋₁₀ fused carbocyclyl, C₆₋₁₀ spirocarbocyclyl, C₅₋₁₀ bridged carbocyclyl, 4- to 8-membered monocyclic heterocyclyl, 7- to 10-membered fused heterocyclyl, 7- to 10-membered spiro heterocyclyl, and 6- to 10-membered bridged heterocyclyl, and the R⁶ is optionally substituted with 1 to 4 R^{6a};
R^{4a}, R^{4b}, R^{4c}, R^{4d}, R^{4e}, and R^{4f} are each independently selected from H, deuterium, and C₁₋₄ alkyl, wherein the alkyl is optionally substituted with 1 to 4 R^{k};
R^{a}, R^{b}, R^{d}, and R^{6a} are each independently selected from H, deuterium, halogen, =O, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₂ alkylene-C₃₋₆ carbocyclyl, -C₁₋₂ alkylene-3- to 7-membered heterocyclyl, - P(=O)R^{5a}R^{5b}, -S(=O)₁₋₂-R^{5c}, -C(=O)R^{5c}, -C(=O)NR^{5d}R^{5e}, -NR^{5d}C(=O)R^{5e}, -C(=O)-M-C(=O)-R^{5c}, -CH₂-M-C(=O)-R^{5c}, -C(=O)-M-R^{5c}, -C(=O)-C₁₋₂ alkylene-OC₀₋₂ alkylene-R^{5c}, -C(=O)-M-C₁₋₂ alkylene-O-R^{5c}, C₃₋₁₁ carbocyclyl, 3- to 11-membered heterocyclyl, 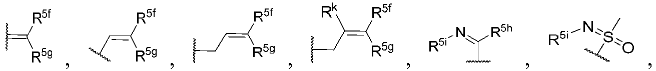 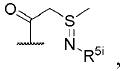 wherein the alkyl, alkenyl, alkynyl, alkylene, carbocyclyl or heterocyclyl is optionally substituted with 1 to 4 R^{k};
M is selected from C₃₋₆ cycloalkyl, phenyl, 4- to 7-membered heterocycloalkyl or 5- to 6-membered heteroaryl, and the M is optionally substituted with 1 to 4 R^{k};
R^{5a}, R^{5b}, and R^{5c} are each independently selected from C₁₋₄ alkyl, -C₁₋₄ alkyl-OC₁₋₄ alkyl, C₁₋₄ alkoxy, C₂₋₄ alkenyl, C₃₋₆ cycloalkyl, C₅₋₈ bridged cycloalkyl, C₅₋₁₁ spirocycloalkyl, C₅₋₁₁ fused cycloalkyl, 4- to 7-membered monocyclic heterocycloalkyl, 6- to 10-membered bridged heterocycloalkyl, 6- to 11-membered spiroheterocycloalkyl, 6- to 11-membered fused heterocycloalkyl, phenyl or 5- to 6-membered heteroaryl, wherein the alkyl, alkoxy, alkenyl, cycloalkyl, heterocycloalkyl, phenyl or heteroaryl is optionally substituted with 1 to 4 R^{k};
R^{5d} and R^{5e} are each independently selected from H, C₁₋₄ alkyl, C₃₋₆ cycloalkyl, 4- to 7-membered heterocycloalkyl, phenyl or 5- to 6-membered heteroaryl, wherein the alkyl, cycloalkyl, heterocycloalkyl, phenyl or heteroaryl is optionally substituted with 1 to 4 R^{k};
R^{5f} and R^{5g} are each independently selected from H, halogen, C₁₋₄ alkyl, and C₃₋₆ cycloalkyl, wherein the alkyl or cycloalkyl is optionally substituted with 1 to 4 R^{k};
or R^{5f} and R^{5g} are directly connected to form C₃₋₆ carbocyclyl or 4- to 7-membered heterocyclyl, wherein the carbocyclyl or heterocyclyl is optionally substituted with 1 to 4 R^{k};
R^{5h} is selected from NH₂, NHC₁₋₄ alkyl, N(C₁₋₄ alkyl)₂, C₁₋₆ alkyl, C₃₋₆ cycloalkyl or 4- to 7-membered heterocycloalkyl, wherein the alkyl, cycloalkyl or heterocycloalkyl is optionally substituted with 1 to 4 R^{k};
R⁵ⁱ is selected from CN, NO₂, -S(=O)₁₋₂-C₁₋₄ alkyl, or -S(=O)₁₋₂-C₃₋₆ cycloalkyl, wherein the alkyl or cycloalkyl is optionally substituted with 1 to 4 R^{k};
R¹, R², R³, R^{L1} and R^{L2} are each independently selected from H, deuterium, halogen, CN, OH, NO₂, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, or -SC₁₋₄ alkyl, wherein the alkyl, alkenyl, or alkynyl is optionally substituted with 1 to 4 R^{k};
alternatively, R^{L1} and R^{L2} together with the carbon atom to which they are attached form C₃₋₆ carbocyclyl or 4- to 6-membered heterocyclyl, wherein the carbocyclyl or heterocyclyl is optionally substituted with 1 to 4 R^{k};
alternatively, R^{5a} and R^{5b} together with the phosphorus atom to which they are attached form 5- to 8-membered monocyclic heterocyclyl, wherein the heterocyclyl is optionally substituted with 1 to 4 R^{k}.

3. The compound or the racemate, stereoisomer, tautomer, deuterated substance, solvate, prodrug, metabolite, pharmaceutically acceptable salt or eutectic crystal thereof according to claim 2, **characterised in that**
ring B is selected from pyrrolothienyl, pyrrolopyrazolyl, pyrrolopyrrolyl, pyrroloimidazolyl, pyrazolothienyl, imidazothienyl, imidazoimidazolyl, pyrazolopyrazolyl, pyrrolothiazolyl, pyrrolofuryl, indolyl, pyrrolopyridyl, pyrrolopyrimidyl, pyrrolopyridazinyl, pyrrolopyrazinyl, pyrrolotriazinyl, pyrazolophenyl, pyrazolopyridyl, pyrazolopyrimidyl, imidazophenyl, imidazopyridyl, imidazopyrimidyl, thienopyridyl, thienophenyl, furopyridyl, and furophenyl, and the ring B is optionally substituted with 1 to 4 R^{b};
L₂ is selected from -(CR^{L1}R^{L2})-, and -(CR^{L1}R^{L2})₂-;
each R^{k} is independently selected from deuterium, halogen, =O, CN, OH, SH, NO₂, COOH, CONH₂, NH₂, SF₅, C₁₋₄ alkyl, OC₁₋₄ alkyl, SC₁₋₄ alkyl, C₂₋₄ alkenyl, C₂₋₄ alkynyl, NHC₁₋₄ alkyl, N(C₁₋₄ alkyl)₂, -O-C₃₋₆ carbocyclyl, -O-3- to 6-membered heterocyclyl, -NH-C₃₋₆ carbocyclyl, -NH-3- to 6-membered heterocyclyl, -C₁₋₂ alkylene-C₃₋₆ carbocyclyl, -C₁₋₂ alkylene-3- to 6-membered heterocyclyl, C₃₋₆ carbocyclyl, 3- to 6-membered heterocyclyl, 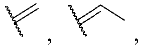 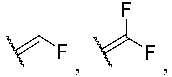 wherein the alkyl, alkylene, alkenyl, alkynyl, carbocyclyl or heterocyclyl is optionally substituted with 1 to 4 substituents selected from deuterium, halogen, CN, OH, NH₂, CH₂CN, C₁₋₄ alkyl, C₁₋₄ alkoxy.

4. The compound or the racemate, stereoisomer, tautomer, deuterated substance, solvate, prodrug, metabolite, pharmaceutically acceptable salt or eutectic crystal thereof according to claim 3, **characterised in that**
ring B is selected from one of the following groups optionally substituted with 1 to 4 R^{b}: 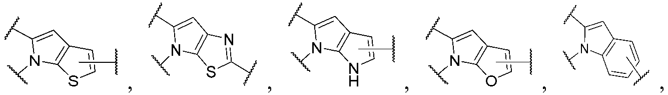 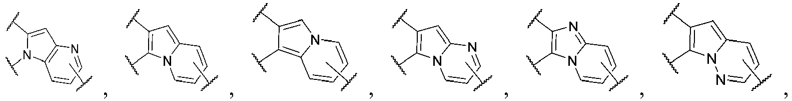 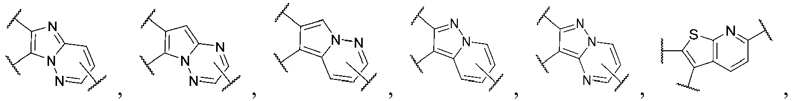 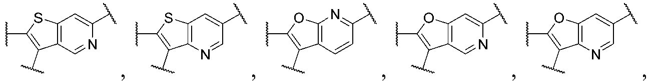 the right side of which is linked to Q;
ring D is selected from 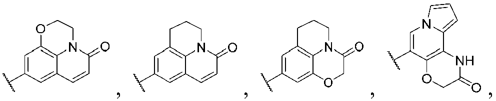 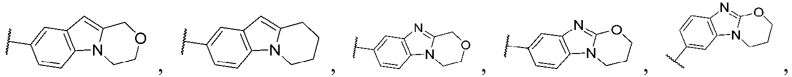 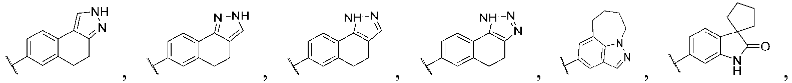 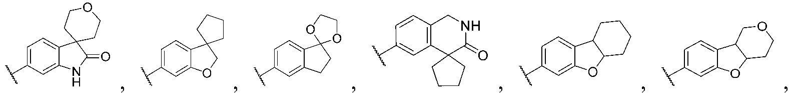 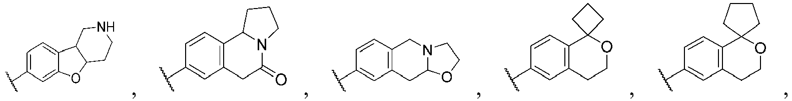 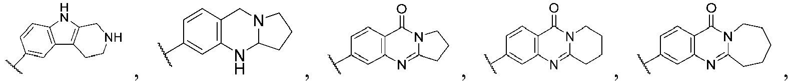 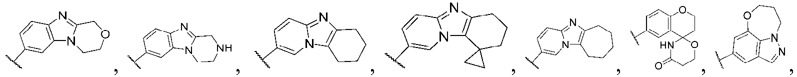 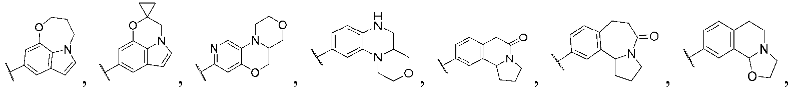 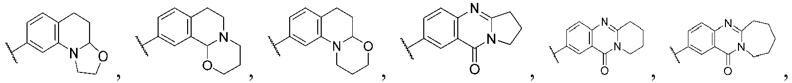 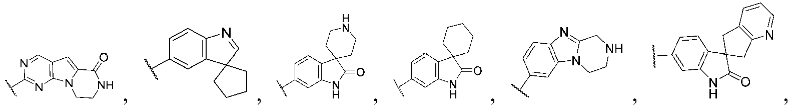 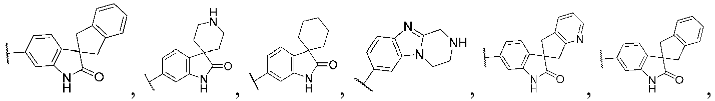 and the ring D is optionally substituted with 1 to 4 R^{d};
preferably, ring D is selected from 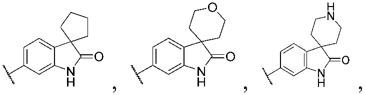 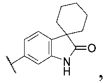 and the ring D is optionally substituted with 1 to 4 R^{d};
Q is selected from a bond, O, S, NH, CH₂, CH(CH₃), 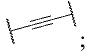
R⁶ is selected from methyl, ethyl, isopropyl, propyl, -CH₂O-CH₃, -CH₂O-CH₂CH₃, -CH₂O-CH(CH₃)₂, -CH₂O-C(CH₃)₃, -CH₂CH₂O-CH₃, -CH₂CH₂O-CH₂CH₃, -CH₂CH₂O-CH(CH₃)₂, -CH₂CH₂O-C(CH₃)₃, cyclopropyl, cyclobutyl, cyclopentyl, cyclohexyl, bicyclo[1.1.1]cyclopentyl, oxetanyl, oxolanyl, oxanyl, azetidinyl, pyrrolidyl, piperidyl, piperazinyl, morpholinyl, phenyl, pyrazolyl, thiazolyl, imidazolyl, oxazolyl, pyrrolyl, thienyl, furyl, pyridyl, pyrimidyl, pyrazinyl, pyridazinyl or 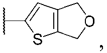 and the R⁶ is optionally substituted with 1 to 4 R^{6a};
R^{4a}, R^{4b}, R^{4c}, R^{4d}, R^{4e}, and R^{4f} are each independently selected from H, deuterium, methyl, ethyl, propyl, and isopropyl;
R^{a}, R^{b}, R^{d}, and R^{6a} are each independently selected from H, deuterium, F, Cl, Br, I, =O, CN, OH, NO₂, NH₂, NH(CH₃), N(CH₃)₂, -P(=O)R^{5a}R^{5b}, 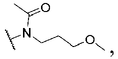 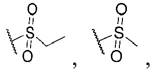 -C(=O)CH₃, -C(=O)CH₂CH₃, or one of the following groups optionally substituted with 1 to 3 R^{k}: methyl, ethyl, propyl, isopropyl, butyl, ethenyl, ethynyl, methoxy, ethoxy, isopropoxy, methylthio, cyclopropyl, cyclobutyl, cyclopentyl, cyclohexyl, cyclobutyl-spiro-cyclobutyl, cyclopentyl-spiro-cyclobutyl, cyclopentyl-spiro-cyclopentyl, cyclohexyl-spiro-cyclobutyl, cyclohexyl-spiro-cyclopentyl, cyclohexyl-spiro-cyclohexyl, azetidinyl, oxetanyl, pyrrolidyl, piperidyl, piperazinyl, morpholinyl, morpholinyl-spiro-cyclohexyl, - C(=O)NHCH₃, -C(=O)NH-cyclopropyl, 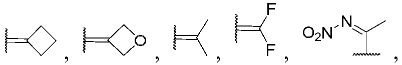 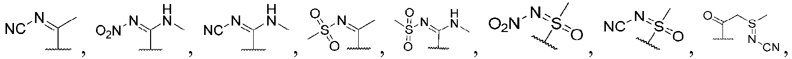 -CH₂-cyclopropyl, -CH₂-cyclobutyl, -CH₂-cyclopentyl, -CH₂-cyclohexyl, -CH₂-azetidinyl, -CH₂-azacyclopentyl, -CH₂-piperidyl, -CH₂-piperazinyl, -CH₂-oxetanyl, -CH2-tetrahydrofuryl, -CH₂-oxanyl, -C(=O)R^{5c}, -C(=O)-M-C(=O)-R^{5c}, -CH₂-M-C(=O)-R^{5c}, -C(=O)-M-CH₂-R^{5c}, -C(=O)-M-R^{5c}, -C(=O)-CH₂-O-R^{5c}, -C(=O)-CH₂-OCH₂-R^{5c}, -C(=O)-M-CH₂-O-R^{5c}, 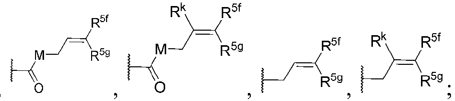
M is selected from cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, and 3-oxabicyclo[3.1.0]hexyl, and the M is optionally substituted with 1 to 4 R^{k};
R^{5c} is selected from one of the following groups optionally substituted with 1 to 4 R^{k}: methyl, ethyl, propyl, isopropyl, methoxy, ethoxy, ethenyl, propenyl, allyl, cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, bicyclo[2.2.1]heptyl, cyclopropyl-spiro-cyclobutyl, cyclobutyl-spiro-cyclobutyl, cyclobutyl-spiro-cyclopentyl, cyclobutyl-spiro-cyclohexyl, cyclopentyl-fused-cyclopentyl, 3-oxabicyclo[3.1.0]hexyl, thienyl, furyl, thiazolyl, oxazolyl, methoxymethyl, and ethoxymethyl;
R^{5f} and R^{5g} are each independently selected from H, F, Cl, Br, methyl, and ethyl;
or R^{5f} and R^{5g} are directly connected to form cyclobutyl, cyclopentyl or cyclohexyl, wherein the cyclobutyl, cyclopentyl or cyclohexyl is optionally substituted with 1 to 4 substituents selected from deuterium, F, Cl, Br, I, CN, OH, SH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, cyclopropyl, and cyclobutyl;
R^{5a} and R^{5b} are each independently selected from methyl, ethyl, propyl, isopropyl, methoxy, ethoxy, cyclopropyl, cyclobutyl, cyclopentyl or cyclohexyl, wherein the methyl, ethyl, propyl, isopropyl, methoxy, ethoxy, cyclopropyl, cyclobutyl, cyclopentyl or cyclohexyl is optionally substituted with 1 to 3 R^{k};
R¹, R², R³, R^{L1}, and R^{L2} are each independently selected from H, deuterium, F, Cl, Br, I, CN, OH, NO₂, NH₂, NH(CH₃), N(CH₃)₂, or one of the following groups optionally substituted with 1 to 3 R^{k}: methyl, ethyl, ethenyl, ethynyl, methoxy, ethoxy, isopropoxy, and methylthio;
alternatively, R^{L1} and R^{L2} together with the carbon atom to which they are attached form the following groups optionally substituted with 1 to 3 R^{k}: cyclopropyl, cyclobutyl, cyclopentyl, cyclohexyl, azetidinyl, pyrrolidyl, piperidyl, oxetanyl, and tetrahydrofuryl;
alternatively, R^{5a} and R^{5b} together with the phosphorus atom to which they are attached form the following groups optionally substituted with 1 to 3 R^{k}: 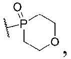 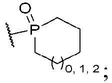
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, morpholinyl, 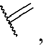 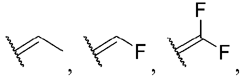 wherein the methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, or morpholinyl is optionally substituted with 1 to 4 substituents selected from deuterium, F, Cl, Br, I, CN, OH, NH₂, C₁₋₄ alkyl, and C₁₋₄ alkoxy.

5. The compound or the racemate, stereoisomer, tautomer, deuterated substance, solvate, prodrug, metabolite, pharmaceutically acceptable salt or eutectic crystal thereof according to claim 4, **characterised in that**
R⁴ is selected from -C(=O)OH, -C(=O)OCH₃, -C(=O)N(CH₃)₂, 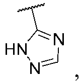 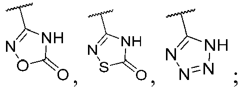
ring A is selected from one of the following groups optionally substituted with 1 to 4 R^{a}: phenyl, cyclopropyl, cyclobutyl, cyclopentyl, cyclohexyl, azetidinyl, pyrrolidyl, piperidyl, oxetanyl, oxolanyl, oxanyl, 1,3-dioxolanyl, 1,4-dioxanyl, piperazinyl, morpholinyl, pyrazolyl, imidazolyl, oxazolyl, thiazolyl, pyrrolyl, thienyl, triazolyl, pyridyl, pyrimidyl, pyridazinyl, pyrazinyl, triazinyl, 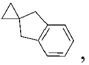 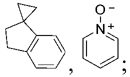
L₂ is selected from -CR^{L1}R^{L2}-, or one of the following groups optionally substituted with 1 to 3 R^{k}: 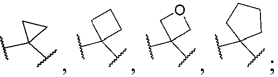
R^{L1} and R^{L2} are each independently selected from H, deuterium, F, Cl, Br, I, CN, OH, NO₂, NH₂, NH(CH₃), N(CH₃)₂, methyl, ethyl, methoxy, ethoxy, isopropoxy, and methylthio;
each R^{k} is independently selected from deuterium, F, Cl, Br, I, =O, CN, OH, SH, NO₂, COOH, CONH₂, NH₂, SF₅, NH(CH₃), NH(CH₂CH₃), N(CH₃)₂, N(CH₂CH₃)₂, methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, morpholinyl, 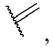 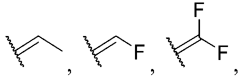 wherein the methyl, ethyl, propyl, isopropyl, methoxy, ethoxy, methylthio, ethenyl, propenyl, allyl, ethynyl, propynyl, propargyl, cyclopropyl, cyclobutyl, azetidinyl, oxetanyl, pyrrolidyl, piperidyl, pyrazolyl, pyrrolyl, or morpholinyl is optionally substituted with 1 to 4 substituents selected from deuterium, F, Cl, Br, I, CN, OH, NH₂, CH₂CN, methyl, ethyl, methoxy, and ethoxy.

6. The compound or the racemate, stereoisomer, tautomer, deuterated substance, solvate, prodrug, metabolite, pharmaceutically acceptable salt or eutectic crystal thereof according to claim 1, **characterised in that**
X is selected from O or S;
L₁ is -C(=O)-;
L₂ is selected from 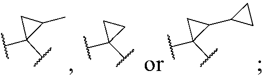
R⁴ is 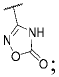
ring A is selected from phenyl, pyridyl, 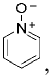 and the ring A is optionally substituted with 1 to 4 R^{a};
ring B is selected from 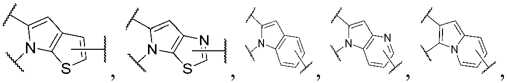 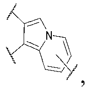 and the ring B is optionally substituted with 1 to 4 R^{b}, the right side of which is linked to Q;
preferably, ring B is selected from 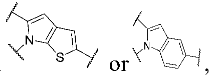 and the ring B is optionally substituted with 1 to 4 R^{b}, the right side of which is linked to Q;
-Q-R⁶ is selected from one of the following optionally substituted groups: ethyl, propyl, isopropyl, 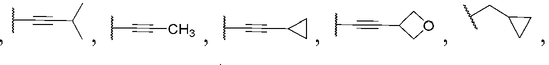 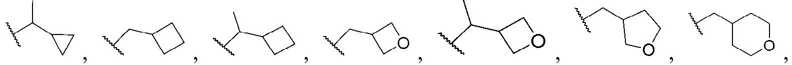 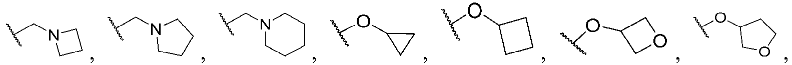 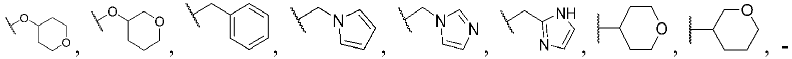 CH₂CH₂CH₂O-CH₂CH₃, -CH₂CH₂CH₂O-CH(CH₃)₂, -CH₂CH₂CH₂O-C(CH₃)₃, 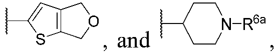 which, when substituted, are substituted with 1 to 4 substituents selected from deuterium, F, Cl, Br, I, =O, CN, OH, CF₃, CHF₂, CH₂F, CD₃, CHD₂, CH₂D, OCD₃, OCF₃, methyl, ethyl, propyl, isopropyl, methoxy or ethoxy;
R^{6a} is selected from methyl, ethyl, propyl, isopropyl, cyclopropyl, cyclobutyl, 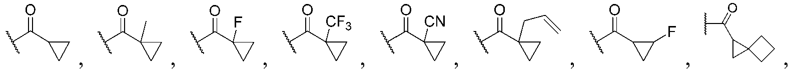 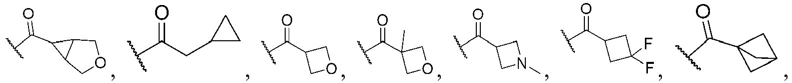 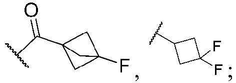
preferably, -Q-R⁶ is selected from one of the following optionally substituted groups: 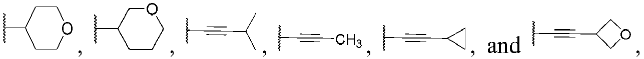 which, when substituted, are substituted with 1 to 4 substituents selected from deuterium, F, Cl, Br, I, =O, CN, OH, CF₃, CHF₂, CH₂F, CD₃, CHD₂, CH₂D, OCD₃, OCF₃, methyl, ethyl, propyl, isopropyl, methoxy or ethoxy;
preferably, -Q-R⁶ is optionally substituted 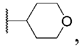 which, when substituted, is substituted with 1 to 4 substituents selected from deuterium, F, Cl, Br, OH, CF₃, CHF₂, CH₂F, CD₃, CHD₂, CH₂D, OCD₃, OCF₃, methyl, ethyl, propyl, isopropyl, methoxy or ethoxy;
R¹, R², and R³ are each independently selected from H, deuterium, F, Cl, Br, I, CN, OH, NO₂, NH₂, NH(CH₃), N(CH₃)₂, CD₃, OCD₃, CF₃, CH₂F, CHF₂, CH₂OH, OCF₃, OCHF₂, OCH₂F, methyl, ethyl, ethenyl, ethynyl, methoxy, ethoxy, isopropoxy, and methylthio;
preferably, R¹ is H;
preferably, R² is selected from H, methyl or CD₃;
preferably, R³ is selected from H, methyl or CD₃;
each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, propynyl, methoxy, ethoxy, isopropoxy, cyclopropyl, -CH₂OH, -CH₂CH₂OH, - CH₂CN, -CH₂N(CH₃)₂, -CH₂-cyclopropyl, 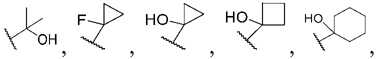 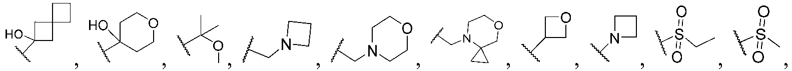 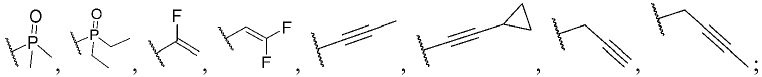
preferably, each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, methoxy, cyclopropyl, 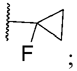
each R^{b} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, methoxy, ethoxy, isopropoxy, and cyclopropyl;
each R^{d} is independently selected from deuterium, F, Cl, Br, I, CN, OH, NH₂, NH(CH₃), N(CH₃)₂, CD₃, -CH₂CH₂OCH₃, -CF₃, -CH₂F, -CHF₂, -CH₂CH₂F, - CH₂CH₂CH₂F, -OCH₂CH₂OH, 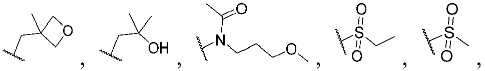 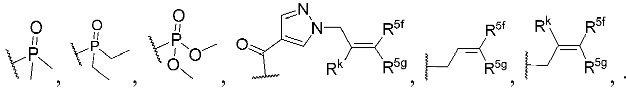 -C(=O)NHCH₃, - C(=O)NH-cyclopropyl, 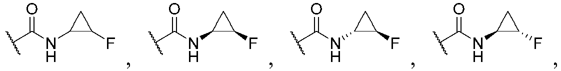 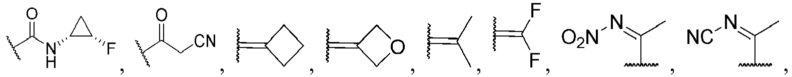 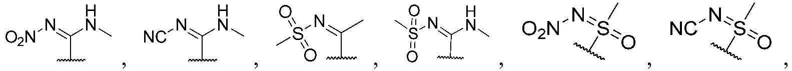 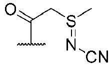 or one of the following groups optionally substituted with 1 to 4 R^{k1}: methyl, ethyl, methoxy, ethoxy, cyclopropyl, oxetanyl, -CH₂-cyclopropyl, -CH₂-cyclobutyl, -CH₂-cyclopentyl, -CH₂-oxetanyl, -CH₂-azetidinyl, -S(=O)₂CH₃, - S(=O)₂ cyclopropyl, -C(=O)NH-cyclopropyl, -C(=O)CH₃, -C(=O)-ethenyl, - C(=O)-cyclopropyl, -C(=O)-cyclobutyl, -C(=O)-cyclopentyl, -C(=O)-bicyclo[2.2.1]heptyl, -C(=O)-bicyclo[1.1.1]pentyl, -C(=O)-cyclobutyl-spiro-cyclobutyl, -C(=O)-oxetanyl, -C(=O)-tetrahydrofuryl, -C(=O)-oxanyl, -C(=O)-azetidinyl, -C(=O)-pyrrolidyl, -C(=O)-piperidyl, -C(=O)-piperazinyl, -C(=O)-pyrazolyl, -C(=O)-phenyl, -C(=O)-azetidinyl-CH₂-cyclopropyl, -C(=O)-pyrazolyl-CH₂-cyclopropyl, -C(=O)-pyrazolyl-CH₂-cyclobutyl, -C(=O)-pyrazolyl-CH₂-cyclopentyl, -C(=O)-pyrrolidyl-CH₂-cyclopropyl, -C(=O)-piperidyl-CH₂-cyclopropyl, -C(=O)-piperazinyl-CH₂-cyclopropyl, -C(=O)-azetidinyl -C(=O)-cyclopropyl, -C(=O)-pyrrolidyl -C(=O)-cyclopropyl, -C(=O)-piperidyl -C(=O)-cyclopropyl, -C(=O)-piperazinyl -C(=O)-cyclopropyl, -CH₂-azetidinyl -C(=O)-cyclopropyl, -CH₂-pyrrolidyl -C(=O)-cyclopropyl, -CH₂-azetidinyl-CH₂-cyclopropyl, -CH₂-pyrrolidyl-CH₂-cyclopropyl, -CH₂-piperidyl-CH₂-cyclopropyl, - CH₂-piperazinyl-CH₂-cyclopropyl, 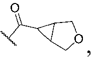 -C(=O)-cyclopropyl-phenyl, -C(=O)-cyclopropyl-pyridyl, -C(=O)-cyclopropyl-thienyl, -C(=O)-cyclopropyl-furyl, - C(=O)-CH₂-O-cyclopropyl, or -C(=O)-CH₂-OCH₂-cyclopropyl;
R^{k1} is selected from deuterium, F, Cl, Br, OH, CN, methyl, ethyl, propyl, isopropyl, ethenyl, propenyl, allyl, -CH₂-cyclopropyl, -CH₂-cyclopropyl, -CH₂-cyclobutyl, cyclopropyl, cyclobutyl, cyclopentyl, cyclohexyl, 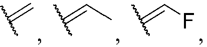 wherein the methyl, ethyl, propyl, isopropyl, ethenyl, propenyl, allyl, -CH₂-cyclopropyl, -CH₂-cyclopropyl, -CH₂-cyclobutyl, cyclopropyl, cyclobutyl, cyclopentyl, or cyclohexyl is optionally substituted with 1 to 4 substituents selected from deuterium, F, Cl, Br, OH, CN, CH₂CN, methyl, ethyl, methoxy or ethoxy;
R^{5f} and R^{5g} are each independently selected from H, F, Cl, Br, methyl, and ethyl; or R^{5f} and R^{5g} are directly connected to form cyclobutyl, cyclopentyl or cyclohexyl;
preferably, each R^{d} is independently selected from deuterium, F, Cl, Br, I, CN, OH, NH₂, NH(CH₃), N(CH₃)₂, CHF₂, CH₂F, CF₃, CD₃, methyl, ethyl, methoxy, ethoxy, cyclopropyl, oxetanyl, -CH₂-cyclopropyl, -CH₂-oxetanyl, -C(=O)CH₃, - C(=O)-cyclopropyl, -C(=O)NHCH₃, -C(=O)NH-cyclopropyl,

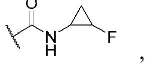

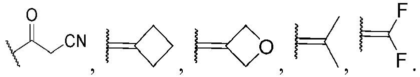

7. The compound or the racemate, stereoisomer, tautomer, deuterated substance, solvate, prodrug, metabolite, pharmaceutically acceptable salt or eutectic crystal thereof according to claim 1, **characterised in that** the compound as shown in general formula (I) is a compound as shown in (Id), 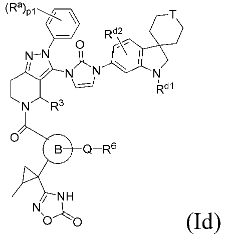
T is selected from a bond, O, S, CH₂, NH, and N (R^{d3}),
p1 is selected from 0, 1, 2, 3 or 4;
R^{d1} is selected from H, CHF₂, CD₃, methyl, cyclopropyl, oxetanyl, -CH₂-cyclopropyl, and -CH₂-oxetanyl;
R^{d2} is selected from H, deuterium, F, Cl, Br, CHF₂, CD₃, methyl, cyclopropyl, oxetanyl, -CH₂-cyclopropyl, and -CH₂-oxetanyl;
R^{d3} is selected from methyl, ethyl, isopropyl, cyclopropyl, cyclobutyl, cyclopentyl, cyclohexyl, -CH₂-cyclopropyl, -CH₂-cyclobutyl, -CH₂-oxetanyl, - CH₂-tetrahydrofuryl, -CH₂-azetidinyl, -CH₂-pyrrolidyl, -CH₂-piperidyl, -CH₂-piperazinyl, -S(=O)₁₋₂-R^{5c}, -C(=O)R^{5c}, -C(=O)NR^{5d}R^{5e}, -NR^{5d}C(=O)R^{5e}, -C(=O)-M-C(=O)-R^{5c}, -CH₂-M-C(=O)-R^{5c}, -C(=O)-M-CH₂-R^{5c}, -C(=O)-M-R^{5c}, -C(=O)-M-CH₂-O-R^{5c}, 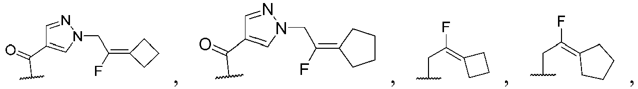 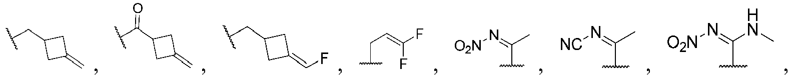 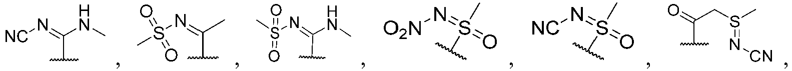 wherein the methyl, ethyl, isopropyl, cyclopropyl, cyclobutyl, cyclopentyl, cyclohexyl, -CH₂-cyclopropyl, -CH₂-cyclobutyl, -CH₂-oxetanyl, -CH₂-tetrahydrofuryl, -CH₂-azetidinyl, -CH₂-pyrrolidyl, -CH₂-piperidyl, or -CH₂-piperazinyl is optionally substituted with 1 to 4 R^{k} (each R^{k} is preferably independently selected from deuterium, F, Cl, Br, CN, OH, CHF₂, CD₃, methyl, ethyl, propyl, isopropyl, cyclopropyl, oxetanyl, -CH₂-cyclopropyl, -CH₂-oxetanyl, methoxy, methoxymethyl, methoxyethyl, CH₂CN, ethenyl, 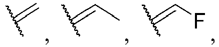 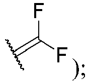
M is selected from cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, 3-oxabicyclo[3.1.0]hexyl, and the M is optionally substituted with 1 to 4 R^{k} (each R^{k} is preferably independently selected from deuterium, F, Cl, Br, CHF₂, CD₃, methyl, cyclopropyl, oxetanyl, - CH₂-cyclopropyl, and -CH₂-oxetanyl);
R^{5c} is selected from methyl, ethyl, propyl, isopropyl, methoxy, ethoxy, ethenyl, propenyl, allyl, cyclopropyl, cyclobutyl, cyclopentyl, cyclohexyl, azetidinyl, pyrrolidyl, piperidyl, piperazinyl, morpholinyl, oxetanyl, tetrahydrofuryl, oxanyl, phenyl, pyrazolyl, pyrrolyl, triazolyl, pyridyl, pyrazinyl, pyridazinyl, pyrazinyl, bicyclo[1.1.1]pentyl, bicyclo[2.2.1]heptyl, cyclopropyl-spiro-cyclobutyl, cyclobutyl-spiro-cyclobutyl, cyclobutyl-spiro-cyclopentyl, cyclobutyl-spiro-cyclohexyl, cyclopentyl-fused-cyclopentyl, 3-oxabicyclo[3.1.0]hexyl, thienyl, furyl, thiazolyl, oxazolyl, methoxymethyl, and ethoxymethyl, and the R^{5c}is optionally substituted with 1 to 4 R^{k} (each R^{k} is preferably independently selected from deuterium, F, Cl, Br, CN, OH, CHF₂, CD₃, methyl, ethyl, propyl, isopropyl, cyclopropyl, oxetanyl, -CH₂-cyclopropyl, -CH₂-oxetanyl, methoxy, methoxymethyl, methoxyethyl, CH₂CN, ethenyl, propenyl, 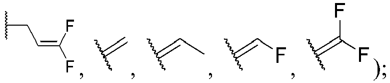
R^{5d} and R^{5e} are each independently selected from H, methyl, ethyl, propyl, isopropyl, cyclopropyl, cyclobutyl, cyclopentyl or cyclohexyl, wherein the methyl, ethyl, propyl, isopropyl, cyclopropyl, cyclobutyl, cyclopentyl or cyclohexyl is optionally substituted with 1 to 4 substituents selected from deuterium, F, Cl, Br, CN, OH, CHF₂, CD₃, methyl, and ethyl;
ring B is selected from 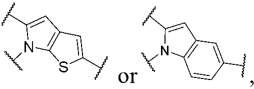 and the ring B is optionally substituted with 1 to 4 R^{b}, the right side of which is linked to Q;
each R^{b} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, methoxy, ethoxy, isopropoxy, and cyclopropyl;
each R^{a} is independently selected from deuterium, F, Cl, Br, I, CN, OH, NH₂, NH(CH₃), N(CH₃)₂, CF₃, CD₃, -OCF₃, -OCD₃, methyl, ethyl, ethenyl, ethynyl, propynyl, methoxy, ethoxy, isopropoxy, cyclopropyl, -CH₂OH, -CH₂CH₂OH, - CH₂CN, -CH₂N(CH₃)₂, -CH₂-cyclopropyl, 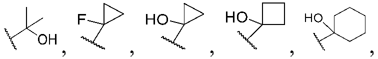 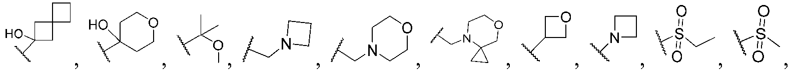 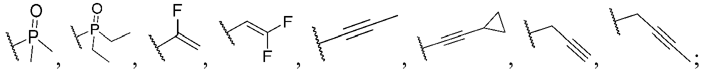
preferably, 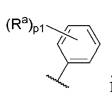 is selected from

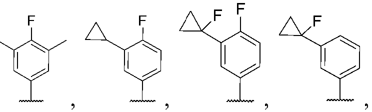

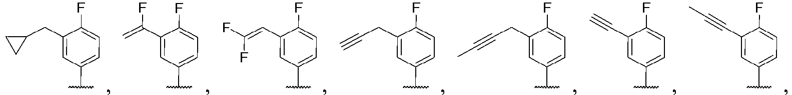

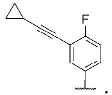

8. The compound or the racemate, stereoisomer, tautomer, deuterated substance, solvate, prodrug, metabolite, pharmaceutically acceptable salt or eutectic crystal thereof according to claim 1, **characterised in that** the compound has a structure selected from one of those in Table E-1.

9. A pharmaceutical composition, **characterised by** comprising the compound or the racemate, stereoisomer, tautomer, deuterated substance, solvate, prodrug, metabolite, pharmaceutically acceptable salt or eutectic crystal thereof according to any one of claims 1-8, and a pharmaceutically acceptable carrier, wherein preferably, the pharmaceutical composition comprises 1-1500 mg of the compound or the racemate, stereoisomer, tautomer, deuterated substance, solvate, prodrug, metabolite, pharmaceutically acceptable salt or eutectic crystal thereof according to any one of claims 1-8.

10. Use of the compound or the racemate, stereoisomer, tautomer, deuterated substance, solvate, prodrug, metabolite, pharmaceutically acceptable salt or eutectic crystal thereof according to any one of claims 1-8, or the pharmaceutical composition according to claim 9 in the preparation of a drug for treating a disease related to GLP-1R activity or expression level, **characterised in that** preferably, the disease is selected from diabetes or obesity.

11. A method for treating a disease in a mammal, **characterised by** comprising administering to a subject a therapeutically effective amount of the compound or the racemate, stereoisomer, deuterated substance, solvate, prodrug, metabolite, pharmaceutically acceptable salt or eutectic crystal thereof according to any one of claims 1-8, wherein the therapeutically effective amount is preferably 1-1500 mg, and the disease is preferably a disease related to GLP-1R activity or expression level.
